(19) **Europäisches Patentamt European Patent Office Office européen des brevets**

(11) **EP 2 179 742 A1**

(12) **EUROPEAN PATENT APPLICATION**

(43) Date of publication:
28.04.2010 Bulletin 2010/17

(51) Int Cl.:
*A61K 38/17* (2006.01)  *C07K 16/18* (2006.01)

(21) Application number: 08020819.2

(22) Date of filing: 24.11.2003

(84) Designated Contracting States:
AT BE BG CH CY CZ DE DK EE ES FI FR GB GR HU IE IT LI LU MC NL PT RO SE SI SK TR

(30) Priority: 26.11.2002 US 429069 P

(62) Document number(s) of the earlier application(s) in accordance with Art. 76 EPC:
03812029.1 / 1 572 116

(27) Previously filed application:
24.11.2003 EP 03812029

(71) Applicant: Genentech, Inc.
South San Francisco CA 94080-4990 (US)

(72) Inventors:
• Clark, Hilary
  San Francisco, CA 94134 (US)
• Hunte, Bridsell
  San Francisco, CA 94110 (US)
• Jackman, Janet
  Half Moon Bay, CA 94019 (US)

• Schoenfeld, Jill
  Ashland, OR 97520 (US)
• Willians, Mickey P.
  Half Moon Bay, CA 94010 (US)
• Wu, Thomas D.
  San Francisco, CA 94110 (US)
• Bodary, Sarah
  Menlo Park, CA 94025 (US)

(74) Representative: **Walton, Seán Malcolm et al**
**Mewburn Ellis LLP**
**33 Gutter Lane**
**London**
**EC2V 8AS (GB)**

Remarks:
•The complete document including Reference Tables and the Sequence Listing is available on CD-ROM from the European Patent Office, Vienna sub-office
•This application was filed on 01-12-2008 as a divisional application to the application mentioned under INID code 62.

(54) **Compositions and methods for the treatment of immune related diseases**

(57) Compositions containing proteins and methods of using those compositions for the diagnosis and treatment of immune related diseases.

**Description**

Field of the Invention

The present invention relates to compositions and methods useful for the diagnosis and treatment of immune related diseases.

Background of the Invention

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, *etc*.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, *etc.* The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, *i.e.*, lymphokines, which play a central role in the activation of B cells, cytotoxic T cells and a variety of other cells which participate in the immune response.

Immune related diseases could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

CD4+ T cells are known to be important regulators of inflammation. Herein, CD4+ T cells were activated and the profile of genes differentially expressed upon activation was analyzed. As such, the activation specific genes may be potential therapeutic targets. *In vivo* co-stimulation is necessary for a productive immune proliferative response. The list of costimulatory molecules is quite extensive and it is still unclear just which co-stimulatory molecules play critical roles in different types and stages of inflammation. In this application the focus is on genes which are specifically upregulated or downregulated by stimulation with anti-CD3/ICAM, or anti-CD3/anti-CD28 and may be useful in targeting inflammatory processes which are associated with these different molecules.

Despite the above identified advances in T cell research, there is a great need for additional diagnostic and therapeutic agents capable of detecting the presence of a T cell mediated disorders in a mammal and for effectively reducing these disorders. Accordingly, it is an objective of the present invention to identify polypeptides that are overexpressed in activated T cells as compared to resting T cells, and to use those polypeptides, and their encoding nucleic acids, to produce compositions of matter useful in the therapeutic treatment and diagnostic detection of T cell mediated disorders in mammals.

Summary of the Invention

A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (*e.g.*, neutralizing antibodies) can be used therapeutically where attenuation of the immune response would be beneficial (*e.g.*, inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) increasing infiltration of inflammatory cells into a tissue of a mammal in need thereof, (b) stimulating or enhancing an immune response in a mammal in need thereof, (c) increasing the proliferation of T-lymphocytes in a mammal in need thereof in response to an antigen, (d) stimulating the activity of T-lymphocytes or (e) increasing the vascular permeability. In a further aspect, when the composition comprises an immune inhibiting molecule, the composition is useful for: (a) decreasing infiltration of inflammatory cells into a tissue of a mammal in need thereof, (b) inhibiting or reducing an immune response in a mammal in need thereof, (c) decreasing the activity of T-lymphocytes or (d) decreasing the proliferation of T-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, rheumatoid arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:

(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:

(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:

(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:

(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is increased.

In a still further embodiment, the invention provides a method of decreasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is decreased.

In a still further embodiment, the invention provides a method of increasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is increased.

In a still further embodiment, the invention provides a method of decreasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is decreased.

B. Additional Embodiments

In other embodiments of the present invention, the invention provides vectors comprising DNA encoding any of the herein described polypeptides. Host cell comprising any such vector are also provided. By way of example, the host cells may be CHO cells, E. *coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier. '

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

**List of Figures**

Figure 1: DNA325395, NP_000973.2, 200012_x_at
Figure 2: PRO81927
Figure 3: DNA329897, NP_031401.1, 200020_at
Figure 4: PRO69676
Figure 5: DNA326769, NP_001000.2, 200024_at
Figure 6: PRO83105
Figure 7: DNA329898, NP_000979.1, 200025_s_at
Figure 8: PRO10643
Figure 9: DNA293451, NP_296374.1, 200026_at
Figure 10: PRO70720
Figure 11: DNA326466, NP_004530.1, 200027_at
Figure 12: PRO60800
Figure 13: DNA329899, NP_002785.1, 200039_s_at
Figure 14: PRO69614
Figure 15: DNA326953, HSPC117, 200042_at
Figure 16: PRO83270
Figure 17: DNA255084, NP_001081.1, 200045_at
Figure 18: PRO50170
Figure 19: DNA272614, NP_004506.1, 200052_s_at
Figure 20: PRO60747
Figure 21: DNA304680, HSPCB, 200064_at
Figure 22: PRO71106
Figure 23: DNA189703, NP_005539.1, 200079_s_at
Figure 24: PRO22637
Figure 25: DNA329900, NP_002905.1, 1053_at
Figure 26: PRO81549
Figure 27: DNA88189, NP_037362.1, 266_s_at
Figure 28: PRO2690
Figure 29: DNA272992, NP_055479.1, 32069_at
Figure 30: PRO61064
Figure 31A-B: DNA329901, BAA32291.2, 32091_at
Figure 32: PRO85218
Figure 33: DNA329902, NP_110419.2, 32502_at
Figure 34: PRO85219
Figure 35: DNA329903, NP_005596.2, 32541_at
Figure 36: PRO85220
Figure 37: DNA327521, NP_002192.2, 33304_at
Figure 38: PRO58320
Figure 39: DNA272223, NP_004444.1, 33494_at
Figure 40: PRO60485
Figure 41A-B: DNA329904, NP_066554.1, 33767_at
Figure 42: PRO85221
Figure 43: DNA210121, NP_001794.1, 34210_at
Figure 44: PRO33667
Figure 45: DNA269828, NP_006691.1, 35254_at
Figure 46: PRO58230
Figure 47: DNA88643, NP_000190.1, 35626_at
Figure 48: PRO2455
Figure 49: DNA331435, NP_006143.1, 35974-at
Figure 50: PRO86495
Figure 51A-B: DNA272022, NP_002607.1, 36829_at
Figure 52: PRO60296
Figure 53: DNA226967, NP_055145.2, 37028_at
Figure 54: PRO37430

Figure 55: DNA226043, NP_006424.2, 37145_at
Figure 56: PRO36506
Figure 57: DNA329906, MGC14258, 37577_at
Figure 58: PRO85223
Figure 59: DNA256295, NP_002310.1, 37796_at
Figure 60: PRO51339
Figure 61: DNA328354, AF237769, 37966_at
Figure 62: PRO84215
Figure 63A-B: DNA329907, NP_036423.1, 38158_at
Figure 64: PRO85224
Figure 65A-B: DNA329908, BAA13246.1, 38892_at
Figure 66: PRO85225
Figure 67: DNA328356, BC013566, 39248_at
Figure 68: PRO38028
Figure 69: DNA327523, NP_004916.1, 39249_at
Figure 70: PRO38028
Figure 71A-B: DNA328358, STK10, 40420_at
Figure 72: PRO84218
Figure 73: DNA329909, NP_077084.1, 40446_at
Figure 74: PRO62251
Figure 75: DNA329910, NP_003251.2, 40837_at
Figure 76: PRO82891
Figure 77A-B: DNA329093, NP_006631.1, 41220_at
Figure 78: PRO84745
Figure 79A-C: DNA331436, 7689629.6, 43427_at
Figure 80: PRO86496
Figure 81: DNA154653, DNA154653, 43511_s_at
Figure 82: DNA262129, NP_079389.1, 44790_s_at
Figure 83: PRO4740
Figure 84: DNA326185, NP_073607.2, 45633_at
Figure 85: PRO82602
Figure 86: DNA329912, NP_004614.1, 46167_at
Figure 87: PRO85227
Figure 88: DNA329913, SSB-3, 46256_at
Figure 89: PRO85228
Figure 90: DNA324145, NP_060259.1, 46665_at
Figure 91: PRO80846
Figure 92: DNA329094, NP_077285.1, 48531_at
Figure 93: PRO84746
Figure 94: NA329914, NP-079175.2, 52285_f_at
Figure 95: PRO85229
Figure 96: DNA328364, NP_068577.1, 52940_at
Figure 97: PRO84223
Figure 98: DNA329915, NP_065093.1, 56197_at
Figure 99: PRO85230
Figure 100A-B: DNA328966, AK024397, 57082_at
Figure 101: PRO84670
Figure 102A-B: DNA226870, NP_000782.1, 48808_at
Figure 103: PRO37333
Figure 104: DNA328366, NP_079233.1, 59375_at
Figure 105: PRO84225
Figure 106: DNA331437, 338326.15, 60084_at
Figure 107: PRO86497
Figure 108: DNA328367, NP_079108.2, 60471_at
Figure 109: PRO84226
Figure 110: DNA327876, NP_005081.1, 60528_at
Figure 111: PRO83815
Figure 112: DNA329917, NP_065174.1, 64486_at

Figure 113: PRO85232
Figure 114: DNA329918, BC008671, 65630_at
Figure 115: PRO85233
Figure 116A-B: DNA196428, BAA31649.1, 76897_s_at
Figure 117: PRO71274
Figure 118: DNA329919, C20orf67, 89948_at
Figure 119: PRO85234
Figure 120: DNA328369, BC007634, 90610-at
Figure 121: DNA269410, NP_002725.1, 200605_s_at
Figure 122: PRO57836
Figure 123A-B: DNA326380, NP_004850.1, 200614_at
Figure 124: PRO82774
Figure 125A-B: DNA194778, NP_055545.1, 200616_s_at
Figure 126: PRO24056
Figure 127: DNA287245, NP_004175.1, 200628_s_at
Figure 128: PRO69520
Figure 129: DNA287245, WARS, 200629_at
Figure 130: PRO69520
Figure 131: DNA327532, GLUL, 200648_s_at
Figure 132: PRO71134
Figure 133: DNA97285, NP_005557.1, 200650_s_at
Figure 134: PRO3632
Figure 135: DNA226125, NP_003136.1, 200652_at
Figure 136: PRO36588
Figure 137: DNA325923, NP_008819.1, 200655_s_at
Figure 138: PRO4904
Figure 139: DNA227055, NP_002625.1, 200658_s_at
Figure 140: PRO37518
Figure 141: DNA275062, NP_006136.1, 200664_s_at
Figure 142: PRO62782
Figure 143: DNA275062, DNAJB1, 200666_s_at
Figure 144: PRO62782
Figure 145A-B: DNA328372,105551.7,200685_at
Figure 146: PRO84229
Figure 147A-B: DNA329920, NP_036558.1, 200687_s_at
Figure 148: PRO85235
Figure 149: DNA324633, BC000478, 200691_s_at
Figure 150: PRO81277
Figure 151: DNA324897, NP_006845.1, 200700_s_at
Figure 152: PRO12468
Figure 153: DNA275267, NP_003737.1, 200703_at
Figure 154: PRO62952
Figure 155: DNA328373, AB034747, 200704_at
Figure 156: PRO84230
Figure 157: DNA328374, NP_004853.1, 200706_s_at
Figure 158: PRO84231
Figure 159: DNA290260, NP_036555.1, 200715_x_at
Figure 160: PRO70385
Figure 161: DNA329921, 1315403.9, 200719_at
Figure 162: PRO85236
Figure 163: DNA329538, M11S1, 200722_s_at
Figure 164: PRO85088
Figure 165: DNA227618, HSGPIP137, 200723_s_at
Figure 166: PRO38081
Figure 167: DNA327114, NP_006004.1, 200725_x_at
Figure 168: PRO62466
Figure 169A-B: DNA327534, NP_003454.1, 200730_s_at
Figure 170: PRO41180

Figure 171A-B: DNA327534, PTP4A1, 200731_s_at
Figure 172: PRO41180
Figure 173: DNA331438, 402431.7, 200732_s_at
Figure 174: PRO86498
Figure 175: DNA327845, NP_000282.1, 200737_at
Figure 176: PRO61271
Figure 177: DNA327845, PGK1, 200738_s_at
Figure 178: PRO61271
Figure 179: DNA287207, NP_006316.1, 200750_s_at
Figure 180: PRO39268
Figure 181A-B: DNA274977, HSU97105, 200762_at
Figure 182: PRO62709
Figure 183: DNA324135, BC001854, 200768_s_at
Figure 184: PRO80837
Figure 185: DNA324135, NP_005902.1, 200769_s_at
Figure 186: PRO80837
Figure 187: DNA271608, NP_055485.1, 200777_s_at
Figure 188: PRO59895
Figure 189: DNA226262, NP_005554.1, 200783_s_at
Figure 190: PRO36725
Figure 191: DNA324060, NP_002530.1, 200790_at
Figure 192: PRO80773
Figure 193: DNA272928, NP_055579.1, 200794_x_at
Figure 194: PRO61012
Figure 195: DNA304668, NP_005336.2, 200799_at
Figure 196: PRO71095
Figure 197: DNA227607, NP_005337.1, 200800_s_at
Figure 198: PRO38070
Figure 199: DNA287211, NP_002147.1, 200806_s_at
Figure 200: PRO69492
Figure 201: DNA287211, HSPD1, 200807_s_at
Figure 202: PRO69492
Figure 203: DNA269874, NP_001271.1, 200810_s_at
Figure 204: PRO58272
Figure 205: DNA269874, CIRBP, 200811_at
Figure 206: PRO58272
Figure 207: DNA227795, NP_006420.1, 200812_at
Figure 208: PRO38258
Figure 209: DNA325596, NP_000356.1, 200822_x_at
Figure 210: PRO69549
Figure 211A-B: DNA328700, AF097514, 200831_s_at
Figure 212: PRO84464
Figure 213A-B: DNA328378, AB032261, 200832_s_at
Figure 214: PRO84233
Figure 215: DNA329922, CTSB, 200838_at
Figure 216: PRO3344
Figure 217: DNA88165, HUMCTSB, 200839_s_at
Figure 218: PRO2678
Figure 219: DNA329923, NP_057211.3, 200847_s_at
Figure 220: PRO85237
Figure 221: DNA324509, NP_002097.1, 200853_at
Figure 222: PRO10297
Figure 223A-C: DNA331439, NP_001447.1, 200859_x_at
Figure 224: PRO86499
Figure 225A-B: DNA228029, NP_055577.1, 200862_at
Figure 226: PRO38492
Figure 227: DNA226112, NP_002769.1, 200866_s_at
Figure 228: PRO36575

Figure 229: DNA226112, PSAP, 200871_s_at
Figure 230: PRO36575
Figure 231: DNA254537, NP_002957.1, 200872_at
Figure 232: PRO49642
Figure 233: DNA271030, NP_006383.1, 200875_s_at
Figure 234: PRO59358
Figure 235: DNA324107, NP_006421.1, 200877 _at
Figure 236: PRO80814
Figure 237: DNA328379, BC015869, 200878_at
Figure 238: PRO84234
Figure 239: DNA329099, 1164406.9, 200880_at
Figure 240: PRO60127
Figure 241: DNA271847, NP_001530.1, 200881_s_at
Figure 242: PRO60127
Figure 243: DNA287187, NP_002620.1, 200886_s_at
Figure 244: PRO69473
Figure 245A-B: DNA327539, NP_009330.1, 200887_s_at
Figure 246: PRO12211
Figure 247: DNA326326, NP_000969.1, 200888_s_at
Figure 248: PRO82724
Figure 249: DNA325584, NP_002005.1, 200894_s_at
Figure 250: PRO59262
Figure 251: DNA325584, FKBP4, 200895_s_at
Figure 252: PRO59262
Figure 253: DNA328380, HSHLAEHCM, 200904_at
Figure 254: DNA304665, NP_000995.1, 200909_s_at
Figure 255: PRO71092
Figure 256: DNA272695, NP_001722.1, 200920_s_at
Figure 257: PRO60817
Figure 258: DNA272695, BTG1, 200921_s_at
Figure 259: PRO60817
Figure 260: DNA227077, NP_005558.1, 200923_at
Figure 261: PRO37540
Figure 262: DNA327255, NP_002385.2, 200924_s_at
Figure 263: PRO57298
Figure 264: DNA225878, NP_004334.1, 200935_at
Figure 265: PRO36341
Figure 266: DNA329925, NP_001528.1, 200942_s_at
Figure 267: PRO85239
Figure 268A-B: DNA287217, NP_001750.1, 200951_s_at
Figure 269: PRO36766
Figure 270A-B: DNA287217, CCND2, 200952_s_at
Figure 271:PRO36766
Figure 272: DNA227491, NP_009027.1, 200960_x_at
Figure 273: PRO37954
Figure 274: DNA331440, NP_036380.2, 200961_at
Figure 275: PRO86500
Figure 276A-B: DNA331289, ABLIM1, 200965_s_at
Figure 277: PRO86390
Figure 278: DNA287355, NP_000025.1, 200966_x_at
Figure 279: PRO69617
Figure 280: DNA324110, NP_005908.1, 200978_at
Figure 281: PRO4918
Figure 282: DNA329928, ANXA6, 200982_s_at
Figure 283: PRO85241
Figure 284A-B: DNA325896, NP_001521.1,200989_at
Figure 285: PRO82352
Figure 286: DNA329929, 400903.6, 200994_at

Figure 287: PRO85242
Figure 288: DNA325778, CKAP4, 200998_s_at
Figure 289: PRO82248
Figure 290: DNA331441, BC015436,200999_s_at
Figure 291: DNA275408, NP_001596.1, 201000_at
Figure 292: PRO63068
Figure 293: DNA304713, NP_006463.2, 201008_s_at
Figure 294: PRO71139
Figure 295: DNA304713, TXNIP, 201009_s_at
Figure 296: PRO71139
Figure 297: DNA304713, 573591, 201010_s_at
Figure 298: PRO71139
Figure 299: DNA89242, NP_000691.1, 201012_at
Figure 300: PRO2907
Figure 301: DNA328388, BC010273, 201013_s_at
Figure 302: PRO84240
Figure 303: DNA328388, NP_006443.1, 201014_s_at
Figure 304: PRO84240
Figure 305: DNA151697, DNA151697, 201016_at
Figure 306: PRO11993
Figure 307A-B: DNA329101, NP-056988.2, 201024_x_at
Figure 308: PRO84751
Figure 309A-B: DNA329101, IF2,201027_s_at
Figure 310: PRO84751
Figure 311: DNA287372, NP-002618.1,201037_at
Figure 312: PRO69632
Figure 313: DNA328391, NP_004408.1, 201041_s_at
Figure 314: PRO84242
Figure 315: DNA328391, DUSP1, 201044_x_at
Figure 316: PRO84242
Figure 317: DNA274743, NP_002850.1, 201087_at
Figure 318: PRO62517
Figure 319: DNA254725, NP_002257.1, 201088_at
Figure 320: PRO49824
Figure 321: DNA329930, ATP6V1B2, 201089_at
Figure 322: PRO85243
Figure 323: DNA287198, NP_006073.1, 201090_x_at
Figure 324: PRO69484
Figure 325A-B: DNA328395, NP_056198.1, 201104_x_at
Figure 326: PRO84245
Figure 327: DNA304719, NP_002296.1, 201105_at
Figure 328: PRO71145
Figure 329: DNA329931, AF053642, 201111_at
Figure 330: DNA331442, NP_002783.1, 201114_x_at
Figure 331: PRO83189
Figure 332: DNA273865, NP_006221.1, 201115_at
Figure 333: PRO61824
Figure 334: DNA326273, Nip_001961.1, 201123_s_at
Figure 335: PRO82678
Figure 336: DNA329255, NP_006267.1, 201129_at
Figure 337: PRO84855
Figure 338: DNA329103, NP_002112.2,201137_s_at
Figure 339: PRO84752
Figure 340: DNA329104, NP_004085.1, 201144_s_at
Figure 341: PRO69550
Figure 342: DNA329105, NP_006109.2, 201145_at
Figure 343: PRO84753
Figure 344: DNA329015, NFE2L2, 201146_at

Figure 345: PRO84691
Figure 346: DNA329932, BC008801, 201160_s_at
Figure 347: PRO85244
Figure 348: DNA151802, NP_003661.1, 201169_s_at
Figure 349: PRO12890
Figure 350: DNA151802, BHLHB2, 201170_s_at
Figure 351: PRO12890
Figure 352: DNA273342, NP_005887.1, 201193_at
Figure 353: PRO61345
Figure 354: DNA331443, NP_003000.1, 201194_at
Figure 355: PRO86501
Figure 356A-B: DNA103453, HUME16GEN, 201195_s_at
Figure 357: PRO4780
Figure 358: DNA272251, NP_002798.1, 201198_s_at
Figure 359: PRO60513
Figure 360: DNA103488, NP_002583.1, 201202_at
Figure 361: PRO4815
Figure 362: DNA327544, NP_002865.1, 201222_s_at
Figure 363: PRO70357
Figure 364: DNA287173, ENO1, 201231_s_at
Figure 365: PRO69463
Figure 366: DNA287331, NP_002645.1, 201251_at
Figure 367: PRO69595
Figure 368: DNA274139, NP_006494.1, 201252_art
Figure 369: PRO62075
Figure 370: DNA270950, NP_003182.1, 201263_at
Figure 371: PRO59281
Figure 372A-B: DNA328404, NP_003321.1, 201266_at
Figure 373: PRO84251
Figure 374: DNA331444, NP_002781.1, 201274_at
Figure 375: PRO60981
Figure 376: DNA323936, NP_001995.1, 201275_at
Figure 377: PRO80669
Figure 378: DNA328405, NP_112556.1, 201277_s_at
Figure 379: PRO84252
Figure 380: DNA270526, NP_001166.1, 201288_at
Figure 381: PRO58903
Figure 382A-B: DNA327545, TOP2A, 201291_s_at
Figure 383: PRO82731
Figure 384: DNA327546, HSTOP2A10, 201292_at
Figure 385: DNA328407, WSB1, 201294_s_at
Figure 386: PRO84254
Figure 387A-B: DNA226778, HSM800772, 201295_s_at
Figure 388: PRO37241
Figure 389: DNA327547, NP_060691.1, 201298_s_at
Figure 390: PRO83583
Figure 391: DNA287222, NP_055555.1, 201303_at
Figure 392: PRO69501
Figure 393: DNA324612, P311, 201310_s_at
Figure 394: PRO81261
Figure 395: DNA325595, NP_001966.1, 201313_at
Figure 396: PRO38010
Figure 397: DNA331445, NP_002778.1, 201317_s_at
Figure 398: PRO71133
Figure 399: DNA274745, NP_006815.1, 201323_at
Figure 400: PRO62518
Figure 401: DNA150781, NP_001414.1, 201324_at
Figure 402: PRO12467

Figure 403: DNA329002, AF385084, 201326-at
Figure 404: PRO4912
Figure 405: DNA329002, NP_001753.1, 201327_s_at
Figure 406: PRO4912
Figure 407: DNA269536, S80343, 201330_at
Figure 408: PRO57952
Figure 409: DNA273323, NP_004243.1, 201349_at
Figure 410: PRO61330
Figure 411: DNA103227, NP_004466.1, 201350_at
Figure 412: PRO4557
Figure 413: DNA329934, NP_000090.1, 201360_at
Figure 414: PRO2721
Figure 415: DNA329107, NP_008818.3,201367_s_at
Figure 416: PRO84754
Figure 417A-B: DNA329108, 1383643.16, 201368_at
Figure 418: PRO84755
Figure 419: DNA329107, ZFP36L2, 201369_s_at
Figure 420: PRO84754
Figure 421A-E: DNA331446, NP_000436.1, 201373_at
Figure 422: PRO86502
Figure 423: DNA329109, NP_004957.1, 201376_s_at
Figure 424: PRO8185
Figure 425: DNA329111, NP_001349.1,201385_at
Figure 426: PRO84756
Figure 427: DNA270979, NP_002800.1, 201388_at
Figure 428: PRO59309
Figure 429: DNA331447, NP_006614.1, 201397_at
Figure 430: PRO85247
Figure 431: DNA329937, NP_002786.2, 201400_at
Figure 432: PRO61014
Figure 433: DNA328412, NP_060428.1, 201411_s_at
Figure 434: PRO84257
Figure 435: DNA329938, 1394805.1, 201416_at
Figure 436: PRO70544
Figure 437: DNA329939, 1393503.1,201417_at
Figure 438: PRO85248
Figure 439: DNA83109, NP_006323.1, 201422_at
Figure 440: PRO2592
Figure 441: DNA226600, NP_003371.1, 201426_s_at
Figure 442: PRO37063
Figure 443: DNA272286, NP_001743.1, 201432_at
Figure 444: PRO60544
Figure 445: DNA327550, NP_001959.1,201437_s_at
Figure 446: PRO81164
Figure 447A-C: DNA88140, COL6A3,201438_at
Figure 448: PRO2670
Figure 449: DNA150535, NP_004809.1, 201440_at
Figure 450: PRO1207
Figure 451: DNA325049, NP_005605.1, 201453_x_at
Figure 452: PRO37938
Figure 453: DNA326736, NP_006657.1,201459_at
Figure 454: PRO83076
Figure 455: DNA226359, NP_002219.1, 201464_x_at
Figure 456: PRO36822
Figure 457: DNA226359, JUN, 201465_s_at
Figure 458: PRO36822
Figure 459: DNA226359, DNA226359, 201466_s_at
Figure 460: PRO36822

Figure 461: DNA328413, NP_004823.1, 201470_at
Figure 462: PRO84258
Figure 463: DNA103320, NP_002220.1, 201473_at
Figure 464: PRO4650
Figure 465: DNA325704, NP_004981.2, 201475_x_at
Figure 466: PRO82188
Figure 467: DNA327551, NP_001024.1, 201476_s_at
Figure 468: PRO59289
Figure 469: DNA327551, RRM1, 201477_s_at
Figure 470: PRO59289
Figure 471: DNA254783, NP_001354.1, 201478_s_at
Figure 472: PRO49881
Figure 473: DNA329940, NP_001805.1,201487_at
Figure 474: PRO2679
Figure 475: DNA304459, BC005020,201489_at
Figure 476: PRO37073
Figure 477: DNA304459, NP_005720.1, 201490_s_at
Figure 478: PRO37073
Figure 479: DNA325920, NP_036243.1, 201491_at
Figure 480: PRO82373
Figure 481: DNA328415, BC006997, 201503_at
Figure 482: PRO60207
Figure 483: DNA329941, NP_001543.1, 201508_at
Figure 484: PRO85249
Figure 485: DNA323741, NP_003123.1, 201516_at
Figure 486: PRO80498
Figure 487: DNA329942, NCBP2, 201521_s_at
Figure 488: PRO85250
Figure 489: DNA328418, NP_003398.1, 201531_at
Figure 490: PRO84261
Figure 491: DNA331292, NP_002779.1, 201532_at
Figure 492: PRO84262
Figure 493: DNA329943, NP_009037.1, 201534_s_at
Figure 494: PRO85251
Figure 495: DNA331448, UBL3, 201535_at
Figure 496: PRO86503
Figure 497: DNA272171, NP-002379.2, 201555_at
Figure 498: PRO60438
Figure 499: DNA226291, NP_055047.1, 201556_s_at
Figure 500: PRO36754
Figure 501: DNA226291, VAMP2, 201557_at
Figure 502: PRO36754
Figure 503A-B: DNA270995, NP_004721.1, 201574_at
Figure 504: PRO59324
Figure 505: DNA227071, NP_000260.1,201577_at
Figure 506: PRO37534
Figure 507: DNA327199, NP_066979.1, 201580_s_at
Figure 508: PRO83475
Figure 509A-B: DNA329944, AB032988, 201581_at
Figure 510: DNA329945, NP-006354.2, 201583_s_at
Figure 511: PRO85252
Figure 512A-B: DNA329946, D80000,201589_at
Figure 513: DNA290280, NP_004359.1, 201605_x_at
Figure 514: PRO70425
Figure 515: DNA329947, NP_536806.1, 201613_s_at
Figure 516: PRO37674
Figure 517: DNA272904, NP_006784.1, 201619_at
Figure 518: PRO60991

Figure 519: DNA255406, NP_005533.1, 201625_s_at
Figure 520: PRO50473
Figure 521: DNA255406, INSIG1, 201627_s_at
Figure 522: PRO50473
Figure 523: DNA329115, NP_434702.1, 201631_s_at
Figure 524: PRO84760
Figure 525: DNA287240, NP_004326.1, 201641_at
Figure 526: PRO29371
Figure 527: DNA327557, NP_004214.1, 201649_at
Figure 528: PRO83588
Figure 529A-B: DNA220748, NP_000201.1, 201656_at
Figure 530: PRO34726
Figure 531A-B: DNA328422, NP_004448.1, 201662_s_at
Figure 532: PRO84263
Figure 533A-B: DNA273732, NP_005487.2, 201663_s_at
Figure 534: PRO61695
Figure 535A-B: DNA273732, HSM801845, 201664_at
Figure 536: PRO61695
Figure 537: DNA273090, NP_002347.4, 201669_s_at
Figure 538: PRO61148
Figure 539: DNA273090, MARCKS, 201670_s_at
Figure 540: PRO61148
Figure 541: DNA290244, NP_000261.1, 201695_s_at
Figure 542: PRO70353
Figure 543: DNA329948, NP_002797.1, 201699_at
Figure 544: PRO85253
Figure 545: DNA324742, NP_001751.1, 201700_at
Figure 546: PRO81367
Figure 547: DNA270883, NP_001061.1, 201714_at
Figure 548: PRO59218
Figure 549A-B: DNA151806, NP_001422.1, 201719_s_at
Figure 550: PRO12768
Figure 551: DNA227461, NP_006753.1, 201720_s_at
Figure 552: PRO37924
Figure 553: DNA227461, LAPTM5, 201721_s_at
Figure 554: PRO37924
Figure 555: DNA329949, BC003376, 201726_at
Figure 556: PRO85254
Figure 557: DNA227576, NP_005618.1, 201739_at
Figure 558: PRO38039
Figure 559: DNA326373, NP_008855.1, 201742_x_at
Figure 560: PRO82769
Figure 561: DNA327559, NP_058432.1, 201752_s_at
Figure 562: PRO83589
Figure 563: DNA331294, ADD3, 201753_s_at
Figure 564: PRO86393
Figure 565: DNA227035, NP_006730.1, 201755_at
Figure 566: PRO37498
Figure 567: DNA329016, NP_006283.1, 201758_at
Figure 568: PRO4887
Figure 569: DNA328427, NP_061109.1, 201760_s_at
Figure 570: PRO84265
Figure 571: DNA287167, NP_006627.1, 201761_at
Figure 572: PRO59136
Figure 573: DNA287625, NP_002809.1, 201762_s_at
Figure 574: PRO69491
Figure 575: DNA329950, NP_076961.1, 201764_at
Figure 576: PRO11558

Figure 577A-B: DNA329951, NP_055680.1, 201774_s_at
Figure 578: PRO85255
Figure 579: DNA151017, NP_004835.1, 201810_s_at
Figure 580: PRO12841
Figure 581: DNA151017, SH3BP5, 201811_x_at
Figure 582: PRO12841
Figure 583: DNA227929, NP_061932.1, 201812_s_at
Figure 584: PRO38392
Figure 585: DNA324015, NP_006326.1, 201821_s_at
Figure 586: PRO80735
Figure 587: DNA329952, BC010285, 201829_at
Figure 588: PRO85256
Figure 589: DNA329952, NET1, 201830_s_at
Figure 590: PRO85256
Figure 591: DNA329954, NP_001518.1, 201833_at
Figure 592: PRO85258
Figure 593A-B: DNA329955, AB029551, 201845_s_at
Figure 594: PRO85259
Figure 595: DNA254350, NP_004043.2, 201848_s_at
Figure 596: PRO49461
Figure 597: DNA254350, BNIP3, 201849_at
Figure 598: PRO49461
Figure 599: DNA329118, NP_068660.1, 2018533_s_at
Figure 600: PRO83123
Figure 601: DNA272066, NP_002931.1, 201872_s_at
Figure 602: PRO60337
Figure 603: DNA150805, NP_055703.1, 201889_at
Figure 604: PRO11583
Figure 605: DNA253582, DNA253582, 201890_at
Figure 606: PRO49181
Figure 607: DNA329956, NP_000875.1, 201892_s_at
Figure 608: PRO85260
Figure 609: DNA328431, NP_001817.1, 201897_s_at
Figure 610: PRO45093
Figure 611: DNA254978, NP_060625.1, 201917_s_at
Figure 612: PRO50067
Figure 613: DNA329057, NP_004116.2, 201921_at
Figure 614: PRO84719
Figure 615: DNA227112, NP_006397.1, 201923_at
Figure 616: PRO37575
Figure 617: DNA275240, NP_005906.2, 201930_at
Figure 618: PRO62927
Figure 619: DNA273014, NP_000117.1, 201931_at
Figure 620: PRO61085
Figure 621A-B: DNA329120, NP_002560.1, 201945_at
Figure 622: PRO2752
Figure 623: DNA274167, NP_006422.1, 201946_s_at
Figure 624: PRO62097
Figure 625: DNA274167, CCT2, 201947_s_at
Figure 626: PRO62097
Figure 627: DNA103481, NP_037417.1, 201948_at
Figure 628: PRO4808
Figure 629A-B: DNA327563, NP_066945.1, 201963_at
Figure 630: PRO83592
Figure 631: DNA275214, NP_002473.1, 201970_s_at
Figure 632: PRO62908
Figure 633A-B: DNA328433, ATP6V1A1, 201971_s_at
Figure 634: PRO84268

Figure 635A-B: DNA272191, NP_002947.1, 201975_at

Figure 636: PRO60456

Figure 637: DNA328809, PTPN12, 202006_at

Figure 638: PRO4803

Figure 639: DNA328437, AF083441, 202021_x_at

Figure 640: PRO84271

Figure 641: DNA329957, NP_005156.1, 202022_at

Figure 642: PRO85261

Figure 643A-B: DNA329958, NP-510880.1, 202039_at

Figure 644: PRO85262

Figure 645: DNA327017, NP_004586.2, 202043_s_at

Figure 646: PRO61744

Figure 647A-B: DNA227985, NP_055107.1, 202047_s_at

Figure 648: PRO38448

Figure 649A-B: DNA225991, NP_000518.1, 202067_s_at

Figure 650: PRO36454

Figure 651A-B: DNA225991, LDLR, 202068_s_at

Figure 652: PRO36454

Figure 653: DNA327567, NP_005521.1, 202069_s_at

Figure 654: PRO83596

Figure 655: DNA226116, NP_002990.1, 202071_at

Figure 656: PRO36579

Figure 657: DNA289522, NP-004994.1, 202077_at

Figure 658: PRO70276

Figure 659: DNA327568, NP_002453.1, 202086_at

Figure 660: PRO57922

Figure 661: DNA327569, CTSL, 202087_s_at

Figure 662: PRO2683

Figure 663: DNA329959, 251651.5, 202094_at

Figure 664: PRO85263

Figure 665: DNA129504, NP_001159.1, 202095_s_at

Figure 666: PRO7143

Figure 667: DNA328440, NP_004517.1, 202107_s_at

Figure 668: PRO84274

Figure 669: DNA329960, 1381890.1, 202136_at

Figure 670: PRO85264

Figure 671: DNA324895, NP_006294.2, 202138_x_at

Figure 672: PRO81501

Figure 673: DNA227150, NP_002337.1, 202145_at

Figure 674: PRO37613

Figure 675: DNA329020, NP_057637.1, 202153_s_at

Figure 676: PRO84695

Figure 677: DNA328442, NP_006078.2, 202154_x_at

Figure 678: PRO84275

Figure 679A-C: DNA331449, NP_004371.1, 202160_at

Figure 680: PRO86504

Figure 681: DNA327573, BC007655, 202165_at

Figure 682: PRO59301

Figure 683: DNA329962, AASDHPPT, 202170_s_at

Figure 684: PRO85266

Figure 685A-B: DNA329963, NP_060700.1, 202184_s_at

Figure 686: PRO85267

Figure 687: DNA254570, NP_055484.1, 202188_at

Figure 688: PRO49673

Figure 689A-B: DNA304479, BC016556, 202194_at

Figure 690: PRO733

Figure 691A-B: DNA329599, NP_003128.2, 202200_s_at

Figure 692: PRO85131

Figure 693A-B: DNA329964, 215949.9, 202206_at
Figure 694: PRO85268
Figure 695: DNA329965, BC001051, 202208_s_at
Figure 696: PRO85269
Figure 697: DNA325477, NP_004256.1, 202218_s_at
Figure 698: PRO12878
Figure 699: DNA328258, SLC16A1, 202236_s_at
Figure 700: PRO84151
Figure 701: DNA326133, NP_005021.2, 202240_at
Figure 702: PRO82557
Figure 703: DNA328444, MGC14458, 202246_s_at
Figure 704: PRO84277
Figure 705A-B: DNA227176, NP_056371.1, 202255_s_at
Figure 706: PRO37639
Figure 707: DNA326120, NP-006101.1, 202257_s_at
Figure 708: PRO82546
Figure 709: DNA150808, NP_002044.1, 202269_s_at
Figure 710: PRO12478
Figure 711: DNA150808, GBP1, 202270_at
Figure 712: PRO12478
Figure 713: DNA329966, NP_006295.1, 202276_at
Figure 714: PRO22705
Figure 715: DNA304716, NP_510867.1, 202284_s_at
Figure 716: PRO71142
Figure 717: DNA331450, NP_004381.1, 202295_s_at
Figure 718: PRO2682
Figure 719A-B: DNA329967, NP_003592.2, 202303_x_at
Figure 720: PRO85270
Figure 721: DNA329524, NP_000584.2, 202307_s_at
Figure 722: PRO36996
Figure 723A-B: DNA151108, NP_004167.3, 202308_at
Figure 724: PRO12105
Figure 725: DNA270142, NP-005947.2, 202309_at
Figure 726: PRO58531
Figure 727: DNA269842, NP_002708.1, 202313_at
Figure 728: PRO58243
Figure 729: DNA328448, NP_000777.1, 202314_at
Figure 730: PRO62362
Figure 731: DNA331451, UNG, 202330_s_at
Figure 732: PRO86505
Figure 733A-B: DNA329970, NP_000910.2, 202336_s_at
Figure 734: PRO85272
Figure 735: DNA255088, NP_003249.1, 202338_at
Figure 736: PRO50174
Figure 737: DNA325115, NP_001435.1, 202345_s_at
Figure 738: PRO81689
Figure 739: DNA270502, NP_002807.1, 202352_s_at
Figure 740: PRO58880
Figure 741A-B: DNA227353, NP_055637.1, 202375_at
Figure 742: PRO37816
Figure 743: DNA328449, NP_005462.1, 202382_s_at
Figure 744: PRO60304
Figure 745: DNA290234, NP_002914.1, 202388_at
Figure 746: PRO70333
Figure 747: DNA325417, NP_001742.1, 202402_s_at
Figure 748: PRO69635
Figure 749: DNA150989, NP_005523.1, 202411_at
Figure 750: PRO12569

Figure 751: DNA326563, NP_036421.2, 202417_at
Figure 752: PRO82927
Figure 753: DNA150514, NP_065203.1, 202418_at
Figure 754: PRO12304
Figure 755: DNA88332, NP_002026.1, 202419_at
Figure 756: PRO2753
Figure 757A-B: DNA329971, NP_075266.1, 202422_s_at
Figure 758: PRO85273
Figure 759: DNA227121, NP_066928.1, 202430_s_at
Figure 760: PRO37584
Figure 761: DNA66487, NP_002458.1, 202431_s_at
Figure 762: PRO1213
Figure 763: DNA103322, NP_005818.1, 202433_at
Figure 764: PRO4652
Figure 765: DNA68868, DNA68868, 202441_at
Figure 766: PRO1460
Figure 767: DNA227121, PLSCR1, 202446_s_at
Figure 768: PRO37584
Figure 769: DNA329972, BC004452, 202451_at
Figure 770: PRO85274
Figure 771A-B: DNA329973, NP_055461.1, 202459_s_at
Figure 772: PRO82824
Figure 773A-B: DNA269642, NP_004557.1, 202464_s_at
Figure 774: PRO58054
Figure 775: DNA227921, NP_003789.1, 202468_s_at
Figure 776: PRO38384
Figure 777A-B: DNA329122, NP_067675.1, 202478_at
Figure 778: PRO84764
Figure 779: DNA329123, NP_002873.1, 202483_s_at
Figure 780: PRO84765
Figure 781A-B: DNA103449, NP_008862.1, 202498_s_at
Figure 782: PRO4776
Figure 783: DNA329974, NP_055083.1, 202501_at
Figure 784: PRO85275
Figure 785: DNA234442, NP_055551.1, 202503_s_at
Figure 786: PRO38852
Figure 787A-B: DNA273879, NP_055753.1, 202519_at
Figure 788: PRO61835
Figure 789A-B: DNA277809, NP_055582.1, 202524_s_at
Figure 790: PRO64556
Figure 791: DNA328452, NP_000394.1, 202528_at
Figure 792: PRO63289
Figure 793A-B: DNA226870, DHFR, 202532_s_at
Figure 794: PRO37333
Figure 795: DNA331452, BC003584, 202533_s_at
Figure 796: PRO86506
Figure 797: DNA331453, NP_060993.1, 202534_x_at
Figure 798: PRO69586
Figure 799: DNA329976, NP_003815.1, 202535_at
Figure 800: PRO4801
Figure 801: DNA329977, BC001553, 202536_at
Figure 802: PRO85276
Figure 803A-B: DNA.255105, NP_000850.1, 202539_s_at
Figure 804: PRO50187
Figure 805A-B: DNA255105, HMGCR, 202540_s_at
Figure 806: PRO50187
Figure 807A-B: DNA274852, NP_004115.1, 202543_s_at
Figure 808: PRO62605

Figure 809: DNA275244, DNA275244, 202557_at
Figure 810A-C: DNA331454, NP_068506.1, 202565_s_at
Figure 811: PRO86507
Figure 812A-C: DNA329978, SVIL, 202566_s_at
Figure 813: PRO85277
Figure 814: DNA326939, NP_004166.1, 202567_at
Figure 815: PRO83257
Figure 816: DNA325587, NP_068772.1, 202580_s_at
Figure 817: PRO82083
Figure 818: DNA227607, HSPALB, 202581_at
Figure 819: PRO38070
Figure 820: DNA328456, NP_000467.1, 202587_s_at
Figure 821: PRO84283
Figure 822: DNA329979, NP_001062.1, 202589_at
Figure 823: PRO82821
Figure 824: DNA329125, NP_056159.1, 202595_s_at
Figure 825: PRO84767
Figure 826A-C: DNA270287, NP_003480.1, 202599_s_at
Figure 827: PRO58675
Figure 828A-C: DNA270287, NRIP1, 202600_s_at
Figure 829: PRO58675
Figure 830A-C: DNA329268, NP_004220.1, 202610_s_at
Figure 831: PRO84864
Figure 832: DNA274881, NP_001896.1, 202613_at
Figure 833: PRO62626
Figure 834A-B: DNA329980, 1134366.16, 202615_at
Figure 835: PRO85278
Figure 836: DNA329126, NP_005025.1, 202635_s_at
Figure 837: PRO84768
Figure 838: DNA59763, NP_000192.1, 202637_s_at
Figure 839: PRO160
Figure 840: DNA59763, CAM1, 202638_s_at
Figure 841: PRO160
Figure 842: DNA289528, NP_004302.1, 202641_at
Figure 843: PRO70286
Figure 844A-B: DNA151841, NP_006281.1, 202643_s_at
Figure 845: PRO12904
Figure 846A-B: DNA151841, TNFAIP3, 202644_s_at
Figure 847: PRO12904
Figure 848: DNA329981, NP_001155.1, 202652_at
Figure 849: PRO49894
Figure 850: DNA254129, NP_006001.1, 202655_at
Figure 851: PRO49244
Figure 852: DNA331455, NP_002792.1, 202659_at
Figure 853: PRO8763
Figure 854: DNA287424, NP_004292.1, 202666_s_-at
Figure 855: PRO69681
Figure 856: DNA326896, NP_003672.1, 202671_s_at
Figure 857: PRO69486
Figure 858: DNA289526, ATF3, 202672_s_at
Figure 859: PRO70282
Figure 860: DNA84130, NP-00380 1.1, 202687_s_at
Figure 861: PRO1096
Figure 862: DNA84130, TNFSF10, 202688_at
Figure 863: PRO1096
Figure 864: DNA329982, NP_008937.1, 202697_at
Figure 865: PRO85279
Figure 866A-B: DNA150467, NP_055513.1, 202699_s_at

Figure 867: PRO12272
Figure 868A-B: DNA150467, KIAA0792, 202700_s_at
Figure 869: PRO1227
Figure 870: DNA326000, NP_004692.1, 202705_at
Figure 871: PRO82442
Figure 872: DNA273371, NP_000364.1, 202706_s_at
Figure 873: PRO61373
Figure 874: DNA329983, BC012595, 202710_at
Figure 875: PRO85280
Figure 876: DNA43010, NP_000588.1, 202718_at
Figure 877: PRO36145
Figure 878A-B: DNA270254, NP _002006.2, 202723_s_at
Figure 879: PRO58642
Figure 880: DNA150713, NP_006570.1, 202735_at
Figure 881: PRO12082
Figure 882: DNA58828, DNA58828, 202746_at
Figure 883: PRO1189
Figure 884: DNA327192, NP_004858.1, 202747_s_at
Figure 885: PRO1189
Figure 886: DNA227133, NP-004111.1, 202748_at
Figure 887: PRO37596
Figure 888: DNA329984, NP_004618.2, 202749_at
Figure 889: PRO11656
Figure 890A-C: DNA329129, NP_009134.1, 202760_s_at
Figure 891: PRO84288
Figure 892: DNA329008, NP_004337.2, 202763_at
Figure 893: PRO12832
Figure 894A-B: DNA328464, 977954.20, 202769_at
Figure 895: PRO84290
Figure 896: DNA226578, NP_004345.1, 202770_s_at
Figure 897: PRO37041
Figure 898: DNA273346, NP_055316.1, 202779_s_at
Figure 899: PRO61349
Figure 900: DNA329985, NP_002185.1, 202794_at
Figure 901: PRO60589
Figure 902: DNA88428, NP_000202.1, 202803_s_at
Figure 903: PRO2787
Figure 904: DNA329986, NP_006454.1, 202811çat
Figure 905: PRO61895
Figure 906A-B: DNA226364, NP_001612.1, 202820_at
Figure 907: PRO36827
Figure 908: DNA328465, NP_005639.1, 202823_at
Figure 909: PRO84291
Figure 910: DNA329987, NP_000286.2, 202833_s_at
Figure 911: PRO85281
Figure 912: DNA269828, FLN29, 202837_at
Figure 913: PRO58230
Figure 914: DNA329988, NP_036460.1, 202842_s_at
Figure 915: PRO1471
Figure 916: DNA329988, DNAJB9, 202843_at
Figure 917: PRO147
Figure 918: DNA103394, NP_004198.1, 202855_s_at
Figure 919: PRO4722
Figure 920: DNA103394, SLC16A3, 202856_s_at
Figure 921: PRO4722
Figure 922A-B: DNA272022, PER1, 202861_at
Figure 923: PRO60296
Figure 924: DNA275144, NP_000128.1, 202862-at

EP 2 179 742 A1

Figure 925: PRO62852
Figure 926: DNA328467, SP100, 202864_s_at
Figure 927: PRO84293
Figure 928: DNA287289, NP_058132.1, 202869_at
Figure 929: PRO69559
Figure 930: DNA273060, NP_001246.1, 202870_s_at
Figure 931: PRO61125
Figure 932: DNA329130, NP_004286.2, 202871_at
Figure 933: PRO20124
Figure 934: DNA328469, NP_001686.1, 202874_s_at
Figure 935: PRO84295
Figure 936: DNA271881, PSCD1, 202880_s_at
Figure 937: PRO60160
Figure 938: DNA329989, HSPPP2R15, 202886_s_at
Figure 939A-B: DNA225538, NP_002476.1, 202906_s_at
Figure 940: PRO36001
Figure 941A-B: DNA225538, NBS1, 202907_s_at
Figure 942: PRO36001
Figure 943: DNA328483, NP_061163.1, 202911_at
Figure 944: PRO84309
Figure 945: DNA327584, NP_002955.2, 202917 _s_at
Figure 946: PRO80649
Figure 947A-B: DNA329132, NP_002648.1, 202925_s_at
Figure 948: PRO83145
Figure 949: DNA272979, NP_003841.1, 202930_s_at
Figure 950: PRO61058
Figure 951: DNA331456, BIN1, 202931_x_at
Figure 952: PRO86508
Figure 953: DNA327585, NP_056518.1, 202937_x_at
Figure 954: PRO83605
Figure 955: DNA328471, ZMPSTE24, 202939_at
Figure 956: PRO84297
Figure 957: DNA304681, NP_066552.1, 202941_at
Figure 958: PRO71107
Figure 959: DNA269481, NP_001976.1, 202942_at
Figure 960: PRO57901
Figure 961: DNA273320, NP_008950.1, 202954_at
Figure 962: PRO61327
Figure 963: DNA273334, NP_000246.1, 202960_s_at
Figure 964: PRO61341
Figure 965A-B: DNA328473, NP_006473.1, 202968_s_at
Figure 966: PRO84299
Figure 967A-B: DNA227293, NP_055698.1, 202972_s_at
Figure 968: PRO37756
Figure 969A-B: DNA227293, KIAA0914, 202973_x_at
Figure 970: PRO37756
Figure 971: DNA329135, NP_002913.2, 2029.88_s_at
Figure 972: PRO58102
Figure 973: DNA274034, NP_006388.1, 203022_at
Figure 974: PRO61977
Figure 975: DNA329136, NP_057475.1, 203023_at
Figure 976: PRO84772
Figure 977A-B: DNA271865, NP_055566.1, 203037_s_at
Figure 978: PRO60145
Figure 979A-B: DNA304464, NP_055733.1, 203044_at
Figure 980: PRO71042
Figure 981A-B: DNA329991, NP_003911.1, 203046_s_at
Figure 982: PRO85284

Figure 983: DNA331457, AF119894, 203047_at
Figure 984: PRO8650
Figure 985: DNA150976, NP_071503.1, 203054_s_at
Figure 986: PRO12565
Figure 987: DNA326693, NP_004697.2, 203055_s_at
Figure 988: PRO83039
Figure 989: DNA188357, NP_000651.1,
Figure 990: PRO21897 203285_s_at
Figure 991: DNA324133, NP_037379.1, 203089_s_at
Figure 992: PRO80835
Figure 993: DNA269984, NP_055443.1, 203094_at
Figure 994: PRO58380
Figure 995: DNA329992, NP_002399.1, 203102_s_at
Figure 996: PRO59267
Figure 997: DNA329993, NP_115754.1, 203113_s_at
Figure 998: PRO85285
Figure 999: DNA329994, PCSK7, 203118_at
Figure 1000: PRO85286
Figure 1001A-B: DNA150447, NP_004854.1, 203128_at
Figure 1002: PRO12256
Figure 1003: DNA254543, NP_006799.1, 203133_at
Figure 1004: PRO49648
Figure 1005: DNA269918, NP_003633.1, 203138_at
Figure 1006: PRO58316
Figure 1007: DNA329001, BCL6, 203140_at
Figure 1008: PRO26296
Figure 1009A-B: DNA329995, NP_006452.1, 203145_at
Figure 1010: PRO85287
Figure 1011A-B: DNA226330, NP_001461.1, 203146_s_at
Figure 1012: PRO36793
Figure 1013: DNA271624, NP_001539.1, 203153_at
Figure 1014: PRO59911
Figure 1015: DNA269660, NP_003192.1, 203177_s_at
Figure 1016: PRO58071
Figure 1017: DNA304720, NP_062427.1, 203186_s_at
Figure 1018: PRO71146
Figure 1019A-B: DNA271744, NP_055476.1, 203206_at
Figure 1020: PRO60028
Figure 1021: DNA329997, BC001866, 203209_at
Figure 1022: PRO61115
Figure 1023: DNA329997, NP_031396.1, 203210_s_at
Figure 1024: PRO61115
Figure 1025A-B: DNA328481, MTMR2, 203211_s_at
Figure 1026: PRO84307
Figure 1027: DNA331458, 995529.4, 203213_at
Figure 1028: PRO86510
Figure 1029: DNA331459, CDC2, 203214_x_at
Figure 1030: PRO70806
Figure 1031: DNA76514, NP_000409.1, 203233_at
Figure 1032: PRO2540
Figure 1033: DNA325507, NP_005842.1, 203252_at
Figure 1034: PRO69461
Figure 1035: DNA330000, NP_036277.1, 203270-at
Figure 1036: PRO85289
Figure 1037: DNA302020, NP_005564.1, 203276_at
Figure 1038: PRO70993
Figure 1039: DNA328486, NP_000149.1, 203282_at
Figure 1040: PRO60119

Figure 1041A-B: DNA330001, NP_036394.1, 203085_s_at
Figure 1042: PRO85290
Figure 1043: DNA225675, NP_005561.1, 203293_s_at
Figure 1044: PRO36138
Figure 1045: DNA330002, BC007195, 203315_at
Figure 1046: PRO80853
Figure 1047A-B: DNA330003, NP_005532.1, 203331_s_at
Figure 1048: PRO85291
Figure 1049: DNA330004, NP_055785.2, 203333_at
Figure 1050: PRO85292
Figure 1051: DNA330005, NP_003696.2, 203340_s_at
Figure 1052: PRO85293
Figure 1053: DNA271959, NP_002885.1, 203344_s_at
Figure 1054: PRO60234
Figure 1055: DNA330006, NP_031384.1, 203347_s_at
Figure 1056: PRO85294
Figure 1057: DNA330007, NP_055111.1, 203357_s_at
Figure 1058: PRO85295
Figure 1059: DNA330008, NP_004447.2, 203358_s_at
Figure 1060: PRO85296
Figure 1061: DNA272449, NP_036465.1, 203360_s_at
Figure 1062: PRO60698
Figure 1063: DNA324514, NP_002349.1, 203362_s_at
Figure 1064: PRO81169
Figure 1065: DNA325749, NP_003868.1, 203372_s_at
Figure 1066: PRO12839
Figure 1067: DNA325749, STATI2, 203373_at
Figure 1068: PRO12839
Figure 1069: DNA274960, NP_008856.1, 203380_x_at
Figure 1070: PRO62694
Figure 1071: DNA151022, NP_001336.1, 203385_at
Figure 1072: PRO12096
Figure 1073: DNA331460, NP_002780.1, 203396_at
Figure 1074: PRO60499
Figure 1075: DNA326892, NP_003711.1, 203405_at
Figure 1076: PRO83213
Figure 1077: DNA274778, NP_005917.1, 203406_at
Figure 1078: PRO62545
Figure 1079: DNA270134, NP_000098.1, 203409_at
Figure 1080: PRO58523
Figure 1081: DNA28759, NP_006150.1, 203413_at
Figure 1082: PRO2520
Figure 1083: DNA287267, NP_001228.1, 203418_at
Figure 1084: PRO37015
Figure 1085A-B: DNA256807, NP_057339.1, 203420_at
Figure 1086: PRO51738
Figure 1087: DNA326745, NP_002682.1, 203422_at
Figure 1088: PRO83083
Figure 1089: DNA330009, NP_054753.1, 203428_s_at
Figure 1090: PRO85297
Figure 1091A-B: DNA275186, DNA275186, 203432_at
Figure 1092A-B: DNA330010, NP_005721.2, 203445_s_at
Figure 1093: PRO85298
Figure 1094: DNA273410, NP_004036.1, 203454_s_at
Figure 1095: PRO61409
Figure 1096: DNA328495, NP_055578.1, 203465_at
Figure 1097: PRO58967
Figure 1098A-C: DNA331461, NP_005493.2, 203504_at

Figure 1099: PRO86511
Figure 1100A-B: DNA331462, NP_003096.1, 203509_ at
Figure 1101: PRO86512
Figure 1102: DNA272911, NP_006545.1, 203517_at
Figure 1103: PRO60997
Figure 1104A-D: DNA331463, NP_000072.1, 203518_at
Figure 1105: PRO86513
Figure 1106A-C: DNA331464, NP_055160.1, 203520_s_at
Figure 1107: PRO86514
Figure 1108A-C: DNA330014, HRIHFB2436, 203521_s_at
Figure 1109: PRO85302
Figure 1110: DNA325404, NP_002330.1, 203523_at
Figure 1111: PRO81936
Figure 1112: DNA323910, NP_002956.1, 203535_at
Figure 1113: PRO80648
Figure 1114A-B: DNA272399, NP_001197.1, 203542_s_at
Figure 1115: PRO60653
Figure 1116A-B: DNA272399, BTEB1, 203543_s_at
Figure 1117: PRO60653
Figure 1118: DNA324684, NP_004210.1, 203554_x_at
Figure 1119; PRO81319
Figure 1120; DNA330015, NP_004620.1, 203564_at
Figure 1121: PRO58704
Figure 1122: DNA330016, NP_006346.1, 203567_s_at
Figure 1123: PRO85303
Figure 1124A-B: DNA150765, NP_003974.1, 203579_s_at
Figure 1125: PRO12458
Figure 1126: DNA273676, NP_0555488.1, 203584_at
Figure 1127: PRO61644
Figure 1128: DNA271003, NP_003720.1, 203594_at
Figure 1129: PRO59332
Figure 1130A-B: DNA270323, NP_036552.1, 203595_s_at
Figure 1131: PRO58710
Figure 1132A-B: DNA270323, R158,203596_s_at
Figure 1133: PRO58710
Figure 1134: DNA330017, NP_009118.1, 203597_s_at
Figure 1135: PRO60916
Figure 1136: DNA329604, NP_003127.1, 203605_at
Figure 1137: PRO85134
Figure 1138: DNA287246, NP_004044.2, 203612_at
Figure 1139: PRO69521
Figure 1140: DNA330018, NP_064528.1, 203622_s_at
Figure 1141: PRO85304
Figure 1142: DNA331465, SKP2, 203625_x_at
Figure 1143: PRO81225
Figure 1144A-B: DNA327596, 345314.2, 203628_at
Figure 1145: PRO1920
Figure 1146A-B: DNA331466, BCL2, 203685_at
Figure 1147: PRO86515
Figure 1148A-B: DNA330021, NP_001940.1, 203693_s_at
Figure 1149: PRO85306
Figure 1150: DNA329900, RFC2, 203696_s_at
Figure 1151: PRO81549
Figure 1152A-C: DNA331467, NP_002213.1, 203710_at
Figure 1153: PRO86516
Figure 1154: DNA329144, KIAA0020, 203712_at
Figure 1155: PRO84779
Figure 1156: DNA326402, NP_004515.1, 203713_s_at

Figure 1157: PRO82793
Figure 1158: DNA324183, DPP4, 203716_s_at
Figure 1159: PRO80881
Figure 1160: DNA150784, NP_001974.1, 203720_s_at
Figure 1161: PRO12800
Figure 1162A-B: DNA269573, NP_002212.1, 203723_at
Figure 1163: PRO57986
Figure 1164: DNA330023, NP_001915.1, 203725_at
Figure 1165: PRO85308
Figure 1166: DNA227020, NP_001416.1, 203729_at
Figure 1167: PRO37483
Figure 1168A-B: DNA325369, NP-055877.2, 203737_s_at
Figure 1169: PRO81905
Figure 1170A-B: DNA150748, NP_001105.1, 203741_s_at
Figure 1171: PRO12446
Figure 1172: DNA327523, AQP3, 203747_at
Figure 1173: PRO38028
Figure 1174: DNA330024, NP_058521.1, 203748_x_at
Figure 1175: PRO85309
Figure 1176: DNA97279, NP-005345.2, 203751_x_at
Figure 1177: PRO3628
Figure 1178A-B: DNA325972, BUB1B, 203755_at
Figure 1179: PRO82417
Figure 1180: DNA330025, NP_055565.2, 203764_at
Figure 1181: PRO85310
Figure 1182: DNA330026, NP_005899.1, 203778_at
Figure 1183: PRO85311
Figure 1184: DNA330027, NP_036578.1, 203787_at
Figure 1185: PRO85312
Figure 1186A-B: DNA150954, NP_055695.1, 203799-at
Figure 1187: PRO12558
Figure 1188: DNA331468, DGUOK, 203816_at
Figure 1189: PRO86517
Figure 1190: DNA274125, NP_071739.1, 203830_at
Figure 1191: PRO62061
Figure 1192A-B: DNA331469, 094680.4, 203845_at
Figure 1193: PRO86518
Figure 1194A-B: DNA325529, GAB2, 203853_s_at
Figure 1195: PRO82037
Figure 1196A-B: DNA275079, NP_056648.1, 203865_s_at
Figure 1197: PRO62797
Figure 1198: DNA275339, NP_005685.1, 203880-at
Figure 1199: PRO63012
Figure 1200: DNA329034, NP_006075.2, 203882_at
Figure 1201: PRO84701
Figure 1202A-B: DNA288692, NP_055719.1, 203884_s_at
Figure 1203: PRO70078
Figure 1204: DNA328513, TAF9, 203893_at
Figure 1205: PRO37815
Figure 1206A-B: DNA330030, NP_055684.1, 203907_s_at
Figure 1207: PRO85315
Figure 1208: DNA82376, NP_002407.1, 203915_at
Figure 1209: PRO1723
Figure 1210: DNA271676, NP_002052.1, 203925-at
Figure 1211: PRO59961
Figure 1212: DNA288249, NP_002940.1, 203931_s_at
Figure 1213: PRO69507
Figure 1214: DNA330031, NP_057210.1, 203960_s_at

Figure 1215: PRO85316
Figure 1216: DNA275012, NP_004679.1, 203964_at
Figure 1217: PRO62740
Figure 1218: DNA272338, NP_001245.1, 203967_at
Figure 1219: PRO60595
Figure 1220: DNA272338, CDC6, 203968_s_at
Figure 1221: PRO60595
Figure 1222: DNA227232, NP_001850.1, 203971_at
Figure 1223: PRO37695
Figure 1224: DNA271374, NP_005474.1, 203976_s_at
Figure 1225: PRO9673
Figure 1226: DNA226133, NP_001983.1, 203989_x_at
Figure 1227: PRO36596
Figure 1228: DNA225915, NP-000561.1, 204006_s_at
Figure 1229: PRO36378
Figure 1230: DNA330032, HUMGCRFC, 204007_at
Figure 1231: PRO85317
Figure 1232: DNA329145, DUSP4, 204014_at
Figure 1233: PRO84780
Figure 1234: DNA331470, HSU48807, 204015_s_at
Figure 1235: PRO86519
Figure 1236: DNA326089, NP_000508.1, 204018_x_at
Figure 1237: PRO3629
Figure 1238: DNA330033, NP_056492.1, 204019_s_at
Figure 1239: PRO85318
Figure 1240: DNA330034, NP_002907.1, 204023_at
Figure 1241: PRO85319
Figure 1242: DNA328271, NP-008988.2, 204026_s_at
Figure 1243: PRO81868
Figure 1244: DNA330035, NP_004228.1, 204033_at
Figure 1245: PRO85320
Figure 1246: DNA325181, CLTA, 204050_s_at
Figure 1247: PRO81742
Figure 1248: DNA226342, NP_000305.1, 204054_at
Figure 1249: PRO36805
Figure 1250A-B: DNA331471, NP_055498.1, 204063_s_art
Figure 1251: PRO61468
Figure 1252: DNA274783, NP_006272.1, 204068_at
Figure 1253: PRO62549
Figure 1254A-C: DNA331472, NP_075463.1, 204072_s_art
Figure 1255: PRO86520
Figure 1256: DNA270476, NP_003591.1, 204092_s_at
Figure 1257: PRO58855
Figure 1258: DNA216689, NP_002975.1, 204103_at
Figure 1259: PRO34276
Figure 1260: DNA328522, NP_001769.2, 204118_at
Figure 1261: PRO2696
Figure 1262: DNA150529, NP_003323.1, 204122_at
Figure 1263: PRO12313
Figure 1264: DNA328524, NP_057097.1, 204125_at
Figure 1265: PRO84336
Figure 1266: DNA304489, NP_003495.1, 204126_s_at
Figure 1267: PRO71058
Figure 1268: DNA330037, BC000149, 204127_at
Figure 1269: PRO82290
Figure 1270: DNA325824, NP_002906.1, 204128_s_at
Figure 1271: PRO82290
Figure 1272: DNA328525, BC021224, 204131_s_at

Figure 1273: PRO84337
Figure 1274: DNA103532, NP_003263.1, 204137_at
Figure 1275: PRO4859
Figure 1276: DNA330038, BC016330, 204146_at
Figure 1277: PRO85322
Figure 1278: DNA330039, NP_002396.2, 204152_s_at
Figure 1279: PRO85323
Figure 1280: DNA330039, MFNG, 204153_s_at
Figure 1281: PRO85323
Figure 1282: DNA330040, NPS23240.1, 204159_at
Figure 1283: PRO59546
Figure 1284: DNA273694, NP_006092.1, 204162_at
Figure 1285: PRO61661
Figure 1286: DNA227116, NP_006738.1, 204164_at
Figure 1287: PRO37579
Figure 1288A-B: DNA254376, NP_055778.1, 204166_at
Figure 1289: PRO49486
Figure 1290: DNA272655, NP_001818.1, 204170_s_at
Figure 1291: PRO60781
Figure 1292: DNA330041, NP-000088.2, 204172_at
Figure 1293: PRO85324
Figure 1294: DNA328528, MLC1SA, 204173_at
Figure 1295: PRO60636
Figure 1296: DNA329148, NP_056955.1, 204175_at
Figure 1297: PRO84782
Figure 1298: DNA226380, NP_001765.1, 204192_at
Figure 1299: PRO4695
Figure 1300: DNA271778, NP_068594.1, 204205_at
Figure 1301: PRO60062
Figure 1302: DNA330042, HSU16307, 204221_x_at
Figure 1303: PRO85325
Figure 1304: DNA150812, NP_006842.1, 204222_s_at
Figure 1305: PRO12481
Figure 1306: DNA227514, NP_000152.1, 204224_s_at
Figure 1307: PRO37977
Figure 1308: DNA88308, NP_004097.1, 204232_at
Figure 1309: PRO2739
Figure 1310: DNA226881, NP_002008.2, 204236_at
Figure 1311: PRO37344
Figure 1312: DNA270434, NP_006434.1, 204238_s_at
Figure 1313: PRO58814
Figure 1314A-B: DNA287273, NP_006435.1, 204240_s_at
Figure 1315: PRO69545
Figure 1316: DNA330043, NP_001789.2, 204252_at
Figure 1317: PRO85326
Figure 1318A-B: DNA103527, NP_000367.1, 204253_s_at
Figure 1319: PRO4854
Figure 1320A-B: DNA103527, VDR, 204254_s_at
Figure 1321: PRO4854
Figure 1322A-B: DNA103527, HUMVDR, 204255_s_at
Figure 1323: PRO4854
Figure 1324: DNA228132, NP_076995.1, 204256_at
Figure 1325: PRO38595
Figure 1326: DNA226577, NP_071390.1, 204265_s_at
Figure 1327: PRO37040
Figure 1328: DNA88643, SGSH, 204293_at
Figure 1329: PRO2455
Figure 1330: DNA330044, GTSE1, 204318_s_at

Figure 1331: PRO85327
Figure 1332: DNA330045, NP_005943.1, 204326_x_at
Figure 1333: PRO82583
Figure 1334: DNA328530, NP_009198.2, 204328_at
Figure 1335: PRO24118
Figure 1336: DNA330046, 987987.10, 204334_at
Figure 1337: PRO85328
Figure 1338: DNA328531, NP_037542.1, 204348_s_at
Figure 1339: PRO84338
Figure 1340: DNA330047, BC005250, 204349_at
Figure 1341: PRO37777
Figure 1342A-B: DNA193847, NP_055518.1, 204377_s_at
Figure 1343: PRO23272
Figure 1344: DNA328533, NP_003647.1, 204392_at
Figure 1345: PRO84340
Figure 1346: DNA226462, NP_002241.1, 204401_at
Figure 1347: PRO36925
Figure 1348A-B: DNA330048, AF080255, 204407_at
Figure 1349: PRO85329
Figure 1350: DNA327616, NP_075011.1, 204415_at
Figure 1351: PRO83624
Figure 1352: DNA331473, NP_000839.1, 204418_s_at
Figure 1353: PRO60552
Figure 1354: DNA226286, NP-001657.1, 204425_at
Figure 1355: PRO36749
Figure 1356: DNA327617, NP_006811.1, 204439_at
Figure 1357: PRO83625
Figure 1358A-B: DNA330049, NP_004514.2, 204444_at
Figure 1359: PRO85330
Figure 1360: DNA329150, NP_000689.1, 204446_s_at
Figure 1361: PRO84783
Figure 1362: DNA270496, NP_001316.1, 204459_at
Figure 1363: PRO58875
Figure 1364: DNA330050, NP_056289.1, 204502_at
Figure 1365: PRO85331
Figure 1366: DNA273612, HSU79274, 204521 -at
Figure 1367: PRO61586
Figure 1368: DNA330051, NP_003431.1, 204523_at
Figure 1369: PRO85332
Figure 1370A-B: DNA330052, NP_009227.1, 204531_s_at
Figure 1371: PRO25103
Figure 1372: DNA82362, NP_001556.1, 204533_at
Figure 1373: PRO1718
Figure 1374A-B: DNA331474, 357276.11, 204552_at
Figure 1375: PRO86521
Figure 1376A-B: DNA329036, NP_002451.1, 204562_at
Figure 1377: PRO84703
Figure 1378: DNA287284, NP_060943.1, 204565_at
Figure 1379: PRO59915
Figure 1380: DNA151910, NP_004906.2, 204567_s_at
Figure 1381: PRO12754
Figure 1382A-B: DNA273627, NP_055739.1, 204568-at
Figure 1383: PRO61599
Figure 1384: DNA272992, N4BP1, 204601_at
Figure 1385: PRO61064
Figure 1386: DNA254157, NP-005245.2, 204618_s_at
Figure 1387: PRO49271
Figure 1388: DNA151048, NP_006177.1, 204621_s_at

Figure 1389: PRO12850
Figure 1390: DNA151048, NR4A2, 204622_x_at
Figure 1391: PRO12850
Figure 1392A-B: DNA330054, NP_004746.1, 204633_s_-at
Figure 1393: PRO85334
Figure 1394: DNA254470, NP_002488.1, 204641_at
Figure 1395: PRO49578
Figure 1396: DNA226182, EDG1, 204642_at
Figure 1397: PRO36645
Figure 1398: DNA210121, CDW52, 204661_at
Figure 1399: PRO33667
Figure 1400: DNA103526, LRMP, 204674_at 2
Figure 1401: PRO4853
Figure 1402: DNA225974, NP_000864.1, 204683_at
Figure 1403: PRO36437
Figure 1404: DNA256295, LRN, 204692_at
Figure 1405: PRO51339
Figure 1406: DNA227573, NP_001780.1, 204696_s_at
Figure 1407: PRO38036
Figure 1408: DNA329151, NP_004280.3, 204702_s_at
Figure 1409: PRO84784
Figure 1410: DNA331475, KNSL5, 204709_s_at
Figure 1411: PRO86522
Figure 1412A-B: DNA331476, NP_000121.1, 204713_s_at
Figure 1413: PRO86523
Figure 1414A-B: DNA225911, F5, 204714_s_at
Figure 1415: PRO36374
Figure 1416A-B: DNA218283, NP_004436.1, 204718_at
Figure 1417: PRO34335
Figure 1418A-B: DNA256461, NP_009017.1, 204728_s_at
Figure 1419: PRO51498
Figure 1420A-C: DNA274487, NP_055562.1, 204730_at
Figure 1421: PRO62389
Figure 1422A-B: DNA83176, NP_003234.1, 204731_at
Figure 1423: PRO2620
Figure 1424A-B: DNA325192, NP_038203.1, 204744_s_at
Figure 1425: PRO81753
Figure 1426: DNA330057, NP_005941.1, 204745_x_at
Figure 1427: PRO85337
Figure 1428: DNA287178, NP_001540.1, 204747_at
Figure 1429: PRO69467
Figure 1430: DNA330058, NP-004529.2, 204749_at
Figure 1431: PRO85338
Figure 1432: DNA329153, NP_001259.1, 204759_at
Figure 1433: PRO84786
Figure 1434: DNA330059, NP_068370.1, 204760_s_at
Figure 1435: PRO85339
Figure 1436: DNA330060, NP_002443.2, 204766_s_at
Figure 1437: PRO85340
Figure 1438: DNA329154, BC000323, 204767_s_at
Figure 1439: PRO69568
Figure 1440: DNA325479, NP_004102.1, 204768_s_at
Figure 1441: PRO69568
Figure 1442: DNA328541, NP_004503.1, 204773_at
Figure 1443: PRO4843
Figure 1444: DNA329155, NP_000034.1, 204780_s_at
Figure 1445: PRO1207
Figure 1446: DNA329155, TNFRSF6, 204781_s_at

Figure 1447: PRO1207
Figure 1448: DNA272121, NP_005895.1, 204790_at
Figure 1449: PRO60391
Figure 1450A-B: DNA330061, NP_055525.1, 04793_at
Figure 1451: PRO85341
Figure 1452: DNA103269, NP_005366.1, 204798_at
Figure 1453: PRO4599
Figure 1454: DNA287168, NP_003132.2, 204804_at
Figure 1455: PRO69460
Figure 1456: DNA330062, NP_006017.1, 204805_s_at
Figure 1457: PRO85342
Figure 1458A-B: DNA329907, ESPL1, 204817_at
Figure 1459: PRO85224
Figure 1460: DNA331477, NP_003309.1, 204822_at
Figure 1461: PRO58276
Figure 1462: DNA255289, NP_055606.1, 204825_at
Figure 1463: PRO50363
Figure 1464A-B: DNA226387, NP_001752.1, 204826-at
Figure 1465: PRO36850
Figure 1466: DNA328544, NP_006673.1, 204834_at
Figure 1467: PRO84347
Figure 1468A-B: DNA270446, NP_058633.1, 204835_at
Figure 1469: PRO58825
Figure 1470: DNA330063, HUMLPTPASE, 204852_s_at
Figure 1471: PRO85343
Figure 1472: DNA150598, NP_003541.1, 204857_at
Figure 1473: PRO12142
Figure 1474: DNA225661, NP_001944.1, 204858_at
Figure 1475: PRO36124
Figure 1476A-B: DNA330064, 332518.2, 204886_at
Figure 1477: PRO85344
Figure 1478: DNA330065, NP_055079.2, 204887_s_at
Figure 1479: PRO85345
Figure 1480: DNA103444, LCK, 204890_s_at
Figure 1481: PRO4771
Figure 1482: DNA331478, BC013200, 204891_s_at
Figure 1483: PRO86524
Figure 1484: DNA194139, DNA194139, 204897_at
Figure 1485: PRO23533
Figure 1486: DNA255326, NP_003855.1, 204900_s_at
Figure 1487: PRO50396
Figure 1488: DNA329157, NP_004271.1, 204905_s_at
Figure 1489: PRO62861
Figure 1490: DNA329011, NP_005169.1, 204908_s_at
Figure 1491: PRO4785
Figure 1492A-B: DNA76503, NP_001549.1, 204912_at
Figure 1493: PRO2536
Figure 1494: DNA330066, NP_004520.1, 204917_s_at
Figure 1495: PRO85346
Figure 1496: DNA228014, NP_002153.1, 204949_at
Figure 1497: PRO38477
Figure 1498: DNA271093, NP_004064.1, 204958_at
Figure 1499: PRO59417
Figure 1500: DNA103283, NP_002423.1, 204959_at
Figure 1501: PRO4613
Figure 1502: DNA330067, NP_001800.1, 204962_s_at
Figure 1503: PRO60368
Figure 1504: DNA287399, NP_058197.1, 204972_at

EP 2 179 742 A1

Figure 1505: PRO69656
Figure 1506: DNA269665, NP_002454.1, 204994_at
Figure 1507: PRO58076
Figure 1508: DNA331479, 411441.5, 204995_at
Figure 1509: PRO86525
Figure 1510: DNA272427, NP_004799.1, 205005_s_at
Figure 1511: PRO60679
Figure 1512: DNA272427, NMT2, 205006_s_at
Figure 1513: PRO60679
Figure 1514: DNA329534, NP_004615.2, 205019_s_at
Figure 1515: PRO2904
Figure 1516: DNA331480, RAD51, 205024_s_at
Figure 1517: PRO86526
Figure 1518: DNA329159, NP_005195.2, 205027_s_at
Figure 1519: PRO4660
Figure 1520: DNA325061, NP_005208.1, 205033_s_at
Figure 1521: PRO9980
Figure 1522: DNA328297, NP_477097.1, 205034_at
Figure 1523: PRO59418
Figure 1524A-C: DNA331481, NP_001804.1, 205046-at
Figure 1525: PRO86527
Figure 1526: DNA324991, ASNS, 205047_s_at
Figure 1527: PRO81585
Figure 1528: DNA271461, NP_000937.1, 205053_at
Figure 1529: PRO59757
Figure 1530A-B: DNA220750, NP_002199.2, 205055_at
Figure 1531: PRO34728
Figure 1532: DNA330071, NP_003607.1, 205063_at
Figure 1533: PRO85350
Figure 1534: DNA330072, NP_071801.1, 205072_s_at
Figure 1535: PRO85351
Figure 1536: DNA304705, NP_002634.1, 205078_at
Figure 1537: PRO71131
Figure 1538: DNA327632, NP-001302.1, 205081_at
Figure 1539: PRO83635
Figure 1540: DNA255336, NP_061332.1, 205084_at
Figure 1541: PRO50406
Figure 1542: DNA330073, NP_004144.1, 205085_at
Figure 1543: PRO85352
Figure 1544: DNA330074, HUMHM145, 205098_at
Figure 1545: PRO85353
Figure 1546: DNA226177, NP_001286.1, 205099_s_at
Figure 1547: PRO36640
Figure 1548: DNA192060, NP_002974.1, 205114_s_at
Figure 1549: PRO21960
Figure 1550: DNA299899, NP_002148.1, 205133_s_at
Figure 1551: PRO62760
Figure 1552: DNA331482, NP_001241.1, 205153_s_at
Figure 1553: PRO34457
Figure 1554: DNA330075, CDC25C, 205167_s_at
Figure 1555: PRO85354
Figure 1556: DNA330076, NP_005410.1, 205170_at
Figure 1557: PRO85355
Figure 1558: DNA328810, NP_001770.1, 205173_x_at
Figure 1559: PRO2557
Figure 1560: DNA330077, ITGB3BP, 205176_s_at
Figure 1561: PRO85356
Figure 1562: DNA151804, NP_006500.1, 205205_at

Figure 1563: PRO12188
Figure 1564: DNA272443, NP_055531.1, 205213_at
Figure 1565: PRO60693
Figure 1566: DNA273535, NP_004217.1, 205214_at
Figure 1567: PRO61515
Figure 1568: DNA325255, NP_001994.2, 205237_at
Figure 1569: PRO1910
Figure 1570: DNA330078, NP_001648.1, 205239_at
Figure 1571: PRO46
Figure 1572: DNA327634, NP_005129.1, 205241_at
Figure 1573: PRO83636
Figure 1574: DNA188333, NP_006410.1, 205242_at
Figure 1575: PRO21708
Figure 1576: DNA227081, NP_000390.2, 205249_at
Figure 1577: PRO37544
Figure 1578: DNA227447, NP_003193.1, 205254_x_at
Figure 1579: PRO37910
Figure 1580: DNA227447, TCF7, 205255x_at
Figure 1581: PRO37910
Figure 1582A-B: DNA226483, NP_000892.1, 205259_at
Figure 1583: PRO36946
Figure 1584A-B: DNA330079, 341358.1, 205263_at
Figure 1585: PRO1162
Figure 1586A-B: DNA188301, NP_002300.1, 205266-at
Figure 1587: PRO21834
Figure 1588: DNA227173, NP_001456.1, 205285_s_at
Figure 1589: PRO37636
Figure 1590A-B: DNA331483, CDC14A, 205288_at
Figure 1591: PRO86528
Figure 1592A-B: DNA331484, NP_000869.1, 205291_at
Figure 1593: PRO3276
Figure 1594: DNA88119, NP_000617.1, 205297_s_at
Figure 1595: PRO2663
Figure 1596A-B: DNA330081, NP_003026.1, 205339_at
Figure 1597: PRO85358
Figure 1598: DNA256854, NP_000456.1, 205345_at
Figure 1599: PRO51785
Figure 1600: DNA270415, NP_002059.1, 205349_at
Figure 1601: PRO58796
Figure 1602: DNA325568, NP_001265.1, 205393_s_at
Figure 1603: PRO12187
Figure 1604: DNA325568, CHEK1, 205394_at
Figure 1605: PRO12187
Figure 1606: DNA330082, NP_005582.1, 205395_s_at
Figure 1607: PRO60497
Figure 1608: DNA328561, NP_004624.1, 205403_at
Figure 1609: PRO2019
Figure 1610: DNA329010, NP_004942.1, 205419_at
Figure 1611: PRO23370
Figure 1612A-B: DNA210654, NP_055726.1, 205434_s_at
Figure 1613: PRO54603
Figure 1614: DNA287337, NP_002096.1, 205436_s_at
Figure 1615: PRO69600
Figure 1616: DNA330083, NP_003073.1, 205443_at
Figure 1617: PRO69499
Figure 1618: DNA272221, NP_037431.1, 205449_at
Figure 1619: PRO60483
Figure 1620: DNA88194, NP_000724.1, 205456-at

Figure 1621: PRO2220
Figure 1622: DNA188355, NP_004582.1, 205476_at
Figure 1623: PRO21885
Figure 1624: DNA287224, NP_005092.1, 205483_s_at
Figure 1625: PRO69503
Figure 1626: DNA330084, NP_055265.1, 205484_at
Figure 1627: PRO9895
Figure 1628: DNA225959, NP_006135.1, 205488_at
Figure 1629: PRO36422
Figure 1630: DNA331485, GNLY, 205495_s_at
Figure 1631: PRO86529
Figure 1632: DNA328566, NP_060446.1, 205510_s_at
Figure 1633: PRO84363
Figure 1634: DNA327639, NP_001053.2, 205513_at
Figure 1635: PRO83640
Figure 1636: DNA330085, D86324, 205518_s_at
Figure 1637: PRO85359
Figure 1638: DNA330086, NP_079184.1, 205519_at
Figure 1639: PRO85360
Figure 1640: DNA254810, NP_056536.1, 205527_s_at
Figure 1641: PRO49906
Figure 1642: DNA331486, OAS1, 205552_s_at
Figure 1643: PRO69559
Figure 1644: DNA330087, PCSK5, 205559_s_at
Figure 1645: PRO85361
Figure 1646: DNA256257, NP_055213.1, 205569_at
Figure 1647: PRO51301
Figure 1648A-B: DNA327643, NP_055712.1, 205594_at
Figure 1649: PRO83644
Figure 1650: DNA329013, NP_005649.1, 205599_at
Figure 1651: PRO20128
Figure 1652: DNA324324, NP_000679.1, 205633_s_at
Figure 1653: PRO8100
Figure 1654: DNA330088, NP_003087.1, 205644_s_at
Figure 1655: PRO61962
Figure 1656: DNA287317, NP_003724.1, 205660_at
Figure 1657: PRO69582
Figure 1658: DNA328570, NP_004040.1, 205681_at
Figure 1659: PRO37843
Figure 1660: DNA330089, NP-004200.2, 205691_at
Figure 1661: PRO12507
Figure 1662: DNA226234, NP_001766.1, 205692_s_at
Figure 1663: PRO36697
Figure 1664: DNA330090, NP_002749.2, 205698_s_at
Figure 1665: PRO62976
Figure 1666: DNA220761, NP_000880.1, 205718_at
Figure 1667: PRO34739
Figure 1668A-B: DNA271762, NP_000048.1, 205733_at
Figure 1669: PRO60046
Figure 1670: DNA331318, NP_003636.1, 205768_s_at
Figure 1671: PRO51139
Figure 1672: DNA331318, SLC27A2, 205769_at
Figure 1673: PRO51139
Figure 1674: DNA330091, NP_057461.1, 205771_s_at
Figure 1675: PRO85362
Figure 1676: DNA330092, NP_004904.1, 205781_at
Figure 1677: PRO85363
Figure 1678A-B: DNA220752, NP_000623.1, 205786_s_at

Figure 1679: PRO34730
Figure 1680: DNA330093, NP_003717.2, 205790_at
Figure 1681: PRO85364
Figure 1682: DNA76517, NP_002176.1, 205798_at
Figure 1683: PRO2541
Figure 1684A-B: DNA271915, NP_056191.1, 205801_s_at
Figure 1685: PRO60192
Figure 1686: DNA194766, NP_079504.1, 205804_s_at
Figure 1687: PRO24046
Figure 1688A-B: DNA328574, NP_004963.1, 205841_at
Figure 1689: PRO8436
Figure 1690A-B: DNA328574, JAK2, 205842_s_at
Figure 1691: PRO84368
Figure 1692: DNA330094, TREX1, 205875_s_at
Figure 1693: PRO85365
Figure 1694: DNA331320, HSU37122, 205882_x_at
Figure 1695: PRO86409
Figure 1696A-B: DNA220746, NP_000876.1, 205884_at
Figure 1697: PRO34724
Figure 1698A-B: DNA220746, ITGA4, 205885_s_at
Figure 1699: PRO34724
Figure 1700: DNA329540, NP_006389.1, 205890_s_at
Figure 1701: PRO85090
Figure 1702: DNA330095, NP_004732.1, 205895_s_at
Figure 1703: PRO85366
Figure 1704: DNA328576, HSU20350, 205898_at
Figure 1705: PRO4940
Figure 1706: DNA287318, NP_002683.1, 205909-at
Figure 1707: PRO69583
Figure 1708: DNA75525, NP-005805.1, 205929_at
Figure 1709: PRO2524
Figure 1710: DNA76516, NP_000556.1, 205945_at
Figure 1711: PRO2022
Figure 1712: DNA329047, NP_006390.1, 205965_at
Figure 1713: PRO58425
Figure 1714: DNA273487, NP_004785.1, 206039_at
Figure 1715: PRO61470
Figure 1716A-B: DNA290265, NP_003421.1, 206059-at
Figure 1717: PRO70395
Figure 1718: DNA330096, NP_057051.1, 206060_s_at
Figure 1719: PRO37163
Figure 1720: DNA271992, NP_006665.1, 206082_at
Figure 1721: PRO60267
Figure 1722: DNA270851, NP_006617.1, 206098_at
Figure 1723: PRO59189
Figure 1724: DNA226105, NP_002925.1, 206111_at
Figure 1725: PRO36568
Figure 1726: DNA83063, NP_004429.1, 206114_at
Figure 1727: PRO2068
Figure 1728A-B: DNA151420, NP_004421.1, 206115_at
Figure 1729: PRO12876
Figure 1730: DNA287306, NP_059993.1, 206133_at
Figure 1731: PRO69572
Figure 1732: DNA330097, NP_001233.1, 206150_at
Figure 1733: PRO2024
Figure 1734: DNA331487, GABPB2, 206173_s_at
Figure 1735: PRO86530
Figure 1736: DNA329005, NP_003028.1, 206181_at

Figure 1737: PRO12612
Figure 1738: DNA330098, NP_073619.1, 206205_at
Figure 1739: PRO85367
Figure 1740: DNA329168, CLC, 206207_at
Figure 1741: PRO84794
Figure 1742: DNA281446, NP_031394.1, 206220_s_at
Figure 1743: PRO66285
Figure 1744: DNA281446, GAP1IP4BP, 206221_at
Figure 1745: PRO66285
Figure 1746A-B: DNA331488, NP_055523.1, 206316_s_at
Figure 1747: PRO86531
Figure 1748: DNA327661, NP_005522.1, 206332_s_at
Figure 1749: PRO83652
Figure 1750: DNA218278, NP_000408.1, 206341_at
Figure 1751: PRO34330
Figure 1752: DNA269870, NP_005382.1, 206348_s_at
Figure 1753: PRO58270
Figure 1754A-B: DNA330100, NP_055690.1, 206364_at
Figure 1755: PRO85369
Figure 1756: DNA329169, NP_002986.1, 206366_x_at
Figure 1757: PRO1610
Figure 1758: DNA271310, NP_004411.1, 206374_at
Figure 1759: PRO59617
Figure 1760A-E: DNA331489, NP_066267.1, 206385_s_at
Figure 1761: PRO86532
Figure 1762: DNA326727, NP_001527.1, 206445_s_at
Figure 1763: PRO83069
Figure 1764A-B: DNA271891, NP_055766.1, 206448_at
Figure 1765: PRO60170
Figure 1766: DNA153751, NP_005942.1, 206461_x_at
Figure 1767: PRO12925
Figure 1768: DNA88203, NP_055022.1, 206485_at
Figure 1769: PRO2698
Figure 1770: DNA288243, NP_002277.3, 206486-at
Figure 1771: PRO36451
Figure 1772: DNA269850, NP_002003.1, 206492_at
Figure 1773: PRO58251
Figure 1774: DNA270444, NP_004824.1, 206513_at
Figure 1775: PRO58823
Figure 1776A-B: DNA188192, NP_006130.1, 206545_at
Figure 1777: PRO21704
Figure 1778A-B: DNA330102, NP_004289.1, 206550_s_at
Figure 1779: PRO85371
Figure 1780: DNA331490, OAS2, 206553_at
Figure 1781: PRO69656
Figure 1782: DNA227540, NP_003036.1, 206566_at
Figure 1783: PRO38003
Figure 1784: DNA330103, NP_056179.1, 206584_at
Figure 1785: PRO19671
Figure 1786: DNA329172, NP_005254.1, 206589_at
Figure 1787: PRO84796
Figure 1788: DNA103451, NP_003846.1, 206618_at
Figure 1789: PRO4778
Figure 1790: DNA227709, NP_000947.1, 206631 at
Figure 1791: PRO38172
Figure 1792: DNA331491, NP_004891.2, 206632_s_at
Figure 1793: PRO62308
Figure 1794: DNA331492, BCL2L1, 206665_s_at

Figure 1795: PRO83141
Figure 1796: DNA88374, NP_002095.1, 206666_at
Figure 1797: PRO2768
Figure 1798: DNA330105, HUMNCAX, 206676_at
Figure 1799: PRO85372
Figure 1800: DNA328590, C6orf32, 206707_x_at
Figure 1801: PRO84375
Figure 1802: DNA330106, NP_003646.1, 206724_at
Figure 1803: PRO85373
Figure 1804A-B: DNA88191, NP_001234.1, 206729_at
Figure 1805: PRO2691
Figure 1806A-B: DNA88650, NP_005807.1, 206761_at
Figure 1807: PRO2460
Figure 1808: DNA226427, NP_002251.1, 206785_s_at
Figure 1809: PRO36890
Figure 1810: DNA88195, NP_000064.1, 206804_at
Figure 1811: PRO2693
Figure 1812: DNA256561, NP_062550.1, 206914_at
Figure 1813: PRO51592
Figure 1814: DNA93439, NP_006555.1, 206974_at
Figure 1815: PRO4515
Figure 1816: DNA35629, NP-000586.2, 206975-at
Figure 1817: PRO7
Figure 1818: DNA328591, NP_006635.1, 206976_s_at
Figure 1819: PRO8437
Figure 1820: DNA331493, CCR2, 206978_at
Figure 1821: PRO84690
Figure 1822: DNA188346, NP_001450.1, 206980_s_at
Figure 1823: PRO21766
Figure 1824A-B: DNA227659, NP_000570.1, 206991_s_at
Figure 1825: PRO38122
Figure 1826A-B: DNA227750, NP_001550.1, 206999_at
Figure 1827: PRO38213
Figure 1828: DNA329903, PPP3CC, 207000_s_at
Figure 1829: PRO85220
Figure 1830: DNA330108, NP_004080.1, 207001_x_at
Figure 1831: PRO85374
Figure 1832: DNA331494, PLAGL1, 207002_s_at
Figure 1833: PRO62736
Figure 1834: DNA331495, HUMBCL2B, 207005_s_at
Figure 1835: PRO86533
Figure 1836: DNA330110, HUMK10A, 207023_x_at
Figure 1837: PRO85375
Figure 1838: DNA225550, NP_003844.1, 207072_at
Figure 1839: PRO36013
Figure 1840: DNA273159, NP_005457.1, 207078_at
Figure 1841: PRO61201
Figure 1842: DNA227481, VAMP1, 207100_s_at
Figure 1843: PRO37944
Figure 1844: DNA218655, NP_000585.1, 207113_s_at
Figure 1845: PRO34451
Figure 1846: DNA330111, NP-002615.2, 207132_x_at
Figure 1847: PRO85376
Figure 1848: DNA330112, NP_444504.1, 207153_s_at
Figure 1849: PRO61610
Figure 1850: DNA103418, NP_036616.1, 207165_at
Figure 1851: PRO4746
Figure 1852: DNA330113, NP_203124.1, 207181_s_at

Figure 1853: PRO85377
Figure 1854: DNA330114, NP_006134.1, 207183_at
Figure 1855: PRO4946
Figure 1856: DNA331496, RBMS1, 207266_x_at
Figure 1857: PRO86534
Figure 1858: DNA83048, NP_001916.1, 207269_at
Figure 1859: PRO2057
Figure 1860A-B: DNA330115, NP_077739.1, 207324_s_at
Figure 1861: PRO85378
Figure 1862A-B: DNA226536, NP_003225.1, 207332_s_at
Figure 1863: PRO36999
Figure 1864: DNA331497, LTB, 207339_s_at
Figure 1865: PRO11604
Figure 1866: DNA330117, NP_003966.1, 207351_s_at
Figure 1867: PRO85379
Figure 1868: DNA330118, NP_036389.2, 207361_at
Figure 1869: PRO85380
Figure 1870: DNA226396, NP_002180.1, 207375_s_at
Figure 1871: PRO36859
Figure 1872: DNA227668, NP_000158.1, 207387_s_at
Figure 1873: PRO38131
Figure 1874A: DNA329093, MSF, 207425_s_at
Figure 1875A-B: PRO84745
Figure 1876: DNA36718, NP_000563.1, 207433_at
Figure 1877: PRO73
Figure 1878A-B: DNA330119, NP_060189.2, 207474_at
Figure 1879: PRO85381
Figure 1880: DNA328597, NP_001680.1, 207507_s_at
Figure 1881: PRO84381
Figure 1882: DNA328597, ATP5G3, 207508_at
Figure 1883: PRO84381
Figure 1884A-B: DNA256059, NP_005164.1, 207521_s_at
Figure 1885: PRO51107
Figure 1886A-B: DNA256059, ATP2A3, 207522_s_at
Figure 1887: PRO51107
Figure 1888: DNA304473, NP_001552.2, 207536_s_at
Figure 1889: PRO2023
Figure 1890: DNA325454, NP_003637.1, 207556_s_at
Figure 1891: PRO81977
Figure 1892: DNA328601, NP_056490.1, 207574_s_at
Figure 1893: PRO84384
Figure 1894A-B: DNA330120, FLJ10971, 207606_s_at
Figure 1895: PRO85382
Figure 1896: DNA255271, NP_038475.1, 207610_s_at
Figure 1897: PRO50348
Figure 1898: DNA331498, TANK, 207616_s_at
Figure 1899: PRO86535
Figure 1900: DNA226337, NP_005683.2, 207622_s_at
Figure 1901: PRO36800
Figure 1902: DNA227606, NP_001872.2, 207630_s_at
Figure 1903: PRO38069
Figure 1904: DNA196426, NP_037440.1, 207651_at
Figure 1905: PRO24924
Figure 1906: DNA328554, NP_038202.1, 207677_s_at
Figure 1907: PRO84354
Figure 1908A-B: DNA226405, NP_006525.1, 207700_s_at
Figure 1909: PRO36868
Figure 1910: DNA329064, NP_060301.1, 207735_at

Figure 1911: PRO84724
Figure 1912: DNA329020, NUP62, 207740_s_at
Figure 1913: PRO84695
Figure 1914: DNA325654, NP_054752.1, 207761_s_at
Figure 1915: PRO4348
Figure 1916A-B: DNA329179, NP_056958.1, 207785_s_at
Figure 1917: PRO84802
Figure 1918: DNA227494, NP_002158.1, 207826_s_at
Figure 1919: PRO37957
Figure 1920A-C: DNA331499, NP-057427.2, 207828_s_at
Figure 1921.: PRO86536
Figure 1922: DNA329182, HPIP, 207838_x_at
Figure 1923: PRO84805
Figure 1924: DNA330123, NP_008984.1, 207840_at
Figures 1925: PRO35080
Figure 1926: DNA227175, NP_006857.1, 207857 at
Figure 1927: PRO37638
Figure 1928: DNA330124, NP_002981.2, 207861_at
Figure 1929: PRO34107
Figure 1930: DNA217245, NP_000579.1, 207906_at
Figure 1931: PRO34287
Figure 1932: DNA218651, NP_003798.1, 207907_at
Figure 1933: PRO34447
Figure 1934: DNA330125, NP_002729.2, 207957_s_at
Figure 1935: PRO85385
Figure 1936A-B: DNA226290, NP_036333.1, 207966_s_at
Figure 1937: PRO36753
Figure 1938: DNA329183, NP_055962.1, 207971_s_at
Figure 1939: PRO84806
Figure 1940A-B: DNA330126, NP_008912.1, 207978_s_at
Figure 1941: PRO85386
Figure 1942: DNA329184, CITED2, 207980_s_at
Figure 1943: PRO84807
Figure 1944A-C: DNA254145, NP_004329.1, 207996_s_at
Figure 1945: PRO49260
Figure 1946: DNA275286, NP_009205.1, 208002_s_at
Figure 1947: PRO62967
Figure 1948: DNA288217, NP_002101.1, 208018_s_at
Figure 1949: PRO69990
Figure 1950: DNA227224, NP_060877.1, 208029_s_at
Figure 1951: PRO37687
Figure 1952A-B: DNA188492, NAB1, 208047_s_at
Figure 1953: PRO22070
Figure 1954: DNA330127, NP_006442.2, 208051_s_at
Figure 1955: PRO85387
Figure 1956A-B: DNA328607, NP_003639.1, 208072_s_at
Figure 1957: PRO84390
Figure 1958A-C: DNA331500, NP_003307.2, 208073_x_at
Figure 1959: PRO86537
Figure 1960A-B: DNA328312, NP_110378.1, 208078_s_at
Figures 1961: PRO84180
Figure 1962: DNA331501, STK6, 208079_s_at
Figure 1963: PRO58855
Figure 1964: DNA323896, NP_112182.1, 208103_s_at
Figure 1965: PRO8063
Figure 1966: DNA330129, NP_112495.1, 208119_s_at
Figure 1967: PRO85389
Figure 1968: DNA325329, NP_004719.1, 208152_s_at

Figure 1969: PRO81872
Figure 1970: DNA36717, NP_000581.1, 208193_at
Figure 1971: PRO72
Figure 1972A-E : DNA330130, HSTITIN, 208195_at
Figure 1973: DNA328611, RASGRP2, 208206_s_at
Figure 1974: PRO84393
Figure 1975: DNA328612, NP_000166.2, 208308_s_at
Figure 1976: PRO84394
Figure 1977A-D: DNA331502, NP_000050.1, 208368_s_at
Figure 1978: PRO86538
Figure 1979: DNA324250, NP-536349.1, 208392_x_-at
Figure 1980: PRO80934
Figure 1981A-B: DNA331503, RAD50, 208393_s_at
Figure 1982: PRO86539
Figure 1983: DNA327690, NP_004022.1, 208436_s_.Jlt
Figure 1984: PRO83673
Figure 1985: DNA103427, NP_005239.1, 208438_s_at
Figure 1986: PRO4755
Figure 1987A-C: DNA331504, ATM, 208442_s_at
Figure 1988: PRO86540
Figure 1989A-B: DNA330134, BAZ1B, 208445_s_at
Figure 1990: PRO85394
Figure 1991A-C: DNA331505, NP_000642.2, 208488_s_at
Figure 1992: PRO86541
Figure 1993: DNA330136, NP_002441.1, 208581_x_at
Figure 1994: PRO82583
Figure 1995A-C: DNA331506, NP_001448.1, 208614_s_at
Figure 1996: PRO86542
Figure 1997A-B: DNA330138, PTP4A2, 2086 17_s_at
Figure 1998: PRO85397
Figure 1999A-B: DNA273567, NP_004944.1, 208624_s_at
Figure 2000: PRO61545
Figure 2001A-B: DNA273567, EIF4G1, 208625_s_at
Figure 2002: PRO61545
Figure 2003: DNA325912, NP_001093.1, 208636_at
Figure 2004: PRO82367
Figure 2005: DNA325912, ACTN1, 208637_x_at
Figure 2006: PRO82367
Figure 2007: DNA329188, BC012142, 208638_at
Figure 2008: PRO84810
Figure 2009: DNA324641, NP_005608.1, 208646_at
Figure 2010: PRO10849
Figure 2011: DNA271268, NP_009057.1, 208649_s_at
Figure 2012: PRO59579
Figure 2013: DNA328617, AF299343, 208653_s_at
Figure 2014: PRO84399
Figure 2015: DNA330139, AK022493, 208657_s_at
Figure 2016: PRO85398
Figure 2017A-C: DNA151898, TTC3, 208661_s_at
Figure 2018: PRO12135
Figure 2019A-C: DNA151898, D84294, 208662_s_at
Figure 2020: PRO12135
Figure 2021A-C: DNA331507, D83327, 208663_s_at
Figure 2022: DNA304686, NP_002565.1, 208680_at
Figure 2023: PRO71112
Figure 2024A-B: DNA328619, BC001188, 208691_at
Figure 2025: PRO84401
Figure 2026: DNA287189, NP_002038.1, 208693_s_at

EP 2 179 742 A1

Figure 2027: PRO69475
Figure 2028: DNA330140, AF275798, 208696_at
Figure 2029: PRO85399
Figure 2030A-C: DNA331508, 198777.9, 208707_at
Figure 2031: PRO8654
Figure 2032: DNA97298, NP_003899.1, 208726_s_at
Figure 2033: PRO3645
Figure 2034: DNA330142, BC003564, 208737at
Figure 2035: PRO85401
Figure 2036: DNA331509, 1138554.23, 208740_at
Figure 2037: PRO86544
Figure 2038: DNA328591, HSP105B, 208744_x_at
Figure 2039: PRO84376
Figure 2040: DNA287285, NP_005794.1, 208748_s_at
Figure 2041: PRO69556
Figure 2042: DNA324217, ATIC, 208758_at
Figure 2043: PRO80908
Figure 2044: DNA327696, AF228339, 208763_s_at
Figure 2045: PRO83679
Figure 2046A-B: DNA331510, 1298307.1, 208776_at
Figure 2047: PRO86545
Figure 2048: DNA287427, NP_002806.1, 208777_s_at
Figure 2049: PRO69684
Figure 2050: DNA287219, NP_110379.1, 208778_s_at
Figure 2051: PRO69498
Figure 2052: DNA329189, NP-009139.1, 208787_at
Figure 2053: PRO4911
Figure 2054: DNA238565, NP_005907.2, 208795_s_at
Figure 2055: PRO39210
Figure 2056: DNA330145, NP_002788.1, 208799_at
Figure 2057: PRO84403
Figure 2058: DNA331511, HSMPIO, 208805_at
Figure 2059A-C: DNA331512, 1397486.26, 208806_at
Figure 2060: PRO86547
Figure 2061A-B: DNA330147, HSU91543, 208807_s_at
Figure 2062: PRO85405
Figure 2063: DNA324531, NP_002120.1, 208808_s_at
Figure 2064: PRO81185
Figure 2065: DNA273521, NP_002070.1, 208813_at
Figure 2066: PRO61502
Figure 2067A-B: DNA330148, AB020636, 208838_at
Figure 2068A-B: DNA330149, HSM801778, 208839_s_at
Figure 2069: PRO82209
Figure 2070: DNA227874, NP_003320.1, 208864_s_at
Figure 2071: PRO38337
Figure 2072: DNA328624, BC003562, 208891_at
Figure 2073: PRO59076
Figure 2074: DNA331513, DUSP6, 208892_s_at
Figure 2075: PRO84404
Figure 2076: DNA331330, BC005047, 208893_s_at
Figure 2077: PRO82215
Figure 2078: DNA329221, NP_061984.1, 208894_at
Figure 2079: PRO4555
Figure 2080A-B: DNA329007, NP_003277.1, 208900_s_at
Figure 2081: PRO37029
Figure 2082A-B: DNA329007, TOP1, 208901_s_at
Figure 2083: PRO37029
Figure 2084: DNA327700, BC015130, 208905_at

43

Figure 2085: PRO83683
Figure 2086: DNA327701, NP_001203.1, 208910_s_at
Figure 2087: PRO82667
Figure 2088: DNA281442, NP_149124.1, 208912_s_art
Figure 2089: PRO66281
Figure 2090A-B: DNA330151, AB029003, 208914_at
Figure 2091: DNA325473, NP_006353.2, 208922_s_at
Figure 2092: PRO81996
Figure 2093: DNA329552, NP_063948.1, 208925_at
Figure 2094: PRO85097
Figure 2095: DNA326233, NP_000968.2, 208929_x_at
Figure 2096: PRO82645
Figure 2097: DNA327702, NP_006490.2, 208934_s_at
Figure 2098: PRO83684
Figure 2099: DNA330152, NP_001939.1, 208956_x_at
Figure 2100: PRO85406
Figure 2101: DNA290261, NP_001291.2, 208960_s_at
Figure 2102: PRO70387
Figure 2103A-B: DNA325478, NP_037534.2, 208962_s_at
Figure 2104: PRO81999
Figure 2105: DNA327661, IFI16, 208965_s_at
Figure 2106: PRO83652
Figure 2107A-B: DNA270277, AF208043, 208966_x_at
Figure 2108: PRO58665
Figure 2109: DNA326343, KPNB1, 208974_x_at
Figure 2110: PRO82739
Figure 2111A-B: DNA330153, HUMIMP90A, 208975_s_at
Figure 2112: PRO82739
Figure 2113: DNA328629, NP-006079.1, 208977_x_at
Figure 2114: PRO84407
Figure 2115: DNA330154, HUMPECAM27, 208981_at
Figure 2116: DNA330155, 7692317.2, 208982_at
Figure 2117: PRO85407
Figure 2118: DNA330156, NP_003749.1, 208985_s_at
Figure 2119: PRO85408
Figure 2120: DNA331514, STAT3, 208992_s_at
Figure 2121: PRO86548
Figure 2122: DNA227552, NP_003346.2, 208997_s_at
Figure 2123: PRO38015
Figure 2124: DNA227552, UCP2, 208998_at
Figure 2125: PRO38015
Figure 2126: DNA328630, NP_036293.1, 209004_s_at
Figure 2127: PRO84408
Figure 2128: DNA331515, FBXL5, 209005 at
Figure 2129: PRO86549
Figure 2130: DNA328631, AK027318, 209006_s_at
Figure 2131: PRO84409
Figure 2132: DNA331516, DNAJB6, 209015_s_at
Figure 2133: PRO83680
Figure 2134: DNA328633, NP_004784.2, 209017_s_at
Figure 2135: PRO84411
Figure 2136: DNA330158, NP_057554.4, 209020_at
Figure 2137: PRO85410
Figure 2138: DNA327851, NP_006363.2, 209024_s_at
Figure 2139: PRO83795
Figure 2140: DNA328635, BC020946, 209026_x_at
Figure 2141: PRO84413
Figure 2142: DNA331517, NP_004150.1, 209040_s_at

Figure 2143: PRO69506
Figure 2144A-C: DNA328637, HSA7042, 209052_s_at
Figure 2145: PRO81109
Figure 2146A-B: DNA331518, AF330040, 209053_s_at
Figure 2147: PRO86550
Figure 2148A-B: DNA226405, NCOA3, 209060_x_at
Figure 2149: PRO36868
Figure 2150: DNA330159, HSM801885, 209064_x_at
Figure 2151: PRO85411
Figure 2152: DNA330160, NP_006285.1, 209066_x_at
Figure 2153: PRO85412
Figure 2154: DNA329194, NP_112740.1, 209068_at
Figure 2155: PRO84814
Figure 2156A-B: DNA330161, NP_085059.1, 209081_s_at
Figure 2157: PRO85413
Figure 2158: DNA330162, NP_057093.1, 209091_s_at
Figure 2159: PRO85414
Figure 2160: DNA330163, NP_060308.1, 209104_s_at
Figure 2161: PRO85415
Figure 2162: DNA330164, NP_004752.1, 209110_s_at
Figure 2163: PRO85416
Figure 2164: DNA327709, NP_000509.1, 209116_x_at
Figure 2165: PRO83690
Figure 2166: DNA288254, NP_006000.2, 209118-s-at
Figure 2167: PRO69536
Figure 2168: DNA325163, NP_001113.1, 209122_at
Figure 2169: PRO81730
Figure 2170: DNA330165, BC015833, 209138_x_at
Figure 2171: PRO85417
Figure 2172: DNA327713, BC010653, 209146_at
Figure 2173: PRO37975
Figure 2174: DNA325285, AKR1C3, 209160_at
Figure 2175: PRO81832
Figure 2176: DNA330166, BC001588, 209161_at
Figure 2177: PRO85418
Figure 2178: DNA271722, NP_004688.1, 209162_s_at
Figure 2179: PRO60006
Figure 2180: DNA330167, CAB43224.1, 209177_at
Figure 2181: PRO85419
Figure 2182A-B: DNA328642, AF073310, 209184_s_at
Figure 2183: PRO84418
Figure 2184: DNA331331, AF161416, 209185_s_at
Figure 2185A-B: DNA328643, HUMHK1A, 209186-at
Figure 2186: PRO84419
Figure 2187: DNA189700, NP_005243.1, 209189_at
Figure 2188: PRO25619
Figure 2189: DNA324766, NP_005443.2, 209196_at
Figure 2190: PRO81387
Figure 2191: DNA226176, NP_003458.1, 209201_x_at
Figure 2192: PRO36639
Figure 2193: DNA326267, NP_004861.1, 209208_at
Figure 2194: PRO82674
Figure 2195: DNA326891, NP_001748.1, 209213_at
Figure 2196: PRO83212
Figure 2197: DNA227483, NP_003120.1, 209218_at
Figure 2198: PRO37946
Figure 2199: DNA330168, NP_006322.1, 209233_at
Figure 2200: PRO85420

Figure 2201: DNA328649, NP_116093.1, 209251 -x-at
Figure 2202: PRO84424
Figure 2203: DNA255255, NP_071437.1, 209267_s_at
Figure 2204: PRO50332
Figure 2205A-B: DNA188492, AF045451, 209272_at
Figure 2206: PRO22070
Figure 2207A-B: DNA226827, NP_001673.1, 209281_s_at
Figure 2208: PRO37290
Figure 2209: DNA328601, GADD45B, 209304_x_at
Figure 2210: PRO84384
Figure 2211: DNA328651, AF087853, 209305_s_at
Figure 2212: PRO82889
Figure 2213: DNA151780, NP_006611.1, 209314_s_at
Figure 2214: PRO12057
Figure 2215: DNA330169, NP_006709.1, 209318_x_at
Figure 2216: PRO62736
Figure 2217: DNA275106, HSU76248, 209339_at
Figure 2218: PRO62821
Figure 2219: DNA269630, NP_003281.1, 209344_at
Figure 2220: PRO58042
Figure 2221A-B: DNA328658, AF055376, 209348_s_at
Figure 2222: PRO84432
Figure 2223: DNA330170, AF109161, 209357_at
Figure 2224: PRO84807
Figure 2225: DNA327720, NP_001970.1, 209368_at
Figure 2226: PRO83699
Figure 2227: DNA330171, CAA34971.1, 209374_s_at
Figure 2228: PRO85421
Figure 2229: DNA330172, BC009529, 209377_s_at
Figure 2230: PRO85422
Figure 2231: DNA330173, HUMAUTOTAX, 209392_at
Figure 2232: PRO85423
Figure 2233: DNA330174, AK027512, 209404_s_at
Figure 2234: PRO85424
Figure 2235: DNA330175, NP_006836.1, 209408_at
Figure 2236: PRO59681
Figure 2237A-B: DNA271241, HSU61500, 209412_at
Figure 2238: PRO59556
Figure 2239: DNA330176, AAB61703.1, 209417_s_at
Figure 2240: PRO85425
Figure 2241: DNA225767, NP_000534.1, 209420_s_at
Figure 2242: PRO36230
Figure 2243: DNA330177, BC001743, 209446_s_at
Figure 2244: PRO10283
Figure 2245: DNA273076, HSU59863, 209451_at
Figure 2246: PRO61137
Figure 2247: DNA326089, HBA2, 209458_x_at
Figure 2248: PRO3629
Figure 2249: DNA287304, AAH00040.1, 209461_x_at
Figure 2250: PRO69571
Figure 2251: DNA297388, NP_004208.1, 209464_at
Figure 2252: PRO70812
Figure 2253: DNA330178, HSTM2CEA, 209498-at
Figure 2254: PRO85426
Figure 2255: DNA330179, NP_067023.1, 209504_s_at
Figure 2256: PRO85427
Figure 2257: DNA324899, NP_002938.1, 209507_at
Figure 2258: PRO81503

Figure 2259: DNA330180, NP_009149.2, 209510_at
Figure 2260: PRO85428
Figure 2261: DNA274027, RAB27A, 209514_s_at
Figure 2262: PRO61971
Figure 2263: DNA274027, HSU38654, 209515_s_at
Figure 2264: PRO61971
Figure 2265: DNA272213, NP_002477.1, 209520_s_at
Figure 2266: PRO60475
Figure 2267: DNA330181, HSM802358, 209523_at
Figure 2268: DNA328663, NP_057157.1, 209524_at
Figure 2269: PRO36183
Figure 2270: DNA330182, PLAA, 209533_s_at
Figure 2271: PRO85430
Figure 2272: DNA330183, AF181265, 209536_s_at
Figure 2273: DNA327724, AF32354257, 209546_s_at
Figure 2274: DNA257501, NP_115688.1, 209551_at
Figure 2275: PRO52073
Figure 2276: DNA330184, BC022475, 209566_at
Figure 2277: PRO85432
Figure 2278: DNA290251, NP_055207.1, 209569_x_at
Figure 2279: PRO70367
Figure 2280: DNA329202, BC001745, 209570_s_at
Figure 2281: PRO70367
Figure 2282: DNA329203, NP_003788.1, 209572..sat
Figure 2283: PRO84819
Figure 2284: DNA304797, NP-005935.3, 209583_s_at
Figure 2285: PRO71209
Figure 2286: DNA328666, AF084943, 209585_s_at
Figure 2287: PRO1917
Figure 2288: DNA270689, NP_002042.1, 209604_s_at
Figure 2289: PRO59053
Figure 2290: DNA271823, NP-004279.2, 209606_at
Figure 2291: PRO60104
Figure 2292A-B: DNA328670, BC001618, 209610_s_at
Figure 2293: PRO70011
Figure 2294: DNA330185, NP_071415.1, 209624_s_at
Figure 2295: PRO85433
Figure 2296: DNA328599, HSNFKBS, 209636_at
Figure 2297: PRO84382
Figure 2298: DNA330186, NP_004327.1, 209642_at
Figure 2299: PRO85434
Figure 2300A-B: DNA330187, HSM801454, 209649_at
Figure 2301: PRO85435
Figure 2302: DNA330188, NP_004356.1, 209662_at
Figure 2303: PRO85436
Figure 2304: DNA323856, PAI-RBP1, 209669_s_at
Figure 2305: PRO80599
Figure 2306: DNA330189, BC000712, 209680_s_at
Figure 2307: PRO85437
Figure 2308: DNA193881, AAF15129.1, 209681_at
Figure 2309: PRO23299
Figure 2310A-B: DNA272671, HSU26710, 209682_at
Figure 2311: PRO60796
Figure 2312: DNA330125, HUMPKB, 209685_s_at
Figure 2313: PRO85385
Figure 2314: DNA331519, HMMR, 209709_s_at
Figure 2315: PRO86551
Figure 2316: DNA328264, NP_005183.2, 209714_s_at

Figure 2317: PRO12087
Figure 2318: DNA330191, NP_036249.1, 209715_at
Figure 2319: PRO85439
Figure 2320A-C: DNA254412, AF008915, 209717_at
Figure 2321: PRO49522
Figure 2322A-B: DNA330192, 234780.1, 209733_at
Figure 2323: PRO85440
Figure 2324: DNA330193, BC015929, 209750-at
Figure 2325: DNA330194, HSU09087, 209754_s_at
Figure 2326: PRO85442
Figure 2327: DNA275195, NP_001025.1, 209773_s_at
Figure 2328: PRO62893
Figure 2329: DNA329205, NP_001343.1, 209782_s_at
Figure 2330: PRO84821
Figure 2331: DNA226436, NP_001772.1, 209795_at
Figure 2332: PRO36899
Figure 2333: DNA327731, NP_003302.1, 209803_s_at
Figure 2334: PRO83707
Figure 2335A-B: DNA271368, HUMKIAAI, 209804_at
Figure 2336: PRO59668
Figure 2337: DNA329206, AF151103, 209813_x_at
Figure 2338: PRO84822
Figure 2339A-C: DNA331520, 1096711.1, 209815_at
Figure 2340: PRO86552
Figure 2341: DNA327732, UMPK, 209825_s_at
Figure 2342: PRO61801
Figure 2343: DNA328676, IL16, 209827_s_at
Figure 2344: PRO84448
Figure 2345A-B: DNA196499, AB002384, 209829_at
Figure 2346: PRO24988
Figure 2347: DNA330197, NP_112190.1, 209832_s_at
Figure 2348: PRO85445
Figure 2349: DNA328677, AF060511, 209836_x_at
Figure 2350: PRO84449
Figure 2351: DNA270180, NP_478123.1, 209849_s_at
Figure 2352: PRO58569
Figure 2353: DNA331521, BC018951, 209868_s_at
Figure 2354: PRO58719
Figure 2355A-B: DNA329536, NP-005494.2, 209870_s_at
Figure 2356: PRO22775
Figure 2357: DNA330198, AB014719, 209871_s_at
Figure 2358: PRO85446
Figure 2359: DNA324184, NP_065726.1, 209891_at
Figure 2360: PRO80882
Figure 2361: DNA328258, HSM802616, 209900_s_at
Figure 2362: PRO84151
Figure 2363: DNA330152, DUT, 209932._s_at
Figure 2364: PRO85406
Figure 2365: DNA150133, AAD01646.1, 209933_s_at
Figure 2366: PRO12219
Figure 2367: DNA329208, CFLAR, 209939_x_at
Figure 2368: PRO84823
Figure 2369: DNA330199, BC004357, 209944_at
Figure 2370: PRO85447
Figure 2371A-B: DNA329065, HSU12767, 209959_at
Figure 2372: PRO84725
Figure 2373: DNA154921, DNA154921, 209967_s_at
Figure 2374: DNA327736, BC002704, 209969_s_at

Figure 2375: PRO83711
Figure 2376: DNA324895, JTV1, 209971_x_at
Figure 2377: PRO81501
Figure 2378: DNA226658, NP_003736.1, 209999_x_at
Figure 2379: PRO37121
Figure 2380: DNA226658, SSI-1, 210001_s_at
Figure 2381: PRO37121
Figure 2382: DNA330200, NP_056222.1, 210006_at
Figure 2383: PRO85448
Figure 2384: DNA269534, NP_002155.1, 210029_at
Figure 2385: PRO57950
Figure 2386: DNA326054, NP_002159.1, 210046_s_at
Figure 2387: PRO82489
Figure 2388: DNA326809, NP_036244.2, 210052_s_at
Figure 2389: PRO83142
Figure 2390: DNA150551, AAB97010.1, 210054_at
Figure 2391: PRO12778
Figure 2392: DNA274960, SFRS5, 210077_s_at
Figure 2393: PRO62694
Figure 2394: DNA324922, BC018962, 210095_s_at
Figure 2395: PRO119
Figure 2396A-B: DNA328685, NP_127497.1, 210113_s_at
Figure 2397: PRO34751
Figure 2398: DNA330201, NP_003774.1, 210121_at
Figure 2399: PRO50625
Figure 2400: DNA330202, NP_005400.1, 210163_at
Figure 2401: PRO19838
Figure 2402: DNA287620, NP_004122.1, 210164_at
Figure 2403: PRO2081
Figure 2404: DNA270196, HUMZFM1B, 210172_at
Figure 2405: PRO58584
Figure 2406: DNA330203, NP_003755.1, 210190_at
Figure 2407: PRO85449
Figure 2408: DNA331335, AF070576, 210201 _x_at
Figure 2409: DNA331522, AF068918, 210202_s_at
Figure 2410: PRO86553
Figure 2411: DNA331523, RAD1, 210216_x_at
Figure 2412: PRO61690
Figure 2413: DNA328467, AF056322, 210218_s_at
Figure 2414: PRO84293
Figure 2415: DNA217253, NP_000749.1, 210229_s_at
Figure 2416: PRO34295
Figure 2417: DNA331084, BC008487, 210254_at
Figure 2418: PRO81984
Figure 2419A-B: DNA270015, NP_003444.1, 2102811_s_at
Figure 2420: PRO58410
Figure 2421: DNA330206, NP_005801.2, 210288_at
Figure 2422: PRO85450
Figure 2423: DNA329945, SEC23B, 210293_s_at
Figure 2424: PRO85252
Figure 2425: DNA218653, NP_003799.1, 210314_x_at
Figure 2426: PRO34449
Figure 2427: DNA326239, NP_006752.1, 210317_s_at
Figure 2428: PRO39530
Figure 2429: DNA329213, NP219491.1, 210321_at
Figure 2430: PRO2313
Figure 2431A-B: DNA329214, NP_001087.1, 210337_s_at
Figure 2432: PRO84826

Figure 2433: DNA225528, NP_000610.1, 210354_at
Figure 2434: PRO35991
Figure 2435: DNA196621, HUMLY9, 210370_s_at
Figure 2436: DNA330207, BC001131, 210387_at
Figure 2437: PRO85451
Figure 2438: DNA226229, NP_002432.1, 210410_s_at
Figure 2439: PRO36692
Figure 2440A-B: DNA330208, AF164622, 210425_s_at
Figure 2441: PRO85452
Figure 2442: DNA329215, NP_036224.1, 210439_at
Figure 2443: PRO7424
Figure 2444: DNA226394, NP_002552.1, 210448_s_at
Figure 2445: PRO36857
Figure 2446: DNA331524, BC003388, 210458_s_at
Figure 2447: PRO86554
Figure 2448: DNA331525, BC002448, 210461_s_at
Figure 2449: PRO86555
Figure 2450: DNA329216, AF022375, 210512_s_at
Figure 2451: PRO84827
Figure 2452: DNA227633, NP_001156.1, 210538_s_at
Figure 2453: PRO38096
Figure 2454: DNA330209, BC000585, 210542_s_at
Figure 2455: PRO85453
Figure 2456: DNA331526, BC014563, 210559_s_art
Figure 2457: PRO58324
Figure 2458: DNA331527, BC001602, 210563_x_at
Figure 2459: PRO86556
Figure 2460: DNA331528, AF00619, 210564_x_at
Figure 2461: PRO86557
Figure 2462: DNA329217, BC003406, 210571_s_at
Figure 2463: PRO84828
Figure 2464: DNA330210, HSU03858, 210607_at
Figure 2465: PRO126
Figure 2466: DNA330211, NP_009092.1, 210629_x_at
Figure 2467: PRO85454
Figure 2468: DNA330212, HUMKRT10A, 210633_x_at
Figure 2469: PRO85455
Figure 2470: DNA331529, LAIR1, 210644_s_at
Figure 2471: PRO86558
Figure 2472A-C: DNA330214, HUMTPRD, 210645_s_at
Figure 2473: PRO12135
Figure 2474: DNA329218, NP_055227.1, 210691_s_at
Figure 2475: PRO84829
Figure 2476: DNA330215, DKFZp762A227Homo, 210692_s_at
Figure 2477: DNA331530, AF064103, 210742_at
Figure 2478: PRO86559
Figure 2479: DNA237817, NP_001307.1, 210766_s_at
Figure 2480: PRO38923
Figure 2481A-B: DNA330216, NP_006445.1, 210778_s_at
Figure 2482: PRO85457
Figure 2483: DNA226881, FLI1, 210786_s_at
Figure 2484: PRO37344
Figure 2485: DNA255402, NP_055288.1, 210802_s_at
Figure 2486: PRO50469
Figure 2487: DNA330027, SSBP2, 210829_s_at
Figure 2488: PRO8531
Figure 2489: DNA329219, BC000385, 210844_x_at
Figure 2490: PRO81278

Figure 2491A-B: DNA331107, HSU26455, 210858_x_at
Figure 2492: PRO86255
Figure 2493: DNA188234, NP_000630.1, 210865_at
Figure 2494: PRO21942
Figure 2495: DNA331531, PFDN5, 210908_s_at
Figure 2496: PRO86560
Figure 2497: DNA330217, AF043183, 210915_x_at
Figure 2498: PRO85458
Figure 2499: DNA274326, NP_003079.1, 210933_s_at
Figure 2500: PRO62244
Figure 2501: DNA329317, NP_057353.1, 210948_s_at
Figure 2502: PRO81157
Figure 2503: DNA331532, AF125393, 210951_x_at
Figure 2504: PRO86561
Figure 2505: DNA330218, HUMTCAXA, 210972_x_at
Figure 2506: DNA273236, NP_004306.1, 210980_s_at
Figure 2507: PRO61263
Figure 2508: DNA269888, NP_002073.1, 210981_s_at
Figure 2509: PRO58286
Figure 2510: DNA329221, HLA-DRA, 210982_s_at
Figure 2511: PRO4555
Figure 2512: DNA238565, MCM7, 210983_s_at
Figure 2513: PRO39210
Figure 2514: DNA326239, YWHAE, 210996_s_at
Figure 2515: PRO39530
Figure 2516A-B: DNA330219, NP_150241.1, 211013_x_at
Figure 2517: PRO85459
Figure 2518: DNA327699, AB023420, 211015_s_at
Figure 2519: PRO83682
Figure 2520: DNA288254, TUBA3, 211058_s_at
Figure 2521: PRO69536
Figure 2522: DNA329992, MGAT2, 211061_s_at
Figure 2523: PRO59267
Figure 2524: DNA324171, NP_065438.1, 211070_s_at
Figure 2525: PRO60753
Figure 2526: DNA330220, NP_006809.1, 211071_s_at
Figure 2527: PRO60769
Figure 2528: DNA287198, K-ALPHA-1, 211072_x_at
Figure 2529: PRO69484
Figure 2530: DNA254470, NEK2, 211080_s_at
Figure 2531: PRO49578
Figure 2532: DNA196432, AF064804,211106_at
Figure 2533: PRO24928
Figure 2534: DNA330202, CCL11, 211122_s_at
Figure 2535: PRO19838
Figure 2536: DNA304765, HUMTCRGAD, 211144_x_at
Figure 2537: PRO71178
Figure 2538: DNA327752, HSDHACTYL, 211150_s_at
Figure 2539A-B: DNA328700, SCD, 211162_x_at
Figure 2540: PRO84464
Figure 2541: DNA330221, NP_056071.1, 211207_s_at
Figure 2542: PRO85460
Figure 2543: DNA330222, NP_003848.1, 211226_at
Figure 2544: PRO45958
Figure 2545: DNA218278, IL2RA, 211269_s_at
Figure 2546: PRO34330
Figure 2547: DNA151022, DGKA, 211272_s_at
Figure 2548: PRO12096

Figure 2549: DNA330223, NP_001790.1, 211297_s_at
Figure 2550: PRO49730
Figure 2551A-C: DNA328811, ITPR1, 211323_s_at
Figure 2552: PRO84551
Figure 2553: DNA188234, TNFSF6, 211333_s_at
Figure 2554: PRO21942
Figure 2555: DNA103395, HSU80737, 211352_s_at
Figure 2556: PRO4723
Figure 2557A-B: DNA275066, NP_000170.1, 211450_s_at
Figure 2558: PRO62786
Figure 2559: DNA327755, NP_115957.1, 211458_s_at
Figure 2560: PRO83725
Figure 2561: DNA93439, CXCR6, 211469_s_at
Figure 2562: PRO4515
Figure 2563: DNA330175, KNSL6, 211519_s_at
Figure 2564: PRO59681
Figure 2565: DNA327756, NP_068814.2, 211538_s_at
Figure 2566: PRO83726
Figure 2567: DNA269888, GPRK6, 211543_s_at
Figure 2568: PRO58286
Figure 2569: DNA226255, NP_003047.1, 211576_s_at
Figure 2570: PRO36718
Figure 2571: DNA330211, LST1, 211581_x_at
Figure 2572: PRO85454
Figure 2573: DNA330224, HUMNCA, 211657_at
Figure 2574: PRO85461
Figure 2575: DNA327709, HBB, 211696_x_at
Figure 2576: PRO83690
Figure 2577: DNA331533, PPARG, 211699_x_at
Figure 2578: PRO86562
Figure 2579: DNA331534, AF116616, 211708_s_at
Figure 2580: DNA226342, PTEN, 211711_s_at
Figure 2581: PRO36805
Figure 2582: DNA328706, BC021909, 211714_x_at
Figure 2583: PRO10347
Figure 2584: DNA88307, NP_001992.1, 211734_s_at
Figure 2585: PRO2280
Figure 2586: DNA329225, EVI2B, 211742_s_at
Figure 2587: PRO84833
Figure 2588: DNA331535, AF105974, 211745_x_at
Figure 2589: PRO3629
Figure 2590: DNA328649, TUBA6, 211750_x_at
Figure 2591: PRO84424
Figure 2592: DNA254725, KPNA2, 211762_s_at
Figure 2593: PRO49824
Figure 2594: DNA330225, NP_115712.1, 211767_at
Figure 2595: PRO85462
Figure 2596A-B: DNA329226, BC006181, 211784_s_at
Figure 2597: PRO60388
Figure 2598: DNA304473, BC006196, 211786_at
Figure 2599: PRO2023
Figure 2600: DNA330226, AF198052, 211794_at
Figure 2601: PRO85463
Figure 2602: DNA227173, FYB, 211795_s_at
Figure 2603: PRO37636
Figure 2604: DNA331536, AAA60662.1, 211796_s_at
Figure 2605: PRO86563
Figure 2606: DNA331537, CCNE2, 211814_s_at

Figure 2607: PRO59418
Figure 2608A-B: DNA331342, DEFCAP, 211822_s_at
Figure 2609: PRO86422
Figure 2610: DNA331343, AK026398, 211824_x_at
Figure 2611: PRO86423
Figure 2612: DNA331538, AF327066, 211825_s_at
Figure 2613: PRO86564
Figure 2614A-B: DNA331539, BRCA1, 211851_x_at
Figure 2615: PRO86565
Figure 2616A-B: DNA188192, CD28, 211856._x_at
Figure 2617: PRO21704
Figure 2618: DNA331540, AF222343, 211861_s_at
Figure 2619: PRO86566
Figure 2620: DNA330228, HUMTCRAZ, 211902_x_at
Figure 2621: PRO85465
Figure 2622: DNA226176, CXCR4, 211919_s_at
Figure 2623: PRO36639
Figure 2624: DNA272286, CAT, 211922_s_at
Figure 2625: PRO60544
Figure 2626: DNA330229, BC011915, 211926_s_at
Figure 2627: PRO85466
Figure 2628: DNA226254, NP_001408.1, 211937_at
Figure 2629: PRO36717
Figure 2630: DNA330230, NP_060977.1, 211938_at
Figure 2631: PRO85467
Figure 2632A-B: DNA325306, ITGB1, 211945_s_at
Figure 2633: PRO81851
Figure 2634A-B: DNA272195, HUMORFGA, 211951_at
Figure 2635: DNA328437, NP_005792.1, 211956_s_at
Figure 2636: PRO84271
Figure 2637: DNA325941, NP_005339.1, 211969_at
Figure 2638: PRO82388
Figure 2639: DNA287194, AAA60258.1, 211974_x_at
Figure 2640: PRO69480
Figure 2641A-C: DNA331541, 1390535.1, 211986_at
Figure 2642: PRO86567
Figure 2643: DNA330232, NP291032.1, 211991_s_at
Figure 2644: PRO85469
Figure 2645: DNA330233, AF218029, 211999_at
Figure 2646: PRO11403
Figure 2647: DNA287433, NP_006810.1, 212009_s_at
Figure 2648: PRO69690
Figure 2649A-D: DNA103461, MKI67, 212020_s_at
Figure 2650: PRO4788
Figure 2651A-D: DNA226463, HSMKI67A, 212021_s_at
Figure 2652: PRO36926
Figure 2653A-D: DNA103461, HSMKI67, 212022_s_at
Figure 2654: PRO4788
Figure 2655A-D: DNA226463, DNA226463, 212023_s_at
Figure 2656: PRO36926
Figure 2657: DNA275447, HSMEMA, 212037_at
Figure 2658: PRO63095
Figure 2659: DNA103380, NP_003365.1, 212038_s_at
Figure 2660: PRO4710
Figure 2661A-B: DNA330234, 215138.24, 212045_at
Figure 2662: PRO85470
Figure 2663: DNA328709, BC004151, 212048_s_at
Figure 2664: PRO37676

Figure 2665A-B: DNA330235, BAA20790.1, 212061_at
Figure 2666: PRO85471
Figure 2667: DNA330236, 228447.20, 212071_s_at
Figure 2668: PRO85472
Figure 2669: DNA154139, DNA154139, 212099_at
Figure 2670A-B: DNA331542, BAA74910.1, 212108_at
Figure 2671: PRO86568
Figure 2672A-B: DNA150956, BAA06685.1, 212110_at
Figure 2673: PRO12560
Figure 2674: DNA328711, AK023154, 212115_at
Figure 2675: PRO84468
Figure 2676: DNA219225, NP_002874.1, 212125_at
Figure 2677: PRO34531
Figure 2678: DNA330238, BC019676, 212127_at
Figure 2679: DNA328713, AF100737, 212130_x_at
Figure 2680: PRO84470
Figure 2681: DNA330239, AK027643, 212131_at
Figure 2682: PRO85474
Figure 2683: DNA330240, CAA52801.1, 212141_at
Figure 2684: PRO85475
Figure 2685: DNA330240, HSP1CDC21, 212142_at
Figure 2686A-B: DNA150829, AB014568, 212144_at
Figure 2687: DNA329602, AK2, 212175_s_at
Figure 2688: PRO85133
Figure 2689: DNA330241, AF314185, 212176_at
Figure 2690: DNA328716, HSM800707, 212179_at
Figure 2691: DNA330242, BC007034, 212185_x_at
Figure 2692: PRO85477
Figure 2693: DNA330243, BC015663, 212190_at
Figure 2694: PRO2584
Figure 2695: DNA326233, RPL13, 212191_x_at
Figure 2696: PRO82645
Figure 2697A-C: DNA330244, 253946.17, 212196_at
Figure 2698: PRO85478
Figure 2699A-B: DNA330245, 230497.7, 212206_at
Figure 2700: PRO85479
Figure 2701: DNA331543, BC008710, 212227_x_at
Figure 2702: PRO84271
Figure 2703: DNA327770, 1384008.4, 212239_at
Figure 2704: PRO83736
Figure 2705: DNA151120, DNA151120, 212240_s_at
Figure 2706: PRO12179
Figure 2707: DNA330246, AF326773, 212241_at
Figure 2708A-B: DNA329229, 1345070.7, 212249_at
Figure 2709: PRO84835
Figure 2710: DNA329182, BC016852, 212259_s_at
Figure 2711: PRO84805
Figure 2712: DNA331544, BC018823, 212266_s_at
Figure 2713: PRO86569
Figure 2714: DNA327771, NP_109591.1, 212268_at
Figure 2715: PRO83737
Figure 2716: DNA326463, NP_000976.1, 212270_x_at
Figure 2717: PRO82846
Figure 2718: DNA150980, HUMMAC30X, 212279_at
Figure 2719: DNA150980, DNA150980, 212281_s_at
Figure 2720: PRO12566
Figure 2721: DNA253017, DNA253017, 212282_s_at
Figure 2722: PRO48926

Figure 2723: DNA328719, BC012895, 212295_s_at
Figure 2724: PRO84475
Figure 2725: DNA271103, NP_005796.1, 212296_at
Figure 2726: PRO59425
Figure 2727: DNA207620, DNA207620, 212300_at
Figure 2728: DNA330247, BC019110, 212313_at
Figure 2729: PRO85481
Figure 2730: DNA330248, BC019924, 212320_at
Figure 2731: PRO10347
Figure 2732A-B: DNA124122, HSP130K, 212331_at
Figure 2733: PRO6323
Figure 2734A-B: DNA124122, NP_005602.2, 212332_at
Figure 2735: PRO6323
Figure 2736: DNA287190, CAB43217.1, 212333_at
Figure 2737: PRO69476
Figure 2738A-B: DNA330216, MAD4, 2123 47_x_at
Figure 2739: PRO85457
Figure 2740A-B: DNA327773, BAA25456.1, 212366_at
Figure 2741: PRO83739
Figure 2742A-C: DNA330249, AAA99177.1, 212372_at
Figure 2743: PRO85482
Figure 2744: DNA329231, NP_000810.1, 212378_at
Figure 2745: PRO84837
Figure 2746: DNA329231, GART, 212379_at
Figure 2747: PRO84837
Figure 2748A-B: DNA150950, HUMKIAAH, 212639_x_at
Figure 2749A-B: DNA328549, NP_002897.1, 212397_at
Figure 2750: PRO84350
Figure 2751A-B: DNA328549, RDX, 212398_at
Figure 2752: PRO84350
Figure 2753: DNA151330, DNA151330, 212400_at
Figure 2754: PRO11708
Figure 2755A-B: DNA330250, NP_060727.1, 212406_s_at
Figure 2756: PRO85483
Figure 2757: DNA254828, AK023204, 212408_at
Figure 2758: PRO49923
Figure 2759: DNA330251, NP_059965.1, 212430_at
Figure 2760: PRO85484
Figure 2761: DNA327774, BC016808, 212460_at
Figure 2762: PRO83740
Figure 2763A-B: DNA330252, NP_055447.1, 212473_s_at
Figure 2764: PRO85485
Figure 2765: DNA269630, TPM4, 212481_s_at
Figure 2766: PRO58042
Figure 2767: DNA330253, BC007665, 212493_s_at
Figure 2768: PRO85486
Figure 2769: DNA330254, AK024029, 212508_at
Figure 2770: PRO85487
Figure 2771A-B: DNA254192, BAA07648.1, 212510_at
Figure 2772: PRO49304
Figure 2773: DNA329233, 383512.16, 212527_at
Figure 2774: PRO84839
Figure 2775: DNA226041, NP_005555.1, 212531_at
Figure 2776: PRO36504
Figure 2777: DNA269882, HSWEE1HU, 212533_at
Figure 2778: PRO58280
Figure 2779A-D: DNA328737, 148650.1, 212560-at
Figure 2780: PRO84490

Figure 2781A-B: DNA330255, AK025499, 212561_at
Figure 2782: PRO85488
Figure 2783: DNA225632, NP_002037.2, 212581_x_at
Figure 2784: PRO36095
Figure 2785A-C: DNA329236, AF392452, 212582_at
Figure 2786: PRO84841
Figure 2787A-B: DNA331545, AB040884, 212585_at
Figure 2788: DNA275100, DNA275100, 212589_at
Figure 2789: DNA330256, BC020889, 212592_at
Figure 2790: PRO81145
Figure 2791: DNA330257, 441179.4, 212605_s_at
Figure 2792: PRO85489
Figure 2793A-B: DNA330258, BAA22955.2, 212619_at
Figure 2794: PRO85490
Figure 2795A-B: DNA330258, AB006624, 212621_at
Figure 2796: DNA330259, NP_008944.1, 212638_s_at
Figure 2797: PRO49366
Figure 2798: DNA331357, 212396_s_at BC010494,
Figure 2799: PRO38556
Figure 2800A-D: DNA327777, HSIL1RECA, 212657_s_at
Figure 2801A-B: DNA327778, AB011154, 212675_s_at
Figure 2802: DNA273465, DNA273465, 212677_s_at
Figure 2803: DNA328744, AF318364, 212680_x_at
Figure 2804: PRO84496
Figure 2805A-B: DNA329901, AB007915, 212683_at
Figure 2806A-B: DNA269508, AB011110, 212706_at
Figure 2807A-B: DNA331546, 332730.12, 212714_at
Figure 2808: PRO86570
Figure 2809: DNA331547, BC010994, 212734_x_at
Figure 2810: PRO82645
Figure 2811: DNA329906, BC007848, 212738_at
Figure 2812: PRO85223
Figure 2813: DNA330261, NP_110383.1, 212762_s_at
Figure 2814: PRO85492
Figure 2815: DNA330262, NP_006409.2, 212768_s_at
Figure 2816: PRO85493
Figure 2817A-B: DNA254149, BAA06224.1, 212789_at
Figure 2818: PRO49264
Figure 2819: DNA331548, BC017356, 212827_at
Figure 2820: PRO86571
Figure 2821A-B: DNA150452, BAA32470.1, 212830_at
Figure 2822: PRO12260
Figure 2823A-B: DNA331549, BAA07892.2, 212832_s_at
Figure 2824: PRO86572
Figure 2825: DNA271714, BAA05039.1, 212836_at
Figure 2826: PRO59998
Figure 2827: DNA331550, AAA59587.1, 212859_v_at
Figure 2828: PRO6386
Figure 2829A-B: DNA328753, BAA13212.1, 212873_at
Figure 2830: PRO84502
Figure 2831: DNA330265, NP_056436.1, 212886_at
Figure 2832: PRO85495
Figure 2833A-B: DNA271215, BAA24380.1, 212892_at
Figure 2834: PRO59530
Figure 2835A-B: DNA330266, CAA10334.1, 212902_at
Figure 2836: PRO85496
Figure 2837A-B: DNA271137, AB014589, 212905_at
Figure 2838: DNA271630, DNA271630, 212907_at

Figure 2839: DNA330267, 235076.14, 212914_at
Figure 2840: PRO85497
Figure 2841: DNA330268, BC009116, 212928_at
Figure 2842: PRO85498
Figure 2843: DNA331551, BC013078, 212933_x_at
Figure 2844: PRO82645
Figure 2845A-B: DNA330269, BC020584, 212936_at
Figure 2846: PRO23868
Figure 2847: DNA330270, HUMORF007, 212949_at
Figure 2848A-B: DNA331552, PAM, 212958_x_at
Figure 2849: PRO86573
Figure 2850: DNA273283, HUMCYSTRNA, 212971_at
Figure 2851: DNA330271, 399773.20, 212980_at
Figure 2852: PRO85500
Figure 2853: DNA330272, AF119896, 212981_s_at
Figure 2854: DNA330273, AK027564, 213007_at
Figure 2855: PRO85502
Figure 2856: DNA254940, BAA91770.1, 213008_at
Figure 2857: PRO50030
Figure 2858: DNA325596, TPI1, 213011_s_at
Figure 2859: PRO69549
Figure 2860: DNA331553, 228519.3, 213021_at
Figure 2861: PRO86574
Figure 2862A-B: DNA253815, BAA20833.2, 213035_at
Figure 2863: PRO49218
Figure 2864A-B: DNA329240, NP_056133.1, 213039_at
Figure 2865: PRO84845
Figure 2866A-B: DNA330275, BAA25487.1, 213045_at
Figure 2867: PRO85504
Figure 2868A-B: DNA329242, BAA76857.1, 213056_at
Figure 2869: PRO84847
Figure 2870: DNA323869, NP_000960.2, 213080_x_at
Figure 2871: PRO80612
Figure 2872: DNA270466, HUMG6PD, 213093_at
Figure 2873: DNA330276, NP_001614.3, 213095_x_at
Figure 2874: PRO85505
Figure 2875: DNA331554, AF118070, 213113_s_at
Figure 2876: PRO86575
Figure 2877: DNA287230, AAA36325.1, 213138_at
Figure 2878: PRO69509
Figure 2879: DNA330277, CAB45152.1, 213142_x_at
Figure 2880: PRO85506
Figure 2881: DNA228053, DNA228053, 213156_at
Figure 2882: DNA151370, DNA151370, 213158_at
Figure 2883: PRO11747
Figure 2884: DNA106374, DNA106374, 213164_at
Figure 2885A-B: DNA330278, BAA13216.1, 213174_at
Figure 2886: PRO85507
Figure 2887: DNA330279, AF043182, 213193_x_at
Figure 2888: PRO85508
Figure 2889: DNA227909, NP_005024.1, 213226_at
Figure 2890: PRO38372
Figure 2891A-B: DNA330280, BAA83045.2, 213254_at
Figure 2892: PRO85509
Figure 2893A-B: DNA328761, BAA82991.1, 213280_at
Figure 2894: PRO84509
Figure 2895: DNA331555, BC009874, 213281_at
Figure 2896A-B: DNA274945, HSACKI10, 213287_s_at

Figure 2897: DNA260974, NP_006065.1, 213293 _s_at
Figure 2898: PRO54720
Figure 2899: DNA328357, 1452321.2, 213295_at
Figure 2900: PRO84217
Figure 2901A-B: DNA329248, BAA20816.1, 213302_at
Figure 2902: PRO84850
Figure 2903A-B: DNA255273, BAA83044.1, 213309_at
Figure 2904: PRO50349
Figure 2905: DNA155418, DNA155418, 213326_at
Figure 2906A-B: DNA331355, AAG24545.1, 213330_s_at
Figure 2907: PRO86431
Figure 2908A-B: DNA330281, AB058688, 213341_at
Figure 2909: DNA327789, 1449824.5, 213348_at
Figure 2910: PRO83753
Figure 2911: DNA287176, AB025254, 213361_at
Figure 2912: DNA327790, 1448999.3, 213364_s_at
Figure 2913: PRO83754
Figure 2914A-B: DNA330282, 217860.13, 213376_at
Figure 2915: PRO85510
Figure 2916: DNA330283, BC020225, 213408_s_at
Figure 2917: PRO85511
Figure 2918: DNA330284, 235806.16, 213434_at
Figure 2919: PRO85512
Figure 2920: DNA225632, GAPD, 213453_x_at
Figure 2921: PRO36095
Figure 2922A-B: DNA330285, 241020.1, 213469_at
Figure 2923: PRO85513
Figure 2924: DNA328766, NP_006077.1, 213476_x_at
Figure 2925: PRO84514
Figure 2926: DNA330286, BC018130, 213506_at
Figure 2927: PRO85514
Figure 2928: DNA326639, NP_001229.1, 213523_at
Figure 2929: PRO82992
Figure 2930A-C: DNA330287, AF380180, 213538_at
Figure 2931: PRO85515
Figure 2932: DNA227483, SQLE, 213562_s_at
Figure 2933: PRO37946
Figure 2934: DNA330288, NP_005606.1, 213566_at
Figure 2935: PRO2869
Figure 2936: DNA330289, 197444.1, 213567_at
Figure 2937: PRO85516
Figure 2938: DNA159560, DNA159560, 213577_at
Figure 2939: DNA330290, 1398807.8, 213581_at
Figure 2940: PRO85517
Figure 2941: DNA327799, HSRP26AA, 213587_s_at
Figure 2942: PRO40011
Figure 2943: DNA273753, AAC39561.1, 213599_at
Figure 2944: PRO61716
Figure 2945: DNA330291, 1500175.9, 213616_at
Figure 2946: PRO85518
Figure 2947A-C: DNA330292, NP_056045.2, 213618-at
Figure 2948: PRO85519
Figure 2949: DNA225974, ICAM2, 213620_s_at
Figure 2950: PRO36437
Figure 2951: DNA331556, BC009513, 213646_x_at
Figure 2952: PRO38556
Figure 2953A-B: DNA273985, BAA07647.1, 213647_at
Figure 2954: PRO61932

Figure 2955: DNA270758, HSU54778, 213655_at
Figure 2956: PRO59117
Figure 2957: DNA330293, BC011922, 213666_at
Figure 2958: PRO85520
Figure 2959: DNA325704, MARS, 213671_s_at
Figure 2960: PRO82188
Figure 2961: DNA304796, NP_443109.1, 213696_s_art
Figure 2962: PRO71208
Figure 2963: DNA273236, ASAH1, 213702_x_at
Figure 2964: PRO61263
Figure 2965: DNA255913, DNA255913, 213725_x_at
Figure 2966: DNA328629, TUBB2, 213726_x_at
Figure 2967: PRO84407
Figure 2968: DNA328771, HSMYOSIE, 213733_at
Figure 2969A-C: DNA151167, FLNA, 213746_s_at
Figure 2970: PRO12867
Figure 2971: DNA326273, BC001832, 213757-at
Figure 2972: PRO82678
Figure 2973A-B: DNA274483, NP_000126.1, 213778_x_at
Figure 2974: PRO62385
Figure 2975: DNA328774, NP_004263.1, 213793_s_at
Figure 2976: PRO60536
Figure 2977: DNA327804, AF442151, 213797_at
Figure 2978: PRO69493
Figure 2979A-B: DNA329967, SMARCA5, 213859_x_at
Figure 2980: PRO85270
Figure 2981: DNA151041, HSAMYB2, 213906_at
Figure 2982: DNA330294, 426625.1, 213908_at
Figure 2983: PRO85521
Figure 2984: DNA330295, NP_037515.1, 213951_s_at
Figure 2985: PRO85522
Figure 2986: DNA327807, NP_115613.1, 213975_s_at
Figure 2987: PRO83768
Figure 2988: DNA327808, NP_002961.1, 213988_s_at
Figure 2989: PRO83769
Figure 2990: DNA329136, HSPC111, 214011_s_at
Figure 2991: PRO84772
Figure 2992: DNA196110, DNA196110, 214016_s_at
Figure 2993: PRO24635
Figure 2994: DNA227224, LC27, 214039_s_at
Figure 2995: PRO37687
Figure 2996: DNA330296, 206955.3, 214054_at
Figure 2997: PRO85523
Figure 2998: DNA273696, DNA273696, 214060_at
Figure 2999A-B: DNA330297, AF378753, 214081_at
Figure 3000: PRO85524
Figure 3001: DNA227091, NP_000256.1, 214084_x_at
Figure 3002: PRO37554
Figure 3003: DNA331557, BC016778, 214085_x_at
Figure 3004: PRO86576
Figure 3005: DNA254686, NP_005475.1, 214086_s_at
Figure 3006: PRO49786
Figure 3007: DNA330298, BC011911, 214095_at
Figure 3008: PRO83772
Figure 3009: DNA329254, BC004215, 214096_s_at
Figure 3010: PRO84854
Figure 3011: DNA330299, AK023737, 214102_at
Figure 3012: PRO85525

Figure 3013: DNA331360, AK022497, 214177_s_at
Figure 3014: PRO86435
Figure 3015A-B: DNA269826, NP_003195.1, 214179_s_art
Figure 3016: PRO58228
Figure 3017: DNA331558, AF000424, 214181_x_at
Figure 3018: PRO86577
Figure 3019: DNA290295, NP_055203.1, 214193_s_at
Figure 3020: PRO70455
Figure 3021: DNA327701, C1QBP, 214214_s_-at
Figure 3022: PRO82667
Figure 3023: DNA331361, NP_003318.1, 214228_x_at
Figure 3024: PRO2398
Figure 3025: DNA154914, DNA154914, 214230_at
Figure 3026: DNA330300, NP_004883.1, 214257_s_at
Figure 3027: PRO41086
Figure 3028: DNA273940, DNA273940, 214272_at
Figure 3029: DNA97279, JUND, 214326_x_at
Figure 3030: PRO3628
Figure 3031: DNA84130, HSU37518, 214329_x_at
Figure 3032: PRO1096
Figure 3033: DNA272928, DAZAP2, 214334_x_at
Figure 3034: PRO61012
Figure 3035: DNA331362, AF275719, 214359_s_at
Figure 3036: PRO86436
Figure 3037: DNA331559, AF043723, 214368_at
Figure 3038: PRO85114
Figure 3039: DNA328611, AF043722, 214369_s_at
Figure 3040: PRO84393
Figure 3041: DNA273138, NP_005495.1, 214390_s_at
Figure 3042: PRO61182
Figure 3043: DNA273174, NP_001951.1, 214394_x_at
Figure 3044: PRO61211
Figure 3045: DNA328782, 337794.1, 214405_at
Figure 3046: PRO84528
Figure 3047: DNA326090, NP_000549.1, 214414_x_at
Figure 3048: PRO3629
Figure 3049: DNA271374, CHAF1A, 214426_x_at
Figure 3050: PRO59673
Figure 3051: DNA287630, NP_000160.1, 214430_at
Figure 3052: PRO2154
Figure 3053: DNA327811, SHMT2, 214437_s_at
Figure 3054: PRO83772
Figure 3055: DNA331363, AF001383, 214439_x_at
Figure 3056: PRO86437
Figure 3057: DNA331560, NP_001326.1, 214450_at
Figure 3058: PRO85081
Figure 3059: DNA273138, BCAT1, 214452_at
Figure 3060: PRO61182
Figure 3061: DNA327812, NP_006408.2, 214453_s_at
Figure 3062: PRO83773
Figure 3063: DNA150971, NP_002249.1, 214470-at
Figure 3064: PRO12564
Figure 3065: DNA330301, NP_008908.1, 214482_at
Figure 3066: PRO85526
Figure 3067: DNA325246, RRP4, 214507_s_at
Figure 3068: PRO81800
Figure 3069: DNA331561, CREM, 214508_x_at
Figure 3070: PRO86578

Figure 3071: DNA331562, NP_003090.1, 214531_s_at
Figure 3072: PRO58654
Figure 3073: DNA216515, NP_003166.1, 214567_s_at
Figure 3074: PRO34267
Figure 3075: DNA331223, HUMPRF1M, 214617_at
Figure 3076: DNA331563, BC004101, 214643_x_at
Figure 3077: PRO86579
Figure 3078: DNA150552, AAB97011.1, 214661_s_at
Figure 3079: PRO12326
Figure 3080: DNA330303, BAA05499.1, 214662_at
Figure 3081: PRO85528
Figure 3082: DNA330304, HSIGVL026, 214677_x_at
Figure 3083: PRO85529
Figure 3084: DNA287355, ALDOA, 214687_x_at
Figure 3085: PRO69617
Figure 3086: DNA330305, HSU79263, 214700_x_at
Figure 3087: DNA288259, NP_114172.1, 214710_s_at
Figure 3088: PRO4676
Figure 3089: DNA324984, NP_115540.1, 214714_at
Figure 3090: PRO81578
Figure 3091: DNA330306, 407311.1, 214743_at
Figure 3092: PRO85531
Figure 3093: DNA331564, BC014654, 214752_x_at
Figure 3094: PRO86580
Figure 3095: DNA254338, AAA60119.1, 214765_s_at
Figure 3096: PRO49449
Figure 3097: DNA275473, DNA275473, 214787_at
Figure 3098A-B: DNA272353, AB007958, 214833_at
Figure 3099: DNA226577, C6orf9, 214847_s_at
Figure 3100: PRO37040
Figure 3101A-B: DNA331565, BAA34472.1, 214945_at
Figure 3102: PRO86581
Figure 3103: DNA328530, LAK-4P, 214958_s_at
Figure 3104: PRO24118
Figure 3105A-B: DNA271654, AB020704, 214978_s_at
Figure 3106A-B: DNA329261, HSM802517, 215001_s_at
Figure 3107: PRO84859
Figure 3108: DNA330308, 307914.1, 215029_at
Figure 3109: PRO85533
Figure 3110: DNA196372, HSBCLXL, 215037_s_at
Figure 3111: PRO24874
Figure 3112: DNA331566, AIF1, 215051_x_at
Figure 3113: PRO86582
Figure 3114: DNA330309, NP_003503.1, 215071_s_at
Figure 3115: PRO85534
Figure 3116A-B: DNA330307, AB018295, 215133_s_at
Figure 3117: DNA331567, 333089.1, 215147_at
Figure 3118: PRO86583
Figure 3119: DNA273371, UMPS, 215165_x_at
Figure 3120: PRO61373
Figure 3121A-B: DNA150496, AB023212, 215175_at
Figure 3122A-B: DNA220748, ITGA6, 215177_s_at
Figure 3123: PRO34726
Figure 3124: DNA330311, 405318.1, 215221_at
Figure 3125: PRO85536
Figure 3126: DNA227597, NP_000627.1, 215223_s_at
Figure 3127: PRO38060
Figure 3128A-B: DNA330312, 406407.1, 215262_at

Figure 3129: PRO85537
Figure 3130: DNA331568, TADA3L, 215273_s_at
Figure 3131: PRO80953
Figure 3132: DNA330314, 026641.5, 215275_at
Figure 3133: PRO85538
Figure 3134: DNA330315, 1500205.1, 215283_at
Figure 3135: PRO85539
Figure 3136: DNA330316, 1448781.1, 215284_at
Figure 3137: PRO85540
Figure 3138: DNA330317, 228133.1, 215330_at
Figure 3139: PRO85541
Figure 3140: DNA331569, NP_000552.1, 215333_x_at
Figure 3141: PRO85542
Figure 3142: DNA327831, NP_076956.1, 215380_s_at
Figure 3143: PRO83783
Figure 3144: DNA328801, 407831.1, 215392_at
Figure 3145: PRO84543
Figure 3146: DNA325174, NP_038470.1, 215416_s_at
Figure 3147: PRO9819
Figure 3148: DNA275385, NP_002085.1, 215438_x_at
Figure 3149: PRO63048
Figure 3150: DNA331570, BC015794, 215440_s_at
Figure 3151: PRO84545
Figure 3152: DNA330319, AF247727, 215483_at
Figure 3153: DNA331571, MAP2K3, 215498_s_at
Figure 3154: PRO86584
Figure 3155: DNA330186, BUB1, 215509_s_at
Figure 3156: PRO85434
Figure 3157: DNA330321, 315726.1, 215605-at
Figure 3158: PRO85545
Figure 3159: DNA331572, AF000426, 215633_x_at
Figure 3160: PRO86585
Figure 3161: DNA330031, LOC51668, 215691_x_at
Figure 3162: PRO85316
Figure 3163: DNA331573, HSAPT1, 215719_x_at
Figure 3164A-B: DNA254376, KIAA0963, 215760_s_at
Figure 3165: PRO49486
Figure 3166A-B: DNA328805, BAA86482.1, 215785_s_at
Figure 3167: PRO84547
Figure 3168: DNA331574, HUMTCGCH, 215806_x_at
Figure 3169: DNA330322, 234025.21, 215855_s_at
Figure 3170: PRO85546
Figure 3171: DNA330323, 335053.1, 215908_at
Figure 3172: PRO85547
Figure 3173: DNA330324, HHEX, 215933_s_at
Figure 3174: PRO58034
Figure 3175: DNA331575, AF223408, 2159428_s_at
Figure 3176: PRO86587
Figure 3177: DNA330325, NP_055057.1, 215948_x_at
Figure 3178: PRO85548
Figure 3179: DNA227668, GK, 215966_x_at
Figure 3180: PRO38131
Figure 3181: DNA330326, AY007142, 215967_s_at
Figure 3182: PRO85549
Figure 3183: DNA331576, HSRNAGLK, 215977_x_at
Figure 3184: DNA188736, U00115, 215990_s_at
Figure 3185: PRO26296
Figure 3186: DNA331577, 208045.1, 216109_at

Figure 3187: PRO86588
Figure 3188: DNA331578, HSTCELD, 216133_at
Figure 3189: PRO86589
Figure 3190: DNA254783, DKC1, 216212_s_at
Figure 3191: PRO49881
Figure 3192A-B: DNA255273, AB029015, 216218_s_at
Figure 3193A-B: DNA256461, AND-1, 216228_s_at
Figure 3194: PRO51498
Figure 3195: DNA329266, BC000142, 216237_s_art
Figure 3196: PRO12845
Figure 3197: DNA151048, HSNOT, 216248_s_at
Figure 3198: PRO12850
Figure 3199: DNA329155, BC012479, 216252_x_at
Figure 3200: PRO1207
Figure 3201: DNA331579, HSCLCA, 216295_s_at
Figure 3202: DNA88189, CD24, 216379_x_at
Figure 3203: PRO2690
Figure 3204: DNA330329, IR1875335, 216483_s_at
Figure 3205: DNA287243, NP_004452.1, 216602_s_at
Figure 3206: PRO69518
Figure 3207: DNA331580, 1099517.2, 216607_s_at
Figure 3208: PRO86590
Figure 3209: DNA227874, TXN, 216609_at
Figure 3210: PRO38337
Figure 3211: DNA88296, NP_005733.1, 216640_s_sat
Figure 3212: PRO2274
Figure 3213: DNA269692, S59049, 216834_at
Figure 3214: PRO58102
Figure 3215: DNA227597, SOD2, 216841_s_at
Figure 3216: PRO38060
Figure 3217A-B: DNA330331, BAA86451.1, 216873_s_at
Figure 3218: PRO85554
Figure 3219: DNA188275, NP_002181.1, 216876_s_at
Figure 3220: PRO21800
Figure 3221A-B: DNA150987, NP_006051.1, 216901_s_at
Figure 3222: PRO12163
Figure 3223: DNA329267, HUMTCRGAAC, 216920_s_at
Figure 3224A-C: DNA328811, HLTMINSP3R1, 216944_s_at
Figure 3225: PRO84551
Figure 3226A-B: DNA151027, AAA80979.1, 216952_s_at
Figure 3227: PRO12843
Figure 3228A-E: DNA269650, PLEC1, 216971_s_at
Figure 3229A-B: PRO58061
Figure 3230: DNA328812, BAA86575.1, 216997_x_at
Figure 3231: PRO84552
Figure 3232: DNA331581, AAB59396.1, 217022_s_at
Figure 3233: PRO86591
Figure 3234: DNA331366, HUMGPCR, 217028_at
Figure 3235: PRO4516
Figure 3236A-B: DNA227293, AB020721, 217047_s_at
Figure 3237: PRO37756
Figure 3238A-B: DNA329269, BAA32292.2, 217122_s_at
Figure 3239: PRO84865
Figure 3240: DNA331582, AAA59588.1, 217165_x_at
Figure 3241: PRO86592
Figure 3242: DNA331583, S70123, 217173_s_at
Figure 3243: DNA331584, AF105973, 217232_x_at
Figure 3244: PRO86593

Figure 3245: DNA330334, NP_114402.1, 217286_s_at
Figure 3246: PRO85557
Figure 3247: DNA331369, HSU88968, 217294_s_at
Figure 3248A-B: DNA331585, AF051334, 217299_s_at
Figure 3249: PRO86594
Figure 3250: DNA331586, S81916, 217356_s_at
Figure 3251: DNA331587, HSNGMRNA, 217398_x_at
Figure 3252: PRO86595
Figure 3253: DNA330335, NP_054765.1, 217408_at
Figure 3254: PRO62166
Figure 3255: DNA331588, AF097635, 217414_x_at
Figure 3256: PRO3629
Figure 3257: DNA329539, HLA-DMA, 217478_s_at
Figure 3258: PRO85089
Figure 3259: DNA331589, 243999.2, 217502_at
Figure 3260: PRO86596
Figure 3261: DNA329271, 406848.1, 217591_at
Figure 3262: PRO84867
Figure 3263: DNA330337, 1447003.1, 217616_at
Figure 3264: PRO85559
Figure 3265: DNA331590, 368556.1, 217655_at
Figure 3266: PRO86597
Figure 3267: DNA330339, HSA012375, 217672_x_at
Figure 3268: DNA323856, HSM800628, 217725_x_at
Figure 3269: PRO80599
Figure 3270: DNA326523, NP_001121.2, 217729_s_at
Figure 3271: PRO71126
Figure 3272: DNA325832, NP_068839.1, 217731_s_at
Figure 3273: PRO1869
Figure 3274A-B: DNA327847, 142131.14, 217738_at
Figure 3275: PRO2834
Figure 3276: DNA88541, NP_005737.1, 217739_s_at
Figure 3277: PRO2834
Figure 3278: DNA327935, NP_079422.1, 217745_s_at
Figure 3279: PRO83866
Figure 3280: DNA327849, NP_057269.1, 217755_at
Figure 3281: PRO83794
Figure 3282A-B: DNA274131, AF183421, 217762_s_at
Figure 3283: PRO62067
Figure 3284: DNA330340, NP_006859.1, 217763_s_at
Figure 3285: PRO85562
Figure 3286A-B: DNA274131, DNA274131, 217764_s_at
Figure 3287: PRO62067
Figure 3288: DNA325821, NP_057016.1, 217769_s_at
Figure 3289: PRO82287
Figure 3290: DNA325910, AF167438, 217776_at
Figure 3291: PRO82365
Figure 3292: DNA227358, NP_057479.1, 217777_s_at
Figure 3293: PRO37821
Figure 3294: DNA328819, NP_057145.1, 217783_s_at
Figure 3295: PRO84557
Figure 3296: DNA325873, SKB1, 217786_at
Figure 3297: PRO82331
Figure 3298: DNA331591, NP_055241.1, 217792_at
Figure 3299: PRO69560
Figure 3300: DNA328303, NP_056525.1, 217807_s_at
Figure 3301: PRO84173
Figure 3302: DNA227223, NP_064583.1, 217814_at

EP 2 179 742 A1

Figure 3303: PRO37686
Figure 3304: DNA327851, NSAP1, 217834_s_at
Figure 3305: PRO83795
Figure 3306: DNA328823, NP_057421.1, 217838_s_at
Figure 3307: PRO84561
Figure 3308: DNA330341, NP_006061.2, 217839_at
Figure 3309: PRO85563
Figure 3310: DNA254773, NP_057231.1, 217841_s_at
Figure 3311: PRO49871
Figure 3312: DNA330342, NP_067021.1, 217844_at
Figure 3313: PRO85564
Figure 3314: DNA329272, NP_055181.1, 217850_at
Figure 3315: PRO84868
Figure 3316A-B: DNA330343, AF403012, 217857_s_at
Figure 3317: DNA330344, NP_057392.1, 217870_s_at
Figure 3318: PRO85565
Figure 3319: DNA326937, NP_002406.1, 217871_s_at
Figure 3320: PRO83255
Figure 3321: DNA330345, NP_055130.1, 217906_at
Figure 3322: PRO85566
Figure 3323: DNA330346, NP-054880.2, 217907_at
Figure 3324: PRO85567
Figure 3325: DNA325780, NP_060371.1, 217914_at
Figure 3326: PRO82250
Figure 3327: DNA327853, NP_054769.1, 217919_s_at
Figure 3328: PRO82223
Figure 3329: DNA330347, 255559.4, 217922_at
Figure 3330: PRO85568
Figure 3331: DNA330348, NP_079150.1, 217929_s_at
Figure 3332: PRO85569
Figure 3333: DNA330349, BC022093, 217931_at
Figure 3334: DNA287241, NP_056991.1, 217933_s_at
Figure 3335: PRO69516
Figure 3336A-B: DNA225648, NP_061165.1, 217941_s_at
Figure 3337: PRO36111
Figure 3338: DNA326730, NP_057037.1, 217950_at
Figure 3339: PRO83072
Figure 3340: DNA329273, NP_037374.1, 217957_at
Figure 3341: PRO84869
Figure 3342: DNA328829, NP_057230.1, 217959_s_at
Figure 3343: PRO84566
Figure 3344: DNA328830, NP_061118.1, 217962_at
Figure 3345: PRO84567
Figure 3346: DNA329274, NP_055195.1, 217963_s_at
Figure 3347: PRO84870
Figure 3348: DNA325496, NP_037397.2, 217969_at
Figure 3349: PRO82013
Figure 3350: DNA327855, NP_057387.1, 217975_at
Figure 3351: PRO83367
Figure 3352: DNA227218, NP_003721.2, 217983._s_at
Figure 3353:PRO37681
Figure 3354: DNA227218, RNASE6PL, 217984_at
Figure 3355: PRO37681
Figure 3356A-B: DNA227238, NP_038476.1, 217985_s_at
Figure 3357: PRO37701
Figure 3358A-B: DNA227238, BAZ1A, 217986_s_at
Figure 3359: PRO37701
Figure 3360: DNA328831, NP_057329.1, 217989_at

EP 2 179 742 A1
Figure 3361: PRO233
Figure 3362: DNA328832, NP_067022.1, 217995_at
Figure 3363: PRO84568
Figure 3364: DNA328833, BC018929, 217996_at
Figure 3365: PRO84569
Figure 3366: DNA328834, AF220656, 217997_at
Figure 3367: DNA287364, NP_031376.1, 218000_s_at
Figure 3368: PRO69625
Figure 3369: DNA273008, NP_003972.1, 218009_s_at
Figure 3370: PRO61079
Figure 3371: DNA330350, NP_006108.1, 218025_s_at
Figure 3372: PRO85570
Figure 3373: DNA328836, NP_054894.1, 218027_at
Figure 3374: PRO84572
Figure 3375: DNA329275, AF070673, 218032_at
Figure 3376: PRO12342
Figure 3377: DNA331592, ANKT, 218039_at
Figure 3378: PRO82424
Figure 3379: DNA328838, NP-054797.2, 218049_s_at
Figure 3380: PRO70319
Figure 3381: DNA330352, NP_075059.1, 218051_s_at
Figure 3382: PRO85571
Figure 3383: DNA329276, NP_077001.1, 218069_at
Figure 3384: PRO12104
Figure 3385: DNA328841, NP-060557.2, 218073_s_at
Figure 3386: PRO84575
Figure 3387: DNA329277, NP_054883.3, 218084_x_at
Figure 3388: PRO6241
Figure 3389: DNA330353, BC020796, 218085_at
Figure 3390: PRO69464
Figure 3391: DNA329278, NP_004495.1, 218092_s_at
Figure 3392: PRO84871
Figure 3393: DNA227313, NP_060945.1, 218095_s_at
Figure 3394: PRO37776
Figure 3395: DNA331593, MRPL4, 218105_s_at
Figure 3396: PRO86598
Figure 3397: DNA326596, NP_060624.1, 218115_at
Figure 3398: PRO82954
Figure 3399: DNA330355, NP_055063.1, 218117_at
Figure 3400: PRO83289
Figure 3401: DNA330356, NP_006318.1, 218118_s_at
Figure 3402: PRO85572
Figure 3403: DNA330357, NP_078786.2, 218130_at
Figure 3404: PRO85573
Figure 3405: DNA227155, NP_057654.1, 218135_at
Figure 3406: PRO37618
Figure 3407: DNA254496, NP_060076.1, 218149_s_at
Figure 3408: PRO49604
Figure 3409: DNA330358, NP_079012.1, 218154_at
Figure 3410: PRO85574
Figure 3411: DNA254739, NP_068766.1, 218156_s_at
Figure 3412: PRO49837
Figure 3413: DNA304470, PRO2577, 218172_s_at
Figure 3414: PRO2577
Figure 3415: DNA330359, NP_065145.1, 218178_s_at
Figure 3416: PRO85575
Figure 3417: DNA304495, NP_057156.1, 218193_s_at
Figure 3418: PRO793
66

Figure 3419A-C: DNA330360, NP_078789.1, 218204_s_at
Figure 3420: PRO85576
Figure 3421: DNA327858, NP_036473.1, 218238_at
Figure 3422: PRO83800
Figure 3423: DNA327858, CRFG, 218239_s_at
Figure 3424: PRO83800
Figure 3425A-B: DNA330361, CKAP2, 218252_at
Figure 3426: PRO85577
Figure 3427: DNA328850, NP_057187.1, 218254_s_at
Figure 3428: PRO84581
Figure 3429: DNA331594, MRPL24, 218270_at
Figure 3430: PRO11652
Figure 3431: DNA273230, NP_060914.1, 218273_s_at
Figure 3432: PRO61257
Figure 3433: DNA324444, NP_006333.1, 218308_at
Figure 3434: PRO81108
Figure 3435: DNA330363, NP_060252.1, 218331_s_at
Figure 3436: PRO85578
Figure 3437: DNA329281, NP_036526.2, 218336_at
Figure 3438: PRO84874
Figure 3439A-B: DNA330364, NP_004417.1, 218338_at
Figure 3440: PRO85579
Figure 3441: DNA272918, NP_055269.1, 218346_s_at
Figure 3442: PRO61003
Figure 3443: DNA327862, NP_060445.1, 218349_s_at
Figure 3444: PRO83803
Figure 3445: DNA328854, NP_056979.1, 218350_s_at
Figure 3446: PRO84585
Figure 3447A-B: DNA273415, KIF4A, 218355_at
Figure 3448: PRO61414
Figure 3449: DNA324890, NP_037525.1, 218356_at
Figure 3450: PRO81496
Figure 3451: DNA330365, NP_036591.1, 218357_s_at
Figure 3452: PRO85580
Figure 3453A-B: DNA331595, NP_073602.2, 218376_s_at
Figure 3454: PRO86599
Figure 3455: DNA330367, NP_057174.1, 218379_at
Figure 3456: PRO85582
Figure 3457: DNA328856, NP_068376.1, 218380_at
Figure 3458: PRO84586
Figure 3459: DNA227248, NP_006287.1, 218397_at
Figure 3460: PRO37711
Figure 3461A-B: DNA287192, NP_006178.1, 218400_at
Figure 3462: PRO69478
Figure 3463: DNA329912, TTC4, 218442_at
Figure 3464: PRO85227
Figure 3465: DNA150661, NP_057162.1, 218446_s_at
Figure 3466: PRO12398
Figure 3467: DNA304781, NP-057385.2, 218461_at
Figure 3468: PRO71191
Figure 3469: DNA328861, NP_057030.2, 218472_s_at
Figure 3470: PRO84589
Figure 3471: DNA330368, NP_064446.1, 218494_s_at
Figure 3472: PRO85583
Figure 3473: DNA150648, NP_037464.1, 218507_at
Figure 3474: PRO11576
Figure 3475: DNA328864, NP_060726.2, 218512_at
Figure 3476: PRO84592

Figure 3477: DNA330369, NP_060822.1, 218513_at
Figure 3478: PRO85584
Figure 3479: DNA330370, NP_060415.1, 218519_at
Figure 3480: PRO190
Figure 3481: DNA327867, NP_061873.2, 218532_s_at
Figure 3482: PRO83808
Figure 3483: DNA330371, NP_060813.1, 218535_s_at
Figure 3484: PRO85585
Figure 3485: DNA327868, NP_060601.2, 218542_at
Figure 3486: PRO83809
Figure 3487: DNA255113, NP_073587.1, 218543_s_at
Figure 3488: PRO50195
Figure 3489: DNA330372, NP_057117.1, 2185498_s_at
Figure 3490: PRO85586
Figure 3491: DNA330373, NP_060751.1, 218552_at
Figure 3492: PRO85587
Figure 3493: DNA330374, NP_054901.1, 218556_at
Figure 3494: PRO23321
Figure 3495: DNA330375, NP_059142.1, 218558_s_at
Figure 3496: PRO85588
Figure 3497: DNA329587, NP_036256.1, 218566_s_at
Figure 3498: PRO85121
Figure 3499: DNA329286, NP_005691.2, 218567_x_at
Figure 3500: PRO69644
Figure 3501: DNA329054, NP_078805.2, 218578_at
Figure 3502: PRO84716
Figure 3503A-B: DNA273435, NP_057532.1, 218585_s_at
Figure 3504: PRO61430
Figure 3505: DNA227327, NP_060547.1, 218593-at
Figure 3506: PRO37790
Figure 3507: DNA328628, NP_060542.2, 218594_at
Figure 3508: PRO84406
Figure 3509: DNA287642, NP_060934.1, 218597_s_at
Figure 3510: PRO9902
Figure 3511A-B: DNA254789, NP_057301.1, 218603_at
Figure 3512: PRO49887
Figure 3513: DNA330376, NP_076962.1, 218622_at
Figure 3514: PRO85589
Figure 3515: DNA327869, NP_057672.1, 218625_at
Figure 3516: PRO1898
Figure 3517: DNA330377, NP_036577.1, 218638_s_at
Figure 3518: PRO85590
Figure 3519: DNA330378, NP_071741.2, 218662_s_at
Figure 3520: PRO81126
Figure 3521: DNA330378, HCAP-G, 218663_at
Figure 3522: PRO81126
Figure 3523: DNA287291, NP_067036.1, 218676_s_at
Figure 3524: PRO69561
Figure 3525: DNA304835, NP_071327.1, 218681_s_at
Figure 3526: PRO71242
Figure 3527: DNA330379, NP_073562.1, 218689_at
Figure 3528: PRO85591
Figure 3529: DNA329288, NP_061910.1, 218695_at
Figure 3530: PRO84880
Figure 3531: DNA287378, NP_060898.1, 218715_at
Figure 3532: PRO69637
Figure 3533: DNA327202, NP_057289.1, 218718_at
Figure 3534: PRO200

Figure 3535: DNA330380, NP_078937.2, 218722_s_at
Figure 3536: PRO85592
Figure 3537: DNA324251, NP_060880.2, 218726_at
Figure 3538: PRO80935
Figure 3539: DNA227617, NP_057161.1, 218732_at
Figure 3540: PRO38080
Figure 3541: DNA330381, NP_076958.1, 218741_at
Figure 3542: PRO38668
Figure 3543: DNA330382, NP_005724.1, 218755_at
Figure 3544: PRO61907
Figure 3545: DNA330383, NP_054828.1, 218782_s_at
Figure 3546: PRO85593
Figure 3547: DNA330384, NP_060388.1, 218802_at
Figure 3548: PRO51129
Figure 3549: DNA88315, NP_004098.1, 218831_s_at
Figure 3550: PRO2743
Figure 3551: DNA330385, NP_057733.2, 218859_s_at
Figure 3552: PRO85594
Figure 3553: DNA330386, NP_057394.1, 218866_s_at
Figure 3554: PRO85595
Figure 3555: DNA330387, NP_036309.1, 218875_s_at
Figure 3556: PRO85596
Figure 3557: DNA327874, BC022791, 218880_at
Figure 3558: PRO4805
Figure 3559A-B: DNA271829, NP_006775.1, 218882_s_at
Figure 3560: PRO60109
Figure 3561: DNA330388, NP_078905.1, 218883_s_at
Figure 3562: PRO85597
Figure 3563: DNA226633, NP_060376.1, 218886_at
Figure 3564: PRO37096
Figure 3565: DNA304780, NP_060562.2, 218888_s_at
Figure 3566: PRO69889
Figure 3567: DNA256762, AK022882, 218889_at
Figure 3568: PRO51695
Figure 3569: DNA328881, NP-057706.2, 218890_x_at
Figure 3570: PRO49469
Figure 3571: DNA325622, NP_060518.1, 218894_s_at
Figure 3572: PRO82113
Figure 3573: DNA225694, NP_060087.1, 218902_at
Figure 3574: PRO36157
Figure 3575: DNA325690, NP_076973.1, 218903_s_at
Figure 3576: PRO82179
Figure 3577: DNA328364, SIGIRR, 218921_at
Figure 3578: PRO84223
Figure 3579: DNA287166, NP_055129.1, 218943_s_at
Figure 3580: PRO69459
Figure 3581: DNA330389, NP_079221.1, 218979_at
Figure 3582: PRO85598
Figure 3583: DNA329050, NP_057053.1, 218982_s_t
Figure 3584: PRO84712
Figure 3585: DNA330390, NP_057630.1, 218983_at
Figure 3586: PRO85599
Figure 3587: DNA288277, NP_061915.1, 218984_at
Figure 3588: PRO70034
Figure 3589: DNA256265, NP_060101.1, 218986_s_at
Figure 3590: PRO51309
Figure 3591: DNA227194, FLJ11000, 218999_at
Figure 3592: PRO37657

Figure 3593: DNA330391, NP_076999.1, 219000_s_at
Figure 3594: PRO34008
Figure 3595: DNA330392, NP_078923.2, 219007_at
Figure 3596: PRO85600
Figure 3597: DNA328885, NP_061108.2, 219017-at
Figure 3598: PRO50294
Figure 3599: DNA330393, NP_067635.1, 219024_at
Figure 3600: PRO85601
Figure 3601: DNA329292, NP_057185.1, 219031_s_at
Figure 3602: PRO84882
Figure 3603: DNA330394, NP_079402.1, 219035_s_at
Figure 3604: PRO85602
Figure 3605: DNA329293, NP_057136.1, 219037_at
Figure 3606: PRO84883
Figure 3607: DNA328886, NP_078811.1, 219040_art
Figure 3608: PRO84610
Figure 3609: DNA331596, NP_060841.1, 219049_at
Figure 3610: PRO84884
Figure 3611: DNA330395, NP_060212.1, 219062_s_at
Figure 3612: PRO85603
Figure 3613: DNA330396, NP_077303.1, 219088_s_at
Figure 3614: PRO85604
Figure 3615: DNA330397, NP_054873.1, 219094_at
Figure 3616: PRO85605
Figure 3617: DNA331597, PLA2G4B, 219095_at
Figure 3618: PRO86600
Figure 3619: DNA330398, NP_060367.1, 219133_at
Figure 3620: PRO85606
Figure 3621: DNA297191, NP-060962.2, 219148_at
Figure 3622: PRO70808
Figure 3623: DNA329295, NP_036549.1, 219155_at
Figure 3624: PRO84885
Figure 3625A-B: DNA329438, NP_476516.1, 219158_s_at
Figure 3626: PRO85008
Figure 3627: DNA328892, NP_067643.2, 219165_at
Figure 3628: PRO84616
Figure 3629: DNA330399, NP_060609.1, 219166_at
Figure 3630: PRO85607
Figure 3631: DNA330400, NP_078796.1, 219176_at
Figure 3632: PRO85608
Figure 3633: DNA271455, NP_057735.1, 219179_at
Figure 3634: PRO59751
Figure 3635: DNA330401, NP_057377.1, 219191_s_at
Figure 3636: PRO85609
Figure 3637: DNA330402, NP_076996.1, 219200_at
Figure 3638: PRO85610
Figure 3639: DNA287235, NP_060598.1, 219204_s_at
Figure 3640: PRO69514
Figure 3641: DNA327879, NP_071451.1, 219209_art
Figure 3642: PRO83818
Figure 3643: DNA330403, NP_059110.1, 219211_at
Figure 3644: PRO85611
Figure 3645: DNA330404, ZNF361, 219228_at
Figure 3646: PRO85612
Figure 3647: DNA225594, NP_037404.1, 219229_at
Figure 3648: PRO36057
Figure 3649: DNA328894, NP_060796.1, 219243_at
Figure 3650: PRO84617

Figure 3651: DNA329296, NP_060328.1, 219258_at
Figure 3652: PRO84886
Figure 3653: DNA304461, NP_054877.1, 219283_at
Figure 3654: PRO71039
Figure 3655: DNA330405, RBM15, 219286_s_at
Figure 3656: PRO85613
Figure 3657A-B: DNA329076, NP_064627.1, 219306_at
Figure 3658: PRO84733
Figure 3659: DNA329914, FLJ12542, 219311_at
Figure 3660: PRO85229
Figure 3661: DNA255939, NP_078876.1, 219315_s_at
Figure 3662: PRO50991
Figure 3663: DNA287404, NP_073748.1, 219334_s_at
Figure 3664: PRO69661
Figure 3665: DNA254710, NP_060382.1, 219352_at
Figure 3666: PRO49810
Figure 3667: DNA325169, HSPC177, 219356_s_at
Figure 3668: PRO81734
Figure 3669: DNA330406, NP_079368.1, 219359_at
Figure 3670: PRO85614
Figure 3671: DNA330407, NP_057026.2, 219363_s_at
Figure 3672: PRO85615
Figure 3673: DNA330408, NP_077024.1, 219364_at
Figure 3674: PRO85616
Figure 3675: DNA254518, NP_057354.1, 219371_s_at
Figure 3676: PRO49625
Figure 3677: DNA327886, NP_060832.1, 219399_at
Figure 3678: PRO41077
Figure 3679: DNA256417, NP_077271.1, 219402_s_at
Figure 3680: PRO51457
Figure 3681A-B: DNA327887, NP_006656.1, 219403_s_art
Figure 3682: PRO83823
Figure 3683: DNA271811, NP_036514.1, 219421_at
Figure 3684: PRO60092
Figure 3685: DNA329014, NP_005746.2, 219424_at
Figure 3686: PRO9998
Figure 3687: DNA328901, FLJ20533, 2199449_s_at
Figure 3688: PRO84622
Figure 3689: DNA328902, NP_071750.1, 219452_at
Figure 3690: PRO84623
Figure 3691: DNA328367, RIN3, 219456_s_at
Figure 3692: PRO84226
Figure 3693: DNA331598, AK026092, 219457_s_at
Figure 3694: PRO86601
Figure 3695: DNA327890, NP_079021.1, 219493_at
Figure 3696: PRO83826
Figure 3697A-B: DNA227179, NP_059120.1, 219505_at
Figure 3698: PRO37642
Figure 3699A-C: DNA331599, BCL11B, 219528_s_at
Figure 3700: PRO86602
Figure 3701: DNA329300, GEMIN6, 219539_at
Figure 3702: PRO84889
Figure 3703: DNA328908, 7691567.2, 219540_at
Figure 3704: PRO84629
Figure 3705: DNA256737, NP_060276.1, 219541_at
Figure 3706: PRO51671
Figure 3707: DNA330410, NP_060925.1, 219555_s_at
Figure 3708: PRO85618

Figure 3709A-B: DNA331600, NP_061985.1, 219577_s_at
Figure 3710: PRO86603
Figure 3711: DNA325053, NP_060230.2, 219588_s_at
Figure 3712: PRO81637
Figure 3713: DNA330412, NP_057617.1, 219594_at
Figure 3714: PRO23600
Figure 3715: DNA331601, NP_071915.1, 219628_at
Figure 3716: PRO85620
Figure 3717: DNA330414, NP_057615.1, 219657_s_at
Figure 3718: PRO81138
Figure 3719A-B: DNA274044, HSM801565, 219671_at
Figure 3720: PRO61987
Figure 3721: DNA293243, RCP, 219681_s_at
Figure 3722: PRO70699
Figure 3723: DNA255161, NP_071430.1, 219684_at
Figure 3724: PRO50241
Figure 3725: DNA287206, NP_060124.1, 219691_at
Figure 3726: PRO69488
Figure 3727A-B: DNA330297, NP_065138.2, 219700_at
Figure 3728: PRO85524
Figure 3729: DNA330416, TDP1, 219715_s_at
Figure 3730: PRO85622
Figure 3731: DNA330417, NP_085144.1, 219716_at
Figure 3732: PRO21341
Figure 3733A-B: DNA227255, NP_036579.1, 219753_at
Figure 3734: PRO37718
Figure 3735: DNA328919, NP_078987.1, 219777_at
Figure 3736: PRO84637
Figure 3737A-B: DNA331602, NP_060568.3, 219787_s_at
Figure 3738: PRO86604
Figure 3739: DNA255822, NP_036346.1, 219797_at
Figure 3740: PRO50877
Figure 3741: DNA227305, NP_064564.1, 219806_s_at
Figure 3742: PRO37768
Figure 3743: DNA329303, NP_054737.1, 219819_s_at
Figure 3744: PRO84892
Figure 3745: DNA287295, NP_078784.1, 219836_at
Figure 3746: PRO69564
Figure 3747: DNA287234, NP_114174.1, 219862_s_at
Figure 3748: PRO69513
Figure 3749: DNA287221, NP_057407.1, 219863_at
Figure 3750: PRO69500
Figure 3751: DNA330419, NP_038469.1, 219864_s_at
Figure 3752: PRO85624
Figure 3753: DNA255255, LOC64116, 219869_s_at
Figure 3754: PRO50332
Figure 3755: DNA330420, NP_078890.1, 219871_at
Figure 3756: PRO85625
Figure 3757: DNA256325, NP_005470.1, 219889_at
Figure 3758: PRO51367
Figure 3759: DNA330421, NP_057438.2, 219911_s_at
Figure 3760: PRO85626
Figure 3761A-B: DNA330422, NP_057736.2, 219913_s_at
Figure 3762: PRO85627
Figure 3763: DNA227787, NP_060606.1, 219918_s_at
Figure 3764: PRO38250
Figure 3765: DNA330423, NP_037466.2, 219920_s_at
Figure 3766: PRO85628

Figure 3767A-B: DNA330424, LTBP3, 219922_s_at
Figure 3768: PRO85629
Figure 3769: DNA328924, NP_057150.2, 219933_at
Figure 3770: PRO84641
Figure 3771: DNA218280, NP_068570.1, 219971_at
Figure 3772: PRO34332
Figure 3773: DNA325979, NP_060924.4, 219978_s_at
Figure 3774: PRO82424
Figure 3775: DNA330425, NP_078956.1, 219990_at
Figure 3776: PRO85630
Figure 3777A-B: DNA330426, SGKL, 220038_at
Figure 3778: PRO85631
Figure 3779: DNA328926, NP_064703.1, 220046_s_at
Figure 3780: PRO84643
Figure 3781A-B: DNA218680, NP_071731.1, 220048_at
Figure 3782: PRO21724
Figure 3783: DNA330427, NP_036593.1, 220052_s_at
Figure 3784: PRO85632
Figure 3785: DNA330428, NP_060385.1, 220060_s_at
Figure 3786: PRO85633
Figure 3787: DNA330537, NP_060533.2, 220085_at
Figure 3788: PRO81892
Figure 3789: DNA256091, NP_071385.1, 220094_s_at
Figure 3790: PRO51141
Figure 3791: DNA330430, NP_078945.1, 220112_at
Figure 3792: PRO85634
Figure 3793: DNA330431, NP_055198.1, 220118_at
Figure 3794: PRO85635
Figure 3795: DNA227302, NP_037401.1, 220132_s_at
Figure 3796: PRO37765
Figure 3797: DNA330432, NP_079219.1, 220169_at
Figure 3798: PRO85636
Figure 3799: DNA331603, TMPRSS3, 220177_s_at
Figure 3800: PRO83482
Figure 3801: DNA256291, NP_079182.1, 220232_at
Figure 3802: PRO51335
Figure 3803: DNA330434, NP_060842.1, 220235_s_at
Figure 3804: PRO85637
Figure 3805: DNA330435, NP_060179.1, 220306_at
Figure 3806: PRO85638
Figure 3807: DNA330436, NP_037394.1, 220319_s_at
Figure 3808: PRO85639
Figure 3809: DNA327904, NP_071419.2, 220330_s_at
Figure 3810: PRO83839
Figure 3811: DNA287186, NP_061134.1, 220358_at
Figure 3812: PRO69472
Figure 3813A-B: DNA330437, NP_079366.1, 220370_s_at
Figure 3814: PRO85640
Figure 3815: DNA330438, NP_061026.1, 220485_s_at
Figure 3816: PRO50795
Figure 3817: DNA327214, NP_078991.2, 220495_s_at
Figure 3818: PRO83483
Figure 3819: DNA324252, NP_060444.1, 220521.s_at
Figure 3820: PRO80936
Figure 3821: DNA331604, PHEMX, 220558_x_at
Figure 3822: PRO86605
Figure 3823: DNA256363, NP_057686.1, 220565_at
Figure 3824: PRO51405

Figure 3825: DNA255798, NP_079265.1, 220576_at
Figure 3826: PRO50853
Figure 3827: DNA330440, NP_079098.1, 220591_s_at
Figure 3828: PRO85642
Figure 3829: DNA255734, NP_057607.1, 220646_s_at
Figure 3830: PRO50791
Figure 3831A-B: DNA327908, MCM10, 220651_s_at
Figure 3832: PRO83843
Figure 3833: DNA329306, NP_079149.2, 220655_at
Figure 3834: PRO84895
Figure 3835A-B: DNA327909, ARNTL2, 220658_s_at
Figure 3836: PRO83844
Figure 3837: DNA329307, NP_037483.1, 220684_at
Figure 3838: PRO84896
Figure 3839: DNA323756, NP_057267.2, 220688_s_at
Figure 3840: PRO80512
Figure 3841: DNA331380, DKFZp566O084Homo, 220690_s_at
Figure 3842: DNA330442, NP_054866.1, 220692_at
Figure 3843: PRO85643
Figure 3844: DNA330443, NP_061086.1, 220702_at
Figure 3845: PRO85644
Figure 3846: DNA288247, NP_478059.1, 220892_s_at
Figure 3847: PRO70011
Figure 3848: DNA327916, NP_079466.1, 220940_at
Figure 3849: PRO83851
Figure 3850: DNA327953, NP_055182.2, 220942_x_at
Figure 3851: PRO83878
Figure 3852: DNA327917, NP_112240.1, 220966_x_at
Figure 3853: PRO83852
Figure 3854: DNA329078, VMP1, 220990_s_at
Figure 3855: PRO23253
Figure 3856A-B: DNA254516, NP_112196.1, 220992_s_at
Figure 3857: PRO49623
Figure 3858: DNA330444, NP_110405.1, 220999_s_at
Figure 3859: PRO85645
Figure 3860: DNA324246, NP_112188.1, 221004._s_at
Figure 3861: PRO80930
Figure 3862: DNA330445, NP_112174.1, 221012_s_at
Figure 3863: PRO85646
Figure 3864A-B: DNA254816, NP_110444.1, 221031_s_at
Figure 3865: PRO49912
Figure 3866: DNA330446, NP_054889.1, 221046_s_at
Figure 3867: PRO85647
Figure 3868: DNA330447, NP_079174.1, 221080_s_at
Figure 3869: PRO85648
Figure 3870: DNA226227, NP_060872.1, 221111_at
Figure 3871: PRO36690
Figure 3872: DNA227267, NP_061130.1, 221123_x_at
Figure 3873: PRO37730
Figure 3874: DNA217256, NP_065386.1, 221165_s_at
Figure 3875: PRO34298
Figure 3876: DNA329310, AK027224, 221185_s_at
Figure 3877: PRO84899
Figure 3878: DNA324408, NP_060493.2, 221203_s_at
Figure 3879: PRO81072
Figure 3880A-B: DNA330448, NP_059111.1, 221221_s_at
Figure 3881: PRO85649
Figure 3882: DNA331605, CISH, 221223_x_at

Figure 3883: PRO86458
Figure 3884: DNA330450, AK025947, 221235_s_at
Figure 3885: PRO85651
Figure 3886: DNA330451, NP_110429.1, 221249_s_at
Figure 3887: PRO85652
Figure 3888: DNA330452, NP-1112494.2, 221258_s_at
Figure 3889: PRO85653
Figure 3890: DNA295327, NP_068575.1, 221271_at
Figure 3891: PRO70773
Figure 3892: DNA330453, NP-1112597.1, 221277_s_at
Figure 3893: PRO85654
Figure 3894: DNA329312, NP_005205.2, 221331_x_at
Figure 3895: PRO84901
Figure 3896: DNA288250, NP_112487.1, 221434_s_at
Figure 3897: PRO70013
Figure 3898: DNA330454, NP_112589.1, 221436_s_at
Figure 3899: PRO85655
Figure 3900: DNA330455, 1097190.16, 221477_s_at
Figure 3901: PRO85656
Figure 3902: DNA150865, NP_057005.1, 221488_s_at
Figure 3903: PRO11587
Figure 3904: DNA272972, NP_057356.1, 221496_s_at
Figure 3905: PRO61052
Figure 3906A-B: DNA329316, AF158555, 221510-s-at
Figure 3907: PRO84904
Figure 3908: DNA330456, NP_060571.1, 221520_s_at
Figure 3909: PRO85657
Figure 3910: DNA326221, NP_057179.1, 221521_s_at
Figure 3911: PRO82634
Figure 3912: DNA328953, NP_004086.1, 221539_at
Figure 3913: PRO70296
Figure 3914: DNA329317, AF288571, 221558_s_at
Figure 3915: PRO81157
Figure 3916: DNA330457, NP_076944.1, 221559_s_at
Figure 3917: PRO85658
Figure 3918: DNA329319, BC006401, 221601_s_at
Figure 3919: PRO1607
Figure 3920: DNA329319, NP_005440.1, 221602_s_at
Figure 3921: PRO1607
Figure 3922: DNA254308, NP_060950.1, 221622_s_at
Figure 3923: PRO49419
Figure 3924: DNA287254, NP_077004.1, 221637_s_at
Figure 3925: PRO69528
Figure 3926: DNA330458, NP_060634.1, 221652_s_at
Figure 3927: PRO85659
Figure 3928: DNA218280, IL21R, 221658_s_at
Figure 3929: PRO34332
Figure 3930: DNA327927, NP-037390.2, 221666_s_at
Figure 3931: PRO57311
Figure 3932: DNA254777, NP_055140.1, 221676_s_at
Figure 3933: PRO49875
Figure 3934: DNA330459, NP_060083.1, 221677_s_at
Figure 3935: PRO50083
Figure 3936: DNA330460, NP_060255.2, 221685_s_at
Figure 3937: PRO85660
Figure 3938: DNA273185, DNA273185, 221727_at
Figure 3939: DNA330461, BC005104, 221732_at
Figure 3940: DNA328961, NP_443112.1, 221756_at

Figure 3941: PRO84667
Figure 3942: DNA328961, MGC17330, 221757_at
Figure 3943: PRO84667
Figure 3944: DNA330462, NP_060103.1, 221766_s_at
Figure 3945: PRO85661
Figure 3946: DNA193901, DNA193901, 221768_at
Figure 3947: PRO23319
Figure 3948: DNA328964, AK056028, 221770_at
Figure 3949: PRO84669
Figure 3950: DNA330463, HSM801191, 221790_s_at
Figure 3951A-B: DNA151745, DNA151745, 221805_at
Figure 3952: PRO12033
Figure 3953: DNA274058, NP_057203.1, 221816_s_at
Figure 3954: PRO61999
Figure 3955: DNA325039, NP_004902.1, 221824_s_at
Figure 3956: PRO2733
Figure 3957: DNA273311, NP_003022.1, 221833_at
Figure 3958: PRO61319
Figure 3959: DNA272419, AF105036, 221841_s_at
Figure 3960: PRO60672
Figure 3961: DNA330464, NP_067082.1, 221882_s_at
Figure 3962: PRO85663
Figure 3963A-B: DNA330465, 253695.2, 221916_at
Figure 3964: PRO85664
Figure 3965A-B: DNA330466, AB018304, 221922_at
Figure 3966: DNA329321, NP_112493.1, 221931_s_at
Figure 3967: PRO84906
Figure 3968: DNA330467, NP_060114.1, 221986_s_at
Figure 3969: PRO85665
Figure 3970: DNA287235, FLJ10534, 221987_s_at
Figure 3971: PRO69514
Figure 3972: DNA327114, RPL10, 221989_at
Figure 3973: PRO62466
Figure 3974: DNA331606, BC018529, 222017_x_at
Figure 3975: PRO86606
Figure 3976: DNA257797, DNA257797, 222036_s_at
Figure 3977: DNA257798, DNA257798, 222037_at
Figure 3978: DNA330468, 1454101.4, 222044_at
Figure 3979: PRO85666
Figure 3980: DNA329919, BC013365, 222045_s_at
Figure 3981: PRO85234
Figure 3982: DNA304466, NP_004834.1, 222062_at
Figure 3983: PRO34336
Figure 3984: DNA325648, NP_037409.2, 2220777_s_at
Figure 3985: PRO82139
Figure 3986: DNA331386, HST000012, 222150_s_at
Figure 3987: DNA287209, NP_056350.1, 222154_s_at
Figure 3988: PRO69490
Figure 3989: DNA256784, NP_075069.1, 222209_s_at
Figure 3990: PRO51716
Figure 3991: DNA328977, NP_071344.1, 222216_s_at
Figure 3992: PRO84678
Figure 3993: DNA330469, NP_056249.1, 222250_s_at
Figure 3994: PRO85667
Figure 3995: DNA328885, EKI1, 222262_s_at
Figure 3996: PRO50294
Figure 3997: DNA330470, 096828.1, 222307_at
Figure 3998: PRO85668

Figure 3999: DNA330471, 027307.1, 222309_at
Figure 4000: PRO85669
Figure 4001: DNA330472, 128864.1, 222326_at
Figure 4002: PRO85670
Figure 4003: DNA330473, NP_060676.2, 222387_s_at
Figure 4004: PRO85671
Figure 4005: DNA330474, AF186382, 222388_s_at
Figure 4006: PRO85672
Figure 4007: DNA331607, HSA251830, 222392_x_at
Figure 4008: PRO86607
Figure 4009A-B: DNA270901, NP_004238.1, 222398_s_at
Figure 4010: PRO59235
Figure 4011: DNA325821, BC014334, 222402_at
Figure 4012: PRO82287
Figure 4013: DNA227358, HSPC121, 222404_x_at
Figure 4014: PRO37821
Figure 4015: DNA330476, AK027421, 222405_at
Figure 4016: PRO85674
Figure 4017: DNA328819, CGI-127, 222408_s_at
Figure 4018: PRO84557
Figure 4019: DNA331608, SNX5, 222417_s_at
Figure 4020: PRO69560
Figure 4021: DNA326307, NP_056399.1, 222425_s_at
Figure 4022: PRO82707
Figure 4023: DNA227223, GK001, 222432_s_at
Figure 4024: PRO37686
Figure 4025A-B: DNA329326, NP_005110.1, 222439_s_at
Figure 4026: PRO84910
Figure 4027: DNA327939, NP_060654.1, 222442_s_at
Figure 4028: PRO83869
Figure 4029: DNA329327, AF198620, 222443_s_at
Figure 4030: PRO84911
Figure 4031A-B: DNA256489, NP_079110.1, 222464_s_at
Figure 4032: PRO51526
Figure 4033A-B: DNA225648, ERBB2IP, 222473_s_at
Figure 4034: PRO36111
Figure 4035: DNA304460, BC003048, 222500_at
Figure 4036: PRO4984
Figure 4037: DNA330477, NP_036227.1, 222516_at
Figure 4038: PRO37979
Figure 4039: DNA329328, NP_067026.2, 222532_at
Figure 4040: PRO84912
Figure 4041: DNA16435, DNA16435, 222543_at
Figure 4042: PRO276
Figure 4043: DNA330478, NP_056978.2, 222557_at
Figure 4044: PRO85675
Figure 4045A-B: DNA330479, 900264.1, 222572_at
Figure 4046: PRO85676
Figure 4047: DNA330480, NP_060697.2, 222600_s_at
Figure 4048: PRO85677
Figure 4049A-B : DNA330481, AB058718, 222603_at
Figure 4050: DNA330482, AK027468, 222606-at
Figure 4051: PRO85678
Figure 4052: DNA330483, AK001472, 222608_s_at
Figure 4053: PRO85679
Figure 4054: DNA329330, NP_057130.1, 222609_s_at
Figure 4055: PRO84914
Figure 4056A-B: DNA331609, 402471.3, 222613_at

Figure 4057: PRO86608
Figure 4058: DNA330485, NP_057415.1, 222624_s_at
Figure 4059: PRO85681
Figure 4060: DNA327942, NP_060596.1, 222642-s_at
Figure 4061: PRO83870
Figure 4062: DNA327943, NP_055399.1, 222646_s_at
Figure 4063: PRO865
Figure 4064A-B: DNA273435, RAMP, 222680_s_at
Figure 4065: PRO61430
Figure 4066: DNA330486, HSM802473, 222692_s_at
Figure 4067: DNA330487, AB052751, 222696_at
Figure 4068: DNA330488, AK025568, 222704_at
Figure 4069: PRO85682
Figure 4070: DNA272874, NP_057111.1, 222714_s_at
Figure 4071: PRO60967
Figure 4072: DNA275116, DNA275116, 222726_s_at
Figure 4073: DNA330489, BC019909, 222740_at
Figure 4074: PRO85683
Figure 4075: DNA330490, 399171.38, 222754_at
Figure 4076: PRO85684
Figure 4077: DNA330491, BC002522, 222759_at
Figure 4078: PRO85685
Figure 4079A-B: DNA330492, FLJ11294, 222763-s-at
Figure 4080: PRO85686
Figure 4081: DNA329332, LOC51605, 222768_s_at
Figure 4082: PRO84916
Figure 4083: DNA330493, AK025248, 222770_s_at
Figure 4084: PRO85687
Figure 4085: DNA304780, NETO2, 222774_s_at
Figure 4086: PRO69889
Figure 4087: DNA330494, BC020651, 222775_s_art
Figure 4088: PRO85688
Figure 4089: DNA330495, NP_060468.1, 222781_s_at
Figure 4090: PRO85689
Figure 4091: DNA330496, HSM802366, 222793_at
Figure 4092: DNA330395, FLJ20281, 222816_s_at
Figure 4093: PRO85603
Figure 4094A-B: DNA331610, TBDN100, 222837_s_at
Figure 4095: PRO86609
Figure 4096: DNA330881, AB027233, 222838_at
Figure 4097: PRO1138
Figure 4098: DNA329335, AK023411, 222843 -at
Figure 4099: PRO84919
Figure 4100: DNA273489, NP_055210.1, 222858_s_at
Figure 4101: PRO61472
Figure 4102: DNA273489, DAPP1, 222859_s_at
Figure 4103: PRO61472
Figure 4104: DNA330498, NP_036225.1, 222862_s_at
Figure 4105: PRO85691
Figure 4106: DNA329336, NP-057144.1, 222867_s_at
Figure 4107: PRO84920
Figure 4108: DNA287404, FLJ22833, 222872_x_at
Figure 4109: PRO69661
Figure 4110: DNA330499, AK026944, 222875_at
Figure 4111: PRO85692
Figure 4112: DNA330500, AK022872, 222889_at
Figure 4113: PRO85693
Figure 4114A-C: DNA330409, HSA404614, 222895_s_at

Figure 4115: PRO85617
Figure 4116: DNA330501, AK022792,222958_s_at
Figure 4117: PRO85694
Figure 4118A-B: DNA330502, AB042719, 222962_s_at
Figure 4119: PRO85695
Figure 4120: DNA329337, AF279437, 222974_at
Figure 4121: PRO10096
Figure 4122: DNA329338, 459502.10, 222977_at
Figure 4123: PRO84921
Figure 4124A-B: DNA329339, 459502.5, 222978_at
Figure 4125: PRO84922
Figure 4126: DNA329340, AF078866, 222979_s_at
Figure 4127: PRO81805
Figure 4128: DNA152786, NP_057215.1, 222980_at
Figure 4129: PRO10928
Figure 4130: DNA152786, RAB 10, 222981_s_at
Figure 4131: PRO10928
Figure 4132A-B: DNA287236, AB024334, 222985_at
Figure 4133: PRO10607
Figure 4134: DNA330503, NP_038466.2, 222989_s_at
Figure 4135: PRO85696
Figure 4136: DNA331611, UBQLN1, 222991_s_at
Figure 4137: PRO86610
Figure 4138: DNA330504, NP_057575.2, 222993_at
Figure 4139: PRO84923
Figure 4140: DNA329571, NP_057547.3, 222996_s_at
Figure 4141: PRO51662
Figure 4142: DNA326195, NP_054781.1, 223018_at
Figure 4143: PRO82611
Figure 4144: DNA330505, BC005937, 223021_x_at
Figure 4145: PRO85697
Figure 4146A-B: DNA329342, AF172847, 223027_at
Figure 4147: PRO84924
Figure 4148: DNA329344, FRSB, 223035_s_at
Figure 4149: PRO84926
Figure 4150: DNA330506, NP_067061.1, 223038_s_at
Figure 4151: PRO82123
Figure 4152: DNA330507, AK054681, 223039_at
Figure 4153: PRO85698
Figure 4154: DNA287260, NP_057184.1, 223040_at
Figure 4155: PRO69532
Figure 4156: DNA324198, HSM801908, 223044_at
Figure 4157: PRO37675
Figure 4158: DNA330508, AF116694, 223047_at
Figure 4159: PRO85699
Figure 4160: DNA189412, NP_057390.1, 223054_at
Figure 4161: PRO25349
Figure 4162A-B: DNA256347, AF298880, 223055_s_at
Figure 4163: PRO51389
Figure 4164A-B : DNA329345, AB033117, 223056_s_at
Figure 4165:DNA327948, NP_060394.1, 223060_at
Figure 4166: PRO69660
Figure 4167: DNA288247, PSA, 223062_s_at
Figure 4168: PRO70011
Figure 4169: DNA330509, AK024555, 223066-at
Figure 4170: PRO80652
Figure 4171: DNA227294, NP_060225.1, 223076_s_at
Figure 4172: PRO37757

Figure 4173: DNA331612, AF097492, 223079_s_at
Figure 4174: PRO86611
Figure 4175: DNA326258, NP_077273.1, 223081_at
Figure 4176: PRO82665
Figure 4177: DNA329346, AK027070, 223085_at
Figure 4178: PRO84928
Figure 4179: DNA327949, NP_057581.2, 223086_x_at
Figure 4180: PRO83874
Figure 4181: DNA331613, 238178.17, 223087_at
Figure 4182: PRO86612
Figure 4183: DNA329347, NP_060949.1, 223088_x_at
Figure 4184: PRO84929
Figure 4185: DNA330511, AK001338, 223090_x_at
Figure 4186: PRO85701
Figure 4187: DNA324209, NP_057018.1, 223096_at
Figure 4188: PRO80902
Figure 4189: DNA329349, NP_054861.1, 223100_s_at
Figure 4190: PRO84931
Figure 4191: DNA327917, MGC3038, 223101_s_at
Figure 4192: PRO83852
Figure 4193: DNA330512, NP_056494.1, 223109_at
Figure 4194: PRO85702
Figure 4195: DNA330436, MIR, 223129_x_at
Figure 4196: PRO85639
Figure 4197: DNA330513, AF212221, 223130_s_at
Figure 4198: PRO85703
Figure 4199A-: DNA330514, DDX36, 223138_s_at
Figure 4200: PRO85704
Figure 4201A-: DNA330514, AF217190, 223139_s_at
Figure 4202: PRO85704
Figure 4203: DNA325557, NP_115675.1, 223151_at
Figure 4204: PRO82060
Figure 4205: DNA329352, NP_057154.2, 223156_at
Figure 4206: PRO84932
Figure 4207: DNA329353, NP_113665.1, 223179_at
Figure 4208: PRO84933
Figure 4209: DNA254276, NP_054896.1, 223180_s_at
Figure 4210: PRO49387
Figure 4211: DNA327953, E2IG5, 223193_x_at
Figure 4212: PRO83878
Figure 4213A-B: DNA281444, NP_064544.1, 223197_s_at
Figure 4214: PRO66283
Figure 4215: DNA304467, NP_115703.1, 223212_at
Figure 4216: PRO71043
Figure 4217: DNA227267, LOC55893, 223216_x_at
Figure 4218: PRO37730
Figure 4219: DNA327954, NP_113646.1, 223220_s_at
Figure 4220: PRO83879
Figure 4221: DNA330515, NP_004580.1, 223221_at
Figure 4222: PRO85705
Figure 4223: DNA329321, SEC13L, 223225_s_at
Figure 4224: PRO84906
Figure 4225: DNA247474, NP_054895.1, 223229_at
Figure 4226: PRO44999
Figure 4227: DNA330516, AK000796, 223239_art
Figure 4228: PRO85706
Figure 4229: DNA287171, NP_036312.1, 223240_at
Figure 4230: PRO69462

Figure 4231: DNA324046, NP_1115700.1, 223272_s_at
Figure 4232: PRO80763
Figure 4233: DNA330517, NP_115879.1, 223273_at
Figure 4234: PRO85707
Figure 4235: DNA330518, BC002493, 223274_at
Figure 4236: PRO85708
Figure 4237: DNA330519, NP_060607.1, 223275_at
Figure 4238: PRO85709
Figure 4239: DNA330520, NP-005777.2, 223283_s_at
Figure 4240: PRO85710
Figure 4241: DNA330521, BC002762,223286_at
Figure 4242: PRO85711
Figure 4243A-B: DNA330522, AF250920, 223287_s_at
Figure 4244: PRO85712
Figure 4245: DNA330523, BC001220, 223294-at
Figure 4246: PRO85713
Figure 4247: DNA330524, MGC4268, 223297_at
Figure 4248: PRO85714
Figure 4249: DNA329356, NP_115671.1, 223304_at
Figure 4250: PRO84935
Figure 4251: DNA330454, BC002551, 223307_at
Figure 4252: PRO85655
Figure 4253: DNA330526, NP_115682.1, 223318_s_at
Figure 4254: PRO34564
Figure 4255A-B: DNA330527, AF272663, 223319_at
Figure 4256: PRO85716
Figure 4257: DNA329358, NP_115649.1, 223334-at
Figure 4258: PRO84937
Figure 4259: DNA330528, AF151063, 223335_at
Figure 4260: PRO50764
Figure 4261: DNA330529, 241399.1, 223343_at
Figure 4262: PRO85717
Figure 4263A-B: DNA255756, HUMPDE7A, 223358_s_at
Figure 4264: PRO5081
Figure 4265: DNA227125, AF132297, 223377_x_at
Figure 4266: PRO37588
Figure 4267: DNA331614, CDCA1, 223381_at
Figure 4268: PRO38881
Figure 4269A-B: DNA329360, NP_115644.1, 223382_s_at
Figure 4270: PRO84939
Figure 4271A-B: DNA329360, NIN283, 223383_at
Figure 4272: PRO84939
Figure 4273: DNA330531, NP_037508.1, 223394_at
Figure 4274: PRO85718
Figure 4275: DNA329361, AF161528, 223397_s_at
Figure 4276: PRO84940
Figure 4277: DNA324156, NP-111558.1, 223403-s-at
Figure 4278: PRO80856
Figure 4279A-B: DNA254516, Clorf25, 223404_s_at
Figure 4280: PRO49623
Figure 4281: DNA256407, NP_055188.1, 223423_at
Figure 4282: PRO51448
Figure 4283: DNA255676, HSM801648, 223434_at
Figure 4284: PRO50738
Figure 4285: DNA330532, NP_078804.1, 223439_at
Figure 4286: PRO85719
Figure 4287: DNA330533, NP_058647.1, 223451_s_at
Figure 4288: PRO772

Figure 4289: DNA329365, CAB56010.1, 223452_8_at
Figure 4290: PRO84944
Figure 4291: DNA327958, NP-111789.1, 223484_at
Figure 4292: PRO23554
Figure 4293: DNA329456, NP_057126.1, 223489xat
Figure 4294: PRO85023
Figure 4295: DNA329456, RRP40, 223490_s_at
Figure 4296: PRO85023
Figure 4297: DNA330534, AF307332, 223494_at
Figure 4298: PRO85720
Figure 4299: DNA304784, NP_006564.1, 223502-s-at.
Figure 4300: PRO738
Figure 4301: DNA330535, NP-115883.1, 223506_at
Figure 4302: PRO85721
Figure 4303: DNA330536, NP_115666.1, 223542_at
Figure 4304: PRO85722
Figure 4305: DNA330537, AF155827, 223556_at
Figure 4306: PRO81892
Figure 4307A-B: DNA327908, HSM801808, 223570-at
Figure 4308: PRO83843
Figure 4309: DNA330538, AF262027, 223598_at
Figure 4310: PRO85723
Figure 4311: DNA330539, NP-055411.1, 223639-s-at
Figure 4312: PRO85724
Figure 4313: DNA330540, NP_055081.1, 223640_at
Figure 4314: PRO85725
Figure 4315: DNA330541, AF277625, 223675_s_at
Figure 4316: PRO85726
Figure 4317: DNA330542, NP_115493.1, 223700-at
Figure 4318: PRO85727
Figure 4319: DNA330543, NAG73, 223725_at
Figure 4320: PRO85728
Figure 4321: DNA329367, TTYH2, 223741_s_at
Figure 4322: PRO84946
Figure 4323: DNA331615, AB049635, 223743_s_at
Figure 4324: PRO62669
Figure 4325: DNA188735, NP_001506.1, 223758_s_at
Figure 4326: PRO26224
Figure 4327: DNA287253, LOC85028, 223774_at
Figure 4328: PRO69527
Figure 4329: DNA331616, BC004277, 223785_at
Figure 4330: PRO86613
Figure 4331: DNA330544, NP277049.1, 223800_s_at
Figure 4332: PRO85729
Figure 4333: DNA256005, NP_004842.1, 223806_s_at
Figure 4334: PRO51056
Figure 4335: DNA330545, AF233516, 223834_at
Figure 4336: PRO70906
Figure 4337: DNA327200, NP_114156.1, 223836_at
Figure 4338: PRO1065
Figure 4339: DNA330546, AF132203, 223839_s_at
Figure 4340: PRO85730
Figure 4341: DNA330547, NP_066014.1, 223849_s_at
Figure 4342: PRO85731
Figure 4343: DNA331392, NP_004186.1, 223851_s_at
Figure 4344: PRO0364
Figure 4345: DNA330548, NP_115590.1, 223880_x_at
Figure 4346: PRO85732

Figure 4347A-B: DNA330522, FOXP1, 223936_s_at
Figure 4348: PRO85712
Figure 4349A-B: DNA330550, HSM801744, 223946_at
Figure 4350: PRO85734
Figure 4351: DNA331393, D83532, 22396_s_at
Figure 4352: PRO86458
Figure 4353: DNA324248, SP110, 223980_s_at
Figure 4354: PRO80932
Figure 4355: DNA330551, BC009946, 223983_s_at
Figure 4356: PRO85735
Figure 4357: DNA330552, BC001104, 223984_s_at
Figure 4358: PRO85736
Figure 4359: DNA328847, NP_056338.1, 223989_s_at
Figure 4360: PRO84579
Figure 4361: DNA331617, AF332652, 224046_s_at
Figure 4362: PRO86614
Figure 4363: DNA329369, AF293026, 224130_s_at
Figure 4364: PRO84948
Figure 4365: DNA330553, AF116653, 224148_at
Figure 4366: DNA331618, AF231339, 224204_x_at
Figure 4367: PRO86615
Figure 4368: DNA330554, AF277993, 224211_at
Figure 4369: PRO85737
Figure 4370A-C: DNA227619, NP_054831.1, 224218_s_at
Figure 4371: PRO38082
Figure 4372: DNA324707, NP_037369.1, 224232_s_at
Figure 4373: PRO81339
Figure 4374: DNA323935, NP_060586.1, 224233_s_at
Figure 4375: PRO80668
Figure 4376: DNA329370, NP_060611.2, 224247_s_at
Figure 4377: PRO84949
Figure 4378A-B: DNA330555, HSM801768, 224308_s_at
Figure 4379: PRO85738
Figure 4380: DNA330556, NP_061881.2, 224319_s_at
Figure 4381: PRO85739
Figure 4382: DNA330557, C20orf154, 224320.s_at
Figure 4383: PRO85740
Figure 4384: DNA330558, NP_057588.1, 224330_s_at
Figure 4385: PRO84950
Figure 4386: DNA327949, MRP64, 224334_s_at
Figure 4387: PRO83874
Figure 4388A-B: DNA330559, BAB21791.1, 224336_s_at
Figure 4389: PRO85741
Figure 4390: DNA331619, BC010896, 224345_x_at
Figure 4391: PRO86616
Figure 4392: DNA331620, NDRG3, 224368_s_at
Figure 4393: PRO86617
Figure 4394: DNA272626, RIP5, 224376_s_at
Figure 4395: PRO60759
Figure 4396: DNA330560, NP_510882.1, 224413_s_at
Figure 4397: PRO85742
Figure 4398: DNA330561, AF321617, 224416_s_at
Figure 4399: PRO85743
Figure 4400: DNA328323, NP_114148.2, 224428_s_at
Figure 4401: PRO69531
Figure 4402: DNA331621, AF060225, 224437_s_at
Figure 4403: PRO86618
Figure 4404: DNA330562, NP_115716.1, 224448_s_at

Figure 4405: PRO85744
Figure 4406: DNA330563, NP_113668.1, 224450_s_at
Figure 4407: PRO85745
Figure 4408: DNA330564, NP_115885.1, 224451_x_at
Figure 4409: PRO85746
Figure 4410: DNA330565, BC006111, 224454_at
Figure 4411: PRO85747
Figure 4412: DNA330566, NP_115720.1, 224464_s_at
Figure 4413: PRO85748
Figure 4414: DNA329373, NP_115722.1, 224467_s_at
Figure 4415: PRO84952
Figure 4416: DNA330567, NP_116114.1, 224504_s_at
Figure 4417: PRO85749
Figure 4418: DNA327976, NP_116120.1, 224511_s_at
Figure 4419: PRO69574
Figure 4420: DNA330568, BC006428, 224516_s_at
Figure 4421: PRO85750
Figure 4422: DNA329374, NP_1115735.1, 224523_s_at
Figure 4423: PRO84953
Figure 4424: DNA331622, TNFRSF18, 224553_s_at
Figure 4425: PRO86619
Figure 4426: DNA330569, BC020516, 224572_s_at
Figure 4427A-B: DNA330570, AB040903, 224578_at
Figure 4428: DNA330571, AK027320, 224607_s_at
Figure 4429: PRO85752
Figure 4430: DNA327980, BC008959, 224615_x_at
Figure 4431: PRO83900
Figure 4432: DNA329376, BAA91036.1, 224632_at
Figure 4433: PRO84954
Figure 4434: DNA330572, CAB82324.1, 224648_at
Figure 4435: PRO85753
Figure 4436A-B: DNA327981, 344095.3, 224654_at
Figure 4437: PRO83901
Figure 4438: DNA330573, C20orf108, 224690_at
Figure 4439: PRO85754
Figure 4440A-B: DNA330574, AB033054, 224698_at
Figure 4441: DNA330575, AK022542, 224701_at
Figure 4442: PRO85756
Figure 4443: DNA331623, BC014138, 224711_at
Figure 4444: PRO86620
Figure 4445: DNA329378, BC022990, 224713_at
Figure 4446: PRO8495
Figure 4447: DNA324173, NP-115766.2, 224714_at
Figure 4448: PRO80871
Figure 4449: DNA330577, NP_443076.1, 224715_at
Figure 4450: PRO85758
Figure 4451A-B: DNA330578, 1353105.1, 224718_at
Figure 4452: PRO85759
Figure 4453: DNA330579, BC009925, 224719_s_at
Figure 4454: PRO85760
Figure 4455: DNA287382, 1383817.3, 224738_x_at
Figure 4456: PRO69641
Figure 4457: DNA257352, DNA257352, 224739_at
Figure 4458: PRO51940
Figure 4459: DNA331624, BC014242, 224740_at
Figure 4460: PRO86621
Figure 4461: DNA330581, MGC16386, 224753_at
Figure 4462: PRO82014

Figure 4463: DNA330582, 1454377.6, 224755_at
Figure 4464: PRO85762
Figure 4465: DNA330583, BC020522, 224759_s_at
Figure 4466: PRO85763
Figure 4467A-B: DNA287330, BAA86479.1, 224799_at
Figure 4468: PRO69594
Figure 4469A-B: DNA330584, FENS-1, 224800_at
Figure 4470: PRO85764
Figure 4471: DNA331397, AK001723, 224802_at
Figure 4472: PRO23259
Figure 4473: DNA330585, 206983.10, 224806_at
Figure 4474: PRO85765
Figure 4475: DNA330586, NP_443183.1, 224825_at
Figure 4476: PRO85766
Figure 4477A-B: DNA330559, AB051487, 224832_at
Figure 4478A-B: DNA330809, 336997.1, 224838_at
Figure 4479: PRO85973
Figure 4480A-B: DNA330587, 1045521.4,
Figure 4481: PRO85767
Figure 4482: DNA329380, BC014868, 224855_at
Figure 4483: PRO80743
Figure 4484: DNA330588, BC019034, 224871_at
Figure 4485: PRO8576
Figure 4486: DNA196374, DNA196374, 224880_at
Figure 4487A-B: DNA331625, 411236.19, 224897_at
Figure 4488: PRO86622
Figure 4489: DNA329382, BC009072, 2249133_s_at
Figure 4490: DNA330590, CIP29, 224914_s_at
Figure 4491: PRO85770
Figure 4492A-B: DNA169523, DNA169523, 224917_at
Figure 4493: PRO23253
Figure 4494: DNA330591, NP-1115865.1, 224919_at
Figure 4495: PRO85771
Figure 4496: DNA330592, AB014733, 224953_at
Figure 4497: DNA287258, C20orf52, 224972_art
Figure 4498: PRO52174
Figure 4499: DNA151170, DNA151170, 224989_at
Figure 4500: PRO12626
Figure 4501: DNA330593, HS126A53, 225005_at
Figure 4502A-B: DNA329385, 330826.1, 225010_at
Figure 4503: PRO84961
Figure 4504: DNA161646, DNA161646, 225036_at
Figure 4505: DNA330594, BC005148, 225039_at
Figure 4506: DNA331626, 412293.2, 225047_at
Figure 4507: PRO86623
Figure 4508: DNA195755, DNA195755, 225051_at
Figure 4509A-B: DNA330596, 998535.1, 225070_at
Figure 4510: PRO85774
Figure 4511A-C: DNA271612, BAA92642.1, 225076_s_at
Figure 4512: PRO59899
Figure 4513: DNA330597, NP_057291.1, 225082_at
Figure 4514: PRO85775
Figure 4515: DNA330598, 1384569.2, 225086_at
Figure 4516: PRO85776
Figure 4517: DNA330599, 898528.3, 225095_at
Figure 4518: PRO85777
Figure 4519: DNA330600, HSA272196, 225096_at
Figure 4520: PRO85778

Figure 4521: DNA330601, 1322727.6, 225098_at
Figure 4522: PRO85779
Figure 4523: DNA331627, BC013920, 225105_at
Figure 4524: PRO86624
Figure 4525: DNA225597, NP_060703.1, 225106_s_at
Figure 4526: PRO36060
Figure 4527A-B: DNA331628, 245994.3, 225113_at
Figure 4528: PRO86625
Figure 4529A-B: DNA327993, 898436.7, 225133_at
Figure 4530: PRO81138
Figure 4531: DNA155396, DNA155396, 225143_at
Figure 4532: DNA330603, 235138.16, 225157_at
Figure 4533: 224839_s_at PRO85781
Figure 4534: DNA329393, NP_079272.4, 225158_at
Figure 4535: PRO84969
Figure 4536: DNA329393, EFG1, 225161_at
Figure 4537: PRO84969
Figure 4538: DNA330604, NP_277050.1, 225171_at
Figure 4539: PRO85782
Figure 4540: DNA327996, BC010181, 225195_at
Figure 4541: PRO83915
Figure 4542: DNA199601, DNA199601, 225199_at
Figure 4543: DNA329394, BC010416, 225201_s_at
Figure 4544: DNA329396, NP_060866.1, 225253_s_at
Figure 4545: PRO84972
Figure 4546: DNA329397, NP_114109.1, 225260_s_at
Figure 4547: PRO84973
Figure 4548A-B: DNA329398, 411135.13, 225262_at
Figure 4549: PRO4805
Figure 4550A-B: DNA258863, DNA258863, 225266_at
Figure 4551A-B: DNA331629, 233102.7, 225269_s_at
Figure 4552: PRO86626
Figure 4553A-B: DNA330606, 475590.1, 225290_at
Figure 4554: PRO85784
Figure 4555: DNA329400, BC005986, 225291_at
Figure 4556: DNA326458, BC014003, 225297_at
Figure 4557: PRO82841
Figure 4558: DNA330607, 167391.12, 225300_at
Figure 4559: PRO85785
Figure 4560: DNA330608, BC016880, 225323_at
Figure 4561: PRO85786
Figure 4562A-B: DNA169918, DNA169918, 225340_s_at
Figure 4563: PRO23256
Figure 4564: DNA330609, AF419331, 225348_at
Figure 4565: PRO22196
Figure 4566: DNA327965, NP_060760.1, 225367_at
Figure 4567: PRO83888
Figure 4568: DNA273635, HSM801117,225371_at
Figure 4569A-B: DNA330610, BAB15739.1, 225372_at
Figure 4570: PRO85787
Figure 4571: DNA331630, BC020568, 225373_at
Figure 4572: PRO86627
Figure 4573A-B: DNA331631, 1383781.5, 225385_s_at
Figure 4574: PRO86628
Figure 4575: DNA329401, BC017480, 225386_s_at
Figure 4576: PRO84976
Figure 4577: DNA329402, 198708.7, 225387_at
Figure 4578: PRO4845

EP 2 179 742 A1

Figure 4579: DNA329403, AF288394, 225399_at
Figure 4580: DNA331632, BC022030, 225400_at
Figure 4581: PRO86629
Figure 4582: DNA330612, C20orf64, 225402_at
Figure 4583: PRO85789
Figure 4584A-B: DNA328893, BC020490, 225406_at
Figure 4585: PRO9914
Figure 4586: DNA330613, BC019355, 225414_at
Figure 4587A-B: DNA331633, 1449606.5, 225433_at
Figure 4588: PRO86630
Figure 4589: DNA304802, AAH00967.1, 225439_at
Figure 4590: PRO71212
Figure 4591: DNA328005, BC004413, 225440_at
Figure 4592: DNA330615, NP_115732.1, 225441_x_at
Figure 4593: PRO85791
Figure 4594: DNA330616, 429555.1, 225443_at
Figure 4595: PRO85792
Figure 4596A-B: DNA330617, 336147.2, 225447_at
Figure 4597: PRO59923
Figure 4598: DNA329404, BC013949, 225454_at
Figure 4599: PRO82972
Figure 4600: DNA330618, CAB55990.1, 225457_s_at
Figure 4601: PRO85793
Figure 4602: DNA330618, HSM801081, 225458_at
Figure 4603: DNA196561, DNA196561, 225470_at
Figure 4604: DNA329405, HSM800962, 225520_at
Figure 4605A-B: DNA330619, BC013128, 225527_at
Figure 4606: PRO12186
Figure 4607A-B: DNA330620, CAB55950.1, 225533_at
Figure 4608: PRO85794
Figure 4609: DNA330621, AF116628, 225535_s_at
Figure 4610: DNA328008, 240051.4, 225541_at
Figure 4611: PRO83926
Figure 4612A-B: DNA330622, 233388.8, 225543_at
Figure 4613: PRO85796
Figure 4614: DNA330623, 1502854.5, 225549_at
Figure 4615: PRO85797
Figure 4616: DNA329406, 1503139.10, 225562_at
Figure 4617: PRO84979
Figure 4618: DNA330624, AK000500, 225580-at
Figure 4619: PRO85798
Figure 4620: DNA330625, AK025643, 225581_s_at
Figure 4621: PRO85799
Figure 4622: DNA304469, NP_149078.1, 225621_at
Figure 4623: PRO71045
Figure 4624: DNA151667, DNA151667, 225634-at
Figure 4625: PRO11970
Figure 4626: DNA330626, 1398905.1, 225638_at
Figure 4627: PRO85800
Figure 4628: DNA331634, CTSC, 225647_s_at
Figure 4629: PRO86631
Figure 4630A-B: DNA288261, NP_037414.2, 225655_at
Figure 4631: PRO70021
Figure 4632: DNA329408, NP_056235.2, 225676_s_at
Figure 4633: PRO38893
Figure 4634A-B: DNA330627, 987725.3, 225679_at
Figure 4635: PRO85801
Figure 4636: DNA329409, BC017248, 225682_s_at

87

EP 2 179 742 A1

Figure 4637: PRO84981
Figure 4638: DNA325272, NP_054891.1, 225683_x_at
Figure 4639: PRO81822
Figure 4640: DNA328012, BC017873, 225686_at
Figure 4641: PRO83930
Figure 4642: DNA328013, AAH01068.1, 225687_at
Figure 4643: PRO83931
Figure 4644A-C: DNA330628, 1400234.13, 225690_at
Figure 4645: PRO85802
Figure 4646A-B: DNA330629, BAA74856.2, 225692-at
Figure 4647: PRO50227
Figure 4543: DNA329394, BC010416, 225201_s_at
Figure 4544: DNA329396, NP_060866.1, 225253_s_at
Figure 4545: PRO84972
Figure 4546: DNA329397, NP_114109.1, 225260_s_at
Figure 4547: PRO84973
Figure 4548A-B: DNA329398, 411135.13, 225262_at
Figure 4549: PRO4805
Figure 4550A-B: DNA258863, DNA258863, 225266_at
Figure 4551A-B: DNA331629, 233102.7, 225269_s_at
Figure 4552: PRO86626
Figure 4553A-B: DNA330606, 475590.1, 225290_at
Figure 4554: PRO85784
Figure 4555: DNA329400, BC005986, 225291_at
Figure 4556: DNA326458, BC014003, 225297_at
Figure 4557: PRO82841
Figure 4558: DNA330607, 167391.12, 225300_at
Figure 4559: PRO85785
Figure 4560: DNA330608, BC016880, 225323_at
Figure 4561: PRO85786
Figure 4562A-B: DNA169918, DNA169918, 225340_s_at
Figure 4563: PRO23256
Figure 4564: DNA330609, AF419331, 225348_at
Figure 4565: PRO22196
Figure 4566: DNA327965, NP_060760.1, 225367_at
Figure 4567: PRO83888
Figure 4568: DNA273635, HSM801117,225371_at
Figure 4569A-B: DNA330610, BAB15739.1, 225372_at
Figure 4570: PRO85787
Figure 4571: DNA331630, BC020568, 225373_at
Figure 4572: PRO86627
Figure 4573A-B: DNA331631, 1383781.5, 225385_s_at
Figure 4574: PRO86628
Figure 4575: DNA329401, BC017480, 225386_s_at
Figure 4576: PRO84976
Figure 4577: DNA329402, 198708.7, 225387_at
Figure 4578: PRO4845
Figure 4579: DNA329403, AF288394, 225399_at
Figure 4580: DNA331632, BC022030, 225400_at
Figure 4581: PRO86629
Figure 4582: DNA330612, C20orf64, 225402_at
Figure 4583: PRO85789
Figure 4584A-B: DNA328893, BC020490, 225406_at
Figure 4585: PRO9914
Figure 4586: DNA330613, BC019355, 225414_at
Figure 4587A-B: DNA331633, 1449606.5, 225433_at
Figure 4588: PRO86630
Figure 4589: DNA304802, AAH00967.1, 225439_at

Figure 4590: PRO71212
Figure 4591: DNA328005, BC004413, 225440_at
Figure 4592: DNA330615, NP_115732.1, 225441_x_at
Figure 4593: PRO85791
Figure 4594: DNA330616, 429555.1, 225443_at
Figure 4595: PRO85792
Figure 4596A-B: DNA330617, 336147.2, 225447_at
Figure 4597: PRO59923
Figure 4598: DNA329404, BC013949, 225454_at
Figure 4599: PRO82972
Figure 4600: DNA330618, CAB55990.1, 225457_s_at
Figure 4601: PRO85793
Figure 4602: DNA330618, HSM801081, 225458_at
Figure 4603: DNA196561, DNA196561, 225470_at
Figure 4604: DNA329405, HSM800962, 225520_at
Figure 4605A-B: DNA330619, BC013128, 225527_at
Figure 4606: PRO12186
Figure 4607A-B: DNA330620, CAB55950.1, 225533_at
Figure 4608: PRO85794
Figure 4609: DNA330621, AF116628, 225535_s_at
Figure 4610: DNA328008, 240051.4, 225541_at
Figure 4611: PRO83926
Figure 4612A-B: DNA330622, 233388.8, 225543_at
Figure 4613: PRO85796
Figure 4614: DNA330623, 1502854.5, 225549_at
Figure 4615: PRO85797
Figure 4616: DNA329406, 1503139.10, 225562_at
Figure 4617: PRO84979
Figure 4618: DNA330624, AK000500, 225580-at
Figure 4619: PRO85798
Figure 4620: DNA330625, AK025643, 225581_s_at
Figure 4621: PRO85799
Figure 4622: DNA304469, NP_149078.1, 225621_at
Figure 4623: PRO71045
Figure 4624: DNA151667, DNA151667, 225634-at
Figure 4625: PRO11970
Figure 4626: DNA330626, 1398905.1, 225638_at
Figure 4627: PRO85800
Figure 4628: DNA331634, CTSC, 225647_s_at
Figure 4629: PRO86631
Figure 4630A-B: DNA288261, NP_037414.2, 225655_at
Figure 4631: PRO70021
Figure 4632: DNA329408, NP_056235.2, 225676_s_at
Figure 4633: PRO38893
Figure 4634A-B: DNA330627, 987725.3, 225679_at
Figure 4635: PRO85801
Figure 4636: DNA329409, BC017248, 225682_s_at
Figure 4637: PRO84981
Figure 4638: DNA325272, NP_054891.1, 225683_x_at
Figure 4639: PRO81822
Figure 4640: DNA328012, BC017873, 225686_at
Figure 4641: PRO83930
Figure 4642: DNA328013, AAH01068.1, 225687_at
Figure 4643: PRO83931
Figure 4644A-C: DNA330628, 1400234.13, 225690_at
Figure 4645: PRO85802
Figure 4646A-B: DNA330629, BAA74856.2, 225692-at
Figure 4647: PRO50227

Figure 4648: DNA273623, AY037153, 225693_s_at
Figure 4649: PRO61596
Figure 4650: DNA330630, TIGA1, 225698_at
Figure 4651: PRO85803
Figure 4652A-B: DNA331635, BAB13371.1, 225704_at
Figure 4653: PRO86632
Figure 4654: DNA331636, 221395.1, 225716_at
Figure 4655: PRO86633
Figure 4656: DNA330633, BC003515, 225723_at
Figure 4657A-B: DNA330634, 243208.1, 225725_at
Figure 4658: PRO85806
Figure 4659A-B: DNA330635, 233691.4, 225736_at
Figure 4660: PRO85807
Figure 4661: DNA324266, NP_056268.1, 225741_at
Figure 4662: PRO80949
Figure 4663: DNA323970, MGC21854, 225763_at
Figure 4664: PRO80699
Figure 4665: DNA331637, 7693984.1, 225768_at
Figure 4666: PRO86634
Figure 4667: DNA330636, NP_201575.2, 225794_s_at
Figure 4668: PRO85808
Figure 4669: DNA330636, LOC91689, 225795_at
Figure 4670: PRO85808
Figure 4671: DNA329414, MGC4677, 225799_at
Figure 4672: PRO84986
Figure 4673: DNA330637, NP_478136.1, 225803_at
Figure 4674: PRO85809
Figure 4675A-C: DNA330638, CAB63749.1, 225814_at
Figure 4676: PRO85810
Figure 4677: DNA330639, 420605.8, 225834_at
Figure 4678: PRO85811
Figure 4679: DNA329417, 411336.1, 225842_at
Figure 4680: PRO84989
Figure 4681: DNA287622, AF041429, 225849_s_at
Figure 4682: DNA329418, BC018969, 225850_at
Figure 4683: PRO19906
Figure 4684: DNA287370, BAB14983.1, 225866_at
Figure 4685: PRO69630
Figure 4686A-B: DNA331638, 1097910.3, 225886_at
Figure 4687: PRO86635
Figure 4688A-B: DNA331639, 1391157.25, 225888_at
Figure 4689: PRO86636
Figure 4690: DNA330642, NP_115494.1, 225898_at
Figure 4691: PRO85814
Figure 4692A-B: DNA331640, 481415.9, 225927_at
Figure 4693: PRO86637
Figure 4694A-B: DNA255887, BAB13380.1, 225929_s_at
Figure 4695: PRO50940
Figure 4696A-B: DNA255887, AB046774,
Figure 4697A-B: DNA330644, 236657.4, 225935_at
Figure 4698: PRO85816
Figure 4699: DNA331641, AK027752, 225959_s_at
Figure 4700: PRO86638
Figure 4701A-B: DNA329360, AF378524, 225962_at
Figure 4702: PRO84939
Figure 4703: DNA329420, BC018014, 225970_at
Figure 4704A-B: DNA330645, 350385.2, 225973_at
Figure 4705: PRO85817

Figure 4706A-B: DNA329421, 343552.1, 225974_at
Figure 4707: PRO84992
Figure 4708A-B: DNA331642, BAB15719.1, 225979_at
Figure 4709: PRO86639
Figure 4710: DNA330647, AK002174, 226001_at
Figure 4711: PRO85819
Figure 4712: DNA330648, 1399123.1, 226005_at
Figure 4713: PRO85820
Figure 4714: DNA330649, AK056957, 226008_at
Figure 4715: PRO85821
Figure 4716A-B: DNA331643, 246054.6, 226021_at
Figure 4717: PRO86640
Figure 4718: DNA331644, 027830.2, 226034_at
Figure 4719: PRO86641
Figure 4720: DNA328021, BC004538, 226038_at
Figure 4721: DNA273736, DNA273736, 226040_at
Figure 4722: DNA330652, CLONE24945, 226055_at
Figure 4723: PRO85824
Figure 4724: DNA330653, 7687670.2, 226068 -at
Figure 4725: PRO85825
Figure 4726: DNA304795, AK056513, 226077_at
Figure 4727: PRO71207
Figure 4728A-B: DNA331645, AAD09327.1, 226082_s_at
Figure 4729: PRO86642
Figure 4730: DNA287271, NP_1116188.2, 226088_at
Figure 4731: PRO69542
Figure 4732: DNA330655, AF114264, 226103_at
Figure 4733: PRO85827
Figure 4734A-B: DNA330656, AK023825, 226109_at
Figure 4735: PRO85828
Figure 4736: DNA329425, BC008294, 226117_at
Figure 4737: DNA330657, 198409.1, 226140_s_at
Figure 4738: PRO85829
Figure 4739: DNA330658, 204262.3, 226157_at
Figure 4740: PRO85830
Figure 4741: DNA330659, AF289605, 226175_at
Figure 4742: PRO85831
Figure 4743A-B: DNA330660, 979126.7, 226178_at
Figure 4744: PRO85832
Figure 4745A-B: DNA259025, DNA259025, 226180_at
Figure 4746: PRO52958
Figure 4747: DNA56350, DNA56350, 226181_at
Figure 4748: 225931_s_at PRO956
Figure 4749: DNA330661, BAB47431.1, 226194_at
Figure 4750: PRO85833
Figure 4751A-B: DNA329428, 1446144.8, 226218_at
Figure 4752: PRO84999
Figure 4753: DNA195822, DNA195822, 226241_s_at
Figure 4754A-B: DNA330662, 334156.1, 226252_at
Figure 4755: PRO85834
Figure 4756: DNA331646, 956845.3, 226261_at
Figure 4757: PRO86643
Figure 4758A-B: DNA330664, 400637.4, 226265_at
Figure 4759: PRO85836
Figure 4760A-B: DNA330665, 233070.3, 226270_at
Figure 4761: PRO85837
Figure 4762: DNA330666, 199829.14, 226272_at
Figure 4763: PRO85838

EP 2 179 742 A1

Figure 4764: DNA193896, DNA193896, 226276_at
Figure 4765: PRO23314
Figure 4766: DNA330667, AF301222, 226287_at
Figure 4767: DNA330668, BC010176, 226308_at
Figure 4768: PRO85840
Figure 4769: DNA328028, NP_005773.1, 226319_s_at
Figure 4770: PRO83945
Figure 4771: DNA328028, ALY, 226320_at
Figure 4772: PRO83945
Figure 4773: DNA330669, 236903.4, 226321 -at
Figure 4774: PRO85841
Figure 4775: DNA330670, BC018453, 226329_s_at
Figure 4776: PRO85842
Figure 4777A-B: DNA330671, 228447.18, 226342_at
Figure 4778: PRO85843
Figure 4779: DNA330672, 255309.4, 226347_at
Figure 4780: PRO85844
Figure 4781: DNA330673, 236879.2, 226348_at
Figure 4782: PRO85845
Figure 4783: DNA329430, NP_116191.2, 226353_at
Figure 4784: PRO38524
Figure 4785: DNA331647, 236137.11, 226354_at
Figure 4786: PRO86644
Figure 4787A-B: DNA330675, 177663.2, 226368_at
Figure 4788: PRO85847
Figure 4789: DNA330676, CAA11393.1, 226388_at
Figure 4790: PRO85848
Figure 4791: DNA330677, 1384190.6, 226390_at
Figure 4792: PRO85849
Figure 4793: DNA151740, DNA151740, 226419_s_at
Figure 4794: PRO12029
Figure 4795: DNA329433, NP-1115937.1, 226442-at
Figure 4796: PRO85003
Figure 4797: DNA330678, 401430.1, 226444-at
Figure 4798: PRO85850
Figure 4799: DNA287657, BC009447, 226448_at
Figure 4800: PRO69688
Figure 4801: DNA330679, BC013040, 226456_at
Figure 4802A-B: DNA330680, BC022792, 226477_at
Figure 4803: PRO85852
Figure 4804: DNA326066, NP_291022.1, 226488_at
Figure 4805: PRO82501
Figure 4806A-B: DNA330681, 971066.5, 226503_at
Figure 4807: PRO85853
Figure 4808: DNA330682, HSM801031, 226510_at
Figure 4809: DNA304794, NP_1115521.2, 226541_at
Figure 4810: PRO71206
Figure 4811: DNA330683, 1446727.8, 226546_at
Figure 4812: PRO85854
Figure 4813: DNA330684, 984114.1, 226548_at
Figure 4814: PRO85855
Figure 4815A-B: DNA328031, 331264.1, 226587_at
Figure 4816: PRO83948
Figure 4817: DNA330685, BAB13430.1, 226588_at
Figure 4818: PRO85856
Figure 4819A-B: DNA330686, 1502531.18, 226602_s_at
Figure 4820: PRO85857
Figure 4821: DNA328033, 1446419.1, 226625_at

92

Figure 4822: PRO83949
Figure 4823: DNA330687, 215158.5, 226650_at
Figure 4824: PRO85858
Figure 4825: DNA258913, DNA258913, 226661_at
Figure 4826: PRO52846
Figure 4827A-C: DNA328462, HSA303079, 226694-at
Figure 4828: PRO84288
Figure 4829: DNA328037, BC016969, 226702_at
Figure 4830: DNA330688, 240121.1, 226725_at
Figure 4831: PRO85859
Figure 4832A-C: DNA330689, 978733.6, 226732_at
Figure 4833: PRO85860
Figure 4834: DNA257914, DNA257914, 226743_at
Figure 4835: PRO52447
Figure 4836: DNA330690, 245065.1, 226745_at
Figure 4837: PRO85861
Figure 4838: DNA330691, BC022075, 226748_at
Figure 4839: PRO85862
Figure 4840: DNA329435, 347092.10, 226750_at
Figure 4841: PRO85005
Figure 4842: DNA331648, 243999.3, 226757_at
Figure 4843: PRO86645
Figure 4844: DNA330692, 1446140.1, 226758_at
Figure 4845: PRO85863
Figure 4846A-B: DNA330331, AB032963, 226771_at
Figure 4847: DNA330693, HSBRN1H12, 226773_at
Figure 4848A-B: DNA330694, 481455.4, 226810_at
Figure 4849: PRO85865
Figure 4850: DNA330695, 404167.9, 226818_at
Figure 4851: PRO85866
Figure 4852A-C: DNA330696, 404167.10, 226841_at
Figure 4853: PRO85867
Figure 4854: DNA330697, BC011808, 226858_at
Figure 4855: PRO85868
Figure 4856A-B: DNA329436, 236863.1, 226869_at
Figure 4857: PRO85006
Figure 4858: DNA330698, BC020852, 226896_at
Figure 4859: PRO85869
Figure 4860: DNA329437, 156503.10, 226901_at
Figure 4861: PRO85007
Figure 4862: DNA330699, BC014203, 226905_at
Figure 4863: DNA330564, ARHGAP9, 226906_s_at
Figure 4864: PRO85746
Figure 4865: DNA327917, BC000798, 226915_s_at
Figure 4866: PRO83852
Figure 4867A-C: DNA331649, 201042.4, 226921_at
Figure 4868: PRO86646
Figure 4869: DNA328044, 039170.3, 226936_at
Figure 4870: PRO83958
Figure 4871: DNA151713, DNA151713, 226943_at
Figure 4872: PRO12003
Figure 4873: DNA330701, NP_115652.1, 226945_at
Figure 4874: PRO85872
Figure 4875: DNA330702, 023085.2, 226965_at
Figure 4876: PRO85873
Figure 4877: DNA330703, 201413.1, 226970_at
Figure 4878: PRO85874
Figure 4879: DNA154627, DNA154627, 226976_at

Figure 4880: DNA330704, BC019075, 226980_at
Figure 4881: PRO85875
Figure 4882A-B: DNA331650, HSA314788, 226998_at
Figure 4883: PRO85008
Figure 4884: DNA329439, HSM802614, 227014_at
Figure 4885A-B: DNA330705, 198782.1, 227020_at
Figure 4886: PRO85876
Figure 4887A-B: DNA330706, AF445027, 227027_at
Figure 4888: PRO85877
Figure 4889: DNA330707, 028375.3, 227044_at
Figure 4890: PRO85878
Figure 4891: DNA330708, 1101317.1, 227066_at
Figure 4892: PRO85879
Figure 4893: DNA329440, 7691797.1, 227068_at
Figure 4894: PRO85009
Figure 4895: DNA330709, 7692923.1, 227117_at
Figure 4896: PRO85880
Figure 4897: DNA323785, NP_116261.1, 227134_at
Figure 4898: PRO80537
Figure 4899: DNA330710, 331040.11, 227135_at
Figure 4900: PRO85881
Figure 4901A-B: DNA330711, 425448.18, 227150_at
Figure 4902: PRO85882
Figure 4903: DNA330712, 1452648.12, 227167_s_at
Figure 4904: PRO85883
Figure 4905: DNA328281, BC000282, 227172_art
Figure 4906: DNA330713, 334485.4, 227187_at
Figure 4907: PRO85884
Figure 4908: DNA330714, 034544.1, 227198_at
Figure 4909: PRO85885
Figure 4910: DNA330715, BC022374, 227211_at
Figure 4911: PRO85886
Figure 4912A-B: DNA330620, HSM800990, 227212_s_art
Figure 4913: DNA330716, BC021675, 227236_at
Figure 4914: PRO85887
Figure 4915: DNA251633, AK023151, 227245_at
Figure 4916: PRO47694
Figure 4917: DNA327206, AY037161, 227262_at
Figure 4918: PRO271
Figure 4919A-B: DNA329442, AH007300S2,227265_at
Figure 4920A-B: DNA331651, 099572.12, 227266_s_at
Figure 4921: PRO86647
Figure 4922: DNA330717, 232831.10, 227290_at
Figure 4923: PRO85888
Figure 4924: DNA329445, 001839.3, 227291_s_at
Figure 4925: PRO85013
Figure 4926: DNA330718, 025465.3, 227295_at
Figure 4927: PRO85889
Figure 4928: DNA330719, 7697121.1, 227307_at
Figure 4929: PRO85890
Figure 4930: DNA330720, 186766.12, 227337_at
Figure 4931: PRO85891
Figure 4932: DNA35664, DNA35664, 227345_at
Figure 4933: PRO34697
Figure 4934A-B: DNA330721, 198680.1, 227350_at
Figure 4935: PRO85892
Figure 4936: DNA226872, NP_001955.1, 227404_s_at
Figure 4937: PRO37335

Figure 4938A-B: DNA330722, AB058729, 227418_at
Figure 4939: DNA330723, AB040960, 227438_at
Figure 4940: DNA329447, BC016981, 227449_at
Figure 4941: PRO85015
Figure 4942: DNA330724, AK056677, 227450_at
Figure 4943: PRO1575
Figure 4944A-B: DNA328054, 233014.1, 227458_at
Figure 4945: PRO83968
Figure 4946A-B: DNA258781, DNA258781, 227466_at
Figure 4947: PRO52715
Figure 4948: DNA329448, BC012948, 227477_at
Figure 4949: PRO85016
Figure 4950: DNA329053, LOC51105, 227523_s_at
Figure 4951: PRO84715
Figure 4952: DNA330725, 337360.7, 227545_at
Figure 4953: PRO85894
Figure 4954: DNA330726, BC014967, 227558_at
Figure 4955: PRO85895
Figure 4956A-B: DNA331652, 1447357.3, 227572_at
Figure 4957: PRO86648
Figure 4958: DNA330728, HSM801012, 227580_s_at
Figure 4959: PRO85897
Figure 4960: DNA330729, 031130.2, 227600_at
Figure 4961: PRO85898
Figure 4962Av: DNA287193, AB037794, 227606_s_at
Figure 4963: DNA330730, BC010846, 227607_at
Figure 4964: PRO85899
Figure 4965: DNA257714, NP_150280.1, 227609_at
Figure 4966: PRO52268
Figure 4967: DNA330731, BC012337, 227614_at
Figure 4968: DNA196237, DNA196237, 227616_at
Figure 4969A-B: DNA330426, AF085233, 227627_at
Figure 4970: PRO85631
Figure 4971A-B: DNA330733, BAA92631.1, 227653_at
Figure 4972: PRO85902
Figure 4973: DNA329449, 979180.1, 227682_at
Figure 4974: PRO85017
Figure 4975: DNA330734, BC008322, 227686_at
Figure 4976: PRO85903
Figure 4977: DNA273987, DNA273987, 227708_at
Figure 4978: DNA330735, 1400830.3, 227722_at
Figure 4979: PRO85904
Figure 4980: DNA329450, BC017226, 227726_at
Figure 4981: PRO85018
Figure 4982A-B: DNA330736, BAA86532.1, 227732-at
Figure 4983: PRO85905
Figure 4984A-B: DNA330737, 331100.8, 227766_at
Figure 4985: PRO85906
Figure 4986: DNA331653, 212641.1,227792_at
Figure 4987: PRO86649
Figure 4988: DNA151733, DNA151733, 227807_at
Figure 4989: PRO12022
Figure 4990A-B: DNA330739, AK000004, 227811_at
Figure 4991: DNA329454, BC022534, 227856_at
Figure 4992: PRO85022
Figure 4993: DNA260485, DNA260485, 227867_at
Figure 4994: PRO54411
Figure 4995: DNA327214, AK056512, 227873_at

EP 2 179 742 A1

Figure 4996: PRO83483
Figure 4997: DNA151503, DNA151503, 227877_at
Figure 4998: PRO11849
Figure 4999: DNA329481, NP-057234.2, 227915_at
Figure 5000: PRO60949
Figure 5001: DNA329456, AF151860, 227916_x_at
Figure 5002: PRO85023
Figure 5003A-B: DNA330740, AY028320S2, 227928_at
Figure 5004: DNA151580, DNA151580, 227930_at
Figure 5005: PRO11901
Figure 5006: DNA330741, 350868.1, 227952_at
Figure 5007: PRO85909
Figure 5008A-B: DNA331654, 476805.1, 228006-at
Figure 5009: PRO86650
Figure 5010: DNA330539, ZNRD1, 228009_x_at
Figure 5011: PRO85724
Figure 5012: DNA150660, NP_057151.1, 228019_s_at
Figure 5013: PRO12397
Figure 5014A-B: DNA328432, NP_005768.1, 228030_art
Figure 5015: PRO61793
Figure 5016: DNA331655, 1449874.3, 228053_s_at
Figure 5017: PRO86651
Figure 5018: DNA330743, AK054689, 228062_at
Figure 5019: PRO85911
Figure 5020: DNA331656, 244771.1, 228063_s_at
Figure 5021: PRO86652
Figure 5022: DNA331657, Blur1, 228065_at
Figure 5023: PRO23970
Figure 5024: DNA329459, 230998.1, 228066_at
Figure 5025: PRO85026
Figure 5026: DNA330745, BC011716, 228069_at
Figure 5027: PRO85913
Figure 5028: DNA196216, DNA196216, 228071_at
Figure 5029: DNA329460, BC017117, 228092_at
Figure 5030: PRO85027
Figure 5031: DNA330746, 346395.7, 228097_at
Figure 5032: PRO85914
Figure 5033: DNA330436, AF187016, 228098_s_at
Figure 5034: PRO85639
Figure 5035A-C: DNA331658, 200650.1, 228109_at
Figure 5036: PRO86653
Figure 5037: DNA329461, BC016615, 228113_at
Figure 5038: PRO85028
Figure 5039: DNA330748, 224725.3, 228159_at
Figure 5040: PRO85916
Figure 5041: DNA330749, 337382.1, 228174_at
Figure 5042: PRO85917
Figure 5043: DNA330750, 984920.1, 228180_at
Figure 5044: PRO85918
Figure 5045: DNA153924, DNA153924, 228188_at
Figure 5046: DNA330751, 334282.2, 228189_at
Figure 5047: PRO85919
Figure 5048: DNA330752, 7694335.3, 22819_at
Figure 5049: PRO85920
Figure 5050A-B: DNA331659, 198497.1, 228201_at
Figure 5051: PRO86654
Figure 5052A-C: DNA328072, AB051556, 228230_at
Figure 5053: DNA330754, 349978.1, 228242_at

96

Figure 5054: PRO85922
Figure 5055: DNA328663, CGI-142, 228266_s_at
Figure 5056: PRO36183
Figure 5057: DNA260948, DNA260948, 228273_at
Figure 5058: PRO54700
Figure 5059: DNA330755, BC020784, 228280_at
Figure 5060: PRO85923
Figure 5061: DNA331660, 230589.4, 228281_at
Figure 5062: PRO86655
Figure 5063A-B: DNA330757, AB046790, 228323_at
Figure 5064: DNA304814, BC016879, 228330_at
Figure 5065: PRO52650
Figure 5066: DNA194202, DNA194202, 228370-at
Figure 5067: PRO23594
Figure 5068: DNA330758, 238545.7, 228381_at
Figure 5069: PRO85925
Figure 5070: DNA330759, 337444.1, 228390_at
Figure 5071: PRO85926
Figure 5072A-B: DNA330760, 330900.8, 228401_at
Figure 5073: PRO85927
Figure 5074: DNA228118, DNA228118, 228456_s_at
Figure 5075: DNA297188, NP_1116233.1, 228468_at
Figure 5076: PRO70805
Figure 5077A-C: DNA331661, 388991.1, 228487_s_at
Figure 5078: PRO86656
Figure 5079: DNA330762, BC010269, 228499_at
Figure 5080: PRO80989
Figure 5081: DNA195938, DNA195938, 228531_at
Figure 5082: DNA329463, 412954.5, 228532_at
Figure 5083: PRO85030
Figure 5084: DNA330763, 1306177.32, 228549_at
Figure 5085: PRO85929
Figure 5086: DNA331662, 1450017.11, 228559_at
Figure 5087: PRO86657
Figure 5088A-C: DNA331663, 475198.1, 228562_at
Figure 5089: PRO86658
Figure 5090: DNA330766, 977419.7, 228597_at
Figure 5091: PRO85932
Figure 5092A-B: DNA331664, 201954.14, 228603_at
Figure 5093: PRO86659
Figure 5094: DNA328079, 239903.1, 228617_at
Figure 5095: PRO83991
Figure 5096: DNA330768, NP_003681.1, 228620_at
Figure 5097: PRO60565
Figure 5098A-B: DNA271477, NP_055774.1, 228641_at
Figure 5099: PRO59770
Figure 5100: DNA330769, 230457.1, 228664_at
Figure 5101: PRO85934
Figure 5102: DNA330770, 1447329.13, 228702_at
Figure 5103: PRO85935
Figure 5104: DNA330771, 1447928.4, 228710_at
Figure 5105: PRO85936
Figure 5106: DNA330772, 286623.2, 228729_at
Figure 5107: PRO85937
Figure 5108: DNA330773, 027401.1, 228758_at
Figure 5109: PRO85938
Figure 5110: DNA330774, 024160.1, 228760_at
Figure 5111: PRO85939

Figure 5112: DNA273232, DNA273232, 228785_at
Figure 5113: DNA330775, 407523.3, 228806-at
Figure 5114: PRO85940
Figure 5115: DNA330776, NP_005740.1, 228834_at
Figure 5116: PRO58014
Figure 5117: DNA256483, HSM802155, 228859_at
Figure 5118: PRO51520
Figure 5119: DNA331665, 330848.1, 228869_at
Figure 5120: PRO86660
Figure 5121: DNA330778, 998621.10, 228891_at
Figure 5122: PRO85942
Figure 5123: DNA328084, 236591.7, 228905_at
Figure 5124: PRO83996
Figure 5125: DNA330779, 244243.1, 228953_at
Figure 5126: PRO85943
Figure 5127: DNA330780, 335374.1, 228955_at
Figure 5128: PRO85944
Figure 5129: DNA330781, 289775.9, 228960_at
Figure 5130: PRO85945
Figure 5131A-B: DNA331666, 7684887.1, 228964_at
Figure 5132: PRO86661
Figure 5133: DNA330783, 239601.22, 228987_at
Figure 5134: PRO85947
Figure 5135: DNA330784, 233595.21, 228990_at
Figure 5136: PRO85948
Figure 5137: DNA330785, 475283.24, 228999_at
Figure 5138: PRO85949
Figure 5139: DNA330786, 233085.1, 229029_at
Figure 5140: PRO85950
Figure 5141: DNA330787, 349981.7, 229040_at
Figure 5142: PRO85951
Figure 5143: DNA330788, AF305195, 229060-at
Figure 5144: PRO85952
Figure 5145: DNA330789, 199829.13, 229064_s_at
Figure 5146: PRO85953
Figure 5147: DNA330790, NP_116133.1, 229070_at
Figure 5148: PRO85954
Figure 5149: DNA330791, 7697349.2, 229072_at
Figure 5150: PRO85955
Figure 5151: DNA330792, 983946.2, 229097_at
Figure 5152: PRO85956
Figure 5153: DNA155281, DNA155281, 229111_at
Figure 5154: DNA330793, 215114.2, 229145_at
Figure 5155: PRO85957
Figure 5156: DNA330794, 481414.8, 229202_at
Figure 5157: PRO85958
Figure 5158: DNA330795, BC017339, 229253_at
Figure 5159: PRO85959
Figure 5160: DNA265865, DNA265865, 229274_at
Figure 5161: DNA151375, DNA151375, 229327_s_at
Figure 5162: PRO11752
Figure 5163: DNA328919, FLJ22690, 229367_s_at
Figure 5164: PRO84637
Figure 5165: DNA257575, DNA257575, 229374_at
Figure 5166: DNA268708, DNA268708, 229391_s_at
Figure 5167A-C: DNA331667, 198342.3, 229394_s_at
Figure 5168: PRO86662
Figure 5169: DNA287421, 234832.1, 229437_at

Figure 5170: PRO69678
Figure 5171: DNA330797, 211332.1, 229442_at
Figure 5172: PRO85961
Figure 5173: DNA328090, 007911.2, 229450-at
Figure 5174: PRO84001
Figure 5175: DNA330798, 984597.1, 229483_at
Figure 5176: PRO85962
Figure 5177: DNA330799, 481875.1, 229551_x_at
Figure 5178: PRO85963
Figure 5179: DNA330800, AK056271, 229595_at
Figure 5180: PRO85964
Figure 5181: DNA330801, 199864.1, 229610_at
Figure 5182: PRO85965
Figure 5183: DNA327205, NP_443174.1, 229625_at
Figure 5184: PRO83478
Figure 5185A-B: DNA226321, NP_006021.1, 229636_art
Figure 5186: PRO36784
Figure 5187A-B: DNA330802, 7694410.1, 229686_at
Figure 5188: PRO85966
Figure 5189: DNA331668, 403448.4, 229699_at
Figure 5190: PRO86663
Figure 5191A-C: DNA330804, NP_055847.1, 229704-at
Figure 5192: PRO85968
Figure 5193: DNA331669, 1466538.1, 229718_at
Figure 5194: PRO86664
Figure 5195A-B: DNA227985, CBX6, 229733_s_at
Figure 5196: PRO38448
Figure 5197: DNA330806, 200298.1, 229809_at
Figure 5198: PRO85970
Figure 5199: DNA330807, 334422.1, 229814_at
Figure 5200: PRO85971
Figure 5201: DNA328092, NP_002598.2, 229830_at
Figure 5202: PRO84003
Figure 5203: DNA330808, 1397087.3, 229838_at
Figure 5204: PRO85972
Figure 5205: DNA328972, BC009950, 229872_s_at
Figure 5206A-C: DNA330810, AF330041, 229881_at
Figure 5207: PRO85974
Figure 5208: DNA287290, AK001793, 229980_s_at
Figure 5209: PRO69560
Figure 5210: DNA330811, 1382987.2, 230000_at
Figure 5211: PRO85975
Figure 5212A-B: DNA330812, 000264.19, 230021_at
Figure 5213: PRO85976
Figure 5214: DNA330813, 246201.1, 230036_art
Figure 5215: PRO85977
Figure 5216: DNA258657, DNA258657, 230060_at
Figure 5217: PRO52596
Figure 5218: DNA330814, 309641.1, 230097_at
Figure 5219: PRO85978
Figure 5220: DNA329467, 029236.1, 230110_at
Figure 5221: PRO85033
Figure 5222: DNA330815, AK057940, 230165_at
Figure 5223: PRO85979
Figure 5224: DNA329468, BC011589, 230170-at
Figure 5225: PRO88
Figure 5226: DNA330816, 980409.1, 230192_at
Figure 5227: PRO85980

Figure 5228A-B: DNA194784, DNA194784, 230218_at
Figure 5229: PRO24061
Figure 5230: DNA331670, 373719.30, 230257_s_at
Figure 5231: PRO86665
Figure 5232A-C: DNA330817, AB020335, 230265_at
Figure 5233: PRO85981
Figure 5234: DNA330818, 212282.1, 230304_at
Figure 5235: PRO85982
Figure 5236: DNA330819, 982802.1, 230337_at
Figure 5237: PRO85983
Figure 5238: DNA330820, 230585.2, 230345_at
Figure 5239: PRO85984
Figure 5240: DNA329470, NP_002756.1, 230352_at
Figure 5241: PRO85035
Figure 5242: DNA331671, 277648.15, 230375_at
Figure 5243: PRO86666
Figure 5244: DNA331672, 332195.1, 230391_at
Figure 5245: PRO86667
Figure 5246: DNA257756, DNA257756, 230405_at
Figure 5247: DNA330823, 010867.1, 230449x_at
Figure 5248: PRO85987
Figure 5249A-B: DNA331673, 333480.5, 230489_at
Figure 5250: PRO86668
Figure 5251A-B: DNA330825, 406864.4, 230526_at
Figure 5252: PRO85989
Figure 5253: DNA331674, 059446.1, 230529-at
Figure 5254: PRO86669
Figure 5255: DNA330827, NP_079521.1, 230536_at
Figure 5256: PRO85991
Figure 5257: DNA330828, 233615.1, 230566-at
Figure 5258: PRO85992
Figure 5259: DNA330829, 007717.1, 230580_at
Figure 5260: PRO85993
Figure 5261: DNA257789, NP_116219.1, 230656_s_at
Figure 5262: PRO52338
Figure 5263: DNA330830, 216899.1, 230703_at
Figure 5264: PRO85994.
Figure 5265A-B: DNA328499, SORL1, 230707-at
Figure 5266: PRO84321
Figure 5267A-B: DNA328099, 335889.1, 230779_at
Figure 5268: PRO84009
Figure 5269: DNA194391, HSM800477, 230848_s_at
Figure 5270: DNA330831, 208876.1, 230913_at
Figure 5271: PRO85995
Figure 5272: DNA330832, 253831.5, 230930_at
Figure 5273: PRO85996
Figure 5274: DNA304827, AF293462, 230966_at
Figure 5275: PRO1265
Figure 5276: DNA330833, 984179.1, 230970_at
Figure 5277: PRO85997
Figure 5278: DNA331675, BC017477, 231094_s_at
Figure 5279: PRO86670
Figure 5280: DNA331676, 980781.1, 231109_at
Figure 5281: PRO86671
Figure 5282: DNA329473, 370473.13, 231124_x_at
Figure 5283: PRO85038
Figure 5284: DNA330835, 399441.1, 231166_at
Figure 5285: PRO85999

Figure 5286A-B: DNA330836, 242968.17, 231169_at
Figure 5287: PRO86000
Figure 5288: DNA330837, 429490.1, 231182_at
Figure 5289: PRO86001
Figure 5290: DNA150808, HUMGBP1, 231577_s_at
Figure 5291: PRO12478
Figure 5292: DNA155700, DNA155700, 231579_s_at
Figure 5293: DNA330838, NP_037460.2, 231715_s_at
Figure 5294: PRO80743
Figure 5295: DNA330839, NP_060908.1, 231769_at
Figure 5296: PRO86002
Figure 5297: DNA330840, BC015355, 231772_x_at
Figure 5298: PRO86003
Figure 5299: DNA208647, DNA208647, 231775_at
Figure 5300: PRO1206
Figure 5301: DNA330841, 983019.1, 231776_at
Figure 5302: PRO86004
Figure 5303: DNA329474, AK001874, 231784_s_at
Figure 5304: PRO38893
Figure 5305: DNA330842, 242234.14, 231193_s_at
Figure 5306: PRO86005
Figure 5307: DNA329312, AF414120, 231794_at
Figure 5308: PRO84901
Figure 5309: DNA330843, 201388.1, 231832_at
Figure 5310: PRO86006
Figure 5311A-B: DNA330844, 243553.2, 231852_at
Figure 5312: PRO86007
Figure 5313: DNA330845, BC009777, 231863_at
Figure 5314: PRO86008
Figure 5315A-B: DNA330846, BC005847, 231876_at
Figure 5316: DNA331677, 981573.1, 231890_at
Figure 5317: PRO86672
Figure 5318: DNA330848, 1447268.2, 231904_at
Figure 5319: PRO86011
Figure 5320: DNA330849, EFG2, 231918_s_at
Figure 5321: PRO86012
Figure 5322A-B: DNA256267, BAB 13444.1, 231956_at
Figure 5323: PRO51311
Figure 5324A-B: DNA271768, AB037834, 231996_at
Figure 5325A-C: DNA330850, 152462.1, 232044_at
Figure 5326: PRO86013
Figure 5327: DNA330851, 337679.3, 232081_at
Figure 5328: PRO86014
Figure 5329: DNA330852, 1383611.1, 232138_at
Figure 5330: PRO86015
Figure 5331: DNA330853, 255956.19, 232141_at
Figure 5332: PRO86016
Figure 5333: DNA328113, 218535.1, 232150_at
Figure 5334: PRO84020
Figure 5335: DNA330854, AK023113, 232155_at
Figure 5336: PRO86017
Figure 5337: DNA331678, ABIN-2, 232160_s_at
Figure 5338: PRO86673
Figure 5339A-E: DNA331679, NP_112598.1, 232164_s_at
Figure 5340: PRO86674
Figure 5341: DNA330856, HSM802268, 232165_at
Figure 5342: DNA330857, 271071.1, 232175_at
Figure 5343: PRO86020

Figure 5344: DNA330858, 252659.1, 232213_at
Figure 5345: PRO86021
Figure 5346: DNA330859, 016890.1, 232216-at
Figure 5347: PRO86022
Figure 5348: DNA331680, 393520.1, 232238_at
Figure 5349: PRO86675
Figure 5350: DNA330861, AK000490, 232278_s_at
Figure 5351: PRO86024
Figure 5352: DNA287658, 199168.2, 232291_at
Figure 5353: PRO69902
Figure 5354: DNA331681, 339154.9, 232304_at
Figure 5355: PRO86676
Figure 5356: DNA330863, 295041.1, 232365_at
Figure 5357: PRO86026
Figure 5358: DNA331682, 1384413.5, 232369_at
Figure 5359: PRO86677
Figure 5360: DNA287182, 424693.25, 232375_at
Figure 5361: PRO69470
Figure 5362: DNA330865, 066613.1, 232412_at
Figure 5363: PRO86028
Figure 5364: DNA331683, 422960.1, 232504_at
Figure 5365: PRO86678
Figure 5366: DNA330867, 333565.1, 232527_at
Figure 5367: PRO86030
Figure 5368: DNA331684, AF161339, 232543_x_at
Figure 5369: DNA330868, 337037.1, 232584_at
Figure 5370: PRO86031
Figure 5371: DNA330869, 406591.1, 232687_art
Figure 5372: PRO86032
Figure 5373: DNA330870, 227719.1, 232883_at
Figure 5374: PRO86033
Figure 5375: DNA330871, 419923.1, 233019_art
Figure 5376: PRO86034
Figure 5377: DNA287404, AK026486, 233085_s_at
Figure 5378: PRO69661
Figure 5379: DNA330872, 056107.1, 233127_at
Figure 5380: PRO86035
Figure 5381A-B: DNA329422, BAA92605.1, 233208_x_at
Figure 5382: PRO84993
Figure 5383A-B: DNA330873, AB040885, 233458_at
Figure 5384: DNA330874, NP_057528.1, 233461_x_at
Figure 5385: PRO86037
Figure 5386: DNA331423, AF176071, 233467_s_at
Figure 5387: DNA330875, 006221.2, 233506-at
Figure 5388: PRO86038
Figure 5389: DNA330876, AK055587, 233528_s_at
Figure 5390: PRO86039
Figure 5391: DNA328812, AB033087, 233575_s_at
Figure 5392: DNA330877, NP_055075.1, 233588_x_at
Figure 5393: PRO86040
Figure 5394A-C: DNA330638, HSM801490, 233632_s_at
Figure 5395: DNA329287, NP_057484.2, 233746_x_at
Figure 5396: PRO84879
Figure 5397: DNA329481, ASB2, 233857_s_at
Figure 5398: PRO60949
Figure 5399: DNA326800, XRN2, 233878_s_at
Figure 5400: PRO83133
Figure 5401: DNA330547, MOV10, 233917_s_at

Figure 5402: PRO85731
Figure 5403: DNA330878, NP_079111.1, 233937_at
Figure 5404: PRO86041
Figure 5405: DNA329571, HSPC195, 233955_x_at
Figure 5406: PRO51662
Figure 5407: DNA329332, BC001262, 233970_s_at
Figure 5408: PRO84916
Figure 5409: DNA331685, AK026111, 233986_s_at
Figure 5410: PRO86680
Figure 5411: DNA331686, HSA271091, 234000_s_at
Figure 5412: PRO86681
Figure 5413: DNA331687, HUMVA25A, 234013_at
Figure 5414: PRO86682
Figure 5415: DNA330880, NP_150283.1, 234284_at
Figure 5416: PRO86043
Figure 5417: DNA330881, CRACC, 234306_s_at
Figure 5418: PRO1138
Figure 5419: DNA329312, CTLA4, 234362_s_at
Figure 5420: PRO84901
Figure 5421: DNA329483, NP_443104.1, 234408_at
Figure 5422: PRO20110
Figure 5423: DNA287425, NP_060979.1, 234464_s_at
Figure 5424: PRO69682
Figure 5425: DNA330387, FBX05, 234863_x_at
Figure 5426: PRO85596
Figure 5427: DNA304813, NP-277053.1, 234973_at
Figure 5428: PRO71222
Figure 5429: DNA330882, 406739.1, 234974_at
Figure 5430: PRO86044
Figure 5431: DNA330883, 1384547.1, 234986_at
Figure 5432: PRO86045
Figure 5433A-B: DNA330884, 267153.16, 234987_at
Figure 5434: PRO86046
Figure 5435: DNA257389, NP_1116248.1, 234993_at
Figure 5436: PRO51974
Figure 5437: DNA330885, AK055618, 235022_at
Figure 5438: PRO86047
Figure 5439: DNA331688, 404157.1, 235052_at
Figure 5440: PRO86683
Figure 5441: DNA331689, 996962.6, 235056_at
Figure 5442: PRO86684
Figure 5443: DNA328143, AK054678, 235061_at
Figure 5444: PRO84048
Figure 5445: DNA330888, 7687712.2, 235088_at
Figure 5446: PRO69581
Figure 5447: DNA330889, AK055762, 235096_at
Figure 5448: PRO86050
Figure 5449: DNA193891, DNA193891, 235099_at
Figure 5450: PRO23309
Figure 5451A-B: DNA330890, AB058722, 235106_at
Figure 5452: DNA330891, AK027315, 235113_at
Figure 5453: PRO86052
Figure 5454: DNA328146, BC019239, 235117_at
Figure 5455: PRO84051
Figure 5456: DNA330892, 198067.4, 235136_at
Figure 5457: PRO86053
Figure 5458: DNA330893, 337392.1, 235157_at
Figure 5459: PRO86054

Figure 5460: DNA329486, 1058246.1, 235175_at
Figure 5461: PRO85047
Figure 5462: DNA330894, AF455817, 235177_at
Figure 5463: PRO86055
Figure 5464: DNA331690, 200228.1, 235199_at
Figure 5465: PRO86685
Figure 5466: DNA330896, 250896.1, 235213_at
Figure 5467: PRO86057
Figure 5468: DNA329488, 1501300.6, 235244_at
Figure 5469: PRO85049
Figure 5470A-C: DNA330897, 332999.23, 235252_at
Figure 5471: PRO86058
Figure 5472: DNA324093, BC019263, 235256_s_at
Figure 5473: PRO80802
Figure 5474: DNA260946, NP-115741.1, 235266_at
Figure 5475: PRO54699
Figure 5476A-C: DNA329379, 010205.2, 235287_at
Figure 5477: PRO84957
Figure 5478: DNA330898, 227608.1, 235299_at
Figure 5479: PRO86059
Figure 5480A-B: DNA330899, 7690822.1, 235306_at
Figure 5481: PRO86060
Figure 5482A-B: DNA330900, 199492.10, 235331_x_at
Figure 5483: PRO86061
Figure 5484: DNA330901, 400258.1, 235360_at
Figure 5485: PRO86062
Figure 5486: DNA330902, 481462.4, 235389_at
Figure 5487: PRO86063
Figure 5488: DNA331691, 405045.1, 235412_at
Figure 5489: PRO86686
Figure 5490: DNA330904, 1446121.1, 235415_at
Figure 5491: PRO86065
Figure 5492: DNA331692, 979330.2, 235425_at
Figure 5493: PRO86687
Figure 5494: DNA257872, DNA257872, 235457_at
Figure 5495: DNA330906, NP-116171.2, 235458_at
Figure 5496: PRO86067
Figure 5497: DNA257302, DNA257302, 235463_s_at
Figure 5498: DNA330907, 7692322.1, 235469_at
Figure 5499: PRO86068
Figure 5500: DNA331693, 203586.1, 235508_at
Figure 5501: PRO86688
Figure 5502: DNA330909, 229234.17, 235523_at
Figure 5503: PRO86070
Figure 5504: DNA304793, NP_443173.1, 235574_at
Figure 5505: PRO71205
Figure 5506: DNA330910, 032253.1, 235581_at
Figure 5507: PRO86071
Figure 5508: DNA330911, 1446080.1, 235607_at
Figure 5509: PRO86072
Figure 5510: DNA330912, 984873.1, 235609_at
Figure 5511: PRO86073
Figure 5512: DNA331694, 222666.9, 235643_at
Figure 5513: PRO86689
Figure 5514: DNA331695, 350462.1, 235652_at
Figure 5515: PRO86690
Figure 5516: DNA330915, 238456.7, 235662_at
Figure 5517: PRO86076

Figure 5518: DNA330916, 234580.1, 235670_at
Figure 5519: PRO86077
Figure 5520: DNA330917, 238496.1, 235696_at
Figure 5521: PRO86078
Figure 5522: DNA330918, 250552.1, 235699_at
Figure 5523: PRO86079
Figure 5524: DNA330919, 423261.6, 235739_at
Figure 5525: PRO86080
Figure 5526: DNA330920, 249518.17, 235783_at
Figure 5527: PRO86081
Figure 5528: DNA330921, 246858.18, 235816_s_at
Figure 5529: PRO86082
Figure 5530: DNA330922, 1447139.1, 235907_at
Figure 5531: PRO86083
Figure 5532: DNA330923, 981520.1, 235940_at
Figure 5533: PRO86084
Figure 5534: DNA330924, 237828.3, 235984-at
Figure 5535: PRO86085
Figure 5536: DNA330925, 257787.1, 235985_at
Figure 5537: PRO86086
Figure 5538: DNA330926, 1446421.2, 236079_at
Figure 5539: PRO86087
Figure 5540: DNA330927, 1499766.1, 236156_at
Figure 5541: PRO86088
Figure 5542A-E: DNA328165, 327340.35, 236172_at
Figure 5543: PRO38220
Figure 5544: DNA330928, 227714.1, 236180_at
Figure 5545: PRO86089
Figure 5546: DNA329489, 338163.1, 236280_at
Figure 5547: PRO85050
Figure 5548: DNA257767, DNA257767, 236285_at
Figure 5549: DNA288255, 236339.1, 236295_s_at
Figure 5550: PRO70016
Figure 5551: DNA331696, 215451.1, 236338_at
Figure 5552: PRO86691
Figure 5553: DNA329490, 267918.1, 236347_at
Figure 5554: PRO85051
Figure 5555: DNA330930, 407665.1, 236379_at
Figure 5556: PRO86091
Figure 5557: DNA331697, 342862.1, 236419_at
Figure 5558: PRO86692
Figure 5559: DNA330932, 231907.4, 236470_at
Figure 5560: PRO86093
Figure 5561: DNA330933, 407881.1, 236506_at
Figure 5562: PRO86094
Figure 5563: DNA330934, 406491.1, 236595_at
Figure 5564: PRO86095
Figure 5565: DNA330935, 229915.1, 236610_at
Figure 5566: PRO86096
Figure 5567A-C: DNA330936, 351043.1, 236641_at
Figure 5568: PRO86097
Figure 5569: DNA330937, 205124.1, 236645_at
Figure 5570: PRO86098
Figure 5571: DNA330938, 7688127.1, 236668_at
Figure 5572: PRO86099
Figure 5573: DNA259749, DNA259749, 236782-at
Figure 5574: DNA329491, 211743.1, 236787_at
Figure 5575: PRO85052

Figure 5576: DNA330939, 214517.1, 236796_at
Figure 5577: PRO86100
Figure 5578: DNA330940, 211136.19, 236832_at
Figure 5579: PRO86101
Figure 5580: DNA330941, 399601.2, 236836_at
Figure 5581: PRO86102
Figure 5582: DNA330942, 337215.1, 236907_at
Figure 5583: PRO86103
Figure 5584: DNA330943, 1042935.2, 237009_at
Figure 5585: PRO86104
Figure 5586: DNA330944, 218030.2, 237180_at
Figure 5587: PRO86105
Figure 5588: DNA330945, 334209.1, 237181_at
Figure 5589: PRO86106
Figure 5590A-B: DNA226536, TFRC, 237215_s_at
Figure 5591: PRO36999
Figure 5592: DNA330946, 023719.1, 237626_at
Figure 5593: PRO86107
Figure 5594: DNA331698, 341647.1, 237741_at
Figure 5595: PRO86693
Figure 5596: DNA330948, 305319.1, 237746_at
Figure 5597: PRO86109
Figure 5598: DNA330949, 089765.7, 237759_at
Figure 5599: PRO86110
Figure 5600: DNA331699, 983684.2, 237953_at
Figure 5601: PRO86694
Figure 5602: DNA330951, 253376.3, 238012_at
Figure 5603: PRO86112
Figure 5604A-B: DNA330952, 333610.10, 238021_s_at
Figure 5605: PRO86113
Figure 5606: DNA330953, BC019600, 238063_at
Figure 5607: DNA331700, 983399.1, 238082_at
Figure 5608: PRO86695
Figure 5609: DNA330955, 311471.1, 238303_at
Figure 5610: PRO86115
Figure 5611: DNA330956, 329762.1, 238311_at
Figure 5612: PRO86116
Figure 5613: DNA329493, 337072.5, 238423_at
Figure 5614: PRO85054
Figure 5615: DNA108695, DNA108695, 238508_at
Figure 5616: PRO9743
Figure 5617: DNA330957, 003538.1, 238509_at
Figure 5618: PRO86117
Figure 5619: DNA330958, 370339.1, 238541_at
Figure 5620: PRO86118
Figure 5621: DNA330959, 358161.17, 238545_at
Figure 5622: PRO86119
Figure 5623: DNA260158, DNA260158, 238551_at
Figure 5624: DNA329495, 1447201.1, 238581_at
Figure 5625: PRO85056
Figure 5626: DNA329496, AK056126, 238600_at
Figure 5627: PRO85057
Figure 5628: DNA329497, 232064.1, 238619_at
Figure 5629: PRO85058
Figure 5630: DNA330960, 001782.1, 238633_at
Figure 5631: PRO86120
Figure 5632: DNA330961, 903323.1, 238651_at
Figure 5633: PRO86121

Figure 5634A-B: DNA329499, 332504.1, 238778_at
Figure 5635: PRO85060
Figure 5636: DNA330962, 336371.2, 238893_at
Figure 5637: PRO86122
Figure 5638A-B: DNA330963, 241110.10, 238910_at
Figure 5639: PRO86123
Figure 5640: DNA331701, 337114.1, 238913_at
Figure 5641: PRO86696
Figure 5642: DNA330965, NP_443111.2, 238960_s_at
Figure 5643: PRO86125
Figure 5644: DNA330966, 213271.1, 238987_at
Figure 5645: PRO86126
Figure 5646: DNA330967, 1499563.1, 238996_x_at
Figure 5647: PRO86127
Figure 5648: DNA330968, 005876.1, 239002_at
Figure 5649: PRO86128
Figure 5650: DNA328194, 998827.1, 239049_at
Figure 5651: PRO84097
Figure 5652: DNA331702, 406864.3, 239062_at
Figure 5653: PRO86697
Figure 5654: DNA330970, 026912.1, 239081_at
Figure 5655: PRO86130
Figure 5656: DNA330971, 413731.1, 239096_at
Figure 5657: PRO86131
Figure 5658A-B: DNA330972, 141588.42, 239133_at
Figure 5659: PRO86132
Figure 5660: DNA330973, NP_003328.1, 239163_at
Figure 5661: PRO10751
Figure 5662: DNA330974, 348211.8, 239219_at
Figure 5663: PRO86133
Figure 5664: DNA330975, 207267.3, 239278_at
Figure 5665: PRO86134
Figure 5666: DNA330976, 023223.1, 239328_at
Figure 5667: PRO86135
Figure 5668: DNA329501, 205323.1, 239331_at
Figure 5669: PRO85062
Figure 5670: DNA330977, 996962.2, 239364_at
Figure 5671: PRO86136
Figure 5672: DNA330978, 039840.1, 239376_at
Figure 5673: PRO86137
Figure 5674: DNA330979, 407730.7, 239388_at
Figure 5675: PRO86138
Figure 5676: DNA330980, 1134871.2, 239401_at
Figure 5677: PRO86139
Figure 5678: DNA330981, 406409.1, 239404_at
Figure 5679: PRO86140
Figure 5680: DNA330982, 980479.1, 239413_at
Figure 5681: PRO86141
Figure 5682: DNA328200, 405394.1, 239442_at
Figure 5683: PRO84103
Figure 5684: DNA330983, 305289.1, 239448_at
Figure 5685: PRO86142
Figure 5686: DNA330984, 015432.1, 239476_at
Figure 5687: PRO86143
Figure 5688: DNA330985, 317098.1, 239494_at
Figure 5689: PRO86144
Figure 5690: DNA330986, 215812.1, 239533_at
Figure 5691: PRO86145

Figure 5692: DNA330987, 116344.1, 239655_at
Figure 5693: PRO86146
Figure 5694: DNA330988, 333476.1, 239680_at
Figure 5695: PRO86147
Figure 5696: DNA330989, 298825.1, 239695_at
Figure 5697: PRO86148
Figure 5698: DNA330990, 004652.1, 239721_at
Figure 5699: PRO86149
Figure 5700: DNA330991, 017874.1, 239757_at
Figure 5701: PRO86150
Figure 5702: DNA330992, AK027783, 239771_at
Figure 5703: PRO86151
Figure 5704: DNA330993, 1439890.1, 239803_at
Figure 5705: PRO86152
Figure 5706: DNA330994, NP_115730.1, 239824_s_at
Figure 5707: PRO86153
Figure 5708A-C: DNA330995, 233142.9, 239897_at
Figure 5709: PRO84857
Figure 5710: DNA258952, DNA258952, 239901_at
Figure 5711: DNA257698, DNA257698, 240070_at
Figure 5712: DNA328206, 1384214.3, 240277_at
Figure 5713: PRO84109
Figure 5714: DNA330996, 144353.1, 240347_at
Figure 5715: PRO86154
Figure 5716: DNA331703, 314831.10, 240452_at
Figure 5717: PRO86698
Figure 5718: DNA330998, 979930.1, 240665_at
Figure 5719: PRO86156
Figure 5720: DNA330999, 1454193.1, 240830_at
Figure 5721: PRO86157
Figure 5722: DNA331000, 7693121.3, 240890_at
Figure 5723: PRO86158
Figure 5724: DNA331704, CARS, 240983_s_at
Figure 5725: PRO86699
Figure 5726: DNA329504, 197187.1, 241365_at
Figure 5727: PRO85065
Figure 5728: DNA331001, 1499887.1, 241370_at
Figure 5729: PRO86159
Figure 5730: DNA331002, 231340.1, 241435_at
Figure 5731: PRO86160
Figure 5732: DNA331003, 391185.30, 241495_at
Figure 5733: PRO86161
Figure 5734: DNA331004, 314498.1, 241505_at
Figure 5735: PRO86162
Figure 5736: DNA331005, 197725.1, 241722_x_at
Figure 5737: PRO86163
Figure 5738: DNA329505, BC017102, 241734_at
Figure 5739: DNA331006, 193718.1, 241740_at
Figure 5740: PRO86164
Figure 5741: DNA331007, 405858.1, 241756_at
Figure 5742: PRO86165
Figure 5743: DNA331705, 428179.1, 241775_at
Figure 5744: PRO86700
Figure 5745: DNA195721, DNA195721, 241819_at
Figure 5746: DNA331009, 222011.1, 241824_at
Figure 5747: PRO86167
Figure 5748: DNA331010, 218800.1, 241843_at
Figure 5749: PRO86168

Figure 5750: DNA331011, 979953.1, 241859-at
Figure 5751: PRO86169
Figure 5752: DNA331012, 030070.1, 241869_at
Figure 5753: PRO86170
Figure 5754: DNA331013, 406509.1, 241924_at
Figure 5755: PRO86171
Figure 5756: DNA329506, NP-387510.1, 241937_s_at
Figure 5757: PRO85067
Figure 5758: DNA331014, 1447958.2, 241985_at
Figure 5759: PRO86172
Figure 5760: DNA331015, 109159.1, 242031_at
Figure 5761: PRO86173
Figure 5762: DNA331016, 229438.1, 242051_at
Figure 5763: PRO86174
Figure 5764: DNA328213, 419856.5, 242059_at
Figure 5765: PRO84116
Figure 5766: DNA331017, 409906.7, 242060_x_at
Figure 5767: PRO86175
Figure 5768: DNA331018, 355930.1, 242110_at
Figure 5769: PRO86176
Figure 5770: DNA331019, 234788.2, 242245_at
Figure 5771: PRO86177
Figure 5772: DNA331020, 403459.1, 242261_at
Figure 5773: PRO86178
Figure 5774: DNA331021, 017309.1, 242268_at
Figure 5775: PRO86179
Figure 5776: DNA331022, BC009627, 242304_at
Figure 5777: DNA331023, 119753.1, 242362-at
Figure 5778: PRO86181
Figure 5779: DNA331024, 028992.1, 242388_x_at
Figure 5780: PRO86182
Figure 5781: DNA328220, 239839.1, 242405_at
Figure 5782: PRO84123
Figure 5783: DNA331025, 127891.1, 242457_at
Figure 5784: PRO86183
Figure 5785: DNA328221, 221374.1, 242471_at
Figure 5786: PRO84124
Figure 5787: DNA257874, DNA257874, 242517_at
Figure 5788: DNA331026, 014632.1, 242518_at
Figure 5789: PRO86184
Figure 5790: DNA331027, 053796.1, 242560_at
Figure 5791: PRO86185
Figure 5792: DNA331028, 7693434.1, 242606_at
Figure 5793: PRO86186
Figure 5794: DNA331706, 351474.1, 242617_at
Figure 5795: PRO86701
Figure 5796: DNA331707, 330870.5, 242625_at
Figure 5797: PRO86702
Figure 5798: DNA331030, 407930.2, 242648_at
Figure 5799: PRO86188
Figure 5800: DNA331031, 405967.1, 242669_at
Figure 5801: PRO86189
Figure 5802A-C: DNA331708, NP_006258.2, 242712_x_at
Figure 5803: PRO86703
Figure 5804A-C: DNA331033, AF330045, 242722_at
Figure 5805: PRO86191
Figure 5806: DNA331034, 7689086.1, 242735_x_at
Figure 5807: PRO86192

Figure 5808: DNA331035, 210512.1, 242783_at
Figure 5809: PRO86193
Figure 5810: DNA331036, 360991.1, 242836_at
Figure 5811: PRO86194
Figure 5812: DNA328224, 028975.1, 242859_at
Figure 5813: PRO84127
Figure 5814: DNA331037, 206873.1, 242890-at
Figure 5815: PRO86195
Figure 5816: DNA331709, 017276.1, 242903_at
Figure 5817: PRO86704
Figure 5818: DNA331710, 227540.15, 242960_at
Figure 5819: PRO86705
Figure 5820: DNA331711, 427600.1, 243006_at
Figure 5821: PRO86706
Figure 5822: DNA331041, 982079.2, 243030_at
Figure 5823: PRO86199
Figure 5824: DNA331042, 019764.1, 243037_at
Figure 5825: PRO86200
Figure 5826: DNA331043, 005042.1, 243134_at
Figure 5827: PRO86201
Figure 5828: DNA331044, 226264.10, 243154_at
Figure 5829: PRO86202
Figure 5830: DNA331045, 066434.1, 243222_at
Figure 5831: PRO86203
Figure 5832: DNA331712, 005752.1, 243271_at
Figure 5833: PRO86707
Figure 5834: DNA331047, BC020624, 243362_s_at
Figure 5835: DNA331048, 7688599.1, 243366_s_at
Figure 5836: PRO86206
Figure 5837: DNA331049, 402027.4, 243395_at
Figure 5838: PRO86207
Figure 5839: DNA331713, 982999.2, 243423_at
Figure 5840: PRO86708
Figure 5841: DNA331051, 306804.1, 243469_at
Figure 5842: PRO86209
Figure 5843: DNA331714, 332965.1, 243496_at
Figure 5844: PRO86709
Figure 5845: DNA331053, 243689.1, 243509_at
Figure 5846: PRO86211
Figure 5847: DNA331715, 7683458.1, 243514_at
Figure 5848: PRO86710
Figure 5849: DNA331055, 1512996.3, 243561_at
Figure 5850: PRO86213
Figure 5851: DNA258957, DNA258957, 243631 at
Figure 5852: DNA331056, 218946.1, 243759_at
Figure 5853: PRO86214
Figure 5854: DNA194184, DNA194184, 243764_at
Figure 5855: PRO23576
Figure 5856: DNA331057, 031316.1, 243888_at
Figure 5857: PRO86215
Figure 5858: DNA331058, 400813.1, 243918_at
Figure 5859: PRO86216
Figure 5860: DNA331059, 035870.32, 243934_at
Figure 5861: PRO86217
Figure 5862: DNA210271, DNA210271, 243999_at
Figure 5863: PRO33803 DNA108728_at
Figure 5864A-B: DNA331060, 406931.1, 244008_at
Figure 5865: PRO86218

Figure 5866: DNA331061, 198683.4, 244026_at
Figure 5867: PRO86219
Figure 5868: DNA331062, BC021973, 244052_at
Figure 5869: PRO23771
Figure 5870: DNA331716, 212607.1, 244267_at
Figure 5871: PRO86711
Figure 5872: DNA331064, 006039.1, 244313_at
Figure 5873: PRO86221
Figure 5874: DNA108738, DNA108738, 244321_at
Figure 5875: PRO9822
Figure 5876: DNA331065, 341348.1, 244382_at
Figure 5877: PRO86222
Figure 5878: DNA331066, 207228.1, 244443_at
Figure 5879: PRO86223
Figure 5880: DNA328239, 331922.4, 244450_at
Figure 5881: PRO84142
Figure 5882: DNA331067, 164869.1, 244599_at
Figure 5883: PRO86224
Figure 5884: DNA331068, 337465.1, 244677-at
Figure 5885: PRO86225
Figure 5886: DNA329512, 336575.1, 244780_at
Figure 5887: PRO85073
Figure 5888: DNA331069, 008651.1, 244798_at
Figure 5889: PRO86226
Figure 5890: DNA331070, 393412.1, 244801_at
Figure 5891: PRO86227
Figure 5892: DNA331071, 343563.1, 244869_at
Figure 5893: PRO86228
Figure 5894A-B: DNA254566, BAA11502.1, D80007_at
Figure 5895: PRO49669
Figure 5896: DNA328961, BC011049, DNA36995_at
Figure 5897: PRO84667
Figure 5898A-B: DNA331072, AB046821, DNA53991_at
Figure 5899: DNA327200, KSP37, DNA59602_at
Figure 5900: PRO1065
Figure 5901: DNA327205, GBP5, DNA61875_at
Figure 5902: PRO83478
Figure 5903: DNA331717, BC020203, DNA71289_at
Figure 5904: PRO86712
Figure 5905: NA331718, AK024409, DNA92232_at
Figure 5906: PRO86713
Figure 5907: DNA96866, DNA96866, DNA96866_at
Figure 5908: PRO6015
Figure 5909: DNA331073, BC011775, DNA101926_at
Figure 5910: PRO86229
Figure 5911: DNA108670, DNA108670, DNA108670_at
Figure 5912: PRO7171
Figure 5913: DNA304467, BC004535, DNA108688_at
Figure 5914: PRO71043
Figure 5915A-B: DNA108728, DNA108728,
Figure 5916: PRO9741
Figure 5917: DNA329215, ICOS, DNA1089177_at
Figure 5918: PRO7424
Figure 5919: DNA331719, BC002424, DNA143288_at
Figure 5920: PRO12705
Figure 5921A-B: DNA150956, HUMORFKG1P, DNA150956_at
Figure 5922: DNA330417, APOL6, DNA164989_at
Figure 5923: PRO21341

Figure 5924: DNA329483, AF384857, DNA166819_at
Figure 5925: PRO20110
Figure 5926: DNA26842, DNA26842, P_Z64949_at
Figure 5927: PRO180
Figure 5928: DNA304468, NP_077300.1, P_Z93700_at
Figure 5929: PRO71044
Figure 5930: DNA39423, DNA39423, P_X52252_at
Figure 5931: PRO271
Figure 5932: DNA330262, GW112, P_Z64962_at
Figure 5933: PRO85493
Figure 5934: DNA331074, AF252257, P_A37030_at
Figure 5935: DNA60764, DNA60764, P-A46906_at
Figure 5936: PRO1265
Figure 5937: DNA331720, AF289594, P_A37063_at
Figure 5938: PRO86714
Figure 5939: DNA331721, BC017876, P-A37079_at
Figure 5940: PRO71045
Figure 5941: DNA76401, DNA76401, P-A37126_at
Figure 5942: PRO1575
Figure 5943: DNA304475, NP-1116246.1, P_A37128_at
Figure 5944: PRO71049
Figure 5945: DNA66480, HSAPO1, NM_000043_at
Figure 5946: PRO1207
Figure 5947: DNA88195, CD3G, NM_000073_at
Figure 5948: PRO2693
Figure 5949: DNA325712, CDK4, NM_000075_at
Figure 5950: PRO82194
Figure 5951: DNA329934, BC013083, NM_000099_at
Figure 5952: PRO2721
Figure 5953A-B: DNA331722, HLTMFVA, NM_000130_at
Figure 5954: PRO36374
Figure 5955: DNA331723, U66095, NM-000161_at
Figure 5956: PRO86715
Figure 5957: DNA227668, HUMGLYKINB, NM_000167_at
Figure 5958: PRO38131
Figure 5959A-D: DNA331724, HSGLA, NM_000169_at
Figure 5960: DNA331725, BC006342, NM_000175_at
Figure 5961: DNA150823, NP_000185.1, NM_000194_t
Figure 5962: PRO12810
Figure 5963: DNA331726, HUMICAMA1A, NM_000201_at
Figure 5964: PRO86716
Figure 5965A-B: DNA88419, HSINTA6R, NM_000210_at
Figure 5966: PRO2339
Figure 5967: DNA88428, HUMLAP, NM_000211_at
Figure 5968: PRO2787
Figure 5969: DNA226014, NP_000230.1, NM_000239_at
Figure 5970: PRO36477
Figure 5971: DNA97287, NP_000240.1, NM_000249_at
Figure 5972: PRO3634
Figure 5973: DNA88554, NP_000241.1, NM_000250_at
Figure 5974: PRO2839
Figure 5975: DNA331727, BC008015, NM_000269_at
Figure 5976: PRO37534
Figure 5977A-E: DNA331728, PTEN4, NM_000314_at
Figure 5978: DNA83020, NP_000349.1, NM_000358_at
Figure 5979: PRO2561
Figure 5980: DNA227081, EGR2, NM-000399_at
Figure 5981: PRO37544

Figure 5982: DNA76512, HSIL2REC, NM_000417_at
Figure 5983: PRO2020
Figure 5984: DNA76514, HSIL4R, NM_000418_at
Figure 5985: PRO2540
Figure 5986: DNA329522, NP_000433.2, NM_000442_at
Figure 5987: PRO85080
Figure 5988: DNA188732, NP_000475.1, NM_000484_at
Figure 5989: PRO25302
Figure 5990: DNA331729, AF281258, NM_000517_at
Figure 5991: DNA331730, BC014514, NM_000527_at
Figure 5992: PRO2915
Figure 5993: DNA331731, HSASM2MR, NM_000543_at
Figure 5994: DNA76516, IL6R, NM_000565_at
Figure 5995: PRO2022
Figure 5996: DNA36718, HUMIL10, NM_000572_at
Figure 5997: PRO73
Figure 5998: DNA324158, NP_000567.1, NM_000576_at
Figure 5999: PRO65
Figure 6000A-B: DNA331732, HSCCR5AB2, NM_000579_at
Figure 6001: PRO25194
Figure 6002: DNA290585, NP_000573.1, NM_000582_f_at
Figure 6003: PRO70536
Figure 6004: DNA216500, NP_000575.1, NM_000584_at
Figure 6005: PRO34252
Figure 6006: DNA36712, HUMIL3, NM_000588_at
Figure 6007: PRO67
Figure 6008A-B: DNA331733, AF361105, NM-000590_at
Figure 6009: DNA331734, BC014081, NM_000593_at
Figure 6010: PRO36996
Figure 6011A-B: DNA331735, AY066019, NM_000594_at
Figure 6012A-B: DNA331736, AY070490, NM_000595_at
Figure 6013: DNA331737, BC009902, NM_000597_at
Figure 6014: PRO2587
Figure 6015: DNA217246, NP_000591.1, NM_000600_at
Figure 6016: PRO34288
Figure 6017: DNA331075, NP_000601.2, NM_000610_at
Figure 6018: PRO86231
Figure 6019A-C: DNA331738, AF375790, NM_000619_at
Figure 6020A-B: DNA220752, ITGAM, NM_000632_at
Figure 6021: PRO34730
Figure 6022A-B: DNA97288, HUMBCL2C, NM_000633_at
Figure 6023: PRO3635
Figure 6024: DNA331739, A12178, NM_000636_at
Figure 6025: PRO86720
Figure 6026: DNA331740, HUMHPC, NM_000639_at
Figure 6027: PRO1208
Figure 6028: DNA329000, HSU03905, NM_000647_at
Figure 6029: PRO84690
Figure 6030: DNA328253, NP_004029.1, NM_000699_at
Figure 6031: PRO84149
Figure 6032: DNA89242, ANXA1, NM_000700_at
Figure 6033: PRO2907
Figure 6034: DNA88194, CD3E, NM_000733_at
Figure 6035: PRO2220
Figure 6036: DNA329975, PRO2325, NM_000791_at
Figure 6037: DNA331741, BC003097, NM_000873_at
Figure 6038: PRO86721
Figure 6039: DNA331076, HSIFNABR, NM_000874_at

Figure 6040: PRO86232
Figure 6041A-B: DNA83101, NP_000868.1, NM_000877_at
Figure 6042: PRO2590
Figure 6043A-B: DNA76508, A07795, NM-000878 _t
Figure 6044: PRO2538
Figure 6045: DNA36714, NP_000870.1, NM_000879_at
Figure 6046: PRO69
Figure 6047A-B: DNA88417, HSINTAL4, NM_000885_at
Figure 6048: PRO2337
Figure 6049: DNA88433, HUMINTB7A, NM_000889_at
Figure 6050: PRO2346
Figure 6051: DNA226053, NP_000908.1, NM_000917_at
Figure 6052: PRO36516
Figure 6053A-B: DNA331742, BC018127, NM_000919_at
Figure 6054: PRO86722
Figure 6055: DNA227709, PTGER2, NM_000956_at
Figure 6056: PRO38172
Figure 6057: DNA226195, NP_000949.1, NM_000958_at
Figure 6058: PRO36658
Figure 6059: DNA327639, TCN1, NM_001062_at
Figure 6060: PRO83640
Figure 6061A-B: DNA150748, ADCY7, NM_001114_at
Figure 6062: PRO12446
Figure 6063: DNA171404, HSU45878, NM_001165_at
Figure 6064: PRO20132
Figure 6065: DNA331743, AAA19687.1, NM_001168_at
Figure 6066: PRO12242
Figure 6067A-B: DNA325972, BC018739, NM_001211_at
Figure 6068: PRO82417
Figure 6069: DNA287267, CCNA2, NM_001237_at
Figure 6070: PRO37015
Figure 6071: DNA196674, D86042, NM_001243_at
Figure 6072: PRO2938
Figure 6073: DNA325568, BC017575, NM_001274_at
Figure 6074: PRO12187
Figure 6075: DNA226177, CCR1, NM_001295_at
Figure 6076: PRO36640
Figure 6077: DNA331744, CTSW, NM_001335_at
Figure 6078: PRO1574
Figure 6079: DNA93466, HUMEDG, NM_001400_at
Figure 6080: PRO4936
Figure 6081: DNA331745, HSU77085, NM_001423_at
Figure 6082: PRO12467
Figure 6083A-C: DNA151167, HSABP280, NM_001456_at
Figure 6084: PRO12867
Figure 6085A-C: DNA331746, AF043045, NM_001457_at
Figure 6086: PRO86723
Figure 6087: DNA188346, FLT3LG, NM_001459_at
Figure 6088: PRO21766
Figure 6089: DNA227173, HSU93049, NM_001465_at
Figure 6090: PRO37636
Figure 6091A-B: DNA331747, GABBR1, NM_001470_at
Figure 6092: PRO86724
Figure 6093A-B: DNA76503, IL10RA, NM_001558_at
Figure 6094: PRO2536
Figure 6095A-B: DNA227750, IL12RB2, NM_001559_at
Figure 6096: PRO38213
Figure 6097: DNA76556, HSU03397, NM-001561_at

Figure 6098: PRO2023
Figure 6099: DNA82362, CXCL10, NM_001565_at
Figure 6100: PRO1718
Figure 6101: DNA227013, NP_001560.1, NM_001569_at
Figure 6102: PRO37476
Figure 6103: DNA331748, BC009799, NM_001657at
Figure 6104: PRO46
Figure 6105: DNA150716, HSZNFNPRA, NM_001706_at
Figure 6106: PRO12790
Figure 6107: DNA331077, HUMBGPAB, NM_001712_at
Figure 6108: PRO86233
Figure 6109: DNA150718, NP_001727.1, NM-001736_at
Figure 6110: PRO12435
Figure 6111A-B: DNA226387, HSCYCLF, NM_001761_at
Figure 6112: PRO36850
Figure 6113: DNA329002, CCT6A, NM_001762_at
Figure 6114: PRO4912
Figure 6115: DNA226380, HSCD37, NM_001774_at
Figure 6116: PRO4695
Figure 6117: DNA331749, D84277, NM_001775_at
Figure 6118: PRO86725
Figure 6119: DNA88199, HUMMEMGL1, NM_001778_at
Figure 6120: PRO2696
Figure 6121: DNA226436, CD69, NM_001781_at
Figure 6122: PRO36899
Figure 6123: DNA331750, A23013, NM_001803_at
Figure 6124: PRO2496
Figure 6125: DNA151798, NP_001797.1, NM_001806_at
Figure 6126: PRO12186
Figure 6127: DNA227232, SLC31A1, NM_001859_at
Figure 6128: PRO37695
Figure 6129: DNA331751, S68134, NM_001881_at
Figure 6130: PRO86726
Figure 6131: DNA331078, NP_001894.1, NM_001903_at
Figure 6132: PRO86234
Figure 6133: DNA331752, BC010240, NM_001908_at
Figure 6134: PRO86727
Figure 6135: DNA225810, HSCATHL, NM_001912_at
Figure 6136: PRO36273
Figure 6137: DNA83048, DEFA4, NM_001925_at
Figure 6138: PRO2057
Figure 6139: DNA88215, NP_001919.1, NM_001928_at
Figure 6140: PRO2703
Figure 6141: DNA196562, HSPCHDP7, NM_001935_at
Figure 6142: PRO25042
Figure 6143: DNA226871, NP_001942.1, NM_001951_at
Figure 6144: PRO37334
Figure 6145: DNA227332, NP_001943.1, NM_001952_at
Figure 6146: PRO37795
Figure 6147: DNA225661, ECGF1, NM_001953_at
Figure 6148: PRO36124
Figure 6149: DNA273174, HSEFLDELA, NM_001960_at
Figure 6150: PRO61211
Figure 6151: DNA150779, HUMETR103, NM_001964_at
Figure 6152: PRO12798
Figure 6153: DNA331753, HUMENOG, NM_001975_at
Figure 6154: PRO38010
Figure 6155: DNA331754, BC002464, NM_001992_at

Figure 6156: PRO86728
Figure 6157: DNA331755, D83920, NM_002003_at
Figure 6158: PRO86729
Figure 6159: DNA226881, HUMERGBFLI, NM_002017_at
Figure 6160: PRO37344
Figure 6161: DNA88332, FVT1, NM_002035_at
Figure 6162: PRO2753
Figure 6163: DNA225979, GIP3, NM_002038_at
Figure 6164: PRO36442
Figure 6165: DNA331756, BC002666, NM_002053_at
Figure 6166: PRO12478
Figure 6167: DNA88374, GZMK, NM_002104_at
Figure 6168: PRO2768
Figure 6169: DNA228014, ICAM3, NM_002162_at
Figure 6170: PRO38477
Figure 6171: DNA331757, A17548, NM_002167_at
Figure 6172: PRO86730
Figure 6173: DNA76517, IL7R, NM_002185_at
Figure 6174: PRO2541
Figure 6175: DNA188271, NP_002179.1, NM_002188_at
Figure 6176: PRO21795
Figure 6177: DNA226396, IL15RA, NM_002189_at
Figure 6178: PRO36859
Figure 6179: DNA227014, NP_002190.1, NM_002199_at
Figure 6180: PRO37477
Figure 6181A-B: DNA88427, HSFNRB, NM_002211_at
Figure 6182: PRO2786
Figure 6183: DNA103215, NP_002210.1, NM_002219_at
Figure 6184: PRO4545
Figure 6185: DNA331758, S82269, NM_002222_at
Figure 6186: PRO86731
Figure 6187: DNA331759, BC002646, NM_002228_at
Figure 6188: PRO4671
Figure 6189: DNA331760, BC009466, NM_002229_at
Figure 6190: PRO4650
Figure 6191A-B: DNA331761, AF305731S5, NM_002250_at
Figure 6192: DNA150971, KLRB1, NM_002258_at
Figure 6193: PRO12564
Figure 6194: DNA326343, BC003572, NM_002265_at
Figure 6195: PRO82739
Figure 6196: DNA288243, LAG3, NM_002286_at
Figure 6197: PRO36451
Figure 6198A-B: DNA188301, LIF, NM_002309_at
Figure 6199: PRO21834
Figure 6200A-B: DNA331762, HUMLYTOXBB, NM_002341_at
Figure 6201: DNA88666, NP_002334.1, NM_002343_at
Figure 6202: PRO2892
Figure 6203: DNA227150, LY6E, NM_002346_at
Figure 6204: PRO37613
Figure 6205: DNA327255, BC001061, NM_002394_at
Figure 6206: PRO57298
Figure 6207: DNA150937, HSU94352, NM_002405_at
Figure 6208: PRO11598
Figure 6209: DNA82376, CXCL9, NM_002416_at
Figure 6210: PRO1723
Figure 6211: DNA103283, MNDA, NM_002432_at
Figure 6212: PRO4613
Figure 6213: DNA103525, NP_002457.1, NM_002466_at

Figure 6214: PRO4852
Figure 6215A-B: DNA331763, AF058696, NM_002485_at
Figure 6216: PRO36001
Figure 6217: DNA103382, HSU49395, NM_002561_at
Figure 6218: PRO4711
Figure 6219A-B: DNA88331, HSFUR, NM_002569_at
Figure 6220: PRO2752
Figure 6221: DNA103488, PCNA, NM_002592_at
Figure 6222: PRO4815
Figure 6223: DNA328587, NP_002612.1, NM_002621_at
Figure 6224: PRO2854
Figure 6225: DNA331764, NP_071438.1, NM_002624_at
Figure 6226: PRO86732
Figure 6227: DNA227067, HSPKCB1A, NM_002738_at
Figure 6228: PRO37530
Figure 6229: DNA227090, NP_002750.1, NM_002759_at
Figure 6230: PRO37553
Figure 6231: DNA88626, HUMSAPABCD, NM_002778_at
Figure 6232: PRO2875
Figure 6233: DNA329098, BC007897, NM_002808_at
Figure 6234: PRO84749
Figure 6235: DNA326853, NP_002818.1, NM_002827_at
Figure 6236: PRO38066
Figure 6237: DNA88607, NP_002892.1, NM_002901_at
Figure 6238: PRO2863
Figure 6239: DNA331765, AF294009, NM_002934_at
Figure 6240: PRO2444
Figure 6241: DNA331766, AF043339, NM_002983_at
Figure 6242: DNA51778, HSHC21, NM_002984_at
Figure 6243: PRO80
Figure 6244: DNA330124, CCL22, NM_002990_at
Figure 6245: PRO34107
Figure 6246: DNA227788, NP_002995.1, NM_003004_at
Figure 6247: PRO38251
Figure 6248: DNA329005, HSU33017, NM_003037_at
Figure 6249: PRO12612
Figure 6250: DNA196489, HUMMCT, NM_003051_at
Figure 6251: PRO24980
Figure 6252A-B: DNA103542, HSLR11, NM_003105_at
Figure 6253: PRO4869
Figure 6254: DNA331767, D78130, NM_003129_at
Figure 6255: PRO37946
Figure 6256: DNA328259, AF029311, NM_003150_at
Figure 6257: DNA227447, HSTCFIC, NM_003202_at
Figure 6258: PRO37910
Figure 6259A-B: DNA226536, HSTRR, NM_003234_at
Figure 6260: PRO36999
Figure 6261A-B: DNA83176, TGFBR3, NM_003243_at
Figure 6262: PRO2620
Figure 6263: DNA103532, TM7SF1, NM_003272_at
Figure 6264: PRO4859
Figure 6265A-B: DNA103585, HUMTOPI, NM_003286_at
Figure 6266: PRO4909
Figure 6267: DNA331768, BC007935, NM_003316_at
Figure 6268: PRO22907
Figure 6269: DNA331769, AF065241, NM_003329_at
Figure 6270A-B: DNA331770, AF019563, NM_003332_at
Figure 6271: DNA331771, HSU76367, NM_003355_at

Figure 6272: PRO86733
Figure 6273: DNA151906, HSUNG, NM_003362_f_at
Figure 6274: PRO12214
Figure 6275: DNA103380, HUMVDAC1X, NM_003374_at
Figure 6276: PRO4710
Figure 6277: DNA225992, NP_003374.1, NM_003383_at
Figure 6278: PRO36455
Figure 6279: DNA227330, NP_003443.1, NM_003452_at
Figure 6280: PRO37793
Figure 6281: DNA93449, AF025375, NM_003467_at
Figure 6282: PRO4516
Figure 6283: DNA331772, BC010022, NM_003504_at
Figure 6284: PRO71058
Figure 6285: DNA331773, AF123318, NM_003550_at
Figure 6286: PRO86734
Figure 6287: DNA331079, AF036342, NM_003650_at
Figure 6288: PRO1191
Figure 6289: DNA328260, AF305428, NM_003661_at
Figure 6290: PRO84152
Figure 6291: DNA151802, AB004066, NM_003670_at
Figure 6292: PRO12890
Figure 6293: DNA227213, NP_003671.1, NM_003680_at
Figure 6294: PRO37676
Figure 6295: DNA331774, AK001769, NM_003730_at
Figure 6296: PRO86735
Figure 6297: DNA150433, AB005043, NM_003745_at
Figure 6298: PRO12771
Figure 6299: DNA328377, NP_003759.1, NM_003768_at
Figure 6300: PRO84232
Figure 6301: DNA194746, HSM800355, NM_003798_at
Figure 6302: DNA196431, AF064090, NM_003807_at
Figure 6303: PRO5810
Figure 6304: DNA61870, HSU57059, NM_003810_at
Figure 6305: PRO1096
Figure 6306A-B: DNA200236, NP_003807.1, NM_003816_at
Figure 6307: PRO34137
Figure 6308: DNA331775, BC000334, NM_003824_at
Figure 6309: PRO4801
Figure 6310: DNA331080, NP_003835.2, NM_003844_at
Figure 6311: PRO86235
Figure 6312: DNA225550, IL18RAP, NM_003853_at
Figure 6313: PRO36013
Figure 6314: DNA103451, IL18R1, NM_003855_at
Figure 6315: PRO4778
Figure 6316: DNA151011, AF037989, NM_003877_at
Figure 6317: PRO12839
Figure 6318: DNA331776, IER3, NM_003897_at
Figure 6319: PRO84760
Figure 6320A-B: DNA150765, SLC7A6, NM_003983_at
Figure 6321: PRO12458
Figure 6322: DNA88308, HUMFCREA, NM_004106_at
Figure 6323: PRO2739
Figure 6324A-B: DNA331777, AF200219S2, NM_004107_at
Figure 6325: DNA227133, GBP2, NM_004120_at
Figure 6326: PRO37596
Figure 6327: DNA83091, HUMSP13E, NM_004131_at
Figure 6328: PRO2081
Figure 6329A-B: DNA151108, SREBF1, NM_004176_at

Figure 6330: PRO12105
Figure 6331: DNA218676, AF125304, NM_004195_at
Figure 6332: PRO34454
Figure 6333: DNA103394, HSU81800, NM_004207_at
Figure 6334: PRO4722
Figure 6335: DNA329533, BC018782, NM_004221_at
Figure 6336: PRO85085
Figure 6337: DNA331778, AK027513, NM_004265_at
Figure 6338: PRO86736
Figure 6339: DNA151142, NP_004321.1, NM_004330_at
Figure 6340: PRO12110
Figure 6341: DNA227303, NP_004322.1, NM_004331_at
Figure 6342: PRO37766
Figure 6343: DNA287240, BST2, NM_004335_at
Figure 6344: PRO29371
Figure 6345: DNA225910, NP_004336.1, NM_004345_at
Figure 6346: PRO36373
Figure 6347: DNA331779, CASP3, NM_004346_at
Figure 6348: PRO12832
Figure 6349A-B: DNA326191, NP_004351.1, NM_004360_at
Figure 6350: PRO2672
Figure 6351A-C: DNA150729, HSU47741, NM_004380_at
Figure 6352: PRO12147
Figure 6353A-B: DNA151420, S40832, NM_004430_at
Figure 6354: PRO12876
Figure 6355A-B: DNA218283, EPHB6,
Figure 6356: PRO34335
Figure 6357: DNA331780, BC003110, NM_004512_at
Figure 6358: PRO4843
Figure 6359: DNA150935, NP_004547.1, NM_004556_at
Figure 6360: PRO12155
Figure 6361A-B: DNA151831, NP_004564.1, NM_004573_at
Figure 6362: PRO12198
Figure 6363: DNA328262, HSU57094, NM_004580_at
Figure 6364: PRO84153
Figure 6365: DNA331781, HSU77035, NM_004591_at
Figure 6366: PRO1724
Figure 6367: DNA331782, HUMVAIPR, NM_004624_at
Figure 6368: DNA329984, WRB, NM_004627_at
Figure 6369: PRO11656
Figure 6370: DNA329119, NP_004633.1, NM_004642_at
Figure 6371: PRO4550
Figure 6372: DNA328578, NP_004656.2, NM_004665_at
Figure 6373: PRO7426
Figure 6374: DNA331783, BC011726, NM_004706_at
Figure 6375: PRO86737
Figure 6376: DNA218284, AF053004, NM_004843_at
Figure 6377: PRO34336
Figure 6378: DNA151017, AB005047, NM_004844_at
Figure 6379: PRO12841
Figure 6380A-B: DNA150447, AB011098, NM_004863_at
Figure 6381: PRO12256
Figure 6382: DNA88295, HUMERP72H, NM_004911_at
Figure 6383: PRO2733
Figure 6384: DNA331784, AB001325, NM_004925_at
Figure 6385: PRO380281
Figure 6386A-B: DNA331785, DSC1, NM_004948_at
Figure 6387: PRO36355

Figure 6388: DNA227563, NP_004946.1, NM_004955_at
Figure 6389: PRO38026
Figure 6390: DNA331786, HUMSTPK13, NM_005030_at
Figure 6391: PRO86738
Figure 6392: DNA329011, BCL3, NM_005178_at
Figure 6393: PRO4785
Figure 6394: DNA331787, AF213050, NM_005192_at
Figure 6395: PRO86739
Figure 6396: DNA103330, HUMPOPSTK, NM_005204_at
Figure 6397: PRO4660
Figure 6398: DNA331788, HUMIGCTL3, NM_005214_at
Figure 6399: NM_004445_at DNA325060, NP_004075.1, NM_005217_at
Figure 6400: PRO2570
Figure 6401: DNA331789, HSCFOS, NM_005252_at
Figure 6402: DNA304668, HSPA1A, NM_005346_at
Figure 6403: PRO71095
Figure 6404: DNA331790, HUMCMYBA, NM_005375_at
Figure 6405: DNA227376, NP_005393.1, NM_005402_at
Figure 6406: PRO37839
Figure 6407: DNA331791, BC005292, NM_005409_at
Figure 6408: PRO19838
Figure 6409: DNA329319, TOSO, NM_005449_at
Figure 6410: PRO1607
Figure 6411A-B: DNA189702, AF047348, NM_005503_at
Figure 6412: PRO22775
Figure 6413: DNA150989, HSP27,
Figure 6414: PRO12569 NM_006021_at
Figure 6415A-C: DNA331792, HUMOP18A, NM_005563_at
Figure 6416: DNA97285, LDHA, NM_005566_at
Figure 6417: PRO3632
Figure 6418: DNA225675, LMAN1, NM_005570_at
Figure 6419: PRO36138
Figure 6420: DNA331793, AF148645, NM_005614_at
Figure 6421: PRO37938
Figure 6422: DNA331794, BC001263, NM_005627_at
Figure 6423: PRO86741
Figure 6424: DNA226500, NP_005619.1, NM_005628_at
Figure 6425: PRO36963
Figure 6426A-B: DNA227206, NP_005648.1, NM_005657_at
Figure 6427: PRO37669
Figure 6428: DNA323937, NP_005689.2, NM_005698_at
Figure 6429: PRO80670
Figure 6430: DNA331081, NP_005714.2, NM_005723_at
Figure 6431: PRO4845
Figure 6432: DNA304459, PPIF, NM_005729_at
Figure 6433: PRO37073
Figure 6434A-B: DNA331082, AF057299, NM_005732_at
Figure 6435: PRO86236
Figure 6436: DNA88541, PBEF, NM_005746_at
Figure 6437: PRO2834
Figure 6438: DNA329014, EBI3, NM_005755_at
Figure 6439: PRO9998
Figure 6440: DNA93548, NP_005758.1, NM_005767_at
Figure 6441: PRO4929
Figure 6442: DNA331083, NP_005759.2, NM_005768_at
Figure 6443: PRO86237
Figure 6444: DNA193866, AF081675, NM_005810_at
Figure 6445: PRO23288

Figure 6446: DNA75525, GPA33, NM_005814_at
Figure 6447: PRO2524
Figure 6448A-B: DNA88650, TACTILE, NM_005816_at
Figure 6449: PRO2460
Figure 6450: DNA150959, NP_005813.1, NM_005822_at
Figure 6451: PRO11599
Figure 6452: DNA329538, BC001731, NM_005898_at
Figure 6453: PRO85088
Figure 6454: DNA324110, MDH1, NM_005917_at
Figure 6455: PRO4918
Figure 6456: DNA328266, NP_005993.1, NM_006002_at
Figure 6457: PRO12125
Figure 6458: DNA150941, NP_006012.1, NM_005532_at
Figure 6459: PRO12548
Figure 6460: DNA227138, NP_006045.1, NM_006054_at
Figure 6461: PRO37601
Figure 6462: DNA88614, HSRING6, NM_006120_at
Figure 6463: PRO2867
Figure 6464: DNA331795, NP_006129.2, NM-006138_at
Figure 6465: PRO81984
Figure 6466: DNA331796, HUMCD284, NM_006139_at
Figure 6467: DNA330114, GPR19, NM_006143_at
Figure 6468: PRO4946
Figure 6469: DNA88372, HUMHFSP, NM_006144_at
Figure 6470: PRO2312
Figure 6471: DNA103526, HSU10485, NM_006152_at
Figure 6472: PRO4853
Figure 6473: DNA331797, BC020544, NM_006159_at
Figure 6474: PRO2520
Figure 6475: DNA151049, S74017, NM_006164_at
Figure 6476: PRO12170
Figure 6477A-B: DNA151841, HUMA20, NM_006290_at
Figure 6478: PRO12904
Figure 6479: DNA331798, TSG101, NM_006292_at
Figure 6480: PRO86742
Figure 6481: DNA83109, HUMIIP, NM_006332_at
Figure 6482: PRO2592
Figure 6483: DNA331799, AY034481, NM_006372_at
Figure 6484: PRO83688
Figure 6485: DNA329540, UBD, NM_006398_at
Figure 6486: PRO85090
Figure 6487: DNA331800, BC007107, NM_006406_at
Figure 6488: PRO12111
Figure 6489: DNA331801, BC012589, NM_006419_at
Figure 6490: PRO21708
Figure 6491: DNA227795, CCT7, NM_006429_at
Figure 6492: PRO38258
Figure 6493: DNA329225, BC005926, NM_006495_at
Figure 6494: PRO84833
Figure 6495: DNA327702, AF074002, NM_006499_at
Figure 6496: PRO83684
Figure 6497: DNA151804, RELB, NM_006509_at
Figure 6498: PRO1218
Figure 6499A-B: DNA331802, AF012108, NM_006534_at
Figure 6500: PRO86743
Figure 6501: DNA93439, HSY13248, NM_006564_at
Figure 6502: PRO4515
Figure 6503: DNA227751, NP_006557.1, NM_006566_at

Figure 6504: PRO38214
Figure 6505: DNA227126, NP_006559.1, NM_006568_at
Figure 6506: PRO37589
Figure 6507: DNA331803, AF116456, NM_006573_at
Figure 6508: PRO738
Figure 6509: DNA331804, BC001572, NM_006579_at
Figure 6510: PRO12082
Figure 6511: DNA324740, NP_006577.1, NM_006586_at
Figure 6512: PRO81365
Figure 6513: DNA331805, HSM801976, NM_006620_at
Figure 6514: PRO86744
Figure 6515: DNA328544, HSFIBLP, NM_006682_at
Figure 6516: PRO84347
Figure 6517: DNA227035, HUMHUMCM5, NM_006739_at
Figure 6518: PRO37498
Figure 6519: DNA227512, NP_006736.1, NM_006745_at
Figure 6520: PRO37975
Figure 6521A-B: DNA331806, AB005666, NM_006747_at
Figure 6522: PRO86745
Figure 6523: DNA227416, NP_006745.1, NM_006754_at
Figure 6524: PRO37879
Figure 6525: DNA331807, HSU30498, NM_006762_at
Figure 6526: PRO86746
Figure 6527: DNA227190, NP_006830.1, NM_006839_at
Figure 6528: PRO37653
Figure 6529: DNA150812, RTVP1, NM_006851_at
Figure 6530: PRO12481
Figure 6531: DNA331808, HSU82278, NM_006866_at
Figure 6532: PRO86747
Figure 6533: DNA103221, NP_006866.1, NM_006875_at
Figure 6534: PRO4551
Figure 6535: DNA328271, ZWINT, NM_007057_at
Figure 6536: PRO81868
Figure 6537: DNA331809, NP_009046.1, NM_007115_at
Figure 6538: PRO86748
Figure 6539: DNA103587, HSMRL3R, NM_007208_at
Figure 6540: PRO4911
Figure 6541: DNA330180, TRC8, NM_007218_at
Figure 6542: PRO85428
Figure 6543: DNA331810, HSU64805, NM_007295_at
Figure 6544: PRO23937
Figure 6545: DNA331086, AB027467, NM_012112_at
Figure 6546: PRO86239
Figure 6547A-B: DNA226290, HSU28811, NM_012201_at
Figure 6548: PRO36753
Figure 6549: DNA227143, NP_036400.1, NM_012268_at
Figure 6550: PRO37606
Figure 6551A-B: DNA150955, NP_036420.1, NM_012288_at
Figure 6552: PRO12559
Figure 6553: DNA331811, AF083247, NM_012328_at
Figure 6554: PRO1471
Figure 6555A-B: DNA227255, STAG3, NM_012447_at
Figure 6556: PRO37718
Figure 6557: DNA304476, NP_036585.1, NM_012453_at
Figure 6558: PRO1125
Figure 6559: DNA88510, HSNKG5, NM_012483_at
Figure 6560: PRO2822
Figure 6561: DNA103418, AF032862, NM_012484_at

EP 2 179 742 A1

Figure 6562: PRO4746
Figure 6563: DNA88189, HUMCD24B, NM_013230_at
Figure 6564: PRO2690
Figure 6565: DNA331812, BC019883, NM_013269_at
Figure 6566: PRO86749
Figure 6567: DNA103481, HUMAUANTIG, NM_013285_at
Figure 6568: PRO4808
Figure 6569: DNA196426, H963, NM_013308_at
Figure 6570: PRO24924
Figure 6571A-B: DNA329017, AF035947, NM_013324_at
Figure 6572: PRO84692
Figure 6573: DNA150648, HIG2, NM_013332_at
Figure 6574: PRO11576
Figure 6575A-B: DNA331813, AF213467, NM_013448_at
Figure 6576: PRO86750
Figure 6577: DNA304461, HSPC067, NM_014158_at
Figure 6578: PRO71039
Figure 6579: DNA331814, BC009642, NM_014164_at
Figure 6580: PRO86751
Figure 6581: DNA330374, ORMDL2, NM_014182_at
Figure 6582: PRO23321
Figure 6583: DNA88203, CD5, NM_014207_at
Figure 6584: PRO2698
Figure 6585A-B: DNA331815, AF135372, NM_014232_at
Figure 6586: DNA331816, BC003067, NM_014330_at
Figure 6587: PRO12543
Figure 6588: DNA331817, NP_055154.2, NM_014339_at
Figure 6589: PRO86240
Figure 6590: DNA227233, NP_055157.1, NM_014342_at
Figure 6591: PRO37696
Figure 6592: DNA227351, AF191020, NM_014367_at
Figure 6593: PRO37814
Figure 6594: DNA331088, NP_055252.2, NM_014437_at
Figure 6595: PRO80674
Figure 6596: DNA330084, SIT, NM_014450_at
Figure 6597: PRO9895
Figure 6598: DNA324198, NP_055400.1, NM_014585_at
Figure 6599: PRO37675
Figure 6600A-B: DNA151879, NP_055463.1, NM_014648_at
Figure 6601: PRO12743
Figure 6602: DNA194805, NP_055500.1, NM_014685_at
Figure 6603: PRO24075
Figure 6604A-B: DNA150467, AB018335, NM_014698_at
Figure 6605: PRO12272
Figure 6606A-B: DNA194778, KIAA0152, NM_014730_at
Figure 6607: PRO24056
Figure 6608A-B: DNA277809, KIAA0275, NM_014767_at
Figure 6609: PRO64556
Figure 6610A-B: DNA227353, SEC24D, NM_014822_at
Figure 6611: PRO37816
Figure 6612: DNA93507, NP_055694.1, NM_014879_at
Figure 6613: PRO4948
Figure 6614A-B: DNA150954, KIAA0022, NM_014880_at
Figure 6615: PRO12558
Figure 6616A-B: DNA227293, DNA227293, NM_014883_at
Figure 6617: PRO37756
Figure 6618: DNA150805, FAM3C, NM_014888_at
Figure 6619: PRO11583

EP 2 179 742 A1

Figure 6620A-B: DNA194837, NP_055714.1, NM_014899_at
Figure 6621: PRO24100
Figure 6622A-B: DNA304464, CHSY1, NM_014918_at
Figure 6623: PRO71042
Figure 6624: DNA330103, MD-2, NM_015364_at
Figure 6625: PRO19671
Figure 6626: DNA150872, NP_056202.1, NM_015387_at
Figure 6627: PRO12814
Figure 6628: DNA328590, BC001232, NM_015864_at
Figure 6629: PRO84375
Figure 6630: DNA196569, NP_056957.1, NM_015873_at
Figure 6631: PRO19859
Figure 6632: DNA150865, LOC51596, NM_015921_at
Figure 6633: PRO11587
Figure 6634: DNA150832, NP_057019.2, NM_015935_at
Figure 6635: PRO1249
Figure 6636: DNA331089, NP_057143.1, NM_016059_at
Figure 6637: PRO4984
Figure 6638: DNA331818, AF151899, NM_016072_at
Figure 6639: PRO793
Figure 6640: DNA328663, AK001280, NM_016073_at
Figure 6641: PRO36183
Figure 6642: DNA331819, BC006807, NM_016077_at
Figure 6643: PRO38080
Figure 6644: DNA150661, LOC51030, NM_016078_at
Figure 6645: PRO12398
Figure 6646: DNA329292, AF085360, NM_016101_at
Figure 6647: PRO84882
Figure 6648: DNA329923, HSPC035, NM_016127_at
Figure 6649: PRO85237
Figure 6650: DNA304832, NP_057327.1, NM_016243_at
Figure 6651: PRO71239
Figure 6652: DNA328831, AF126780, NM_016245_at
Figure 6653: PRO233
Figure 6654: DNA328513, AF151895, NM_016283_at
Figure 6655: PRO37815
Figure 6656: DNA304781, LOC51184, NM_016301_at
Figure 6657: PRO71191
Figure 6658: DNA331820, BC001144, NM_016306_at
Figure 6659: PRO1080
Figure 6660: DNA331821, AK023410, NM_016354_at
Figure 6661: PRO86752
Figure 6662: DNA330390, AF178985, NM_016546_at
Figure 6663: PRO85599
Figure 6664: DNA331822, AF318357, NM_016553_at
Figure 6665: PRO86753
Figure 6666: DNA227298, NP_057649.1, NM_016565_at
Figure 6667: PRO37761
Figure 6668: DNA327869, NRN1, NM_016588_at
Figure 6669: PRO1898
Figure 6670: DNA331823, AK027682, NM_017424_at
Figure 6671: PRO86754
Figure 6672: DNA225694, FLJ20005, NM_017617_at
Figure 6673: PRO36157
Figure 6674: DNA326385, NP_060117.2, NM_017647_at
Figure 6675: PRO82778
Figure 6676: DNA287206, FLJ20073, NM_017654_at
Figure 6677: PRO69488

124

Figure 6678: DNA227294, FLJ20303, NM_017755_at
Figure 6679: PRO37757
Figure 6680: DNA226646, NP_060352.1, NM_017882_at
Figure 6681: PRO37109
Figure 6682: DNA331824, BC010907,
Figure 6683: PRO86755 NM_021618_at
Figure 6684: DNA330537, HELLS, NM_018063_at
Figure 6685: PRO81892
Figure 6686: DNA328628, BC011983, NM_018072_at
Figure 6687: PRO84406
Figure 6688: DNA328841, BC003082, NM_018087_at
Figure 6689: PRO84575
Figure 6690: DNA304780, AK027630, NM_018092_at
Figure 6691: PRO69889
Figure 6692: DNA227787, ASPM, NM_018123_at
Figure 6693: PRO38250
Figure 6694: DNA327942, AK001387, NM_18126_at
Figure 6695: PRO83870
Figure 6696: DNA325030, MGC5242, NM-018295_at
Figure 6697: PRO81619
Figure 6698: DNA331825, ChGn, NM_018371_ at
Figure 6699: PRO86756
Figure 6700: DNA226227, IL26, NM-018402_at
Figure 6701: PRO36690 Figure
Figure 6702: DNA331826, BC014129, NM_018407_at
Figure 6703: PRO81696
Figure 6704: DNA331827, BC007043, NM_018464_at
Figure 6705: PRO9902
Figure 6706: DNA227313, TPARL, NM_018475_at
Figure 6707: PRO37776
Figure 6708A-B: DNA331828, AB033051, NM_018695_at
Figure 6709: DNA304494, NP_061195.1, NM_018725_at
Figure 6710: PRO71061
Figure 6711: DNA227929, LOC54543, NM_019059_at
Figure 6712: PRO38392
Figure 6713: DNA329553, NP_064535.1, NM_020150_at
Figure 6714: PRO38313
Figure 6715: DNA227305, FN5, NM_020179_at
Figure 6716: PRO37768
Figure 6717: DNA227223, AF226054, NM_020198_at
Figure 6718: PRO37686
Figure 6719: DNA227280, NP_064615.1, NM_020230_at
Figure 6720: PRO37743
Figure 6721: DNA328926, AF180920, NM_020307_at
Figure 6722: PRO84643
Figure 6723: DNA331829, AF279144, NM_020405_at
Figure 6724: PRO85524
Figure 6725: DNA331830, BC001299, NM_020470_at
Figure 6726: PRO86757
Figure 6727A-B: DNA331831, HSA277248, NM_020525_at
Figure 6728: DNA151185, AF098642, NM_021105_at
Figure 6729: PRO12628
Figure 6730: DNA327199, DJ971N18.2, NM_021156_at
Figure 6731: PRO83475
Figure 6732: DNA227276, NP_005702.1, NM_017906_at
Figure 6733: PRO37739
Figure 6734A-B: DNA331832, AF051850, NM_021738_at
Figure 6735: PRO86758

Figure 6736: DNA331833, AF269133, NM_021798_at
Figure 6737: PRO86759
Figure 6738: DNA328364, AK025099, NM_021805_at
Figure 6739: PRO84223
Figure 6740: DNA331834, AF246221, NM_021999_at
Figure 6741: PRO86760
Figure 6742: DNA304465, AF277316, NM_022044_at
Figure 6743: PRO4424
Figure 6744: DNA331835, BC018724, NM_022107_at
Figure 6745: PRO37040
Figure 6746: DNA331836, BC002337, NM_022346_at
Figure 6747: DNA328902, LOC64174, NM_022355 _at
Figure 6749: DNA331837, AF201380, NM_022364_at
Figure 6748: PRO84623
Figure 6750: PRO71059
Figure 6751: DNA331090, FLJ22313, NM_022373_at
Figure 6752: PRO86241
Figure 6753: DNA327928, NP_072092.2, NM_022570_at
Figure 6754: PRO1082
Figure 6755: DNA331091, NP_076414.1, P-Z07192_at
Figure 6756: PRO86242
Figure 6757: DNA331838, HUMMYCDT, HSMYC1_at
Figure 6758: DNA228179, DNA228179, AF041429_at
Figure 6759: PRO38642
Figure 6760: DNA331092, NP_078918.2, AK024865_at
Figure 6761: PRO86243
Figure 6762: DNA331093, BC018094, AB029032_at
Figure 6763: DNA331094, HUMTCRGAAA, HSTCRGR_at
Figure 6764: DNA331839, AY027765, HUMCYCB_at
Figure 6765: DNA326640, CCNE1, HUMCYCE_at
Figure 6766: PRO4756
Figure 6767: DNA228020, DNA228020, AK026923_at
Figure 6768: PRO38483
Figure 6769A-B: DNA150452, AB011542, AB011542_at
Figure 6770A-B: DNA150476, AB020637, AB020637_at
Figure 6771: DNA331840, BC003648, AF040964_at
Figure 6772: PRO86761
Figure 6773: DNA150552, AF040965, AF040965 _at
Figure 6774A-B: DNA328805, AB032994, AP160973_at
Figure 6775A-B: DNA331841, HSCHTOG, HUMKG1BB_at
Figure 6776: PRO86762
Figure 6777A-B: DNA331842, BC004375, AF261758_at
Figure 6778: PRO38492
Figure 6779: DNA331095, NP_005216.1, HUME2F_at
Figure 6780: PRO86245
Figure 6781: DNA331843, AF202723, AB014568_at
Figure 6782: DNA159542, DNA159542, HUMMAC30X_at
Figure 6783: DNA331844, BC009267, HUMLAMBBA_at
Figure 6784: PRO82888
Figure 6785: DNA331096, 575881, P_V84330_at
Figure 6786: PRO86246
Figure 6787: DNA287239, AF212242, AK024843_at
Figure 6788: PRO38497
Figure 6789: DNA154390, DNA154390, HUMP13KIN_at
Figure 6790: DNA151247, DNA151247, P_V43601_at
Figure 6791: PRO11643
Figure 6792: DNA329950, MGC5576, P_V43613_at
Figure 6793: PRO11558

Figure 6794: DNA161927, DNA161927, P_Z29229_at
Figure 6795: DNA155316, DNA155316, P_A09058_at
Figure 6796: DNA329026, AF230200, AK021966_at
Figure 6797A-B: DNA228052, DNA228052, AB006624_at
Figure 6798: PRO38515
Figure 6799: DNA161913, DNA161913, HSM800208_at
Figure 6800: DNA331845, AK027432, HSM800284_at
Figure 6801: PRO86763
Figure 6802: DNA329430, SPPL2A, AX027882_at
Figure 6803: PRO38524
Figure 6804: DNA151422, DNA151422, P_X04312_at
Figure 6805: PRO11792
Figure 6806: DNA228066, NP_079431.1, AK021910_at
Figure 6807: PRO38529
Figure 6808A-C: DNA330360, FYCO1, AK023397_at
Figure 6809: PRO85576
Figure 6810: DNA287185, DNA287185, P_V84564_at
Figure 6811: PRO37492
Figure 6812: DNA331846, AF272741, HUMTCBYY_at
Figure 6813: DNA331097, AK027322, AX041977_at
Figure 6814: PRO86247
Figure 6815: DNA151756, DNA151756, P_X84947_at
Figure 6816: PRO12037
Figure 6817: DNA151761, DNA151761, P_X84970_at
Figure 6818: PRO12039
Figure 6819: DNA331847, BC008330, AK026632_at
Figure 6820: PRO38556
Figure 6821: DNA326258, MGC2941, AK026537_at
Figure 6822: PRO82665
Figure 6823A-B: DNA287330, AB032991, AB032991_at
Figure 6824: DNA331848, 1510819.1, P_X99863_at
Figure 6825: PRO86764
Figure 6826: DNA331849, HSINSP4BP, HSINSP4BP_at
Figure 6827: PRO66285
Figure 6828A-B: DNA331850, HSA237724, HSA237724_at
Figure 6829: DNA328049, 981676.1, HSM800856_at
Figure 6830: PRO83963
Figure 6831: DNA331851, AK027334, P_A51904_at
Figure 6832: PRO23392
Figure 6833: DNA193996, DNA193996, P_A40502_at
Figure 6834: PRO23400
Figure 6835: DNA194019, DNA194019, AK000004_at
Figure 6836: PRO23421
Figure 6837: DNA194063, DNA194063, P_V84608_at
Figure 6838: PRO23460
Figure 6839: DNA83046, NP_000565.1, P_X30170_at
Figure 6840: PRO2569
Figure 6841: DNA331852, 985629.1, P_Z59467_at
Figure 6842: PRO86765
Figure 6843: DNA195915, DNA195915, P_X85020_at
Figure 6844: DNA331853, BC001305, AK027031_at
Figure 6845: PRO23769
Figure 6846A-B: DNA328720, NP-078800.2, P_X35729_at
Figure 6847: PRO84476
Figure 6848: DNA194679, BAA05062.1, HUMORFT_at
Figure 6849: PRO23989
Figure 6850: DNA331854, AF244129, AF244129_at
Figure 6851: PRO86766

EP 2 179 742 A1

Figure 6852: DNA194766, DJ434O14.3, HS434O143_at
Figure 6853: PRO24046
Figure 6854: DNA331855, BLP1, P-Z98236_at
Figure 6855: PRO85742
Figure 6856: DNA330358, BC008904, AX011617_at
Figure 6857: PRO85574
Figure 6858: DNA330380, FLJ12436, AK022498_at
Figure 6859: PRO85592
Figure 6860: DNA328288, NP_073591.1, AK022938_at
Figure 6861: PRO69876
Figure 6862: DNA196036, DNA196036, AI471699_RC_at
Figure 6863: DNA331098, AY052405, AX047348_at
Figure 6864: PRO86248
Figure 6865A-B: DNA331099, AB058685, AX048187_at
Figure 6866: DNA331100, BC021238, P_X84987_at
Figure 6867: PRO86249
Figure 6868: DNA331101, NP_114143.1, 250446.2_at
Figure 6869: PRO86250
Figure 6870: DNA331856, BC022522, 237658.8_at
Figure 6871: PRO71209
Figure 6872: DNA106360, DNA106360, 164869.1_at
Figure 6873: DNA155526, DNA155526, 251178.1_at
Figure 6874: DNA331102, NP_116052.1, 481267.1_at
Figure 6875: PRO86251
Figure 6876: DNA196289, DNA196289, 230230.2_at
Figure 6877: DNA323696, BC015160, 428335.22_at
Figure 6878: DNA326749, NP_116101.1, DNA167237_at
Figure 6879: PRO23238
Figure 6880: DNA210391, DNA210391, P_X85039_at
Figure 6881: PRO34886
Figure 6882: DNA331103, NP_009125.1, NM_007194_at
Figure 6883: PRO34956
Figure 6884: DNA331857, SIRPB2, NM_018556_at
Figure 6885: PRO86767
Figure 6886: DNA254406, NP_060854.1, NM_018384_at
Figure 6887: PRO49516
Figure 6888A-B: DNA331858, ABCA7, NM_019112_at
Figure 6889: PRO86768
Figure 6890: DNA255704, NP_057570.1, NM_016486_at
Figure 6891: PRO50764
Figure 6892: DNA331859, AF267245, NM_016523_at
Figure 6893: PRO86769
Figure 6894: DNA254470, HSU11050, NM_002497_at
Figure 6895: PRO49578
Figure 6896A-B: DNA256461, HSAJ6266, NM_007086_at
Figure 6897: PRO51498
Figure 6898: DNA330480, FLJ10808, NM_018227_at
Figure 6899: PRO85677
Figure 6900: DNA328669, NP_005882.1, NM_005891_at
Figure 6901: PRO84441
Figure 6902: DNA254777, CORO1C,
Figure 6903: PRO49875 NM_014373_at
Figure 6904A-B: DNA329991, TIMELESS, NM_003920_at
Figure 6905: PRO85284
Figure 6906: DNA255161, IFRG28, NM_022147_at
Figure 6907: PRO50241
Figure 6908: DNA327812, IFI44, NM_006417_at
Figure 6909: PRO83773

128

Figure 6910: DNA304716, CDKN1A, NM_000389_at
Figure 6911: PRO71142
Figure 6912: DNA328431, HSCKSHS1, NM_001826_at
Figure 6913: PRO45093
Figure 6914: DNA269926, HSCDC2R, NM_001786_at
Figure 6915: PRO58324
Figure 6916: DNA331104, NP_066280.1, NM_021000_f_at
Figure 6917: PRO86252
Figure 6918A-B: DNA274893, NP_006282.1, NM_006291_at
Figure 6919: PRO62634
Figure 6920: DNA271455, LOC51339, NM_016651_at
Figure 6921: PRO59751
Figure 6922: DNA329172, GFI1, NM_005263_at
Figure 6923: PRO84796
Figure 6924: DNA331860, BC010940, NM_004833_at
Figure 6925: PRO86770
Figure 6926: DNA329274, AF187064, NM_014380_at
Figure 6927: PRO84870
Figure 6928A-B: DNA328535, NP_009147.2, NM_007216_at
Figure 6929: PRO60044
Figure 6930: DNA331861, MAP2K6, NM_002758_at
Figure 6931: PRO86771
Figure 6932: DNA331105, NP_009012.1, NM_007081_at
Figure 6933: PRO86253
Figure 6934: DNA256257, LAMP3, NM_014398_at
Figure 6935: PRO51301
Figure 6936: DNA256033, NP_060164.1, NM_017694_at
Figure 6937: PRO51081
Figure 6938A-B: DNA331106, NP_065107.1, NM_020374_at
Figure 6939: PRO86254
Figure 6940A-B: DNA254789, LOC51696, NM-016217_at
Figure 6941: PRO49887
Figure 6942A-B: DNA254376, AB023180, NM_014963_at
Figure 6943: PRO49486
Figure 6944: DNA254129, ARMET, NM_006010_at
Figure 6945: PRO49244
Figure 6946: DNA331862, AX008892, NM_005484_at
Figure 6947: PRO86772
Figure 6948: DNA256407, HSA249248, NM_014325_at
Figure 6949: PRO51448
Figure 6950A-C: DNA256495, HSU33841, NM-000051_at
Figure 6951: PRO51531
Figure 6952: DNA330384, FLJ20647, NM_017918_at
Figure 6953: PRO51129
Figure 6954: DNA331863, AK000318, NM_017760_at
Figure 6955: PRO86773
Figure 6956: DNA256533, NP_006105.1, NM_006114_at
Figure 6957: PRO51565
Figure 6958A-B: DNA287273, AF092563, NM_006444_at
Figure 6959: PRO69545
Figure 6960: DNA256295, DNA256295, NM_002319_at
Figure 6961: PRO51339
Figure 6962A-C: DNA331864, HSA303086, NM_002266_at
Figure 6963: DNA254350, AF002697, NM_004052_at
Figure 6964: PRO49461
Figure 6965: DNA255010, NP_061869.1, NM_018996_at
Figure 6966: PRO50099
Figure 6967: DNA329900, HUMA1SBU, NM_002914_at

Figure 6968: PRO8154
Figure 6969: DNA323838, CDKN2C, NM_001262_at
Figure 6970: PRO59546
Figure 6971: DNA271093, CNK, NM_004073_at
Figure 6972: PRO59417
Figure 6973: DNA331108, NP_005265.1, NM_005274_at
Figure 6974: PRO10780
Figure 6975: DNA290234, RGS2, NM_002923_at
Figure 6976: PRO70333
Figure 6977: DNA275015, HSU31383, NM_004125_at
Figure 6978: PRO62743
Figure 6979: DNA256854, HSU76638, NM_000465_at
Figure 6980: PRO51785
Figure 6981: DNA331865, IRF7, NM_004031_at
Figure 6982: PRO86774
Figure 6983: DNA255271, EMR2, NM_013447_at
Figure 6984: PRO50348
Figure 6985: DNA331866, AB020970, NM_0221544_at
Figure 6986: DNA331867, AF058762, NM_003857_at
Figure 6987A-B: DNA331109, NP_005155.1, NM_005164_at
Figure 6988: PRO50662
Figure 6989: DNA331110, NP_057563.3, NM_016479_at
Figure 6990: PRO86256
Figure 6991: DNA254276, HSPC154, NM_014177_at
Figure 6992: PRO49387
Figure 6993: DNA287241, LAP3, NM_015907_at
Figure 6994: PRO69516
Figure 6995A-B: DNA331111, NP_004229.1, NM_004238_at
Figure 6996: PRO86257
Figure 6997: DNA255261, NP_060262.1, NM_017792_at
Figure 6998: PRO50338
Figure 6999A-B: DNA331868, HSM802180, NM_017631_at
Figure 7000: DNA331869, NP_067024.1, NM_021201_at
Figure 7001: PRO50191
Figure 7002A-B: DNA255846, NP_057424.1, NM_016340_at
Figure 7003: PRO50900
Figure 7004: DNA331870, AK001274, NM_017613_at
Figure 7005: PRO86776
Figure 7006: DNA287221, LOC51191, NM_016323_at
Figure 7007: PRO69500
Figure 7008: DNA331871, BC017842, NM_004851_at
Figure 7009: PRO86777
Figure 7010: DNA260982, NP_060819.1, NM_018349_at
Figure 7011: PRO54728
Figure 7012: DNA329033, NP_005375.1, NM_005384_at
Figure 7013: PRO84700
Figure 7014: DNA271095, AF091433, NM_004702_at
Figure 7015: PRO59418
Figure 7016: DNA297387, NP_003494.1, NM_003503_at
Figure 7017: PRO58394
Figure 7018: DNA331872, AF099644, NM_001255_at
Figure 7019: PRO86778
Figure 7020: DNA331873, PTPN7, NM_002832_at
Figure 7021: PRO69609
Figure 7022: DNA269750, NP_002919.1, NM_002928_at
Figure 7023: PRO58159
Figure 7024: DNA268036, AB023416, NM_013258_at
Figure 7025: PRO57311

Figure 7026: DNA270522, NP_006013.1, NM_006022_at
Figure 7027: PRO58899
Figure 7028: DNA330057, MT1G, NM_005950_at
Figure 7029: PRO85337
Figure 7030A-B: DNA331113, NP_005914.1, NM_005923_at
Figure 7031: PRO60244
Figure 7032: DNA331874, BC002847, NM_016103_at
Figure 7033: PRO84581
Figure 7034: DNA269791, NP_001168.1, NM-001177_at
Figure 7035: PRO58197
Figure 7036: DNA331114, AF291719, NM_007182_at
Figure 7037: PRO86258
Figure 7038: DNA329580, HSU78170, NM_005825_at
Figure 7039: PRO85114
Figure 7040: DNA281436, NP_003286.1, NM_017627_at
Figure 7041: PRO66275
Figure 7042: DNA331875, CDC25B, NM_021874_at
Figure 7043: PRO83123
Figure 7044: DNA331876, BC005912, NM_002001_at
Figure 7045: PRO2280
Figure 7046: DNA256737, FLJ20406, NM_017806_at
Figure 7047: PRO51671
Figure 7048: DNA255432, NP_060283.1, NM_017813_at
Figure 7049: PRO50499
Figure 7050A-B: DNA254262, NP_055197.1, NM_014382_at
Figure 7051: PRO49373
Figure 7052: DNA331877, HUMHMGAB, NM_000859_at
Figure 7053: DNA328901, BC002748, NM_017866_at
Figure 7054: PRO84622
Figure 7055: DNA254416, NP_060915.1, NM_018445_at
Figure 7056: PRO49526
Figure 7057: DNA331115, AF221521, NM_020524_at
Figure 7058: PRO86259
Figure 7059: DNA255326, AF055993, NM_003864_at
Figure 7060: PRO50396
Figure 7061A-C: DNA328498, AF285167, NM_005502_at
Figure 7062: PRO84320
Figure 7063: DNA331878, AF246240, NM_018480_at
Figure 7064: PRO86779
Figure 7065: DNA331879, AK021999, NM_022765_at
Figure 7066: PRO86780
Figure 7067: DNA255135, AB016068, NM_005857_at
Figure 7068: PRO50216
Figure 7069: DNA331116, NP_060656.1, NM_018186_at
Figure 7070: PRO86260
Figure 7071: DNA237817, HSU33286, NM_001316_at
Figure 7072: PRO38923
Figure 7073A-B: DNA329904, AF203032, NM_021076_at
Figure 7074: PRO85221
Figure 7075: DNA329583, AD24, NM_022451_at
Figure 7076: PRO85117
Figure 7077: DNA331117, NP_065170.1, NM_020437_at
Figure 7078: PRO86261
Figure 7079: DNA254710, FLJ20637, NM_017912_at
Figure 7080: PRO49810
Figure 7081: DNA331880, HSIFI56R, NM_001548_at
Figure 7082: PRO59911
Figure 7083A-B: DNA270323, HSU34605, NM_012420_at

Figure 7084: PRO58710
Figure 7085: DNA287224, ISG15, NM_005101_at
Figure 7086: PRO69503
Figure 7087: DNA269922, HSISG20GN, NM_002201_at
Figure 7088: PRO58320
Figure 7089: DNA331118, NP_201569.1, NM_003672_at
Figure 7090: PRO86262
Figure 7091: DNA272655, HSCKSHS2, NM_001827_at
Figure 7092: PRO60781
Figure 7093A-B: DNA329160, NP_002821.1, NM_002830_at
Figure 7094: PRO84789
Figure 7095: DNA270415, GNA15, NM_002068_at
Figure 7096: PRO58796
Figure 7097: DNA331881, AK023223, NM-016131_at
Figure 7098: PRO10928
Figure 7099: DNA270059, NP_003920.1, NM_003929_at
Figure 7100: PRO58452
Figure 7101: DNA273487, RAB33A, NM-_04794_at
Figure 7102: PRO61470
Figure 7103: DNA326306, NP_066960.1, NM_021137_at
Figure 7104: PRO62566
Figure 7105: DNA287378, AF244135, NM_018428_at
Figure 7106: PRO69637
Figure 7107: DNA327879, MDA5, NM_022168_at
Figure 7108: PRO83818
Figure 7109A-B: DNA327674, NP_002739.1, NM_002748_at
Figure 7110: PRO83661
Figure 7111: DNA331882, AB030251, NM_013277_at
Figure 7112: PRO86781
Figure 7113: DNA254518, LOC51713, NM_016270_at
Figure 7114: PRO49625
Figure 7115: DNA256561, CRTAM, NM_019604_at
Figure 7116: PRO51592
Figure 7117: DNA331883, AF096290, NM_003645_at
Figure 7118: PRO51139
Figure 7119: DNA255215, AF207600, NM_018638_at
Figure 7120: PRO50294
Figure 7121A-B: DNA256807, FAM8A1, NM_016255_at
Figure 7122: PRO51738
Figure 7123: DNA260974, TRIM22, NM_006074_at
Figure 7124: PRO54720
Figure 7125: DNA330443, PRO2037, NM_018616_at
Figure 7126: PRO2037
Figure 7127: DNA331119, NP_005433.2, NM_005442_at
Figure 7128: PRO50745
Figure 7129: DNA254274, NP_073573.1, NM_022736_at
Figure 7130: PRO49385
Figure 7131: DNA255088, HUMTK, NM_003258_at
Figure 7132: PRO50174
Figure 7133: DNA255113, FLJ22693, NM_022750_at
Figure 7134: PRO50195
Figure 7135: DNA331884, BC008870, NM_017606_at
Figure 7136: PRO49604
Figure 7137: DNA331885, MRPL35, NM_016622_at
Figure 7138: PRO86782
Figure 7139: DNA272245, NP_055301.1, NM_014486_F_at
Figure 7140: PRO60507
Figure 7141A-D: DNA331886, AF051160, NM_003463_at

Figure 7142: DNA330877, HKE2, NM_014260_at
Figure 7143: PRO86040
Figure 7144: DNA295327, IL21, NM_021803_at
Figure 7145: PRO70773
Figure 7146: DNA287178, HSU52513, NM_001549_at
Figure 7147: PRO69467
Figure 7148: DNA327661, HUMIFI16A, NM_005531_at
Figure 7149: PRO83652
Figure 7150A-B: DNA329036, HSU63738, NM_002460_at
Figure 7151: PRO84703
Figure 7152: DNA273523, NP_002154.1, NM_002163_at
Figure 7153: PRO61504
Figure 7154: DNA331887, AF002822, NM_004701_at
Figure 7155: PRO82442
Figure 7156: DNA331888, AF022109, NM_001254_at
Figure 7157: PRO60595
Figure 7158: DNA273535, AB011421, NM-004226_at
Figure 7159: PRO61515
Figure 7160: DNA275012, NMI, NM_004688_at
Figure 7161: PRO62740
Figure 7162: DNA331120, NP_008976.1, NM-007045_at
Figure 7163: PRO86263
Figure 7164: DNA331889, AF182076, NM_015710_at
Figure 7165: PRO84173
Figure 7166: DNA331890, AF095287, NM_004219_f_at
Figure 7167: PRO81319
Figure 7168: DNA331121, AF175306, NM_014288_at
Figure 7169: PRO86264
Figure 7170: DNA327858, AF120334, NM_012341_at
Figure 7171: PRO83800
Figure 7172: DNA331122, NP_005728.2, NM_005737_at
Figure 7173: PRO86265
Figure 7174: DNA289528, ARL3, NM_004311_at AB037815_at
Figure 7175: PRO70286
Figure 7176: DNA270526, HUMLYGDI, NM_001175_at
Figure 7177: PRO58903
Figure 7178: DNA271931, NP_005745.1, NM_005754_at
Figure 7179: PRO60207
Figure 7180: DNA329123, RANBP1, NM_002882_at
Figure 7181: PRO84765
Figure 7182A-B: DNA331891, HSM800983, NM_014922_at
Figure 7183: DNA331892, BC019255, NM_006452_at
Figure 7184: PRO84240
Figure 7185: DNA329587, AF192466, NM_012124_at
Figure 7186: PRO85121
Figure 7187A-B: DNA329248, AB002359, AB002359_at
Figure 7188: DNA254668, AB002437, AB002437_at
Figure 7189: DNA256233, DNA256233, AB017268_F_at
Figure 7190: PRO51278
Figure 7191A-B: DNA255448, BAA92554.1, AB037737_at
Figure 7192: PRO50515
Figure 7193A-B: DNA255619, AF054589, AF054589_at
Figure 7194: PRO50682
Figure 7195: DNA331123, AF062649, AF062649_f_at
Figure 7196: PRO86266
Figure 7197A-B: DNA331893, AB058697, AK001581_at
Figure 7198: DNA331894, HSM802273, AB032963_at
Figure 7199: DNA331895, HUMTLEIV, AB033087_at

Figure 7200: PRO86785
Figure 7201A-B: DNA329039, DORFIN, AK027070_at
Figure 7202: PRO84706
Figure 7203: DNA255040, CAB55998.1, HSM801103_at
Figure 7204: PRO50128
Figure 7205: DNA328509, NP_006739.1, HSU44403_at
Figure 7206: PRo57996
Figure 7207: DNA254338, HUMPLT, HLTMPLT_at
Figure 7208: PRO49449
Figure 7209: DNA331896, AF067008, NM_001363_at
Figure 7210: PRO49881
Figure 7211: DNA331897, BC008843, AB007915_at
Figure 7212: PRO86786
Figure 7213: DNA331124, NP_079430.1, AB018353_at
Figure 7214: PRO86267
Figure 7215A-B: DNA330736, AB033044, AB033044_at
Figure 7216A-B: DNA331125, AB037815,
Figure 7217A-B: DNA331898, AF058925, AF058925_at
Figure 7218: PRO86787
Figure 7219: DNA331126, AF078867, AF078866_at
Figure 7220: PRO86269
Figure 7221: DNA254836, BAA91233.1, AK000529_at
Figure 7222: PRO49931
Figure 7223: DNA88277, NP_006721.1, AK027197_at
Figure 7224: PRO2724
Figure 7225: DNA331899, 1399286.1, AW290940_RC_at
Figure 7226: PRO86788
Figure 7227: DNA256872, HSM801990, HSM801990_at
Figure 7228A-B: DNA254192, HUMKIAAK, HUMKIAAK_at
Figure 7229: DNA331900, BIN2, NM_016293_at
Figure 7230: PRO86789
Figure 7231A-B: DNA256731, BAA83028.1, AB028999_at
Figure 7232: PRO51665
Figure 7233: DNA331901, HSM801036, AB029015_at
Figure 7234A-B: DNA331127, BAA86477.1, AB032989_at
Figure 7235: PRO86270
Figure 7236A-B: DNA254672, BAA92652.1, AB037835_at
Figure 7237: PRO49773
Figure 7238A-C: DNA331128, NP_065892.1, AB040884_at
Figure 7239: PRO84841
Figure 7240: DNA269976, AAC14260.1, AF039023_at
Figure 7241: PRO58372
Figure 7242: DNA331129, HSA227869, HSA227869_r_at
Figure 7243: DNA256422, HSA227900, HSA227900_at
Figure 7244: DNA331902, BC014522, HSSOM172M_at
Figure 7245: PRO86790
Figure 7246: DNA329040, BC001356, HSU72882_at
Figure 7247: PRO84707
Figure 7248: DNA331130, AAK50430.1, HUMTI227HC_at
Figure 7249: PRO86272
Figure 7250A-B: DNA331131, HSA223948, AY013288_at
Figure 7251: DNA326056, NP_072088.1, AY007810_at
Figure 7252: PRO82491
Figure 7253: DNA329041, HSM800399, AF132199_at
Figure 7254: DNA255780, AK022209, AK022209_at
Figure 7255: PRO50835
Figure 7256: DNA254922, AK022604, AK022604_at
Figure 7257: PRO50012

Figure 7258: DNA330432, FLJ23235, AK026888_at
Figure 7259: PRO85636
Figure 7260A-B: DNA256299, AB051489, ABO51489_at
Figure 7261: DNA331903, BC015380, HSM801707_at
Figure 7262: DNA255626, HSM802849, HSM802849_at
Figure 7263: PRO50690
Figure 7264: DNA331132, NP_115524.1, HSM801796_at
Figure 7265: PRO86273
Figure 7266: DNA255964, NP_079113.1, AK025125_at
Figure 7267: PRO51015
Figure 7268: DNA255465, AK024313, AK024313_at
Figure 7269: PRO50532
Figure 7270: DNA329597, AK022178, AK022178_at
Figure 7271: PRO85129
Figure 7272: DNA254228, NP_079236.1, AK021791_at
Figure 7273: PRO49340
Figure 7274: DNA331904, AK023431, AF298880_at
Figure 7275: PRO86791
Figure 7276: DNA329078, AF214006, HSM801679_at
Figure 7277: PRO23253
Figure 7278: DNA256784, FLJ22104, AK025757_at
Figure 7279: PRO51716
Figure 7280: DNA331905, AK001823, HSM801648_at
Figure 7281: PRO86792
Figure 7282: DNA329044, NP_064562.1, AK025265_at
Figure 7283: PRO84709
Figure 7284: DNA331906, HSA227916, NM_001530_at
Figure 7285: DNA330023, GADD45A, NM_001924_at
Figure 7286: PRO85308
Figure 7287A-B: DNA272191, RSN, NM_002956_at
Figure 7288: PRO60456
Figure 7289: DNA328418, HUMG0S24A, NM_003407_at
Figure 7290: PRO84261
Figure 7291: DNA331133, HSU63830, NM_004180_at
Figure 7292: PRO86274
Figure 7293: DNA271310, DUSP8, NM-004420_at
Figure 7294: PRO59617
Figure 7295: DNA331907, AKAP7, NM_004842_at
Figure 7296: PRO63228
Figure 7297: DNA287203, NP_006182.1, NM_006191_at
Figure 7298: PRO69487
Figure 7299: DNA274783, HSU26424, NM_006281_at
Figure 7300: PRO62549
Figure 7301A-B: DNA255281, NP_006380.1, NM_006389_at
Figure 7302: PRO50357
Figure 7303: DNA328712, NP_006501.1, NM_006510_at
Figure 7304: PRO84469
Figure 7305: DNA331908, AF161440, NM_012111_at
Figure 7306: DNA330065, STK18, NM_014264_at
Figure 7307:PRO85345
Figure 7308: DNA152148, DNA152148, HSP1CDC21_at
Figure 7309: PRO10290
Figure 7310: DNA329925, HSBP1, NM_001537_at
Figure 7311: PRO85239
Figure 7312: DNA331909, HSCFANT, NM_002964_at
Figure 7313: PRO86795
Figure 7314: DNA329139, NP_003893.2, NM_003902_at
Figure 7315: PRO84774

Figure 7316: DNA331910, HSSEC232, NM_006363_at
Figure 7317: PRO86796
Figure 7318: DNA329047, BATF, NM_006399_at
Figure 7319: PRO58425
Figure 7320: DNA274167, AF026166, NM_006431_at
Figure 7321: PRO62097
Figure 7322: DNA254572, NP_006576.1, NM_006585_at
Figure 7323: PRO49675
Figure 7324A-B: DNA331911, AB003334, NM_006644_at
Figure 7325: PRO86797
Figure 7326: DNA331912, BC009405, NM_013411_at
Figure 7327: PRO86798
Figure 7328: DNA255289, MELK, NM_014791_at
Figure 7329: PRO50363
Figure 7330A-B: DNA331913, BAB21784.1, NM_015383_at
Figure 7331: PRO86799
Figure 7332: DNA329148, LOC51042, NM_015871_at
Figure 7333: PRO84782
Figure 7334: DNA326221, AF125098, NM_016095_at
Figure 7335: PRO82634
Figure 7336: DNA331914, BC009398, HUMP1CDC47_at
Figure 7337: PRO86800
Figure 7338A-B: DNA328312, HUMAREB6, HUMAREB6_at
Figure 7339: PRO84180
Figure 7340: DNA325941, HSPCA, HSHSP90R_at
Figure 7341: PRO82388
Figure 7342: DNA328483, VIT1, NM_000179_at
Figure 7343: PRO84309
Figure 7344: DNA271847, HUMDNAJHOM, NM_001539_at
Figure 7345: PRO60127
Figure 7346: DNA331915, BC001786, NM_002014_at
Figure 7347: PRO59262
Figure 7348: DNA331916, HUMMIF, NM_002415_at
Figure 7349: DNA331917, PHF1, NM_002636_at
Figure 7350: PRO86802
Figure 7351: DNA329604, SRP54, NM_003136_at
Figure 7352: PRO85134
Figure 7353A-B: DNA331134, NP_003381.1, NM_003390_at
Figure 7354: PRO86275
Figure 7355A-B: DNA290265, ZNF91, NM_003430_f_at
Figure 7356: PRO70395
Figure 7357A-C: DNA331918, AF009425, NM_004338_at
Figure 7358: PRO86803
Figure 7359: DNA254582, NP_004626.1, NM_004635_at
Figure 7360: PRO49685
Figure 7361A-B: DNA275334, NP_112162.1, NM_004749_at
Figure 7362: PRO63009
Figure 7363: DNA254157, HSU13045, NM_005254_at
Figure 7364: PRO49271
Figure 7365A-B: DNA124122, RBL2, NM_005611_at
Figure 7366: PRO6323
Figure 7367: DNA330776, TOB 1, NM_005749_at
Figure 7368: PRO58014
Figure 7369: DNA326980, AF140598, NM_014248_at
Figure 7370: PRO83289
Figure 7371: DNA271608, HUMRSC419, NM_014670_at
Figure 7372: PRO59895
Figure 7373: DNA272928, HUMORFKG1F, NM_014764_at

EP 2 179 742 A1

Figure 7374: PRO61012
Figure 7375: DNA290235, NP_057121.1, NM_016037_at
Figure 7376: PRO70335
Figure 7377: DNA331135, HUMKG1DD, HUMKG1DD_at
Figure 7378A-B: DNA330119, AF226044, HUMKIAAQ_at
Figure 7379: RO85381
Figure 7380: DNA331137, HS24P52, HUMHSP70H_at
Figure 7381: PRO86278
Figure 7382A-B: DNA269805, NP_001263.1, NM_001272_at
Figure 7383: PRO58209
Figure 7384: DNA270689, HSGATA3R, NM_002051_at
Figure 7385: PRO59053
Figure 7386: DNA331919, HUMCFA, NM_002965_at
Figure 7387: PRO80648
Figure 7388A-B: DNA304800, NP_004146.1, NM_004155_at
Figure 7389: PRO69458
Figure 7390: DNA273418, AAG01157.1, NM_004301_at
Figure7391 : PRO61417
Figure 7392: DNA330066, MLLT3, NM_004529_at
Figure 7393: PRO85346
Figure 7394: DNA270733, S46622, NM_005605_at
Figure 7395: PRO59094
Figure 7396: DNA331138, NP_005997.2, NM_006006_at
Figure 7397: PRO86279
Figure 7398: DNA331139, NP_006865.1, NM_006874_at
Figure 7399: PRO81172
Figure 7400: DNA331920, AF090950, NM_015675_at
Figure 7401: PRO84384
Figure 7402: DNA329050, MRPS17, NM_015969_at
Figure 7403: PRO84712
Figure 7404A-B: DNA329122, GS3955, NM_021643_at
Figure 7405: PRO84764
Figure 7406: DNA331921, 244055.1, AF320911-at
Figure 7407: PRO86804
Figure 7408: DNA331922, AK026275, AK026275_at
Figure 7409: PRO86805
Figure 7410A-B: DNA254516, AF288399, AF288399_at
Figure 7411: PRO49623
Figure 7412: DNA328313, NP_115579.1, AK025076_at
Figure 7413: PRO84181
Figure 7414: DNA327865, NP_079105.1, AK026315_at
Figure 7415: PRO83806
Figure 7416: DNA294813, NP-444283.1, P-T67134_at
Figure 7417: PRO70763
Figure 7418A-B: DNA254706, AB046851, AB046851_at
Figure 7419: DNA329052, NP_078801.1, AK026237_at
Figure 7420: PRO84714
Figure 7421: DNA256890, BC008988, P200467_at
Figure 7422: PRO51824
Figure 7423: DNA256291, FLJ21032, AK024685_f_at
Figure 7424: PRO51335
Figure 7425: DNA331923, HSUCP2X12, P-C69111_at
Figure 7426: DNA213665, DNA213665, P_X30166_at
Figure 7427: PRO35126
Figure 7428: DNA331140, 332752.10, AK023798_at
Figure 7429: PRO86280
Figure 7430A-B: DNA331141, BAB 13420.1, AB046814_at
Figure 7431: PRO86281

Figure 7432: DNA331924, BC004932, AK024551_at
Figure 7433: PRO21434
Figure 7434A-B: DNA256267, AB046838, AB046838_at
Figure 7435: DNA327954, BAL, P_D00629_at
Figure 7436: PRO83879
Figure 7437: DNA255798, FLJ12377, AK022439_at
Figure 7438: PRO50853
Figure 7439: DNA330389, FLJ12888, AK022950_at
Figure 7440: PRO85598
Figure 7441: DNA330086, FLJ12973, AK023035_at
Figure 7442: PRO85360
Figure 7443: DNA331142, NP_116325.1, P.Z98137at
Figure 7444: PRO51781
Figure 7445: DNA329384, BC008502, P-Z33372_at
Figure 7446: PRO84960
Figure 7447A-B: DNA331143, NP_149075.2, AK022613_at
Figure 7448: PRO86282
Figure 7449: DNA331925, 424693.10, AK022231_at
Figure 7450: PRO86806
Figure 7451: DNA331144, NP_078834.1, AK023982-at
Figure 7452: PRO86283
Figure 7453A-B: DNA331926, BAB13449.1, AB046843_at
Figure 7454: PRO51258
Figure 7455: DNA255197, DNA255197, P_Z50392_at
Figure 7456: PRO50276
Figure 7457: DNA328010, NP_149016.1, HSM801092_at
Figure 7458: PRO83928
Figure 7459: DNA262805, DNA262805, HSM800425_at
Figure 7460: DNA331146, 1400830.1, HUMJNLTRA_at
Figure 7461: PRO86284
Figure 7462: DNA328317, cig5, AF026941_at
Figure 7463: PRO69493
Figure 7464: DNA331147, NP_079104.1, AF131768_at
Figure 7465: PRO86285
Figure 7466: DNA255770, DNA255770, AK022106_at
Figure 7467A-C: DNA254412, EVI5, AF008915_at
Figure 7468: PRO49522
Figure 7469: DNA331148, 978273.10, AK023244_at
Figure 7470: PRO86286
Figure 7471: DNA330532, AK026279, AK026279_at
Figure 7472: PRO85719
Figure 7473: DNA330388, FLJ23468, AK027121_at
Figure 7474: PRO85597
Figure 7475: DNA331927, AK026969, AK026969_at
Figure 7476: PRO86807
Figure 7477: DNA330447, FLJ22757, AK026410_t
Figure 7478: PRO85648
Figure 7479: DNA324984, FLJ12298, AK022360_at
Figure 7480: PRO81578
Figure 7481: DNA331149, 7697327.1, HSM802839_at
Figure 7482: PRO86287
Figure 7483A-B: DNA256267, DNA256267, AK023113_at
Figure 7484: PRO51311
Figure 7485: DNA331150, BC017725, 1387341.2_at
Figure 7486: PRO86288
Figure 7487: DNA257606, DNA257606, 428093.1_at
Figure 7488: DNA258375, AF283301, 413231.5_at
Figure 7489: PRO52516

Figure 7490: DNA331928, AK027419, 154551.10_at
Figure 7491: PRO86808
Figure 7492: DNA328319, BC019562, 411364.2_at
Figure 7493: DNA290812, DNA290812, 220495.3_CON_at
Figure 7494: PRO70559
Figure 7495: DNA304799, BC022410, 337588.1_at
Figure 7496: PRO52633
Figure 7497: DNA257403, DNA257403, 012814.1_at
Figure 7498: DNA304820, NP_115940.1, 317557.1_at
Figure 7499: PRO47351
Figure 7500: DNA331929, BC019246, 441855.8_CON_at
Figure 7501: PRO83338
Figure 7502: DNA260581, DNA260581, 127987.6_at
Figure 7503: PRO54507
Figure 7504: DNA257576, DNA257576, 334945.2-at
Figure 7505: DNA304819, BC004398, 202113.2_at
Figure 7506: DNA304794, FBXO30, 222128.1_at
Figure 7507: PRO71206
Figure 7508: DNA259323, DNA259323, 022997.1_at
Figure 7509: PRO53256
Figure 7510: DNA304796, MED8, 237428.13_at
Figure 7511: PRO71208
Figure 7512: DNA259615, DNA259615, 1000203.1_at
Figure 7513: DNA304805, AK027628, 475113.7_at
Figure 7514: PRO69531
Figure 7515: DNA304793, GBP4, 206425.2_at
Figure 7516: PRO71205
Figure 7517: DNA331151, 018033.1, 018033.1_CON_at
Figure 7518: PRO86289
Figure 7519: DNA304068, AK057631, 1091656.1_at
Figure 7520: PRO71035
Figure 7521: DNA257714, EPSTI1, 337352.17_at
Figure 7522: PRO52268
Figure 7523: DNA304798, NP-443097.1, 246119.7_at
Figure 7524: PRO71210
Figure 7525: DNA258721, DNA258721, 197627.1_at
Figure 7526A-B: DNA257461, NP_113607.1, 086533.1_at
Figure 7527: PRO52040
Figure 7528: DNA331152, 1042156.3, 1042156.3_at
Figure 7529: PRO86290
Figure 7530: DNA331153, 004052.1, 004052.1_at
Figure 7531: PRO86291
Figure 7532: DNA331930, AK054582, 978231.1_at
Figure 7533: PRO86809
Figure 7534: DNA259587, DNA259587, 220866.1_at
Figure 7535: DNA106195, DNA106195, 359193.13_at
Figure 7536: DNA331154, 212376.1, 212376.1_at
Figure 7537: PRO86292
Figure 7538: DNA331155, 112652.1, 112652.1_at
Figure 7539: PRO86293
Figure 7540: DNA304806, BC019022, 983343.1_at
Figure 7541: PRO71215
Figure 7542: DNA262708, DNA262708, 118516.1_RC_at
Figure 7543: DNA259475, DNA259475, 239162.1_at
Figure 7544: DNA269148, DNA269148, 411192.2_at
Figure 7545: DNA304817, BC015532, 211436.3_at
Figure 7546: PRO71224
Figure 7547: DNA260313, DNA260313, 1098929.1_at

Figure 7548: PRO54242
Figure 7549A-B: DNA328325, NP_061142.1, 445188.4_at
Figure 7550: PRO84190
Figure 7551A-B: DNA304800, SERPINB9, 354740.1_at
Figure 7552: PRO69458
Figure 7553: DNA331156, 118180.1, 118180.1_at
Figure 7554: PRO86294
Figure 7555: DNA287659, AK027790, 406833.1_at
Figure 7556: PRO69903
Figure 7557: DNA331931, 092555.3, 092555.4_at
Figure 7558: PRO86810
Figure 7559: DNA331157, NP-439896.1, 022541.5_at
Figure 7560: PRO86295
Figure 7561: DNA260573, DNA260573, 899597.1_at
Figure 7562: PRO54499
Figure 7563: DNA260157, DNA260157, 236833.1_at
Figure 7564: PRO54086
Figure 7565: DNA174145, DNA174145, 100083.2_at
Figure 7566: PRO35770
Figure 7567: DNA260167, DNA260167, 264556.1-at
Figure 7568A-B: DNA331932, 239260.1, 239260.1_at
Figure 7569: PRO86811
Figure 7570: DNA260031, DNA260031, 161526.1_at
Figure 7571: DNA258907, DNA258907, 347940.2_at
Figure 7572: PRO52840
Figure 7573: DNA257455, DNA257455, 977723.3_at
Figure 7574: PRO52035
Figure 7575: DNA304807, BC014978, 005415.2_at
Figure 7576: PRO71216
Figure 7577: DNA258864, DNA258864, 331965.1_at
Figure 7578: DNA304811, 428051.2, 428051.2_at
Figure 7579: PRO71220
Figure 7580: DNA257389, FLJ14906, 987098.1_at
Figure 7581: PRO51974
Figure 7582: DNA331158, 130352.1, 130352.1_at
Figure 7583: PRO86296
Figure 7584: DNA258951, DNA258951, 222361.1_at
Figure 7585: DNA331159, NP_077291.1, 411426.29_at
Figure 7586: PRO86297
Figure 7587: DNA257784, DNA257784, 481853.1_at
Figure 7588: DNA331933, AF272148, 074299.1_at
Figure 7589: PRO86812

BRIEF DESCRIPTION OF THE DRAWINGS

In the list of figures for the present application, specific cDNA sequences which are differentially expressed in differentiated macrophages as compared to normal undifferentiated monocytes are individually identified with a specific alphanumerical designation. These cDNA sequences are differentially expressed in monocytes that are specifically treated as described in Example 1 below. If start and/or stop codons have been identified in a cDNA sequence shown in the attached figures, they are shown in bold and underlined font, and the encoded polypeptide is shown in the next consecutive figure.

The Figures 1-7589 show the nucleic acids of the invention and their encoded PRO polypeptides. Also included, for convenience is a List of Figures, which gives the figure number and the corresponding DNA or PRO number.

DETAILED DESCRIPTION OF THE PREFERRED EMBODIMENTS

I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X", "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http:l/www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficall/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')$_2$, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')$_2$ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')$_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$-$V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc.*

The term "T cell mediated disease" means a disease in which T cells directly or indirectly mediate or otherwise contribute to a morbidity in a mammal. The T cell mediated disease may be associated with cell mediated effects, lymphokine mediated effects, *etc.,* and even effects associated with B cells if the B cells are stimulated, for example, by the lymphokines secreted by T cells.

Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease. Infectious diseases including viral diseases such as AIDS (HIV infection), hepatitis A, B, C, D, and E, herpes, *etc.,* bacterial infections, fungal infections, protozoal infections and parasitic infections.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (e.g., $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-a, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1$\alpha$, IL-2, IL-3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

## Table 1

```
/*
 *
 * C-C increased from 12 to 15
 * Z is average of EQ
 * B is average of ND
 * match with stop is _M; stop-stop = 0; J (joker) match = 0
 */
#define  _M      -8        /* value of a match with a stop */

int      _day[26][26] = {
/*     A B C D E F G H I J K L M N O P Q R S T U V W X Y Z*/
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */   {_M,_M,_M,_M,_M,_M,_M,_M,_M,_M,_M,_M,_M,_M, 0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   { 1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

## Table 1 (cont')

```
/*
*/
#include <stdio.h>
#include <ctype.h>

#define   MAXJMP      16        /* max jumps in a diag */
#define   MAXGAP      24        /* don't continue to penalize gaps larger than this */
#define   JMPS        1024      /* max jmps in an path */
#define   MX          4         /* save if there's at least MX-1 bases since last jmp */

#define   DMAT        3         /* value of matching bases */
#define   DMIS        0         /* penalty for mismatched bases */
#define   DINS0       8         /* penalty for a gap */
#define   DINS1       1         /* penalty per base */
#define   PINS0       8         /* penalty for a gap */
#define   PINS1       4         /* penalty per residue */

struct jmp {
          short           n[MAXJMP];        /* size of jmp (neg for dely) */
          unsigned short  x[MAXJMP];        /* base no. of jmp in seq x */
};                                          /* limits seq to 2^16 -1 */

struct diag {
          int             score;            /* score at last jmp */
          long            offset;           /* offset of prev block */
          short           ijmp;             /* current jmp index */
          struct jmp      jp;               /* list of jmps */
};

struct path {
          int     spc;                /* number of leading spaces */
          short   n[JMPS]; /* size of jmp (gap) */
          int     x[JMPS]; /* loc of jmp (last elem before gap) */
};

char            *ofile;                  /* output file name */
char            *namex[2];               /* seq names: getseqs() */
char            *prog;                   /* prog name for err msgs */
char            *seqx[2];                /* seqs: getseqs() */
int             dmax;                    /* best diag: nw() */
int             dmax0;                   /* final diag */
int             dna;                     /* set if dna: main() */
int             endgaps;                 /* set if penalizing end gaps */
int             gapx, gapy;              /* total gaps in seqs */
int             len0, len1;              /* seq lens */
int             ngapx, ngapy;            /* total size of gaps */
int             smax;                    /* max score: nw() */
int             *xbm;                    /* bitmap for matching */
long            offset;                  /* current offset in jmp file */
struct   diag   *dx;                     /* holds diagonals */
struct   path   pp[2];                   /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"


static   _dbval[26] = {
         1,14,2,13,0,0,4,11,0,0,12,0,3,15,0,0,0,5,6,8,8,7,9,0,10,0
};


static   _pbval[26] = {
         1, 2|(1<<('D'-'A'))|(1<<('N'-'A')), 4, 8, 16, 32, 64,
         128, 256, 0xFFFFFFF, 1<<10, 1<<11, 1<<12, 1<<13, 1<<14,
         1<<15, 1<<16, 1<<17, 1<<18, 1<<19, 1<<20, 1<<21, 1<<22,
         1<<23, 1<<24, 1<<25|(1<<('E'-'A'))|(1<<('Q'-'A'))
};


main(ac, av)
                 main
         int     ac;
         char    *av[ ];
{
         prog = av[0];
         if (ac != 3) {
                 fprintf(stderr,"usage: %s file1 file2\n", prog);
                 fprintf(stderr,"where file1 and file2 are two dna or two protein sequences.\n");
                 fprintf(stderr,"The sequences can be in upper- or lower-case\n");
                 fprintf(stderr,"Any lines beginning with ';' or '<' are ignored\n");
                 fprintf(stderr,"Output is in the file \"align.out\"\n");
                 exit(1);
         }
         namex[0] = av[1];
         namex[1] = av[2];
         seqx[0] = getseq(namex[0], &len0);
         seqx[1] = getseq(namex[1], &len1);
         xbm = (dna)? _dbval : _pbval;

         endgaps = 0;                            /* 1 to penalize endgaps */
         ofile = "align.out";         /* output file */

         nw();            /* fill in the matrix, get the possible jmps */
         readjmps();      /* get the actual jmps */
         print();         /* print stats, alignment */

         cleanup(0);      /* unlink any tmp files */
}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()

nw

{
        char        *px, *py;           /* seqs and ptrs */
        int         *ndely, *dely;      /* keep track of dely */
        int         ndelx, delx;        /* keep track of delx */
        int         *tmp;               /* for swapping row0, row1 */
        int         mis;                /* score for each type */
        int         ins0, ins1;         /* insertion penalties */
        register    id;                 /* diagonal index */
        register    ij;                 /* jmp index */
        register    *col0, *col1;       /* score for curr, last row */
        register    xx, yy;             /* index into seqs */


        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;            /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

## Table 1 (cont')

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

## Table 1 (cont')

...nw

```
                        id = xx - yy + len1 - 1;
                        if (mis >= delx && mis >= dely[yy])
                                col1[yy] = mis;
                        else if (delx >= dely[yy]) {
                                col1[yy] = delx;
                                ij = dx[id].ijmp;
                                if (dx[id].jp.n[0] && (!dna || (ndelx >= MAXJMP
                                && xx > dx[id].jp.x[ij]+MX) || mis > dx[id].score+DINS0)) {
                                        dx[id].ijmp++;
                                        if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                        }
                                }
                                dx[id].jp.n[ij] = ndelx;
                                dx[id].jp.x[ij] = xx;
                                dx[id].score = delx;
                        }
                        else {
                                col1[yy] = dely[yy];
                                ij = dx[id].ijmp;
                if (dx[id].jp.n[0] && (!dna || (ndely[yy] >= MAXJMP
                                && xx > dx[id].jp.x[ij]+MX) || mis > dx[id].score+DINS0)) {
                                        dx[id].ijmp++;
                                        if (++ij >= MAXJMP) {
                                                writejmps(id);
                                                ij = dx[id].ijmp = 0;
                                                dx[id].offset = offset;
                                                offset += sizeof(struct jmp) + sizeof(offset);
                                        }
                                }
                                dx[id].jp.n[ij] = -ndely[yy];
                                dx[id].jp.x[ij] = xx;
                                dx[id].score = dely[yy];
                        }
                        if (xx == len0 && yy < len1) {
                                /* last col
                                */
                                if (endgaps)
                                        col1[yy] -= ins0+ins1*(len1-yy);
                                if (col1[yy] > smax) {
                                        smax = col1[yy];
                                        dmax = id;
                                }
                        }
                }
                if (endgaps && xx < len0)
                        col1[yy-1] -= ins0+ins1*(len0-xx);
                if (col1[yy-1] > smax) {
                        smax = col1[yy-1];
                        dmax = id;
                }
                tmp = col0; col0 = col1; col1 = tmp;
        }
        (void) free((char *)ndely);
        (void) free((char *)dely);
        (void) free((char *)col0);
        (void) free((char *)col1);                      }
```

## Table 1 (cont')

```
/*
 *
 * print() -- only routine visible outside this module
 *
 * static:
 * getmat() -- trace back best path, count matches: print()
 * pr_align() -- print alignment of described in array p[ ]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256      /* maximum output line */
#define P_SPC    3        /* space between name or num and seq */

extern  _day[26][26];
int     olen;             /* set output line length */
FILE    *fx;              /* output file */

print()

print
{
        int     lx, ly, firstgap, lastgap;       /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {     /* leading gap in y */
                pp[1].spc = firstgap = dmax - (len1 - 1);
                lx -= pp[1].spc;
        }
        if (dmax0 < len0 - 1) {          /* trailing gap in x */
                lastgap = len0 - dmax0 -1;
                lx -= lastgap;
        }
        else if (dmax0 > len0 - 1) {    /* trailing gap in y */
                lastgap = dmax0 - (len0 - 1);
                ly -= lastgap;
        }
        getmat(lx, ly, firstgap, lastgap);
        pr_align();
}
```

## Table 1 (cont')

```
/*
 * trace back the best path, count matches
 */
static
getmat(lx, ly, firstgap, lastgap)                                    getmat
        int      lx, ly;               /* "core" (minus endgaps) */
        int      firstgap, lastgap;    /* leading trailing overlap */
{
        int              nm, i0, i1, siz0, siz1;
        char             outx[32];
        double           pct;
        register         n0, n1;
        register char    *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

## Table 1 (cont')

```
        fprintf(fx, "<gaps in first sequence: %d", gapx);                      ...getmat
        if (gapx) {
                (void) sprintf(outx, " (%d %s%s)",
                        ngapx, (dna)? "base":"residue", (ngapx == 1)? "":"s");
                fprintf(fx,"%s", outx);


        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);

        }
        if (dna)
                fprintf(fx,
                "\n<score: %d (match = %d, mismatch = %d, gap penalty = %d + %d per base)\n",
                smax, DMAT, DMIS, DINS0, DINS1);

        else
                fprintf(fx,
                "\n<score: %d (Dayhoff PAM 250 matrix, gap penalty = %d + %d per residue)\n",
                smax, PINS0, PINS1);

        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");

        else
                fprintf(fx, "<endgaps not penalized\n");

}
static          nm;                     /* matches in core -- for checking */
static          lmax;                   /* lengths of stripped file names */
static          ij[2];                  /* jmp index for a path */
static          nc[2];                  /* number at start of current line */
static          ni[2];                  /* current elem number -- for gapping */
static          siz[2];
static char     *ps[2];                 /* ptr to current element */
static char     *po[2];                 /* ptr to next output char slot */
static char     out[2][P_LINE];         /* output line */
static char     star[P_LINE];           /* set by stars() */


/*
 * print alignment of described in struct path pp[ ]
 */
static
pr_align()                                                                     pr_align
{
        int             nn;     /* char count */
        int             more;
        register        i;

        for (i =  0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];                                 }
```

## Table 1 (cont')

<code>
        for (nn = nm = 0, more = 1; more; ) {
                for (i = more = 0; i < 2; i++) {
                        /*
                         * do we have more of this sequence?
                         */
                        if (!*ps[i])
                                        continue;

                        more++;

                        if (pp[i].spc) {        /* leading space */
                                *po[i]++ = ' ';
                                pp[i].spc--;
                        }
                        else if (siz[i]) {        /* in a gap */
                                *po[i]++ = '-';
                                siz[i]--;
                        }
                        else {                        /* we're putting a seq element
                                                 */
                                *po[i] = *ps[i];
                                if (islower(*ps[i]))
                                                *ps[i] = toupper(*ps[i]);
                                po[i]++;
                                ps[i]++;

                                /*
                                 * are we at next gap for this seq?
                                 */
                                if (ni[i] == pp[i].x[ij[i]]) {
                                        /*
                                         * we need to merge all gaps
                                         * at this location
                                         */
                                        siz[i] = pp[i].n[ij[i]++];
                                        while (ni[i] == pp[i].x[ij[i]])
                                                        siz[i] += pp[i].n[ij[i]++];
                                }
                                ni[i]++;
                        }
                }
                if (++nn == olen || !more && nn) {
                        dumpblock();
                        for (i = 0; i < 2; i++)
                                        po[i] = out[i];
                        nn = 0;
                }
        }
}

/*
 * dump a block of lines, including numbers, stars: pr_align()
 */
static
dumpblock()
</code>

## dumpblock

<code>
{
        register  i;
        for (i = 0; i < 2; i++)
                        *po[i]-- = '\0';
</code>

...pr_align

## Table 1 (cont')

...dumpblock

```
        (void) putc('\n', fx);
        for (i = 0; i < 2; i++) {
                if (*out[i] && (*out[i] != ' ' || *(po[i]) != ' ')) {
                        if (i == 0)
                                nums(i);
                        if (i == 0 && *out[1])
                                stars();
                        putline(i);
                        if (i == 0 && *out[1])
                                fprintf(fx, star);
                        if (i == 1)
                                nums(i);
                }
        }
}

/*
 * put out a number line: dumpblock()
 */
static
nums(ix)
        int     ix;     /* index in out[ ] holding seq line */
{
        char            nline[P_LINE];
        register        i, j;
        register char   *pn, *px, *py;

        for (pn = nline, i = 0; i < lmax+P_SPC; i++, pn++)
                *pn = ' ';
        for (i = nc[ix], py = out[ix]; *py; py++, pn++) {
                if (*py == ' ' || *py == '-')
                        *pn = ' ';
                else {
                        if (i%10 == 0 || (i == 1 && nc[ix] != 1)) {
                                j = (i < 0)? -i : i;
                                for (px = pn; j; j /= 10, px--)
                                        *px = j%10 + '0';
                                if (i < 0)
                                        *px = '-';
                        }
                        else
                                *pn = ' ';
                        i++;
                }
        }
        *pn = '\0';
        nc[ix] = i;
        for (pn = nline; *pn; pn++)
                (void) putc(*pn, fx);
        (void) putc('\n', fx);
}

/*
 * put out a line (name, [num], seq, [num]): dumpblock()
 */
static
putline(ix)
        int     ix;                     {
```

nums

putline

## Table 1 (cont')

...putline

```
        int             i;
        register char   *px;

        for (px = namex[ix], i = 0; *px && *px != ':'; px++, i++)
                (void) putc(*px, fx);
        for (; i < lmax+P_SPC; i++)
                (void) putc(' ', fx);


        /* these count from 1:
         * ni[ ] is current element (from 1)
         * nc[ ] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
```

**stars**

```
{
        int             i;
        register char   *p0, *p1, cx, *px;

        if (!*out[0] || (*out[0] == ' ' && *(po[0]) == ' ') ||
            !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

## Table 1 (cont')

```
/*
 * strip path or prefix from pn, return len: pr_align()
 */
static
stripname(pn)

stripname
    char     *pn;        /* file name (may be path) */
{
    register char     *px, *py;

    py = 0;
    for (px = pn; *px; px++)
            if (*px == '/')
                    py = px + 1;
    if (py)
            (void) strcpy(pn, py);
    return(strlen(pn));

}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>


char      *jname = "/tmp/homgXXXXXX";              /* tmp file for jmps */
FILE      *fj;


int       cleanup();                               /* cleanup tmp file */
long      lseek();


/*
 * remove any tmp file if we blow
 */
cleanup(i)                                                                cleanup
        int       i;
{
        if (fj)
                (void) unlink(jname);
        exit(i);
}


/*
 * read, return ptr to seq, set dna, len, maxlen
 * skip lines starting with ';', '<', or '>'
 * seq in upper or lower case
 */
char      *
getseq(file, len)                                                        getseq
        char      *file;     /* file name */
        int       *len;      /* seq len */
{
        char          line[1024], *pseq;
        register char *px, *py;
        int           natgc, tlen;
        FILE          *fp;


        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

## Table 1 (cont')

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}


char     *
g_calloc(msg, nx, sz)
```

```
        char     *msg;          /* program, calling routine */
        int      nx, sz;        /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}


/*
 * get final jmps from dx[ ] or tmp file, set pp[ ], reset dmax: main()
 */
readjmps()
```

### readjmps

```
{
        int             fd = -1;
        int             siz, i0, i1;
        register  i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

## Table 1 (cont')

```
                if (j < 0 && dx[dmax].offset && fj) {
                        (void) lseek(fd, dx[dmax].offset, 0);
                        (void) read(fd, (char *)&dx[dmax].jp, sizeof(struct jmp));
                        (void) read(fd, (char *)&dx[dmax].offset, sizeof(dx[dmax].offset));
                        dx[dmax].ijmp = MAXJMP-1;
                }
                else
                        break;
        }
        if (i >= JMPS) {
                fprintf(stderr, "%s: too many gaps in alignment\n", prog);
                cleanup(1);
        }
        if (j >= 0) {
                siz = dx[dmax].jp.n[j];
                xx = dx[dmax].jp.x[j];
                dmax += siz;
                if (siz < 0) {                  /* gap in second seq */
                        pp[1].n[i1] = -siz;
                        xx += siz;
                        /* id = xx - yy + len1 - 1
                        */
                        pp[1].x[i1] = xx - dmax + len1 - 1;
                        gapy++;
                        ngapy -= siz;
/* ignore MAXGAP when doing endgaps */
                        siz = (-siz < MAXGAP || endgaps)? -siz : MAXGAP;
                        i1++;
                }
                else if (siz > 0) {     /* gap in first seq */
                        pp[0].n[i0] = siz;
                        pp[0].x[i0] = xx;
                        gapx++;
                        ngapx += siz;
/* ignore MAXGAP when doing endgaps */
                        siz = (siz < MAXGAP || endgaps)? siz : MAXGAP;
                        i0++;
                }
        }
        else
                break;
}

/* reverse the order of jmps
*/
for (j = 0, i0--; j < i0; j++, i0--) {
        i = pp[0].n[j]; pp[0].n[j] = pp[0].n[i0]; pp[0].n[i0] = i;
        i = pp[0].x[j]; pp[0].x[j] = pp[0].x[i0]; pp[0].x[i0] = i;
}
for (j = 0, i1--; j < i1; j++, i1--) {
        i = pp[1].n[j]; pp[1].n[j] = pp[1].n[i1]; pp[1].n[i1] = i;
        i = pp[1].x[j]; pp[1].x[j] = pp[1].x[i1]; pp[1].x[i1] = i;
}
if (fd >= 0)
        (void) close(fd);
if (fj) {
        (void) unlink(jname);
        fj = 0;
        offset = 0;
}                                       }
```

## Table 1 (cont')

```
/*
 * write a filled jmp struct offset of the prev one (if any): nw()
 */
writejmps(ix)
    writejmps
    int     ix;
{
    char    *mktemp();

    if (!fj) {
        if (mktemp(jname) < 0) {
            fprintf(stderr, "%s: can't mktemp() %s\n", prog, jname);
            cleanup(1);
        }
        if ((fj = fopen(jname, "w")) == 0) {
            fprintf(stderr, "%s: can't write %s\n", prog, jname);
            exit(1);
        }
    }
    (void) fwrite((char *)&dx[ix].jp, sizeof(struct jmp), 1, fj);
    (void) fwrite((char *)&dx[ix].offset, sizeof(dx[ix].offset), 1, fj);
}
```

### Table 2

| PRO | XXXXXXXXXXXXXXX | (Length = 15 amino acids) |
|---|---|---|
| Comparison Protein | XXXXXYYYYYYY | (Length = 12 amino acids) |

% amino acid sequence identity =

(the number of identically matching amino acid residues between the two polypeptide sequences as determined by ALIGN-2) divided by (the total number of amino acid residues of the PRO polypeptide) = 5 divided by 15 = 33.3%

### Table 3

| PRO | XXXXXXXXXX | (Length = 10 amino acids) |
|---|---|---|
| Comparison Protein | XXXXXYYYYYYYZZYZ | (Length = 15 amino acids) |

% amino acid sequence identity =

(the number of identically matching amino acid residues between the two polypeptide sequences as determined by ALIGN-2) divided by (the total number of amino acid residues of the PRO polypeptide) = 5 divided by 10 = 50%

**Table 4**

| PRO-DNA | NNNNNNNNNNNNNN | (Length = 14 nucleotides) |
|---|---|---|
| Comparison DNA | NNNNNNLLLLLLLLLL | (Length = 16 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

6 divided by 14 = 42.9%

**Table 5**

| PRO-DNA | NNNNNNNNNNNN | (Length = 12 nucleotides) |
|---|---|---|
| Comparison | DNA NNNNLLLVV | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

II. Compositions and Methods of the Invention

A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been disclosed. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile val | |
| Arg (R) lys; | gln; asn lys | |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13: 4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Propertied W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}$P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology. 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwirzia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonga ptr3 phoA E15 (argF-lac)169 degP ompT kan^r^; E. coli* W3110 strain 37D6, which has the complete genotype *tonA ptr3 phoA E15 (argF-lac)169 degP ompT rbs7 ilvG kan^r^; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharormyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hanse$_{II}$ula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. <u>Selection and Use of a Replicable Vector</u>

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275: 615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

4. <u>Detecting Gene Amplification/Expression</u>

Gene amplification and/or expression may be measured in a sample directly, for example, by conventional Southern blotting, Northern blotting to quantitate the transcription of mRNA [Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

5. <u>Purification of Polypeptide</u>

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.*, US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate T-cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.,* Small et al., Mol. Cell. Biol. 5: 642-648 [1985]).

One suitable cell based assay is the mixed lymphocyte reaction (MLR). Current Protocols in Immunology, unit 3.12; edited by J E Coligan, A M Kruisbeek, D H Marglies, E M Shevach, W Strober, National Institutes of Health, Published by John Wiley & Sons, Inc. In this assay, the ability of a test compound to stimulate or inhibit the proliferation of activated T cells is assayed. A suspension of responder T cells is cultured with allogeneic stimulator cells and the proliferation of T cells is measured by uptake of tritiated thymidine. This assay is a general measure of T cell reactivity. Since the majority of T cells respond to and produce IL-2 upon activation, differences in responsiveness in this assay in part reflect differences in IL-2 production by the responding cells. The MLR results can be verified by a standard lymphokine (IL-2) detection assay. *Current Protocols in Immunology,* above, 3.15, 6.3.

A proliferative T cell response in an MLR assay may be due to direct mitogenic properties of an assayed molecule or to external antigen induced activation. Additional verification of the T cell stimulatory activity of the PRO polypeptides can be obtained by a costimulation assay. T cell activation requires an antigen specific signal mediated through the T-cell receptor (TCR) and a costimulatory signal mediated through a second ligand binding interaction, for example, the B7 (CD80, CD86)/CD28 binding interaction. CD28 crosslinking increases lymphokine secretion by activated T cells. T cell activation has both negative and positive controls through the binding of ligands which have a negative or positive effect. CD28 and CTLA-4 are related glycoproteins in the Ig superfamily which bind to B7. CD28 binding to B7 has a positive costimulation effect of T cell activation; conversely, CTLA-4 binding to B7 has a T cell deactivating effect. Chambers, C. A. and Allison, J. P., Curr. Opin. Immunol. (1997) 9:396. Schwartz, R. H., Cell (1992) 71:1065; Linsey, P. S. and Ledbetter, J. A., Annu. Rev. Immunol. (1993) 11:191; June, C. H. et al, Immunol. Today (1994) 15: 321; Jenkins, M. K., Immunity (1994) 1:405. In a costimulation assay, the PRO polypeptides are assayed for T cell costimulatory or inhibitory activity.

Direct use of a stimulating compound as in the invention has been validated in experiments with 4-1BB glycoprotein, a member of the tumor necrosis factor receptor family, which binds to a ligand (4-1BBL) expressed on primed T cells and signals T cell activation and growth. Alderson, M. E. et al., J. Immunol. (1994) 24:2219.

The use of an agonist stimulating compound has also been validated experimentally. Activation of 4-1BB by treatment with an agonist anti-4-1BB antibody enhances eradication of tumors. Hellstrom, I. and Hellstrom, K. E., Crit. Rev. Immunol. (1998) 18:1. Immunoadjuvant therapy for treatment of tumors, described in more detail below, is another example of the use of the stimulating compounds of the invention.

Alternatively, an immune stimulating or enhancing effect can also be achieved by administration of a PRO which has vascular permeability enhancing properties. Enhanced vascular permeability would be beneficial to disorders which can be attenuated by local infiltration of immune cells (*e.g.*, monocytes, eosinophils, PMNs) and inflammation.

On the other hand, PRO polypeptides, as well as other compounds of the invention, which are direct inhibitors of T cell proliferation/activation, lymphokine secretion, and/or vascular permeability can be directly used to suppress the immune response. These compounds are useful to reduce the degree of the immune response and to treat immune related diseases characterized by a hyperactive, superoptimal, or autoimmune response. This use of the compounds of the invention has been validated by the experiments described above in which CTLA-4 binding to receptor B7 deactivates T cells. The direct inhibitory compounds of the invention function in an analogous manner. The use of compound which suppress vascular permeability would be expected to reduce inflammation. Such uses would be beneficial in treating conditions associated with excessive inflammation.

Alternatively, compounds, *e.g.*, antibodies, which bind to stimulating PRO polypeptides and block the stimulating effect of these molecules produce a net inhibitory effect and can be used to suppress the T cell mediated immune response by inhibiting T cell proliferation/activation and/or lymphokine secretion. Blocking the stimulating effect of the polypeptides suppresses the immune response of the mammal. This use has been validated in experiments using an anti-IL2 antibody. In these experiments, the antibody binds to IL2 and blocks binding of IL2 to its receptor thereby achieving a T cell inhibitory effect.

H. Animal Models

The results of the cell based in vitro assays can be further verified using *in vivo* animal models and assays for T-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e.g.*, murine models. Such models can be generated by introducing cells into syngeneic mice using standard techniques, *e.g.*, subcutaneous injection, tail vein injection, spleen implantation, intraperitoneal implantation, implantation under the renal capsule, *etc.*

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test the compounds of the invention are the allogeneic heart transplant models described by Tanabe, M. et al, Transplantation (1994) 58:23 and Tinubu, S. A. et al, J. Immunol. (1994) 4330-4338.

Animal models for delayed type hypersensitivity provides an assay of cell mediated immune function as well. Delayed type hypersensitivity reactions are a T cell mediated *in vivo* immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in *Current Protocols in Immunology,* above, unit 4.5.

EAE is a T cell mediated autoimmune disease characterized by T cell and mononuclear cell inflammation and subsequent demyelination of axons in the central nervous system. EAE is generally considered to be a relevant animal model for MS in humans. Bolton, C., Multiple Sclerosis (1995) 1:143. Both acute and relapsing-remitting models have been developed. The compounds of the invention can be tested for T cell stimulatory or inhibitory activity against immune mediated demyelinating disease using the protocol described in *Current Protocols in Immunology,* above, units 15.1 and 15.2. See also the models for myelin disease in which oligodendrocytes or Schwann cells are grafted into the central nervous system as described in Duncan, I. D. et al, Molec. Med. Today (1997) 554-561.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998).

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The compounds of the invention can be tested for activity against autoimmune arthritis using the protocols described in *Current Protocols in Immunology,* above, units 15.5. See also the model using a monoclonal antibody to CD18 and VLA-4 integrins described in Issekutz, A.C. et al., Immunology (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compounds of the invention for activity and effectiveness in the treatment of asthma are described by Wolyniec, W. W. et al, Am. J. Respir. Cell Mol. Biol. (1998) 18:777 and the references cited therein.

Additionally, the compounds of the invention can be tested on animal models for psoriasis like diseases. Evidence suggests a T cell pathogenesis for psoriasis. The compounds of the invention can be tested in the scid/scid mouse model described by Schon, M. P. et al, Nat. Med. (1997) 3:183, in which the mice demonstrate histopathologic skin lesions resembling psoriasis. Another suitable model is the human skin/scid mouse chimera prepared as described by Nickoloff, B. J. et al, Am. J. Path. (1995) 146:580.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.*, Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.,* head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the T cell proliferation stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.*, Thomas and Capecchi, Cell, 51:503 (1987) for a description of homologous recombination vectors]. The vector is introduced into an embryonic stem cell line (*e.g.*, by electroporation) and cells in which the introduced DNA has homologously recombined with the endogenous DNA are selected [see *e.g.,* Li et al.., Cell, 69:915 (1992)]. The selected cells are then injected into a blastocyst of an animal (*e.g.*, a mouse or rat) to form aggregation chimeras [see *e.g.,* Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

I. <u>ImmunoAdjuvant Therapy</u>

In one embodiment, the immunostimulating compounds of the invention can be used in immunoadjuvant therapy for the treatment of tumors (cancer). It is now well established that T cells recognize human tumor specific antigens. One group of tumor antigens, encoded by the MAGE, BAGE and GAGE families of genes, are silent in all adult normal tissues , but are expressed in significant amounts in tumors, such as melanomas, lung tumors, head and neck tumors, and bladder carcinomas. DeSmet, C. et al., (1996) Proc. Natl. Acad. Sci. USA, 93:7149. It has been shown that costimulation of T cells induces tumor regression and an antitumor response both *in vitro* and *in vivo.* Melero, I. et al., Nature Medicine (1997) 3:682; Kwon, E. D. et al., Proc. Natl. Acad. Sci. USA (1997) 94: 8099; Lynch, D. H. et al, Nature Medicine (1997) 3:625; Finn, O. J. and Lotze, M. T., J. Immunol. (1998) 21:114. The stimulatory compounds of the invention can be administered as adjuvants, alone or together with a growth regulating agent, cytotoxic agent or chemotherapeutic agent, to stimulate T cell proliferation/activation and an antitumor response to tumor antigens. The growth regulating, cytotoxic, or chemotherapeutic agent may be administered in conventional amounts using known administration regimes. Immunostimulating activity by the compounds of the invention allows reduced amounts of the growth regulating, cytotoxic, or chemotherapeutic agents thereby potentially lowering the toxicity to the patient.

J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g.*, on a microtiter plate, by covalent or non-covalent attachments. Non-covalent attachment generally is accomplished by coating the solid surface with a solution of the polypeptide and drying. Alternatively, an immobilized antibody, *e.g.*, a monoclonal antibody, specific for the polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, T cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g.*, between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.,* Rossi, *Current Biology* 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e.g.,* PCT publication No. WO 97/33551, *supra.*

These molecules can be identified by any or any combination of the screening assays discussed above and/or by any other screening techniques well known for those skilled in the art.

L. Anti-PRO Antibodies

The present invention further provides anti-PRO antibodies. Exemplary antibodies include polyclonal, monoclonal, humanized, bispecific, and heteroconjugate antibodies.

1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp.59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')$_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain.

Humanization can be essentially performed following the method of Winter and co-workers [Jones et al., Nature, 321: 522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. $F(ab')_2$ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody F(ab')$_2$ molecule. Each Fab' fragment was separately secreted from *E. coli* and subjected to directed chemical *coupling in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V$_H$) connected to a light-chain variable domain (V$_L$) by a linker which is too short to allow pairing between the two domains on the same chain. Accordingly, the V$_H$ and V$_L$ domains of one fragment are forced to pair with the complementary V$_L$ and V$_H$ domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994). Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *e.g.*, the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g.*, an enzymatically active toxin of bacterial, fungal, plant, or animal origin, or fragments thereof), or a radioactive isotope *(i.e.,* a radioconjugate).

Chemotherapeutic agents useful in the generation of such immunoconjugates have been described above.

Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}$Bi, $^{131}$I, $^{131}$In, $^{90}$Y, and $^{186}$Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent *(e.g.,* a radionucleotide).

8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

M. Pharmaceutical Compositions

The active PRO molecules of the invention (*e.g.*, PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (*e.g.*, Zn-protein complexes); and/or non-ionic surfactants such as TWEEN™, PLURONICS™ or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.,* Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

N. Methods of Treatment

It is contemplated that the polypeptides, antibodies and other active compounds of the present invention may be used to treat various immune related diseases and conditions, such as T cell mediated diseases, including those characterized by infiltration of inflammatory cells into a tissue, stimulation of T-cell proliferation, inhibition of T-cell proliferation, increased or decreased vascular permeability or the inhibition thereof.

Exemplary conditions or disorders to be treated with the polypeptides, antibodies and other compounds of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease.

In systemic lupus erythematosus, the central mediator of disease is the production of auto-reactive antibodies to self proteins/tissues and the subsequent generation of immune-mediated inflammation. Antibodies either directly or indirectly mediate tissue injury. Though T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjögren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas

EP 2 179 742 A1

in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet $\beta$ cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barré syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+ T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (*i.e.*, as from chemotherapy) immunodeficiency, and neoplasia.

It has been demonstrated that some human cancer patients develop an antibody and/or T lymphocyte response to antigens on neoplastic cells. It has also been shown in animal models of neoplasia that enhancement of the immune response can result in rejection or regression of that particular neoplasm. Molecules that enhance the T lymphocyte response in the MLR have utility *in vivo* in enhancing the immune response against neoplasia. Molecules which enhance

186

the T lymphocyte proliferative response in the MLR (or small molecule agonists or antibodies that affected the same receptor in an agonistic fashion) can be used therapeutically to treat cancer. Molecules that inhibit the lymphocyte response in the MLR also function *in vivo* during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other means) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g.*, polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (*e.g.*, 0.1-20 mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

O. Articles of Manufacture

In another embodiment of the invention, an article of manufacture containing materials (*e.g.*, comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, *e.g.*, fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

EXAMPLE 1: Microarray analysis of stimulated T-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this instance, activated CD4+ T cells) sample is greater than hybridization signal of a probe from a control (in this instance, non-stimulated CD4 + T cells) sample, the gene or genes overexpressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

In this experiment, CD4+ T cells were purified from a single donor using the RossetteSep™ protocol from (Stem Cell Technologies, Vancouver BC) which contains anti-CD8, anti-CD16, anti-CD19, anti-CD36 and anti-CD56 antibodies used to produce a population of isolated CD4 + T cells. Isolated CD4+ T cells were activated with an anti-CD3 antibody (used at a concentration that does not stimulate proliferation) together with either ICAM-1 or anti-CD28 antibody. At 24 or 72 hours cells were harvested, RNA extracted and analysis run on Affimax (Affymetrix Inc. Santa Clara, CA) microarray chips. Non-stimulated (resting) cells were harvested immediately after purification, and subjected to the same analysis. Genes were compared whose expression was upregulated at either of the two timepoints in activated vs. resting cells.

Below are the results of these experiments, demonstrating that various PRO polypeptides of the present invention are differentially expressed in isolated CD4 + T cells activated by anti-CD3/ICAM-1 or anti-CD3/anti-CD28 as compared to isolated resting CD4+ T cells. As described above, these data demonstrate that the PRO polypeptides of the present invention are useful not only as diagnostic markers for the presence of one or more immune disorders, but also serve as therapeutic targets for the treatment of those immune disorders.

The results of this experment are Figures 1-7589 show increase or decrease in expression upon stimulation with anti-CD3/ICAM1 and also show increase or decrease in expression upon stimulation with anti-CD3/anti-CD28. The nucleic acids and encoded proteins of Figure 946, Figure 1520, Figure 1574, Figure 1622, Figure 1816, Figure 2433, Figure 2986, Figure 3220, Figure 4120 and Figure 5421 are significantly overexpressed in isolated CD4 + T cells activated by anti-CD3/ICAM-1 or anti-CD3/anti-CD28 as compared to isolated resting CD4+ T cells.

EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO can then be identified using standard techniques known in the art.

EXAMPLE 3: Expression of PRO in *E. coli*

This example illustrates preparation of an unglycosylated form of PRO by recombinant expression in *E. coli*.

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate·2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1 % TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 $\mu$g pRK5-PRO DNA is mixed with about 1 $\mu$g DNA encoding the VA RNA gene [Thimmappaya et al., Cell, 31:543 (1982)] and dissolved in 500 $\mu$l of 1 mM Tris-HCl, 0.1 mM EDTA, 0.227 M CaCl$_2$. To this mixture is added, dropwise, 500 $\mu$l of 50 mM HEPES (pH 7.35), 280 mM NaCl, 1.5 mM NaPO$_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 $\mu$Ci/ml $^{35}$S-cysteine and 200 $\mu$Ci/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 $\mu$g pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 $\mu$g/ml bovine insulin and 0.1 $\mu$g/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO$_4$ or DEAE-dextran. As described above, the cell cultures can be incubated, and the medium replaced with culture medium (alone) or medium containing a radiolabel such as $^{35}$S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by $Ni^{2+}$-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Quiagen), Dosper® or Fugene® (Boehringer Mannheim). The cells are grown as described in Lucas et al., supra. Approximately $3 \times 10^{-7}$ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 $\mu$m filtered PS20 with 5% 0.2 $\mu$m diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with $3 \times 10^5$ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at $1.2 \times 10^6$ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 $\mu$m filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 $\mu$l of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB 110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by $Ni^{2+}$-chelate affinity chromatography as follows. Extracts are prepared from recombinant virus-infected Sf9 cells as described by Rupert et al., Nature, 362: 175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KC1), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 $\mu$m filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (*e.g.*, high ionic strength buffers in the presence of detergent). Then, the column is eluted under conditions that disrupt antibody/PRO polypeptide binding (*e.g.*, a low pH buffer such as approximately pH 2-3, or a high concentration of a chaotrope such as urea or thiocyanate ion), and PRO polypeptide is collected.

EXAMPLE 9: Drugs Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

EXAMPLE 10: Rational Drug Design

The goal of rational drug design is to produce structural analogs of biologically active polypeptide of interest (*i.e.*, a PRO polypeptide) or of small molecules with which they interact, *e.g.*, agonists, antagonists, or inhibitors. Any of these examples can be used to fashion drugs which are more active or stable forms of the PRO polypeptide or which enhance or interfere with the function of the PRO polypeptide *in vivo* (*c.f.*, Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

**Claims**

1. A composition comprising (a) a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), (b) an agonist of said polypeptide, (c) an antagonist of said polypeptide, or (d) an antibody that binds to said polypeptide, for use in a method for treating an immune related disorder in a mammal.

2. A composition for use according to Claim 1 wherein the disorder is a T cell mediated disease.

3. A composition for use according to Claim 1 or Claim 2, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

4. A method for determining the presence of a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), in a sample suspected of containing said polypeptide, said method comprising exposing said sample to an antibody to the polypeptide, and determining binding of said antibody to a component of said sample.

5. A method of diagnosing an immune related disease or an inflammatory immune response in a mammal, said method comprising detecting the level of expression of a gene encoding a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.

6. A method of diagnosing an immune related disease in a mammal, said method comprising (a) contacting an anti-antibody to a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69) with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.

7. A method of identifying a compound that inhibits the activity of a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), said method comprising contacting cells which normally respond to said polypeptide with (a) said polypeptide and (b) a candidate compound, and determining the lack responsiveness by said cell to (a).

8. A method of identifying a compound that inhibits the expression of a gene encoding a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), said method comprising contacting cells which normally express said polypeptide with a candidate compound, and determining the lack of expression said gene.

9. The method of Claim 8, wherein said candidate compound is an antisense nucleic acid.

10. A method of identifying a compound that mimics the activity of a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69), said method comprising contacting cells which normally respond to said polypeptide with a candidate compound, and determining the responsiveness by said cell to said candidate compound.

11. An antagonist of a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69) for use in a method for stimulating the immune response in a mammal.

12. An antibody which specifically binds to a polypeptide having at least 80% amino acid sequence identity to the polypeptide shown in Figure 70 (SEQ ID NO: 70) or a polypeptide encoded by the full length coding region of the nucleotide sequence shown in Figure 69 (SEQ ID NO: 69).

13. The antibody of Claim 12, wherein said antibody is a monoclonal antibody, a humanized antibody or a single-chain antibody.

14. A composition of matter comprising (a) an agonist of a polypeptide having at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69, (b) an antagonist of said polypeptide, or (c) an antibody that binds to said polypeptide, in combination with a carrier.

15. An article of manufacture, comprising:

a container;
a label on said container; and
a composition of matter comprising (a) an anti-polypeptide antibody, wherein the polypeptide has at least 80% amino acid sequence identity to the polypeptide shown in Figure 70 (SEQ ID NO: 70) or to a polypeptide encoded by the full length coding region of the nucleotide sequence shown in Figure 69 (SEQ ID NO: 69) , (b) an agonist antibody of said polypeptide, or (c) an antagonist antibody of said polypeptide, contained within said container, wherein label on said container indicates that said composition of matter can be used for treating an immune related disease.

Europäisches
Patentamt

European
Patent Office

Office européen
des brevets

# EUROPEAN SEARCH REPORT

**Application Number**

EP 08 02 0819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | DATABASE Geneseq [Online] 13 February 2003 (2003-02-13), "Human MDDT polypeptide SEQ ID 637." XP002566752 retrieved from EBI accession no. GSP:ABU11690 Database accession no. ABU11690 * compound * | 1-3 | INV. A61K38/17 C07K16/18 |
| X | -& WO 02/079449 A2 (INCYTE GENOMICS INC [US]; DAFFO ABEL [US]; JONES ANISSA L [US]; TRAN A) 10 October 2002 (2002-10-10) * SEQ ID NO: 637 (page 315) residues 15-307; page 27 - page 28 * | 1-15 | |
| X | WO 01/77291 A2 (GENETICS INST [US] INST GENETICS LLC [US]) 18 October 2001 (2001-10-18) * SEQ ID NO: 415 (page 225); page 38 * | 1-15 | |
| X | WO 01/83510 A1 (HUMAN GENOME SCIENCES INC [US]; KOMATSOULIS GEORGE [US]; RUBEN STEVEN) 8 November 2001 (2001-11-08) *  SEQ ID NO: 86 and 26; page 36 - page 38 * | 1-15 | **TECHNICAL FIELDS SEARCHED (IPC)** A61K C07K |
| X | DATABASE UniProt [Online] 1 November 1997 (1997-11-01), "RecName: Full=Aquaporin-3; Short=AQP-3;" XP002566707 retrieved from EBI accession no. UNIPROT:Q92482 Database accession no. Q92482 * the whole document * | 1-4 | |

-/--

The present search report has been drawn up for all claims

| Place of search | Date of completion of the search | Examiner |
|---|---|---|
| The Hague | 8 February 2010 | Cupido, Marinus |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**EUROPEAN SEARCH REPORT**

Application Number

EP 08 02 0819

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE EMBL [Online] 10 September 2001 (2001-09-10), "Homo sapiens aquaporin 3 (Gill blood group), mRNA (cDNA clone MGC:8708 IMAGE:3877096), complete cds." XP002566708 retrieved from EBI accession no. EMBL:BC013566 Database accession no. BC013566 * the whole document * ----- | 1-4 | |

TECHNICAL FIELDS SEARCHED (IPC)

The present search report has been drawn up for all claims

| Place of search | Date of completion of the search | Examiner |
|---|---|---|
| The Hague | 8 February 2010 | Cupido, Marinus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 2 179 742 A1

**ANNEX TO THE EUROPEAN SEARCH REPORT
ON EUROPEAN PATENT APPLICATION NO.**

EP 08 02 0819

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2010

| Patent document cited in search report | | Publication date | Patent family member(s) | | Publication date |
|---|---|---|---|---|---|
| WO 02079449 | A2 | 10-10-2002 | AU | 2002338265 A1 | 15-10-2002 |
| | | | AU | 2002344208 A1 | 09-12-2002 |
| | | | CA | 2442705 A1 | 05-12-2002 |
| | | | CA | 2447183 A1 | 10-10-2002 |
| | | | EP | 1383894 A2 | 28-01-2004 |
| | | | WO | 02097031 A2 | 05-12-2002 |
| WO 0177291 | A2 | 18-10-2001 | AU | 5119901 A | 23-10-2001 |
| | | | EP | 1274831 A2 | 15-01-2003 |
| WO 0183510 | A1 | 08-11-2001 | AU | 5566801 A | 12-11-2001 |
| | | | CA | 2403901 A1 | 08-11-2001 |
| | | | EP | 1280815 A1 | 05-02-2003 |
| | | | JP | 2004527205 T | 09-09-2004 |

**EP 2 179 742 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 404097 A
- WO 9311161 A
- US 4275149 A
- US 4675187 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A

- EP 117060 A
- EP 117058 A
- US 4376110 A
- US 4873191 A, Hoppe and Wanger
- US 4736866 A
- WO 9733551 A
- US 4816567 A
- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- US 3773919 A
- EP 616812 A
- US 0110482 W
- EP 307247 A
- US 5122469 A
- WO 8403564 A

**Non-patent literature cited in the description**

- **Nielsen et al.** *Prot. Eng.,* 1997, vol. 10, 1-6
- **von Heinje et al.** *Nucl. Acids. Res.,* 1986, vol. 14, 4683-4690
- **Altschul et al.** *Methods in Enzymology,* 1996, vol. 266, 460-480
- **Altschul et al.** *Nucleic Acids Res,* 1997, vol. 25, 3389-3402
- **Ausubel et al.** Current Protocols in Molecular Biology. Wiley Interscience Publishers, 1995
- **Sambrook et al.** Molecular Cloning: A Laboratory Manual. Cold Spring Harbor Press, 1989
- **Zapata et al.** *Protein Eng.,* 1995, vol. 8 (10), 1057-1062

- **Pluckthun.** The Pharmacology of Monoclonal Antibodies. Springer-Verlag, 1994, vol. 113, 269-315
- **Hollinger et al.** *Proc. Natl. Acad. Sci. USA,* 1993, vol. 90, 6444-6448
- Cell cycle regulation, oncogens, and antineoplastic drugs. **Murakami et al.** The Molecular Basis of Cancer. WB Saunders, 1995, 13
- **Carter et al.** *Nucl. Acids Res.,* 1986, vol. 13, 4331
- **Zoller et al.** *Nucl. Acids Res.,* 1987, vol. 10, 6487
- **Wells et al.** *Gene,* 1985, vol. 34, 315
- **Wells et al.** *Philos. Trans. R. Soc. London SerA,* 1986, vol. 317, 415

- **Cunningham ; Wells.** *Science,* 1989, vol. 244, 1081-1085
- **Creighton.** The Proteins. W.H. Freeman & Co,
- **Chothia.** *J. Mol. Biol.,* 1976, vol. 150, 1
- **T.E. Creighton.** Proteins: Structure and Molecular Propertied. W.H. Freeman & Co, 1983, 79-86
- **Aplin ; Wriston.** *CRC Crit. Rev. Biochem.,* 1981, 259-306
- **Hakimuddin et al.** *Arch. Biochem. Biophys.,* 1987, vol. 259, 52
- **Edge et al.** *Anal. Biochem.,* 1981, vol. 118, 131
- **Thotakura et al.** *Meth. Enzymol.,* 1987, vol. 138, 350
- **Field et al.** *Mol. Cell. Biol.,* 1988, vol. 8, 2159-2165
- **Evan et al.** *Molecular and Cellular Biology,* 1985, vol. 5, 3610-3616
- **Paborsky et al.** *Protein Engineering,* 1990, vol. 3 (6), 547-553
- **Hopp et al.** *BioTechnology,* 1988, vol. 6, 1204-1210
- **Martin et al.** *Science,* 1992, vol. 255, 192-194
- **Skinner et al.** *J. Biol. Chem.,* 1991, vol. 266, 15163-15166
- **Lutz-Freyermuth et al.** *Proc. Natl. Acad. Sci. USA,* 1990, vol. 87, 6393-6397
- **Stewart et al.** Solid-Phase Peptide Synthesis. W.H. Freeman Co, 1969
- **Merrifield.** *J. Am. Chem. Soc.,* 1963, vol. 85, 2149-2154
- **Sambrook et al.** Molecular Cloning: A Laboratory Manual. Cold Spring Harbor Laboratory Press, 1989
- **Dieffenbach et al.** PCR Primer: A Laboratory Manual. Cold Spring Harbor Laboratory Press, 1995
- Mammalian Cell Biotechnology: a Practical Approach. IRL Press, 1991
- **Shaw et al.** *Gene,* 1983, vol. 23, 315
- **Graham ; van der Eb.** *Virology,* 1978, vol. 52, 456-457
- **Van Solingen et al.** *J. Bact.,* 1977, vol. 130, 946
- **Hsiao et al.** *Proc. Natl. Acad. Sci. (USA),* 1979, vol. 76, 3829
- **Keown et al.** *Methods in Enzymology,* 1990, vol. 185, 527-537
- **Mansour et al.** *Nature,* 1988, vol. 336, 348-352
- **Beach ; Nurse.** *Nature,* 1981, vol. 290, 140
- **Fleer et al.** *Bio/Technology,* 1991, vol. 9, 968-975
- **Louvencourt et al.** *J. Bacteriol.,* 1983, vol. 154 (2), 737-742
- **Van den Berg et al.** *Bio/Technology,* 1990, vol. 8, 135
- **Sreekrishna et al.** *J. Basic Microbiol.,* 1988, vol. 28, 265-278
- **Case et al.** *Proc. Natl. Acad. Sci. USA,* 1979, vol. 76, 5259-5263
- **Ballance et al.** *Biochem. Biophys. Res. Commun.,* 1983, vol. 112, 284-289
- **Tilburn et al.** *Gene,* 1983, vol. 26, 205-221
- **Yelton et al.** *Proc. Natl. Acad. Sci. USA,* 1984, vol. 81, 1470-1474
- **Kelly ; Hynes.** *EMBO J,* 1985, vol. 4, 475-479
- **C. Anthony.** The Biochemistry of Methylotrophs, 1982, 269
- **Graham et al.** *J. Gen Virol.,* 1977, vol. 36, 59
- **Urlaub ; Chasin.** *Proc. Natl. Acad. Sci. USA,* 1980, vol. 77, 4216
- **Mather.** *Biol. Reprod.,* 1980, vol. 23, 243-251
- **Urlaub et al.** *Proc. Natl. Acad. Sci. USA,* 1980, vol. 77, 4216
- **Stinchcomb et al.** *Nature,* 1979, vol. 282, 39
- **Kingsman et al.** *Gene,* 1979, vol. 7, 141
- **Tschemper et al.** *Gene,* 1980, vol. 10, 157
- **Jones.** *Genetics,* 1977, vol. 85, 12
- **Chang et al.** *Nature,* 1978, vol. 275, 615
- **Goeddel et al.** *Nature,* 1979, vol. 281, 544
- **Goeddel.** *Nucleic Acids Res,* 1980, vol. 8, 4057
- **deBoer et al.** *Proc. Natl. Acad. Sci. USA,* 1983, vol. 80, 21-25
- **Hitzeman et al.** *J. Biol. Chem.,* 1980, vol. 255, 2073
- **Hess et al.** *J. Adv. Enzyme Reg.,* 1968, vol. 7, 149
- **Holland.** *Biochemistry,* 1978, vol. 17, 4900
- **Gething et al.** *Nature,* 1981, vol. 293, 620-625
- **Mantei et al.** *Nature,* 1979, vol. 281, 40-46
- **Thomas.** *Proc. Natl. Acad. Sci. USA,* 1980, vol. 77, 5201-5205
- **Deutscher.** *Methods in Enzymology,* 1990, 182
- **Scopes.** Protein Purification: Principles and Practice. Springer-Verlag, 1982
- **Zola.** Monoclonal Antibodies: A Manual of Techniques. CRC Press, Inc, 1987, 147-158
- **Small et al.** *Mol. Cell. Biol.,* 1985, vol. 5, 642-648
- Current Protocols in Immunology. John Wiley & Sons, Inc
- **Chambers, C. A. ; Allison, J. P.** *Curr. Opin. Immunol.,* 1997, vol. 9, 396
- **Schwartz, R. H.** *Cell,* 1992, vol. 71, 1065
- **Linsey, P. S. ; Ledbetter, J. A.** *Annu. Rev. Immunol.,* 1993, vol. 11, 191
- **June, C. H. et al.** *Immunol. Today,* 1994, vol. 15, 321
- **Jenkins, M. K.** *Immunity,* 1994, vol. 1, 405
- **Alderson, M. E. et al.** *J. Immunol.,* 1994, vol. 24, 2219

- **Hellstrom, I. ; Hellstrom, K. E.** *Crit. Rev. Immunol.,* 1998, vol. 18, 1
- **Auchincloss, H. Jr. ; Sachs, D. H.** Fundamental Immunology. Raven Press, 1989, 889-992
- **Tanabe, M. et al.** *Transplantation,* 1994, vol. 58, 23
- **Tinubu, S. A. et al.** *J. Immunol.,* 1994, 4330-4338
- **Bolton, C.** *Multiple Sclerosis,* 1995, vol. 1, 143
- **Duncan, I. D. et al.** *Molec. Med. Today,* 1997, 554-561
- Current Protocols in Immunology. John Wiley & Sons, Inc, 1994
- **Grabbe, S. ; Schwarz, T.** *Immun. Today,* 1998, vol. 19 (1), 37-44
- **Issekutz, A.C. et al.** *Immunology,* 1996, vol. 88, 569
- **Wolyniec, W. W. et al.** *Am. J. Respir. Cell Mol. Biol.,* 1998, vol. 18, 777
- **Schon, M. P. et al.** *Nat. Med.,* 1997, vol. 3, 183
- **Nickoloff, B. J. et al.** *Am. J. Path.,* 1995, vol. 146, 580
- **Van der Putten et al.** *Proc. Natl. Acad. Sci. USA,* 1985, vol. 82, 6148-615
- **Thompson et al.** *Cell,* 1989, vol. 56, 313-321
- **Lo.** *Mol. Cel. Biol.,* 1983, vol. 3, 1803-1814
- **Lavitrano et al.** *Cell,* 1989, vol. 57, 717-73
- **Lasko et al.** *Proc. Natl. Acad. Sci. USA,* 1992, vol. 89, 6232-636
- **Thomas ; Capecchi.** *Cell,* 1987, vol. 51, 503
- **Li et al.** *Cell,* 1992, vol. 69, 915
- **Bradley.** Teratocarcinomas and Embryonic Stem Cells: A Practical Approach. IRL, 1987, 113-152
- **DeSmet, C. et al.** *Proc. Natl. Acad. Sci. USA,* 1996, vol. 93, 7149
- **Melero, I. et al.** *Nature Medicine,* 1997, vol. 3, 682
- **Kwon, E. D. et al.** *Proc. Natl. Acad. Sci. USA,* 1997, vol. 94, 8099
- **Lynch, D. H. et al.** *Nature Medicine,* 1997, vol. 3, 625
- **Finn, O. J. ; Lotze, M. T.** *J. Immunol.,* 1998, vol. 21, 114
- **Fields ; Song.** *Nature (London),* 1989, vol. 340, 245-246
- **Chien et al.** *Proc. Natl. Acad. Sci. USA,* 1991, vol. 88, 9578-9582
- **Chevray ; Nathans.** *Proc. Natl. Acad. Sci. USA,* 1991, vol. 89, 5789-5793
- **Kohler ; Milstein.** *Nature,* 1975, vol. 256, 495
- **Goding.** Monoclonal Antibodies: Principles and Practice. Academic Press, 1986, 59-103
- **Kozbor.** *J. Immunol.,* 1984, vol. 133, 3001
- **Brodeur et al.** Monoclonal Antibody Production Techniques and Applications. Marcel Dekker, Inc, 1987, 51-63
- **Munson ; Pollard.** *Anal. Biochem.,* 1980, vol. 107, 220
- **Jones et al.** *Nature,* 1986, vol. 321, 522-525
- **Riechmann et al.** *Nature,* 1988, vol. 332, 323-329
- **Presta.** *Curr. Op. Struct. Biol.,* 1992, vol. 2, 593-596
- **Riechmann et al.** *Nature,* 1988, vol. 332, 323-327
- **Verhoeyen et al.** *Science,* 1988, vol. 239, 1534-1536
- **Hoogenboom ; Winter.** *J. Mol. Biol.,* 1991, vol. 227, 381
- **Marks et al.** *J. Mol. Biol.,* 1991, vol. 222, 581
- **Cole et al.** Monoclonal Antibodies and Cancer Therapy. Alan R. Liss, 1985, 77
- **Boerner et al.** *J. Immunol.,* 1991, vol. 147 (1), 86-95
- **Marks et al.** *Bio/Technology,* 1992, vol. 10, 779-783
- **Lonberg et al.** *Nature,* 1994, vol. 368, 856-859
- **Morrison.** *Nature,* 1994, vol. 368, 812-13
- **Fishwild et al.** *Nature Biotechnology,* 1996, vol. 14, 845-51
- **Neuberger.** *Nature Biotechnology,* 1996, vol. 14, 826
- **Lonberg ; Huszar.** *Intern. Rev. Immunol.,* 1995, vol. 13, 65-93
- **Milstein ; Cuello.** *Nature,* 1983, vol. 305, 537-539
- **Traunecker et al.** *EMBO J.,* 1991, vol. 10, 3655-3659
- **Suresh et al.** *Methods in Enzymology,* 1986, vol. 121, 210
- **Brennan et al.** *Science,* 1985, vol. 229, 81
- **Shalaby et al.** *J. Exp. Med.,* 1992, vol. 175, 217-225
- **Kostelny et al.** *J. Immunol.,* 1992, vol. 148 (5), 1547-1553
- **Gruber et al.** *J. Immunol.,* 1994, vol. 152, 5368
- **Tutt et al.** *J. Immunol.,* 1991, vol. 147, 60
- **Caron et al.** *J. Exp Med.,* 1992, vol. 176, 1191-1195
- **Shopes.** *J. Immunol,* 1992, vol. 148, 2918-2922
- **Wolff et al.** *Cancer Research,* 1993, vol. 53, 2560-2565
- **Stevenson et al.** *Anti-Cancer Drug Design,* 1989, vol. 3, 219-230
- **Vitetta et al.** *Science,* 1987, vol. 238, 1098
- **Epstein et al.** *Proc. Natl. Acad. Sci. USA,* 1985, vol. 82, 3688

- **Hwang et al.** *Proc. Natl Acad. Sci. USA,* 1980, vol. 77, 4030
- **Martin et al.** *J. Biol. Chem.,* 1982, vol. 257, 286-288
- **Gabizon et al.** *J. National Cancer Inst.,* 1989, vol. 81 (19), 1484
- Remington's Pharmaceutical Sciences. 1980
- **Marasco et al.** *Proc. Natl. Acad. Sci. USA,* 1993, vol. 90, 7889-7893
- Chemotherapy Service. Williams & Wilkins, 1992
- **Bolivar et al.** *Gene,* 1977, vol. 2, 95
- **Thimmappaya et al.** *Cell,* 1982, vol. 31, 543
- **Somparyrac et al.** *Proc. Natl. Acad. Sci.,* 1981, vol. 12, 7575
- **Ausubel et al.** Current Protocols of Molecular Biology. John Wiley and Sons, 1997
- **Lucas et al.** *Nucl. Acids Res.,* 1996, vol. 24 (9), 1774-1779
- **O'Reilley et al.** Baculovirus expression vectors: A Laboratory Manual. Oxford University Press, 1994
- **Rupert et al.** *Nature,* 1993, vol. 362, 175-179
- **Hodgson.** *Bio/Technology,* 1991, vol. 9, 19-21
- **Braxton ; Wells.** *Biochemistry,* 1992, vol. 31, 7796-7801
- **Athauda et al.** *J. Biochem.,* 1993, vol. 113, 742-746